# EUROPEAN PATENT APPLICATION

(11) **EP 4 847 017 A1**
(43) Date of publication of application: **02.12.2026**
(21) Application number: 25744675.7
(22) Date of filing: 23.01.2025
(51) Int. Cl.: A61K 31/155, A61P 35/00

(54) **FORMULATION COMPRISING N,N-DIMETHYLBIGUANIDE, PHARMACEUTICAL COMBINATION COMPRISING N,N-DIMETHYLBIGUANIDE AND DRUG, COMPOSITION, KIT, USE THEREOF, AND METHOD FOR PREPARING SAME**

(30) Priority: 23.01.2024 CN 202410104044; 05.12.2024 CN 202411779600
(71) Applicant: Chengdu Kuachangaopu Medical Technology Co., Ltd., Chengdu, Sichuan 610000 (CN); Kuachang Inc., Anaheim, CA 92804 (US); Human Plasma Product Services Asia Limited, Hong Kong (HK)
(72) Inventor: ZOU, Fanglin, Chengdu, Sichuan 610041 (CN); ZOU, Lichang, Chengdu, Sichuan 610041 (CN); WANG, Jianxia, Chengdu, Sichuan 610041 (CN); ZHONG, Xiaoli, Chengdu, Sichuan 610041 (CN); LU, Xing, Chengdu, Sichuan 610041 (CN)
(74) Representative: ABG Intellectual Property Law, S.L.
(86) International application number: PCT/CN2025/074262
(87) International publication number: WO 2025/157212

(57) **Abstract**

Provided are a formulation comprising N,N-dimethylbiguanide and use thereof in preparing a lesion interventional drug. Also provided are a pharmaceutical combination comprising N, N-dimethylbiguanide and a drug with a synergistic effect, a pharmaceutical composition, formulation and kit comprising the pharmaceutical combination, a method for preparing same, and use thereof.

## Description

### Technical Field

The present application disclosure relates to new uses of dimethylbiguanide compounds, and to advantages (such as dosage reduction with enhanced efficacy, decreased biological barrier function, and improved heterogeneity adaptability, among others) based on the new uses, to combinations with different components (Z) and uses thereof. The Z includes or is selected from at least one of following groups: cell-reactive antineoplastic agents (abbreviated as B), (injectable) weakly basic sodium salts (abbreviated as C), methylene blue dyes (abbreviated as D), and immunomodulators (abbreviated as E), such as injectable immunomodulators.

### Background Technology

Many substances exhibit antineoplastic activity in cell-based tests. Even substances having relatively strong activity often have a low probability of exhibiting the expected effect in animal experiments, and substances showing relatively good effects in animal experiments likewise often have a low probability of exhibiting the expected therapeutic efficacy in clinical trials. Only a very small number of the best candidates are approved and granted indications for the treatment of the most suitably selected tumors. However, despite such selection from tens of thousands of candidates, clinical practice has further shown that therapeutic efficacy, even within an approved indication, varies among individuals and is often unsatisfactory. The key limitations are as follows:
Biological barrier problem. These cell-reactive drugs or their active forms can produce an antineoplastic reaction only upon contact with tumor cells. A cell suspension is overly simple compared with the tumor tissue of a model animal, while the tumor tissue of an animal patient is more complex. The degree of biological barriers (including tissue barriers, cell membrane barriers, etc.) is therefore greatly increased and becomes a key control step for the cellular reaction.

Heterogeneity problem. Heterogeneity includes tissue heterogeneity, spatial heterogeneity, and safety-impact heterogeneity. Different tumor masses of the same type, or different regions of the same tumor mass, may differ greatly in tissue-barrier function, morphological distribution, or safety impact. If the above heterogeneity problems are not fully considered and reliance is placed simply on the results of cell-based or animal experiments, it is difficult to administer antineoplastic drugs according to individual circumstances, even when the drugs are administered directly to a tumor mass, and difficult to produce the expected therapeutic effect.

Dose-effect problem. The therapeutic efficacy of cell-reactive drug is highly dependent on its antineoplastic reaction concentration, whereas its dosage is generally limited by side effects. Many drugs provide therapeutic efficacy at the cost of partially reduced safety, causing patients to be troubled by side effects during treatment; nevertheless, the therapeutic performance provided at relatively high doses (including timeliness, effectiveness, long-term effectiveness, and heterogeneity adaptability) still needs to be improved. For example, owing to the combined effects of the biological barriers and heterogeneity described above, the amount of drug delivered to different regions and the duration for which that amount is maintained may vary greatly. If, in addition, the dose-effect performance of the drug is not strong, the antineoplastic action in some regions may be insufficient to inhibit tumor cells effectively, and such regions may become a source of tumor drug resistance and recurrence. Accordingly, weak dose-effect performance of a drug also includes low heterogeneity adaptability despite a large dosage.

In the prior art, dimethylbiguanide (generally C4H11N5·HCl) is indicated for lowering blood glucose in type 2 diabetes. Research into new uses of dimethylbiguanide has continued. All reported antineoplastic actions of dimethylbiguanide (hereinafter abbreviated as conventional antineoplastic actions of dimethylbiguanide) are directed to tumor cells, for example, affecting tumor-cell proliferation through activation of the AMP-activated protein kinase pathway (Morales D R, Morris A D. Metformin in Cancer Treatment and Prevention [J]. Annual Review of Medicine, 2015). In cell-based tests, the half-maximal effective concentration (EC50) against selected sensitive cells is at the mM level, more than 100 times higher than the M level of conventional antineoplastic drugs such as 5-FU, demonstrating that any antineoplastic action of dimethylbiguanide at the cellular level is very weak.

Compared with approved drugs indicated for the treatment of solid tumors, dimethylbiguanide presents the three key limitations described above (the biological barrier problem, the heterogeneity problem, and the dose-effect problem) even more prominently. In summary, conventional dimethylbiguanide drugs or formulations exhibit the basic characteristics of a large dosage and low therapeutic efficacy (weak dose-effect performance): long-term administration (for example, continuous administration for more than 30 days in mouse experiments) and high-dose administration (for example, 1,000-2,000 mg per day) are required, yet the resulting therapeutic efficacy remains very low. For example, among five clinical studies of dimethylbiguanide using Ki-67 as a tumor-proliferation marker, only two showed a significant reduction in Ki-67, two showed no significant change in Ki-67, and one showed an increase in Ki-67-(Vancura A, Bu P, Bhagwat M, et al. Metformin as an Anticancer Agent [J]. Trends Pharmacol. Sci., 2018, Vol. 39, No. 10, pp. 867-878).

The combined use of a conventional dimethylbiguanide drug with another drug (taking a cell-reactive antineoplastic drug as an example) essentially utilizes interaction between them at the cellular level (Kan Guanting, Yu Jiandong, Xiong Yang. Research Progress of Metformin Combined with Chemotherapeutic Drugs in Antitumor Therapy. Chinese Journal of Modern Applied Pharmacy, August 2020, Vol. 37, No. 16, pp. 2025-2030). Such combined use does not change the antineoplastic pharmacology of the respective active components; the weak dose-effect performance of the conventional dimethylbiguanide drug described above remains, and the problem to be solved does not involve the biological barrier and heterogeneity problems described above.

The present application relates to localized lesions and conducts research using tumor masses as an example, with the objective of solving the above problems of conventional dimethylbiguanide drugs and their combined use with other drugs (taking a cell-reactive antineoplastic drug as an example): Problem 1, the dose-effect performance is too weak, particularly under conditions involving biological barriers and heterogeneity; and Problem 2, when used in combination with another drug, dimethylbiguanide can provide only a cytotoxic action limited by Problem 1 and does not help solve the biological barrier and heterogeneity problems of the other drug. The objective is to impart to dimethylbiguanide, and to the existing technology for its combined use with an antineoplastic drug, new therapeutic value (for example, a new indication) that cannot be provided by the prior art but is urgently needed clinically, thereby meeting various clinical needs that remain unmet by the prior art.

### Summary of Invention

The inventors of the present invention unexpectedly discovered that an aqueous dimethylbiguanide hydrochloride solution and an aqueous N,N-dimethylbiguanide solution, which are generally regarded as pharmaceutical equivalents, show differences in intratumoral cell morphology within one hour and differences in tumor-inhibition rate at the end of the experiment, respectively, after being introduced into tumors containing drug-resistant tumor cells. These results are clearly conflict with the existing structure-activity concept that "where basic chemical structures are identical and the pharmacological effects are basically identical" (this is also the basis for the common practice in the prior art of referring to dimethylbiguanide salts collectively as dimethylbiguanide), and exceed expectations based on the conventional antineoplastic action of dimethylbiguanide.

Therefore, according to the first aspect of the present invention, provided is a product specifically for lesion intervention, specifically a formulationcomprising N,N-dimethylbiguanide (abbreviated as R) and optionally a medium, such as a solvent, the formulation does not contain an acid capable of converting R into a salt thereof, such as an acidic pH-adjusting agent required to satisfy cell-culture requirements or conventional injection safety requirements, wherein the concentration of the N,N-dimethylbiguanide is greater than or equal to its administration concentration, and wherein the administration concentration (w/w) is 0.5% ≤ CR ≤ 51%, preferably 1% - 50%, more preferably 3% - 50% or 5% - 30%.

More specifically, the formulation of the present invention is preferably used for treatment of a lesion such as a tumor or a nodule in a subject, preferably, the treatment is a local treatment.

In some embodiments, the tumor includes a solid tumor.

In some embodiments, the above formulation contains a medium such as a solvent, preferably the solvent includes water, more preferably such as water for injection.

In some embodiments, the formulation does not include a salt of N,N-dimethylbiguanide (abbreviated as a dimethylbiguanide salt, abbreviated as R').

In some embodiments, the concentration (w/w) of the salt of N,N-dimethylbiguanide in the formulation is ≤ 30%, preferably ≤ 25%, preferably ≤ 10%, more preferably < 5%, ≤ 4%, or ≤ 3%, and even more preferably ≤ 2%, particularly preferably ≤ 1%, such as 1% or 0.5%, and most preferably 0%, that is, the formulation does not contain a salt of N,N-dimethylbiguanide.

In some embodiments, an administration amount of N,N-dimethylbiguanide in the above formulation is: ${Q}_{R} = {\sum }_{i = 1}^{nR} {q}_{R \left(i\right)}$ , where n_{R} is the number of times that R is administered to a tumor in one course of treatment, q_{R(i)} is an amount of R in the i-th (i = 1,..., n_{R}) administration, and: ${q}_{R \left(i\right)} = {\sum }_{ii = 1}^{nRi} {c}_{R \left(ii\right)} \times {v}_{R \left(ii\right)}$, where n_{Ri} is the number of administration points, such as the number of needle insertion points, in the i-th administration of R, C_{R(ii)} and V_{R(ii)} are the concentration and volume of R administered at the ii-th administration point, such as the needle insertion point, respectively, where v_{R(ii)} ≤ 3 times the target volume (abbreviated as V_{target(ii)}) of the ii-th administration point, and C_{R} is 0.5 - 51%, where 0.5% is a co-adminstration concentration threshold of R, and 51% is upper limit of the solubility threshold of R.

In some embodiments, the formulation is a fast-acting formulation only for rapidly reducing a volume of a lesion.

In some embodiments, the formulation is a low-frequency effective formulation only for low-frequency administration.

In some embodiments, the formulation is a liquid supply agent, where the liquid is a micro-volume liquid, and the micro-volume is 2 - 1000 µL, 5 - 500 µL, 5 - 100 µL, 5 - 50 µL, or 5 - 20 µL.

In some embodiments, the salt of N,N-dimethylbiguanide includes hydrochloride, sulfate, acetate, phosphate, citrate, lactate, nitrate, carbonate of N,N-dimethylbiguanide, and the like.

In some embodiments, the acidic pH-adjusting agent includes or is selected from strong acids such as hydrochloric acid, sulfuric acid; or weak acids such as acetic acid, citric acid, phosphoric acid, malic acid, tartaric acid, lactic acid, and carbonic acid, and the like.

In some embodiments, N,N-dimethylbiguanide is an organic compound (a) of the formula C₄H₁₁N₅, which has the following formula 1:

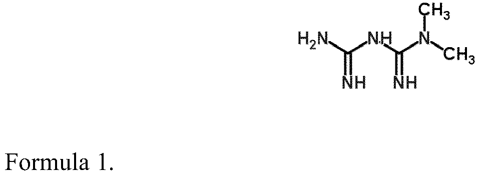

In some embodiments, a dimethylbiguanide medicament, which is used as a conventional medication, is typically a complex of N,N-dimethylbiguanide with an acid (referred to as dimethylbiguanide salt, abbreviated as R'), with the chemical formula C₄H₁₁N₅·acid(a+b). The most commonly used dimethylbiguanide medication is dimethylbiguanide hydrochloride (C₄H₁₁N₅·HCl), also known as dimethylbiguanide hydrochloride, which has the following formula 2:

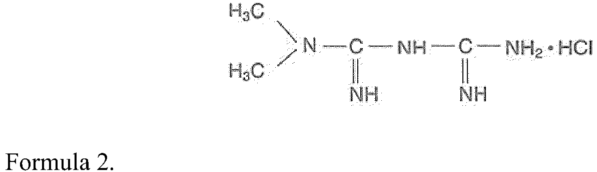

In some embodiments, the formulation consists of N,N-dimethylbiguanide and water. In one embodiment, an administration concentration (C_{R}, w/w) of N,N-dimethylbiguanide in the formulation can be 0.75%, 1%, 2%, 3%, 5%, 10%, 15%, 20%, 25%, 30%, 35%, 40%, or 50%, or any range therebetween, such as 0.75% to 20%, 1% to 20%, 2% to 20%, 3% to 20%, 5% to 20%, 5% to 40%, and so on. In another embodiment, the administration concentration (C_{R}, w/w) of N,N-dimethylbiguanide in the formulation is 20%, 30%, 35%, or 40% to the maximum solubility, such as 51%, or it can be within a range of 0.5% - 51%, 0.5% - 50%, 1% - 50%, 5% - 50%, 15% - 50%, 25% - 50%, 35% - 50%.

In some embodiments, the formulation does not comprise an acid that can significantly convert N, N-dimethylbiguanide to dimethylbiguanide salt in an aqueous solution. Specifically, the acid is an acidic pH-adjusting agent required to meet cell-culture requirements or conventional injection safety requirements, including or selected from the group consisting of, for example, hydrochloric acid, sulfuric acid, acetic acid, citric acid, phosphoric acid, malic acid, tartaric acid, lactic acid, and carbonic acid, and the like.

In some embodiments, the formulation is specifically used for lesion intervention, and is preferably used for treating lesions such as tumors or nodules in a subject.

According to the second aspect of the present invention, provided is a formulation specifically used for lesion intervention, comprising N,N-dimethylbiguanide (abbreviated as R) and optionally a solvent , wherein the formulation does not comprise an acid that can convert the R to its salt, such as an acidic pH-adjusting agent required to meet cell-culture requirements or conventional injection safety requirements, and a concentration of the N,N-dimethylbiguanide is greater than or equal to its intervention concentration, wherein the intervention concentration (w/w) is 0.5% ≤ C ≤ 51%, preferably 1% - 50%, more preferably 3% - 40% or 5% - 35%. Preferably, the formulation is used for interventional treatment of lesions such as tumors or nodules in a subject.

In some embodiments, the formulation may comprise a solvent, preferably, the solvent comprises water, and more preferably is water for injection.

In some embodiments, the above formulation may not include dimethylbiguanide salt.

In some embodiments, the N,N-dimethylbiguanide in the above formulation has the structure (Formula 1) as described in the first aspect, and its concentration (w/w) can be ≤ 51%, ≤ 50%, ≤ 40%, ≤ 30%, ≤ 25%, ≤ 20%, or ≤ 10%, ≤ 5%, ≤ 2%, such as 1%, 0.75%, or 0.5%.

In some embodiments, a concentration (w/w) of the N,N-dimethylbiguanide (R) in the formulation can be 0.5% - 51%, 0.5% - 50%, 1% - 40%, 5% - 50%, 5% - 40%, 5% - 30%, 5% - 25%, 5% - 20%, 1% - 30%, 1% - 25%, 1% - 20%, 15% - 50%, 25% - 50%, 35% - 50%, 5% - 20%, 0.5% - 10%, or 1% - 5%, or ≤ 2% such as 1%, 0.75%, or 0.5%.

In some embodiments, the acidic pH-adjusting agent can include or be selected from hydrochloric acid, sulfuric acid, acetic acid, citric acid, phosphoric acid, malic acid, tartaric acid, lactic acid, and carbonic acid.

In some embodiments, the formulation consists of N,N-dimethylbiguanide (R) and water.

In some embodiments, the formulation does not comprise an acid that can cause significant conversion of N,N-dimethylbiguanide (R) to dimethylbiguanide salts in an aqueous solution.

According to the third aspect of the present invention, a pharmaceutical combination comprising N,N-dimethylbiguanide (R) and a component (Z) is provided, wherein an administration concentration of the N,N-dimethylbiguanide is 0.1% - 51%, preferably 0.2% - 50% by weight/weight (w/w), and the component (Z) includes or is selected from at least one of the following groups: cell-reactive antineoplastic agents (referred to as B), near-neutral or weakly basic sodium salts (referred to as C), methylene blue dyes (referred to as D), and immunomodulators (referred to as E). In some embodiments, in the above pharmaceutical combination, N,N-dimethylbiguanide (R) has the structure (Formula 1) as described in the first aspect.

In some embodiments, a pharmaceutical combination which can be used to treat lesions such as tumors or nodules in a subject is provided.

In some embodiments, in the above pharmaceutical combination, the N,N-dimethylbiguanide is used to provide an active form different from a conventional metformin salt (whose active ingredient is a salt of N,N-dimethylbiguanide (R')) through the following limitations:
4). not mixed with an acid that causes significant conversion of R into R';
5). local intralesional administration;
6). an administration amount for one course of treatment is: ${Q}_{R} = {\sum }_{i = 1}^{nR} {q}_{R \left(i\right)}$,
where Q_{R} < 50% or 25% of the equivalent administration amount of said R', n_{R} is the number of times that R is administered to a tumor in one course of treatment, and q_{R(i)} is an amount of R in the i-th (i = 1,..., nR) administration, and: ${q}_{R \left(i\right)} = {\sum }_{i i - 1}^{n R i} {c}_{R \left(ii\right)} \times {v}_{R \left(ii\right)} ,$
where n_{Ri} is the number of administration points for R in the i-th administration, for example, the number of needle insertion points, c_{R(ii)} and v_{R(ii)} are concentration and volume of R administered at the ii-th administration point, for example, the needle insertion point, respectively, and a concentration threshold of c_{R(ii)} (W/W) is 0.1% or 0.2%, and the upper limit of the concentration threshold is 51%, a volume threshold of v_{R(ii)} is 1/800 or 1/300 of a target volume (abbreviated as V_{target(ii)}), and the upper limit of the volume threshold is 3 times of V_{target(ii)}.

Here, the active form of R' is presented as being a form that fully complexed with acid in solution; while the active form of R is presented as being less complexed with acid in solution, mainly existing in a form of minimal or partial complexation, thereby providing an activity (abbreviated as activity A) through a transient local action of distinguishing active forms, which is difficult for R' to provide, including at least one of the following: effectively reducing a function of biological barrier (abbreviated as activity A1), effectively damaging a lesion tissue (abbreviated as activity A2), and/or effectively releasing immune substances and/or inflammatory signals of alesion within a lesion tissue (abbreviated as activity A3).

In some embodiments, the pharmaceutical combination is used for treating a solid tumor, and comprises R and B, wherein:
- the activity A of R at least includes effectively reducing a function of biological barrier (activity A1);
- the cell-reactive antineoplastic agent (B) provides the cellular response (abbreviated as activity B) through the following limitations, one of key control steps of which is crossing the above-mentioned biological barrier:
   1). intratumoral administration and/or systemic administration;
   2). the administration amount for one course of treatment is: ${Q}_{B} = {\sum }_{j - l}^{nB} {q}_{B \left(j\right)} + {\sum }_{i ′ - l}^{nB ′} {c}_{B \left(j′\right)} \times {v}_{B \left(j′\right)}$ ,
where Q_{B} is ≤a conventional administration amount in the sensitive tumor of B, n_{B} and q_{B(j)} are the number of systemic administrations and the administration amount in the j-th (j = 1,..., n_{B}) administration of B in one course of treatment, respectively, and n_{B'}, cB_{(j')}, and v_{B(j')} are the number of intratumoral administrations and the concentration and volume in the j'-th (j' = 1,..., nB') administration of B in one course of treatment, respectively.

In some embodiments, in the above pharmaceutical combination, the pharmaceutical combination comprises R, C, and/or D, where:
- the activity A of R at least includes effectively damaging the lesion tissue (activity A2);
- the C is used to provide the effect of improving a local efficacy-toxicity ratio of R (abbreviated as activity C) through the following limitations:
   1). mixed administration with the R;
   2). the mixed amount ratio of C to R (Q_{C}/Q_{R}) is 0.5 to 4.0;
- D provides a pharmacological effect of enhancing a drug effect of the R through the following limitations:
   1). intralesional administration, including mixed administration with R or separate administration;
   2). the administration amount for one course of treatment QE is ≤ the effective amount for transient local action.

In some embodiments, the above pharmaceutical combination comprises R and E, where:
- the activity A of R at least includes effectively reducing a function of biological barrier (activity A1), and/or effectively releasing immune substances and/or inflammatory signals of the lesion within a lesion (activity A3).
- the E is used to provide an immunomodulatory effect benefiting from the activity A1 and/or an action of optimizing a local and/or systemic immune response by utilizing the activity A3, through the following limitations (referred to as activity E):
   1). intralesional administration and/or systemic administration;
   2). the administration amount for one course of treatment is: ${Q}_{E} = {\sum }_{k = 1}^{n E} {q}_{B \left(k\right)} + {\sum }_{k ′ = 1}^{n E ′} cE \left(k′\right) \times {v}_{E \left(k′\right)} ,$
wherein Q_{E} is ≦ a conventional administration amount in an anti-lesion application of E, n_{E} and q_{E(k)} are the number of systemic administrations and an amount of the k-th (j = 1,..., nₖ) administration in one course of treatment, respectively, n_{E'}, c_{E(k')}, and v_{E(k')} are the number of intratumoral administration, the concentration and the volume of the k'th (k' = 1,..., nE') administration in one course of treatment, respectively.

In some embodiments, in the above pharmaceutical combination, the tumor is a solid tumor suitable for intratumoral administration. In some embodiments, in the above pharmaceutical combination, the component (Z) includes a cell-reactive antineoplastic agents (B), and the R provides an activity (referred to as activity A) that cannot be provided by a conventional dimethylbiguanide (active ingredient of which is dimethylbiguanide salt, referred to as R'), through the following limitations, including effectively reducing the function of the biological barrier (referred to as activity A1):
1). intratumoral administration;
2). the administration amount is: ${Q}_{R} = {\sum }_{i = 1}^{nR} {q}_{R \left(i\right)}$,
   wherein n_{R} is the number of intratumoral administrations of R in one course of treatment, q_{R(i)} is the amount of R in the i-th (i = 1,..., nR) administration, and: ${q}_{R \left(i\right)} = {\sum }_{i i - 1}^{n R i} {c}_{R \left(ii\right)} \times {v}_{R \left(ii\right)} ,$
   wherein n_{Ri} is the number of injection points in the i-th administration of R, C_{R(ii)} and v_{R(ii)} are the concentration and volume of R administered at the ii-th injection point, respectively, and the concentration threshold of c_{R(ii)} (W/W) is 0.1% or 0.2%, the upper limit of the concentration threshold is 51%, and the volume threshold of v_{R(ii)} is 1/800 or 1/300 of a target volume (referred to as V_{target(ii)}), and the upper limit of the volume threshold is 3 times of V_{target(ii)};

- the B provided one of the key control steps through the following limitations, i.e., the specific antitumor effect of crossing a tumor tissue barrier (referred to as activity B):
   1). intratumoral administration and/or systemic administration;
   2). the aministration amount is: ${Q}_{B} = {\sum }_{j = 1}^{nB} {q}_{B \left(j\right)} + {\sum }_{j ′ = 1}^{nB ′} {C}_{B \left(j′\right)} \times {V}_{B \left(j′\right)}$,
   wherein Q_{B} is ≦ a conventional aministration amount in the sensitive tumor of said B, n_{B}, q_{B(j)} are the number of systemic administrations and the amount of the j-th (j = 1,..., n_{B}) administration of B in one course of treatment, respectively, n_{B'}, c_{B(j')}, and v_{B(j')} are the number of tumor administrations, the concentration and the volume of the j'-th (j' = 1,..., n_{B'}) administration of B in one course of treatment, respectively;
- the type of the combination is a synergistic combination of the activity A of R and the activity B of B, where the drug effects of A and B enhance each other.

In some embodiments, in the above pharmaceutical combination, the component (Z) includes a nearly neutral or weakly basic sodium salt (C); R is limited to intralesional administration, C increases an efficacy-toxicity ratio of the R intralesional administration by mixing with the R; and a mixing amount ratio of C to R (Q_{C}/Q_{R}) is 0.5 to 4.0.

In some embodiments, in the above pharmaceutical combination, the component (Z) includes methylene blue dyes (D); R and D are limited to intralesional administration, and D enhances the efficacy of R by mixing with R or being administered independently.

In some embodiments, in the above pharmaceutical combination, the component (Z) includes an immunomodulator (E), wherein:
- R is used to provide an activity (referred to as activity A) that conventional dimethylbiguanide (active ingredient of which is dimethylbiguanide salt, referred to as R') cannot provide, through the following limitation, including effectively reducing the function of a biological barrier (referred to as activity A1), and/or effectively releasing immune substances and/or inflammatory sgnals of a lesion within the lesion (referred to as activity A3):
   1). intralesional administration;
   2). the administration amount in one course of treatment is: ${Q}_{R} = {\sum }_{i = 1}^{nR} {q}_{R \left(i\right)}$,
      wherein nR is the number of intralesional administrations of R in one course of treatment, qR(i) is the amount of R in the i-th (i = 1,..., nR) administration, and: ${q}_{R \left(i\right)} = {\sum }_{i i = 1}^{n R i} {c}_{R \left(ii\right)} \times {v}_{R \left(ii\right)} ,$
      wherein nRi is the number of administration points, such as the number of needle insertion points, in the i-th administration of R, c_{R(ii)} and v_{R(ii)} are the concentration and volume of R at the ii-th administration point, such as the needle insertion point, respectively, where the concentration threshold of c_{R(ii)} (W/V) is 0.1% or 0.2%, and the upper limit of the concentration threshold is 51%, the volume threshold of v_{R(ii)} is 1/800 or 1/300 of the target volume (referred to as V_{target(ii)}), and the upper limit of the volume threshold is 3 times of V_{target(ii)};
- E is used to provide an immunomodulatory effect that benefits from the reduction of a biological barrier function, and/or optimizing the local and/or systemic immune response by utilizing the immune substances and/or inflammatory signals effectively released of a lesion within the lesion (referred to as activity E):
   1). intralesional administration and/or systemic administration;
   2). the administration amount for one course of treatment is: ${Q}_{E} = {\sum }_{k = 1}^{n E} {q}_{B \left(k\right)} + {\sum }_{k ′ = 1}^{n E ′} cE \left(k′\right) \times {v}_{E \left(k′\right)} ,$ wherein Q_{E} is a conventional amount of E in the anti-lesion application, n_{E} and q_{E(k)} are the number of systemic administrations and the amount of the k-th (j = 1,..., nₖ) administration of E in one course of treatment, respectively, and n_{E',} c_{E(k')}, and v_{E(k')} are the number of intratumoral administrations, the concentration and the volume of the k'-th (k' = 1,..., n_{k'}) administration of E in one course of treatment, respectively.

In some embodiments, the concentration threshold of c_{R(ii)} (W/W) can be 0.1% (synergistic threshold) or 0.2% (effective threshold), and the upper limit of the concentration threshold is 51%; the volume threshold of v_{R(ii)} can be 1/800 or 1/300 of the target volume (denoted as v_{target(ii)}), and the upper limit of the volume threshold is 3 times of V_{target(ii)}.

In some embodiments, in the above pharmaceutical combination, N,N-dimethylbiguanide (R) includes less than 30% (w/w), preferably no more than 5% a salt of N,N-dimethylbiguanide (R'), or more preferably does not comprise a salt of N,N-dimethylbiguanide (R'). Preferably, N,N-dimethylbiguanide (R) is in the form of a powder injection.

In some embodiments, the above-mentioned pharmaceutical combination is suitable for local lesions such as tumors. Preferably, the pharmaceutical combination comprises B and/or E, and is used for refractory malignant tumors. Preferably, the refractory malignant tumors include or are selected from at least one of the following groups: chemotherapy drug-resistant tumors, tumors with unfavorable microenvironments, tumors after discontinuation of antineoplastic agents, tumors without effective chemotherapeutic drugs, and tumors in patients with contraindication to or unsuitability for conventional chemotherapeutic drugs.

In some embodiments, the above-mentioned pharmaceutical combination is suitable for heterogeneous tumors, where the heterogeneity includes at least one of tissue heterogeneity, spatial heterogeneity, and safety heterogeneity. The heterogeneous tumors are those where different tumors of the same type or different regions of the same tumor are set as different target areas for differentiated administration to facilitate treatment.

In some embodiments, in the above-mentioned pharmaceutical combination, the component (Z) includes antineoplastic agents, and the antineoplastic agents include or are selected from cell-reactive drugs (B) that one of whose key control steps is to cross the tumor tissue barrier.

In some embodiments, in the above-mentioned pharmaceutical combination, the cell-reactive drugs include cytotoxic drugs and targeted antineoplastic agents.

In some embodiments, in the above-mentioned pharmaceutical combination, the cytotoxic drugs include DNA-damaging agents, antimetabolites, microtubule inhibitors, and topoisomerase inhibitors.

In some embodiments, in the above-mentioned pharmaceutical combination, representative compounds of DNA-damaging agents include cisplatin, carboplatin, oxaliplatin, ifosfamide, and doxorubicin.

In some embodiments, in the above-mentioned pharmaceutical combination, representative compounds of antimetabolites include fluorouracil, gemcitabine, and methotrexate.

In some embodiments, in the above-mentioned pharmaceutical combination, representative compounds of microtubule inhibitors include paclitaxel and vincristine.

In some embodiments, in the above-mentioned pharmaceutical combination, targeted antineoplastic agents include gefitinib, osimertinib, larotrectinib, pazopanib, trastuzumab, and derivatives thereof.

In some embodiments, in the above-mentioned pharmaceutical combination, medication B includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin, mitoxantrone, gefitinib, osimertinib, larotrectinib, pazopanib, trastuzumab, and derivatives thereof.

In some embodiments, in the above-mentioned pharmaceutical combination, the near-neutral or weakly basic sodium salt (C) includes or is selected from one or more of the following: sodium chloride, sodium bicarbonate, sodium dihydrogen phosphate, sodium lactate, sodium acetate, and the like. Preferably, the weakly basic sodium salt (C) includes injectable weakly basic sodium salts, such as sodium bicarbonate.

In some embodiments, in the above-mentioned pharmaceutical combination, the methylene blue dye (D) includes or is selected from one or more of the following: methylene blue, patent blue, isosulfan blue, and new methylene blue.

In some embodiments, in the above-mentioned pharmaceutical combination, the immunomodulator (E) includes immunomodulators and/or immunosuppressants. Specifically, the immunomodulators can include or be selected from one or more of the following: biological macromolecule immunomodulators (abbreviated as E1), immune cell immunomodulators (abbreviated as E2), and vaccine immunomodulators (abbreviated as E3).

In some embodiments, biological macromolecule immunomodulators can include, for example, immune checkpoint inhibitors, cytokines, and TLR agonists. In one embodiment, the immune checkpoint inhibitors can include Anti-PD-1 (CD279), cytokines can include interleukin-15, and TLR agonists can include CpG ODN 1826 (abbreviated as CpG).

In some embodiments, the immune cell immunomodulators include, for example, CAR-T cell therapy, DC cell therapy, TIL cell therapy, and the like.

In some embodiments, vaccine immunomodulators include Bacillus Calmette-Guérin (BCG).

In some embodiments, in the pharmaceutical combination, the administration concentration (w/w) of dimethylbiguanide (R) can be 0.5% - 51%, 0.5 - 50%, 0.5 - 45%, 1% - 45%, 5% - 45%, 5% - 40%, 5% - 30%, 5% - 25%, 5% - 20%, 1% - 30%, 1% - 25%, 1% - 20%, 0.5% - 10%, 1% - 5%, 15% - 50%, 25% - 50%, 35% - 50%, 5% - 20%, or ≤ 2% such as 1%, 0.75%, or 0.5%.

In some embodiments, the above pharmaceutical combination includes dimethylbiguanide (R) and doxorubicin (DOX), where the dimethylbiguanide (R) can be administered at a concentration of 0.2% - 50%, and the doxorubicin (DOX) can be administered at a concentration of 0.025% - 1%. Preferably, the dimethylbiguanide (R) is administered at a concentration of 0.5% - 50%, and the doxorubicin (DOX) is administered at a concentration of 0.1% - 1%. Optionally, both dimethylbiguanide (R) and doxorubicin (DOX) are administered intratumorally.

In some embodiments, the pharmaceutical combination includes dimethylbiguanide (R) and ifosfamide (IFO), where the dimethylbiguanide (R) can be administered at a concentration of 0.2% - 50%, and the ifosfamide (IFO) can be administered at a concentration of 0.1% - 15%. Preferably, the dimethylbiguanide (R) is administered at a concentration of 0.2% - 45%, and the ifosfamide (IFO) is administered at a concentration of 0.1% - 10%. More preferably, the dimethylbiguanide (R) is administered at a concentration of 5% - 45%, and the ifosfamide (IFO) is administered at a concentration of 0.5% - 10%.

In some embodiments, the pharmaceutical combination comprises dimethylbiguanide (R) and cisplatin (DDP), wherein the dimethylbiguanide (R) can be administered at a concentration of 0.2% to 50%, and the cisplatin (DDP) can be administered at a concentration of 0.01% to 0.4%. Preferably, dimethylbiguanide (R) is administered at a concentration of 0.5% to 50%, and the cisplatin (DDP) is administered at a concentration of 0.01% to 0.04%.

In some embodiments, the pharmaceutical combination comprises dimethylbiguanide (R) and 5-fluorouracil (5-FU), wherein the dimethylbiguanide (R) can be administered at a concentration of 0.2% to 50%, and the 5-fluorouracil (5-FU) can be administered at a concentration of 0.05% to 9%. Preferably, the dimethylbiguanide (R) is administered at a concentration of 0.2% to 45%, and the 5-fluorouracil (5-FU) is administered at a concentration of 0.5% to 9%. More preferably, the dimethylbiguanide (R) is administered at a concentration of 5% to 45%, and the 5-fluorouracil (5-FU) is administered at a concentration of 0.9% to 9%.

In some embodiments, the pharmaceutical combination comprises dimethylbiguanide (R), cisplatin (DDP), and gemcitabine (GEM), wherein the dimethylbiguanide (R) can be administered at a concentration of 0.2% to 50%, the cisplatin (DDP) can be administered at a concentration of 0.05% to 1%, and the gemcitabine (GEM) can be administered at a concentration of 0.5% to 2%, preferably at a concentration of 1%.

In some embodiments, the pharmaceutical combination comprises dimethylbiguanide (R), paclitaxel (PTX), and 5-fluorouracil (5-FU), wherein the dimethylbiguanide (R) can be administered at a concentration of 0.2% to 50%, the 5-fluorouracil (5-FU) can be administered at a concentration of 0.1% to 10%, and the paclitaxel can be administered at a concentration of 0.02% to 1%, preferably at a concentration of 0.03%.

In some embodiments, the pharmaceutical combination comprises dimethylbiguanide (R) and osimertinib, wherein the dimethylbiguanide (R) can be administered at a concentration of 0.2% to 50%, and the osimertinib can be administered at a concentration of 0.05% to 2%. Preferably, the dimethylbiguanide (R) is administered at a concentration of 5%, and the osimertinib is administered at a concentration of 0.15%.

In some embodiments, the pharmaceutical combination comprises dimethylbiguanide (R) and gefitinib (GEF), wherein the dimethylbiguanide (R) can be administered at a concentration of 0.2% to 50%, and the gefitinib (GR) can be administered at a concentration of 0.1% to 2%. Preferably, the dimethylbiguanide (R) is administered at a concentration of 1% to 50%, and the gefitinib (GR) is administered at a concentration of 0.5%.

In some embodiments, the pharmaceutical combination comprises dimethylbiguanide (R), cisplatin (DDP), and gefitinib (GR), wherein the dimethylbiguanide (R) is administered at a concentration of 5%, the gefitinib (GR) is administered at a concentration of 0.5%, and the cisplatin (DDP) is administered at a concentration of 0.15.

In some embodiments, the pharmaceutical combination comprises dimethylbiguanide (R), ifosfamide (IFO), and gefitinib (GR), wherein the dimethylbiguanide (R) is administered at a concentration of 5%, the ifosfamide (IFO) is administered at a concentration of 1%, and the gefitinib (GR) is administered at a concentration of 0.5%.

In some embodiments, the pharmaceutical combination comprises dimethylbiguanide (R), doxorubicin (DOX), and gefitinib (GR), wherein the dimethylbiguanide (R) is administered at a concentration of 5%, the doxorubicin (DOX) at a concentration of 0.05%, and the gefitinib (GR) at a concentration of 0.5%.

In some embodiments, the pharmaceutical combination comprises dimethylbiguanide (R) and sodium bicarbonate (SB), wherein the dimethylbiguanide (R) can be administered at a concentration of 0.5% to 4%, and the sodium bicarbonate (SB) can be administered at a concentration of 0.5% to 15%, preferably 0.5% to 10%, and more preferably 1.5% to 9%; preferably, the dimethylbiguanide (R) is administered at a concentration of 1% to 3%, and the sodium bicarbonate (SB) at a concentration of 1% to 9%; more preferably, the dimethylbiguanide (R) is administered at a concentration of 3%, and the sodium bicarbonate (SB) at a concentration of 3% to 9%. Optionally, the pharmaceutical combination may also include ifosfamide (IFO) at a concentration of 0.5%, 5-fluorouracil (5-FU) at a concentration of 0.4%, or osimertinib at a concentration of 0.3%.

In some embodiments, the pharmaceutical combination comprises dimethylbiguanide (R) and methylene blue dye (D) such as methylene blue (MB). In one embodiment, the pharmaceutical combination comprises dimethylbiguanide (R) and methylene blue (MB), wherein the dimethylbiguanide (R) can be administered at a concentration of 1% to 50%, and the MB can be administered at a concentration of 0.3% to 5%, preferably 0.5% to 5%; more preferably, the dimethylbiguanide (R) is administered at a concentration of 5% to 50%, and the MB at a concentration of 0.5% to 5%; preferably, the dimethylbiguanide (R) is administered at a concentration of 5% to 50%, and the MB at a concentration of 0.5% to 1%. Optionally, the pharmaceutical combination may also include one or more of ifosfamide (IFO) at a concentration of 0.5% to 1%, 5-fluorouracil (5-FU) at a concentration of 0.4% to 1%, and osimertinib at a concentration of 0.3%. Preferably, the pharmaceutical combination comprising dimethylbiguanide (R) and methylene blue (MB) can be administered intratumorally and/or systemically. In some embodiments, the pharmaceutical combination comprises dimethylbiguanide (R), MB, and any one of IFO, 5-FU, and osimertinib, wherein the dimethylbiguanide (R) is administered at a concentration of 5%, the MB is administered at a concentration of 1%, the IFO is administered at a concentration of 0.5%, the 5-FU is administered at a concentration of 0.4%, and the osimertinib is administered at a concentration of 0.3%. In some embodiments, the pharmaceutical combination comprises dimethylbiguanide (R), SB, MB, and any one of IFO, 5-FU, and osimertinib, wherein the dimethylbiguanide (R) is administered at a concentration of 5%, the SB is administered at a concentration of 5% to 10%, the MB is administered at a concentration of 0.5%, the IFO is administered at a concentration of 0.5%, the 5-FU is administered at a concentration of 0.4%, and the osimertinib is administered at a concentration of 0.3%.

In some embodiments, the pharmaceutical combination comprises dimethylbiguanide (R) and an immunomodulator (E), wherein the immunomodulator includes or is selected from biological macromolecule immunomodulators (abbreviated as E1), immune cell immunomodulators (abbreviated as E2), and vaccine immunomodulators (abbreviated as E3). In some embodiments, biological macromolecule immunomodulators can be, for example, immune checkpoint inhibitors, cytokines, and TLR agonists. In one embodiment, the immune checkpoint inhibitor can be Anti-PD-1 (CD279), the cytokine can be interleukin-15, the TLR agonist includes CpG ODN 1826 (abbreviated as CpG), and the vaccine-immunomodulator can be BCG.

In some embodiments, the pharmaceutical combination comprises dimethylbiguanide (R) and CpG ODN 1826 (CpG), wherein the dimethylbiguanide (R) can be administered at a concentration of 1% to 50%, and the CpG can be administered at a concentration of 0.1%; preferably, the dimethylbiguanide (R) is administered at a concentration of 2.5% to 50%, and the CpG is administered at a concentration of 0.1%. Preferably, the pharmaceutical combination further includes MB at a concentration of 1%, IFO at a concentration of 0.5%, and/or SB at a concentration of 10%. In some embodiments, the pharmaceutical combination comprises dimethylbiguanide (R) and CpG and one or two of the following: IFO, MB, and SB, wherein the dimethylbiguanide (R) is administered at a concentration of 2.5% to 50%, the IFO at a concentration of 0.5%, the CpG at a concentration of 0.1%, the MB at a concentration of 1%, and the SB is administered at a concentration of 10%.

In some embodiments, the pharmaceutical combination comprises dimethylbiguanide (R) and the immune checkpoint inhibitor Anti-PD-1 (CD279), such as RMP1-14, wherein the dimethylbiguanide (R) can be administered at a concentration of 0.2% to 50%, and the RMP1-14 can be administered at a concentration of 0.1%; preferably, the dimethylbiguanide (R) is administered at a concentration of 1% to 50%, and the RMP1-14 is administered at a concentration of 0.1%.

In some embodiments, the pharmaceutical combination comprises dimethylbiguanide (R) and BCG, wherein the dimethylbiguanide (R) can be administered at a concentration of 1% to 50%, preferably 2.5% to 50%, and more preferably 5% to 50%, and the BCG can be administered at a dose of 1×10⁶ CFU; preferably, the dimethylbiguanide (R) is administered at a concentration of 50%, and the BCG is administered at a dose of 1×10⁶ CFU. Optionally, the pharmaceutical combination further includes any one of IFO at a concentration of 0.5% and SB at a concentration of 10%. In some embodiments, the pharmaceutical combination comprises dimethylbiguanide (R) and BCG and one of the following: IFO and SB, wherein the dimethylbiguanide (R) is administered at a concentration of 5% to 50%, the BCG is administered at a dose of 1×10⁶ CFU, the IFO is administered at a concentration of 0.5%, and the SB is administered at a concentration of 10%.

In some embodiments, dimethylbiguanide (R) and component (Z) in the pharmaceutical combination can be administered simultaneously, sequentially, or at intervals.

In some embodiments, dimethylbiguanide (R) and component (Z) in the pharmaceutical combination are both administered intratumorally (combination relationship I); the R and Z are administered intratumorally and systemically, respectively (combination relationship II); or a portion of Z is administered intratumorally and the other portion of Z is administered systemically, while R is administered intratumorally (combination relationship III).

In some embodiments, the pharmaceutical combination can be used to treat lesions such as tumors or nodules in a subject.

According to the fourth aspect of the present invention, a pharmaceutical combination comprising formulation I and formulation II is provided, wherein the formulation I comprises N,N-dimethylbiguanide (R) or R and a portion of component (Z), and the formulation II comprises Z but not R, wherein:
1) in the formulation I, the N,N-dimethylbiguanide (R) comprises ≤5% or does not comprise a salt of N,N-dimethylbiguanide;
2) a co-adminstration mode of the formulation I and the formulation II is:
   formulation I is only used for intratumoral administration and cannot be used for systemic administration,
   formulation II can be used for intratumoral administration and/or systemic administration;
3) a co-adminstration amount of the formulation I and the formulation II is limited by the co-adminstration amount of Z and R:
   (1). the adminstration amount of R is: ${Q}_{R} = {\sum }_{i = 1}^{nR} {q}_{R \left(i\right)}$,
      wherein n_{R} is the number of intratumoral administrations of R in one course of treatment, q_{R(i)} is the amount of R in the i-th (i = 1,..., n_{R}) administration, and: ${q}_{R \left(i\right)} = {\sum }_{i i - 1}^{n R i} {c}_{R \left(ii\right)} \times {v}_{R \left(ii\right)} ,$
      wherein n_{Ri} is the number of administration points, for example, the number of needle insertion points of R in the i-th administration, c_{R(ii)} and v_{R(ii)} are the concentration and volume of R administered at the ii-th administration point, for example, the needle insertion point, wherein the v_{R(ii)} is ≤ 3 times of the target volume of the ii-th administration point (denoted as v_{target(ii)}), and c_{R} is 0.2-51%, wherein 0.2% is the co-adminstration concentration threshold of R, and 51% is the upper limit of the dissolution threshold of R;
   (2). the adminstration amount of Z is: ${Q}_{Z} = {\sum }_{j - l}^{nB} {q}_{Z \left(j\right)} + {\sum }_{i ′ - l}^{nB ′} {c}_{Z \left(j′\right)} \times {v}_{Z \left(j′\right)} ,$ wherein Q_{Z} is ≤ the conventional amount of Z, n_{Z} and q_{Z(j)} are the number of systemic administrations of Z in one course of treatment and the adminstration amount in the j-th (j = 1,..., nB) administration, respectively, n_{Z'}, c_{Z(j')} and v_{Z(j')} are the number of intratumoral administrations of Z in one course of treatment, and the concentration and volume at the j'-th (j' = 1,..., n_{Z'}) administration, respectively.

In some embodiments, in the above pharmaceutical combination, the component Z includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin and mitoxantrone.

In some embodiments, in the above pharmaceutical combination, the mixing contraindications of R are any one of the following groups:
1) a salt of N,N-dimethylbiguanide (R');
2) an acidifying agent that significantly converts R to R', wherein the acidifying agent includes acidic pH regulators and other acidic substances;
3) a conventional dimethylbiguanide pharmaceutical adjuvant having an amount ratio with R (Q adjuvant/Q_{R}) of >3.0 or >4.0, including its conventional sustained-release dosage form carriers, such as gel carriers and nano-carriers;
4) adjuvants that are unstable in strongly alkaline aqueous solutions, including, for example, reducing sugars, cellulose, liposomes, carbomers, and polyethylene glycol-modified nano-carriers,
wherein the mixing contraindications refer to the need to avoid mixing with them throughout the entire process of formulation, preparation, product, and use.

In some embodiments, in the above pharmaceutical combination, the co-adminstration mode includes:
1) R and Z are mixed for intralesional or intratumoral administration,
2) R and Z are used for intratumoral or intratumoral administration, respectively,
3) R and Z are used for intralesional or intratumoral administration, and systemic administration, respectively,
4) R and a portion of Z are mixed or separately used for intralesional or intratumoral administration, while the other portion of Z is used for systemic administration.

In some embodiments, in the above pharmaceutical combination, the co-adminstration threshold is 0.5%, 1%, 3.3%, 5%, or 10%.

According to the fifth aspect of the present invention, a pharmaceutical composition comprising N,N-dimethylbiguanide in free-base form (R) or any of the above-mentioned pharmaceutical compositions of the present invention is provided.

According to the sixth aspect of the present invention, a formulation comprising any of the above-mentioned pharmaceutical combinations or compositions of the present invention is provided.

According to the seventh aspect of the present invention, a use of N,N-dimethylbiguanide (R) and an antineoplastic drug in the preparation of a medicament, a pharmaceutical combination or a kit for treating tumors is provided.

According to the eighth aspect of the present invention, a use of the above-mentioned formulation, pharmaceutical combination or composition of the present invention in the preparation of a medicament, a pharmaceutical combination or a kit for treating lesions such as tumors or nodules in a subject is provided.

Specifically, the above-mentioned formulation, pharmaceutical combination or composition of the present invention can be used to treat lesions such as tumors or nodules in a subject.

The R in the above-mentioned formulation, pharmaceutical combination or composition of the present invention is used to provide transient activity, where the transient activity refers to a local activity provided within a transient period before the active form of R is converted to the active form of R'.

In some embodiments, in the above uses, the administration concentration (w/w) of the dimethylbiguanide is ≤51%, ≤50%, ≤40%, ≤30%, ≤25%, ≤20%, ≤10%, ≤5%, or ≤2%, such as 1%, 0.75%, or 0.5%.

In some embodiments, in the above uses, the administration concentration (w/w) of N,N-dimethylbiguanide (R) is 0.2%-51%, 0.5-51%, 0.5-50%, 1%-40%, 5%-50%, 5%-40%, 5%-30%, 5%-25%, 5%-20%, 1%-30%, 1%-25%, 1%-20%, 0.5%-10%, 1%-5%, or ≤2%, such as 1%, 0.75%, or 0.5%.

In some embodiments, in the above uses, the concentration (w/w) of the salt of N,N-dimethylbiguanide contained in the formulation, pharmaceutical combination or composition is ≤30%, preferably ≤20%, preferably ≤10%, more preferably ≤5%, ≤4%, or ≤3%, more preferably ≤2%, particularly preferably ≤1%, such as 1% or 0.5%, and most preferably 0%, that is, the formulation, pharmaceutical combination or composition does not contain the salt of N,N-dimethylbiguanide.

In some embodiments, the formulation of the present invention comprises N,N-dimethylbiguanide and optionally a solvent, and the formulation does not contain additional agents required for cell culture or the safety of conventional injection. The concentration of N,N-dimethylbiguanide contained in the formulation is ≥ its administration or intervention concentration, where the administration or intervention concentration (w/w) is 0.5% ≤ C ≤ 51%, preferably, 1-51%, 2-50%, 3-50%, 4-50%, 5-50%, 6-50%, 7-50%, 8-50%, 9-50%, 10-50%, 15-50%, 20-50%, 25-50%, 30-50%, 35-50%, 40-50%, 1-40%, 5-50%, 10-40%, 15-40%, 20-40%, 25-40%, 30-40%, 35-40%, or 1-35%, 5%-30%, etc., or any value and range therebetween. For example, 1.5%, 2%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 21%, 21.5%, 22%, 22.5%, 23%, 23.5%, 24%, 24.5%, 25%, 26%, 27%, 28%, 29%, 30%, 31%, 32%, 33%, 34%, 35%, 36%, 37%, 38%, 39%, 40%, 45%, 50%.

In some embodiments, the formulation comprises a solvent, such as a pharmaceutically acceptable carrier, preferably water, and more preferably (sterile) water for injection.

In some embodiments, the formulation does not include a salt of N,N-dimethylbiguanide.

In some embodiments, the concentration (w/w) of the salt of N,N-dimethylbiguanide included in the formulation is ≤ 30%, ≤ 25%, preferably, ≤ 10%, preferably ≤ 5%, more preferably ≤ 4%, more preferably ≤ 3%, even more preferably ≤ 2%, particularly preferably ≤ 1%, and most preferably 0%, that is, the formulation does not contain a salt of N,N-dimethylbiguanide.

In one embodiment, the additional agent includes an acidic pH adjusting agnt.

In one embodiment, the acidic pH adjusting agnt includes or is selected from hydrochloric acid, sulfuric acid, acetic acid, citric acid, phosphoric acid, malic acid, tartaric acid, lactic acid, carbonic acid, and the like.

In some embodiments, the pharmaceutical combination or composition for the above uses includes an antineoplastic drug or agent, and the antineoplastic drug or agent includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin and mitoxantrone.

In some embodiments, in the above uses, the antineoplastic drug is doxorubicin (DOX), where the dimethylbiguanide (R) can be administered at a concentration (w/w) of 0.2% - 50%, and the doxorubicin (DOX) can be administered at a concentration (w/w) of 0.025% - 1%, preferably, the dimethylbiguanide (R) is administered at a concentration (w/w) of 5% - 50%, and the doxorubicin (DOX) is administered at a concentration (w/w) of 0.1% - 1%.

In some embodiments, in the above uses, the antineoplastic drug is ifosfamide (IFO), where the dimethylbiguanide (R) can be administered at a concentration (w/w) of 0.2% - 50%, and the ifosfamide (IFO) can be administered at a concentration (w/w) of 0.1% - 10%, preferably, the dimethylbiguanide (R) is administered at a concentration (w/w) of 0.2% - 45%, and the ifosfamide (IFO) is administered at a concentration (w/w) of 0.1% - 10%, more preferably, the dimethylbiguanide (R) is administered at a concentration (w/w) of 5% - 45%, and the ifosfamide (IFO) is administered at a concentration (w/w) of 0.5% - 10%.

In some embodiments, in the above uses, the antineoplastic drug is cisplatin (DDP), where the dimethylbiguanide (R) can be administered at a concentration (w/w) of 0.2% - 50%, and the cisplatin (DDP) can be administered at a concentration (w/w) of 0.01% - 0.4%, preferably, the dimethylbiguanide (R) is administered at a concentration (w/w) of 0.5% - 50%, and the cisplatin (DDP) is administered at a concentration (w/w) of 0.01% - 0.04%.

In some embodiments, in the above uses, the antineoplastic drug is 5-fluorouracil (5-Fu), wherein the dimethylbiguanide (R) can be administered at a concentration of 0.2% to 50%, and the 5-fluorouracil (5-FU) can be administered at a concentration of 0.05% to 9% (w/w). Preferably, the dimethylbiguanide (R) is administered at a concentration of 0.2% to 45% (w/w), and the 5-fluorouracil (5-Fu) is administered at a concentration of 0.5% to 9% (w/w). More preferably, the dimethylbiguanide (R) is administered at a concentration (w/w) of 5% to 45%, and the 5-fluorouracil (5-Fu) is administered at a concentration (w/w) of 0.9% to 9%.

In some embodiments, in the above uses, the antineoplastic drug includes cisplatin (DDP) and gemcitabine (GEM), wherein the dimethylbiguanide (R) can be administered at a concentration (w/w) of 0.2% to 50%, the cisplatin (DDP) can be administered at a concentration (w/w) of 0.05% to 1%, and the gemcitabine (GEM) can be administered at a concentration (w/w) of 0.5% to 2%, preferably at a concentration (w/w) of 1%.

In some embodiments, in the above uses, the antineoplastic drug includes paclitaxel (PTX) and 5-fluorouracil (5-Fu), wherein the dimethylbiguanide (R) can be administered at a concentration (w/w) of 0.2% to 50%, the 5-fluorouracil (5-Fu) can be administered at a concentration (w/w) of 0.1% to 10%, and the paclitaxel can be administered at a concentration (w/w) of 0.02% to 1%, preferably at a concentration (w/w) of 0.03%.

In some embodiments, in the above uses, the antineoplastic drug is osimertinib, wherein the dimethylbiguanide (R) can be administered at a concentration (w/w) of 0.2% to 50%, and the osimertinib can be administered at a concentration (w/w) of 0.05% to 2%. Preferably, the dimethylbiguanide (R) is administered at a concentration (w/w) of 5%, and the osimertinib is administered at a concentration (w/w) of 0.15%.

In some embodiments, in the above uses, the antineoplastic drug is gefitinib (GR), wherein the dimethylbiguanide (R) can be administered at a concentration (w/w) of 0.2% to 50%, and the gefitinib (GR) can be administered at a concentration (w/w) of 0.1% to 2%. Preferably, the dimethylbiguanide (R) is administered at a concentration (w/w) of 1% to 50%, and the gefitinib (GR) is administered at a concentration (w/w) of 0.5%.

In some embodiments, in the above uses, the antineoplastic drug includes cisplatin (DDP) and gefitinib (GR), wherein the dimethylbiguanide (R) is administered at a concentration (w/w) of 5%, the gefitinib (GR) is administered at a concentration (w/w) of 0.5%, and the cisplatin (DDP) is administered at a concentration (w/w) of 0.15%.

In some embodiments, in the above uses, the antineoplastic drug includes ifosfamide (IFO) and gefitinib (GR), wherein the dimethylbiguanide (R) is administered at a concentration (w/w) of 5%, the ifosfamide (IFO) is administered at a concentration (w/w) of 1%, and the gefitinib (GR) is administered at a concentration (w/w) of 0.5%.

In some embodiments, in the above uses, the antineoplastic drug includes doxorubicin (DOX) and gefitinib (GR), wherein the N,N-dimethylbiguanide (R) is administered at a concentration (w/w) of 5%, the doxorubicin (DOX) is administered at a concentration(w/w) of 0.05%, and the gefitinib (GR) is administered at a concentration (w/w) of 0.5%.

In some embodiments, in the above uses, the N,N-dimethylbiguanide and the antineoplastic drug can be administered simultaneously, sequentially, or at intervals.

In some embodiments, in the above uses, both the N,N-dimethylbiguanide and the antineoplastic drug are administered intralesionally or intratumorally (combination relationship I); the R and B are administered intralesionally or intratumorally and systemically, respectively (combination relationship II); or R and a portion of the B is administered intralesionally or intratumorally, and the other portion of the B is administered systemically (combination relationship III).

In some embodiments, in the above uses, the N,N-dimethylbiguanide is used to provide a morphological manifestation different from that of conventional metformin drug (whose active ingredient is R'), wherein the active form of R' is completely complexed with acid in solution; while the active form of R is less complexed with acid in solution, mainly existing in a form of minimal or partial acid complexation, thereby providing an activity (referred to as activity A) that is difficult to be provided by R' through the transient local effect of this different active form, including the activity of effectively reducing the function of the biological barrier (referred to as activity A1), the activity of effectively damaging the lesion (referred to as activity A2), and/or the activity of effectively releasing the pathological immune substances and/or inflammatory signals within the pathological tissue (referred to as activity A3).

In some embodiments, the N,N-dimethylbiguanide is used to provide an action that maximizes the effect of the antineoplastic drug, including the activity of effectively reducing the function of the tumor tissue barrier (referred to as activity A).

In some embodiments, the antineoplastic drug is used to provide an antineoplastic effect that maximizes the transient effect of the N,N-dimethylbiguanide, including the antineoplastic effect triggered at the tumor cell level (referred to as activity B).

In some embodiments, the N,N-dimethylbiguanide (R) shall not be mixed with a sufficient amount of R', or an acidifying agent that can convert R to a sufficient amount of R', wherein the sufficient amount refers to an amount that makes the ratio of R' to R (R'/R) be ≥ 1/3.

In some embodiments, in the above uses, a synergy is produced between N,N-dimethylbiguanide and the antineoplastic drug, the synergy being a dose reduction and efficacy enhancement relative to a conventional co-administered combination (R'/B combination), which is manifested as providing the following better effects when the dosage of dimethylbiguanide is at least halved (QR ≤ 1/2QR') and the dosage of B is reduced by at least 20%:
1). the pharmaceutical use performance, including adaptability to tumor heterogeneity, is significantly improved, manifested as at least one of the pharmaceutical adjustability indexes increasing by at least 100%, where the pharmaceutical adjustability refers to the performance of the medicament being able to provide differentiated administration parameters to different target areas while still maintaining efficacy; and/or
2). the pharmaceutical efficacy is significantly enhanced, manifested as at least one of the pharmaceutical efficacy indexes increasing by at least 25%.

In some embodiments, in the above uses, the synergy is further manifested as a further enhanced efficacy relative to the highest single agent scheme of R and B, manifested as at least one of the pharmaceutical adjustability indexes increasing by at least 10%, and/or at least one of the pharmaceutical efficacy indexes increasing by at least 10%.

In some embodiments, the pharmaceutical adjustability indexes include at least one of the following intratumoral administration adjustability coefficients:
1). the concentration adjustability coefficient of R, which is a ratio of the maximum value of c_{R} to the threshold (cR's maximum value/threshold), and an increase in the adjustability index is an increase in the coefficient, preferably the coefficient increases to >10, 50, 100 or 150, which enables R to provide highly variable concentrations to different regions while still maintaining its activity stable, including for example, tailored medicament for heterogeneous tumors;
2). the target volume adjustability coefficient of R, which is a ratio of the maximum value of target volume (v_{target)} to the threshold (v_{target}'s maximum value/threshold), and an increase in the adjustability index is an increase in the coefficient, preferably the coefficient increases to >2, 10, 50, or 100, which enables R to select different v_{target} according to different treatment situations, including for example, tailoredmedicament for heterogeneous tumors;
3). the administration volume ratio adjustability coefficient of R, which is a ratio of the maximum value to threshold of the ratio of administration volume (v_{R}) to the threshold (maximum value/threshold of v_{R}/v_{target}), and an increase in the adjustability index is an increase in the coefficient, preferably the coefficient increases to >5, 10, or 100, which enables R to provide highly variable volume ratios to different regions while still maintaining its activity stable, including for example, tailoredmedicament for heterogeneous tumors;
4). the single-dose adjustability coefficient of R, which is a ratio of the maximum value of single dose (c_{Ri}×v_{Ri}) to the threshold (c_{Ri}×v_{Ri}'s maximum value/threshold), and an increase in the adjustability index is an increase in the coefficient, preferably the coefficient increases to >2, 6, or 10, which enables R to select different single doses according to different treatment situations, including for example, tailoredmedicament for heterogeneous tumors; and
5). the density adjustable coefficient of the administration points of R, which is a ratio of the maximum value of the density of administration point (nᵢᵢ/ v_{target}) to the threshold (maximum value of nᵢᵢ/ v_{target}/threshold), and an increase in the adjustability index is an increase in the coefficient, preferably the coefficient is increased to > 3, 10, 50, or 100, which enables R to provide highly variable density to different regions while maintaining its activity stable, including for example, tailoredmedicament for heterogeneous tumors.

In some embodiments, the pharmaceutical efficacy indexes include at least one of the following:
1). Timeliness indicator, manifested as the onset time in mouse experiments (the day when the tumor proliferation rate < 42%), and the onset time is reduced by at least 25% compared to the R' scheme, R'/B coadminsteration scheme, and B single agent scheme, which makes the combination a fast-acting or further fast-acting combination, especially suitable for tumors that require rapid reduction in tumor size;
2). Effectiveness indicator, manifested as the tumor inhibition rate or objective response rate in mouse experiments, and the tumor inhibition rate or objective response rate is increased by at least 25% compared to the comparison schemes (the R' scheme, coadminsteration schemes of R' with B, D, and/or E, B, D, and/or E single agent schemes), which makes the combination a highly effective or further highly effective combination, especially suitable for tumors that require maximum inhibition of tumor size;
3). Long-term efficacy indicator, manifested as the progression-free time or survival rate in mouse experiments, and the progression-free time or survival rate is increased by at least 25% compared to the comparison schemes (the R' scheme, coadminsteration schemes of R' with B, D, and/or E, B, D, and/or E single agent schemes), which makes the combination a long-acting or further long-acting combination, especially suitable for tumors that require maximum extension of progression-free time.

**According to the ninth aspect of the present invention,** a method for treating cells of a lesion, such as a tumor is provided, comprising administering or introducing the above-mentioned formulation, pharmaceutical combination, and composition of the present invention to the lesion, penetrating into the lesion, and contacting with cells within the tissue.

**According to the tenth aspect of the present invention,** a method for treating a tumor is provided, comprising administering the above-mentioned formulation, pharmaceutical combination, and composition of the present invention to the tumor. Preferably, the administration includes intratumoral administration of the formulation of the present invention, and/or intratumoral administration of the dimethylbiguanide, and intratumoral or systemical administration of themedicament.

**According to the eleventh aspect of the present invention,** a method for treating a tumor in a subject is provided, including administering the above-mentioned formulation or pharmaceutical combination of the present invention to the subject.

In the above methods, the formulation contains N,N-dimethylbiguanide and optionally a solvent, wherein the formulation does not contain an acidic pH-adjusting agent required to meet cell-culture requirements or conventional injection safety requirements, and the concentration of N,N-dimethylbiguanide is not lower than its intervention concentration, and the intervention concentration (w/w) is 0.5% ≤ C ≤ 51%, preferably 1% - 50%, more preferably 3% - 40% or 5% - 35%.

In some embodiments, the formulation may contain a solvent, preferably the solvent is water, more preferably water for injection.

In some embodiments, the formulation may not include dimethylbiguanide salts.

In some embodiments, the concentration of N,N-dimethylbiguanide in the formulation (w/w) may be ≤ 51%, ≤ 50%, ≤ 40%, ≤ 30%, ≤ 25%, ≤ 20%, ≤ 10%, ≤ 5%, or ≤ 2%, such as 1%, 0.75%, or 0.5%.

In some embodiments, the concentration(w/w) of N,N-dimethylbiguanide (R) in the formulation can be 0.2% - 51%, 0.2% - 50%, 0.5% - 50%, 1% - 40%, 5% - 50%, 5% - 40%, 5% - 30%, 5% - 25%, 5% - 20%, 1% - 30%, 1% - 25%, 1% - 20%, 1% - 10%, 0.5% - 10%, 1% - 5%, or ≤ 2% such as 1%, 0.75%, or 0.5%.

In some embodiments, the acidic pH-adjusting agent can include or be selected from hydrochloric acid, sulfuric acid, acetic acid, citric acid, phosphoric acid, malic acid, tartaric acid, lactic acid, and carbonic acid.

In some embodiments, in the above method, R is contained in a powder.

In some embodiments, in the above method, the powder consists of dry powder containing R and a solvent.

In some embodiments, in the above method, the dry powder is dry powder of R, and the solvent is water for injection.

In some embodiments, in the above method, the formulation does not contain an acid that can significantly convert N,N-dimethylbiguanide (R) in the aqueous solution to dimethylbiguanide salt.

In the above method, the administered pharmaceutical combination or composition comprises N,N-dimethylbiguanide (R) and component (Z), wherein the administration concentration of N,N-dimethylbiguanide is 0.1% - 51%, preferably 0.2% - 50% by weight/weight (w/w), and themedicament includes a cell-reactive antineoplastic agent (abbreviated as B).

In some embodiments, in the above method, R in the pharmaceutical combination is used to provide an activity (abbreviated as activity A) that conventional dimethylbiguanide (active ingredient of which is dimethylbiguanide salt, abbreviated as R') is difficult to provide, including the effect of effectively damaging pathological tissue (abbreviated as activity A2), through following limitations:
1). Intralesional administration;
2). The adminsteration amount for one course of treatment is: ${Q}_{R} = {\sum }_{i = 1}^{nR} {q}_{R \left(i\right)}$,
   wherein, n_{R} is the number of intralesional administrations of R in one course of treatment, and q_{R(i)} is the amount of R in the i-th (i = 1,..., n_{R}) administration, and: ${q}_{R \left(i\right)} = {\sum }_{i i = 1}^{n R i} {c}_{R \left(ii\right)} \times {v}_{R \left(ii\right)} ,$
   wherein, nRi is the number of administration points, for example, the number of needle insertion points of R in the i-th administration, and c_{R(ii)} and v_{R(ii)} are the concentration and volume of R administered at the ii-th administration point, for example, the needle insertion point, respectively. The concentration threshold of c_{R(ii)} (W/V) is 0.1% or 0.2%, and the upper limit of the concentration threshold is 51%. The volume threshold of v_{R(ii)} is 1/800 or 1/300 of the target volume (abbreviated as V_{target(ii)}), and the upper limit of the volume threshold is 3 times of V_{target(ii)};

- The B provides the cellular response (abbreviated as active B) through the following limitations, one of key control steps of which is crossing the above-mentioned biological barrier:
   1). Intratumoral administration and/or systemic administration;
   2). The adminsteration amount for one course of treatment is: ${Q}_{B} = {\sum }_{j - l}^{nB} {q}_{B \left(j\right)} + {\sum }_{i ′ - l}^{nB ′} cB \left(j′\right) \times {v}_{B \left(j′\right)}$ ,
   wherein, Q_{B} is ≦ the conventional administration amount in the sensitive tumor of B, n_{B} and q_{B(j)} are the number of systemic administrations of B in one course of treatment and the amount in the j-th (j = 1,..., n_{B}) administration, respectively, and n_{B'}, c_{B(j')} and v_{B(j')} are the number of tumor administrations of B in one course of treatment and the concentration and volume in the j'-th (j' = 1,..., n_{B'}) administration, respectively;
- The type of the combination is a synergistic combination in which the pharmaceutical effects of the activity A of R and the activity B of B enhance each other.

In some embodiments, the N,N-dimethylbiguanide (R) includes < 30%, preferably ≦ 5% of a salt of N,N-dimethylbiguanide (R'), or does not comprise a salt of N,N-dimethylbiguanide (R'), and preferably, the -N,N-dimethylbiguanide (R) is in the form of a powder injection.

In some embodiments, in the above method, the component (Z) in the pharmaceutical combination or composition includes an antineoplastic drug, which is selected from a cell-reactive drug whose key control step is crossing the tumor tissue barrier.

In some embodiments, the cell-reactive drugs include cytotoxic drugs and targeted antineoplastic agents.

In some embodiments, the cytotoxic drugs include DNA damaging agents, antimetabolites, microtubule inhibitors, and topoisomerase inhibitors.

In some embodiments, the representative compounds of the DNA damaging agents include cisplatin, carboplatin, oxaliplatin, ifosfamide, and doxorubicin.

In some embodiments, the representative compounds of the antimetabolites include fluorouracil, gemcitabine, and methotrexate.

In some embodiments, the representative compounds of the microtubule inhibitors include paclitaxel, vincristine.

In some embodiments, in the above method, the targeted antineoplastic agents include kinase inhibitors.

In some embodiments, in the above method, the kinase inhibitors include gefitinib, osimertinib, larotrectinib, pazopanib, trastuzumab and derivatives thereof.

In some embodiments, in the above method, the B includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin, mitoxantrone, gefitinib, osimertinib, larotrectinib, pazopanib, trastuzumab and derivatives thereof.

In some embodiments, in the above-mentioned method, the administration concentration (w/w) of N,N-dimethylbiguanide (R) can be 0.2% - 51%, 0.2% - 50%, 0.5% - 50%, 0.5% - 45%, 1% - 45%, 5% - 45%, 5% - 40%, 5% - 30%, 5% - 25%, 5% - 20%, 1% - 30%, 1% - 25%, 1% - 20%, 0.5% - 10%, 1% - 5%, or ≤ 2%, such as 1%, 0.75%, or 0.5%.

In some embodiments, in the above-mentioned methods, the pharmaceutical combination or composition comprises dimethylbiguanide (R) and doxorubicin (DOX), wherein the dimethylbiguanide (R) can be administered at a concentration of 0.2% to 50% (w/w), and the doxorubicin (DOX) can be administered at a concentration of 0.025% to 1% (w/w). Preferably, the dimethylbiguanide (R) is administered at a concentration of 5% to 50% (w/w), and the doxorubicin (DOX) is administered at a concentration of 0.1% to 1% (w/w). Optionally, both dimethylbiguanide (R) and doxorubicin (DOX) are administered intratumorally.

In one embodiment, the dimethylbiguanide (R) is administered intratumorally at a concentration of 0.2% to 50% (w/w), and the doxorubicin (DOX) is administered intratumorally at a concentration of 0.025% to 1% (w/w), and the doxorubicin (DOX) is administered systemically, such as intraperitoneally at a concentration of 0.05% to 0.06% (w/w).

In some embodiments, in the above-mentioned methods, the pharmaceutical combination or composition comprises dimethylbiguanide (R) and ifosfamide (IFO), wherein the dimethylbiguanide (R) can be administered at a concentration of 0.2% to 50% (w/w), and the ifosfamide (IFO) can be administered at a concentration of 0.1% to 10% (w/w). Preferably, the dimethylbiguanide (R) is administered at a concentration of 0.2% to 45% (w/w), and the ifosfamide (IFO) is administered at a concentration of 0.1% to 10% (w/w). More preferably, the dimethylbiguanide (R) is administered at a concentration of 5% to 45% (w/w), and the ifosfamide (IFO) is administered at a concentration of 0.5% to 10% (w/w). Optionally, both dimethylbiguanide (R) and ifosfamide (IFO) are administered intratumorally.

In one embodiment, the dimethylbiguanide (R) is administered intratumorally at a concentration of 5% to 50% (w/w), and the ifosfamide (IFO) is administered intratumorally at a concentration of 0.5% to 10% (w/w), and the ifosfamide (IFO) is administered systemically such as intraperitoneally at a concentration of 0.5% (w/w).

In some embodiments, in the above-mentioned methods, the pharmaceutical combination or composition comprises dimethylbiguanide (R) and cisplatin (DDP), wherein the dimethylbiguanide (R) can be administered at a concentration of 0.2% to 50% (w/w), and the cisplatin (DDP) can be administered at a concentration of 0.01% to 0.4% (w/w). Preferably, the dimethylbiguanide (R) is administered at a concentration of 0.5% to 50% (w/w), and the cisplatin (DDP) is administered at a concentration of 0.01% to 0.04% (w/w). Optionally, both dimethylbiguanide (R) and cisplatin (DDP) are administered intratumorally.

In one embodiment, the dimethylbiguanide (R) is administered intratumorally at a concentration of 0.5% to 50% (w/w), and the cisplatin (DDP) is administered intratumorally at a concentration of 0.013% to 0.4% (w/w), and preferably, the cisplatin (DDP) is administered systemically, such as intraperitoneally at a concentration of 0.015% (w/w).

In some embodiments, in the above-mentioned methods, the pharmaceutical combination or composition comprises dimethylbiguanide (R) and 5-fluorouracil (5-FU), wherein the dimethylbiguanide (R) can be administered at a concentration of 0.2% to 50% (w/w), and the 5-fluorouracil (5-FU) can be administered at a concentration of 0.05% to 9% (w/w). Preferably, the dimethylbiguanide (R) is administered at a concentration of 0.2% to 45% (w/w), and the 5-fluorouracil (5-FU) is administered at a concentration of 0.5% to 9% (w/w). More preferably, the dimethylbiguanide (R) is administered at a concentration of 5% to 45% (w/w), and the 5-fluorouracil (5-FU) is administered at a concentration of 0.9% to 9% (w/w). Optionally, both dimethylbiguanide (R) and 5-fluorouracil (5-FU) are administered intratumorally.

In one embodiment, the dimethylbiguanide (R) is administered intratumorally at a concentration of 5% to 45% (w/w), the 5-fluorouracil (5-FU) is administered intratumorally at a concentration of 0.9% to 9% (w/w), and the 5-fluorouracil (5-FU) is administered systemically, such as intraperitoneally at a concentration of 0.5% (w/w).

In some embodiments, in the above-mentioned methods, the pharmaceutical combination or composition comprises dimethylbiguanide (R), cisplatin (DDP), and gemcitabine (GEM), wherein the dimethylbiguanide (R) can be administered at a concentration of 0.2% to 50% (w/w), the cisplatin (DDP) can be administered at a concentration of 0.05% to 1% (w/w), and the gemcitabine (GEM) can be administered at a concentration of 0.5% to 2% (w/w), preferably at a concentration of 1% (w/w). Optionally, or, dimethylbiguanide (R) and cisplatin (DDP) are administered intratumorally and gemcitabine (GEM) is administered systemically, such as intraperitoneally.

In some embodiments, in the above-mentioned methods, the pharmaceutical combination or composition comprises dimethylbiguanide (R), paclitaxel (PTX), and 5-fluorouracil (5-FU), wherein the dimethylbiguanide (R) can be administered at a concentration of 0.2% to 50% (w/w), the 5-fluorouracil (5-FU) can be administered at a concentration of 0.1% to 10% (w/w), and the paclitaxel can be administered at a concentration of 0.02% to 1% (w/w), preferably at a concentration of 0.03% (w/w). Optionally, dimethylbiguanide (R) and 5-fluorouracil (5-FU) are administered intratumorally and paclitaxel (PTX) is administered systemically, such as intraperitoneally.

In some embodiments, in the above-mentioned methods, the pharmaceutical combination or composition comprises dimethylbiguanide (R) and osimertinib, wherein the dimethylbiguanide (R) can be administered at a concentration of 0.2% to 50% (w/w), and the osimertinib can be administered at a concentration of 0.05% to 2% (w/w). Preferably, the dimethylbiguanide (R) is administered at a concentration of 5% (w/w), and the osimertinib is administered at a concentration of 0.15% (w/w). Optionally, the dimethylbiguanide (R) is administered intratumorally, and the osimertinib is administered systemically, such as intraperitoneally.

In some embodiments, in the above-mentioned method, the pharmaceutical combination or composition comprises dimethylbiguanide (R) and gefitinib (GR), wherein the dimethylbiguanide (R) can be administered at a concentration of 0.2% to 50% (w/w), and the gefitinib (GR) can be administered at a concentration of 0.1% to 2% (w/w). Preferably, the dimethylbiguanide (R) is administered at a concentration of 1% to 50% (w/w), and the gefitinib (GR) is administered at a concentration of 0.5% (w/w). Optionally, the dimethylbiguanide (R) is administered intratumorally, and the gefitinib (GR) is administered systemically, for example, intraperitoneally.

In some embodiments, in the above-mentioned method, the pharmaceutical combination comprises dimethylbiguanide (R), cisplatin (DDP), and gefitinib (GR), wherein the dimethylbiguanide (R) is administered at a concentration of 5% (w/w) (e.g., intratumorally), the gefitinib (GR) is administered at a concentration of 0.5% (w/w) (systemically, for example, intraperitoneally), and the cisplatin (DDP) is administered at a concentration of 0.15% (w/w) (e.g., intratumorally).

In some embodiments, in the above-mentioned method, the pharmaceutical combination or composition comprises dimethylbiguanide (R), ifosfamide (IFO), and gefitinib (GR), wherein the dimethylbiguanide (R) is administered at a concentration of 5% (w/w) (e.g., intratumorally), the ifosfamide (IFO) is administered at a concentration of 1% (w/w) (e.g., intratumorally), and the gefitinib (GR) is administered at a concentration of 0.5% (w/w) (systemically, for example, intraperitoneally).

In some embodiments, in the above-mentioned method, the pharmaceutical combination or composition comprises dimethylbiguanide (R), doxorubicin (DOX), and gefitinib (GR), wherein the dimethylbiguanide (R) is administered at a concentration of 5% (w/w) (e.g., intratumorally), the doxorubicin (DOX) is administered at a concentration of 0.05% (w/w) (e.g., intratumorally), and the gefitinib (GR) is administered at a concentration of 0.5% (w/w) (systemically, for example, intraperitoneally).

In some embodiments, in the above-mentioned method, the pharmaceutical combination or composition comprises dimethylbiguanide (R) and sodium bicarbonate (SB), wherein the dimethylbiguanide (R) can be administered at a concentration of 0.5% to 4%, and the sodium bicarbonate (SB) can be administered at a concentration of 0.5% to 15%, preferably 0.5% to 10%, and more preferably 1.5% to 9%. Preferably, the dimethylbiguanide (R) is administered at a concentration of 1% to 3%, and the sodium bicarbonate (SB) is administered at a concentration of 1% to 9%. More preferably, the dimethylbiguanide (R) is administered at a concentration of 3%, and the sodium bicarbonate (SB) is administered at a concentration of 3% to 9%. Optionally, the pharmaceutical combination also includes ifosfamide (IFO) at a concentration of 0.5%, 5-fluorouracil (5-FU) at a concentration of 0.4%, or osimertinib at a concentration of 0.3%. Preferably, the above-mentioned pharmaceutical combination comprising dimethylbiguanide (R) and sodium bicarbonate (SB) is administered intratumorally.

In some embodiments, in the above-mentioned method, the pharmaceutical combination or composition comprises dimethylbiguanide (R) and methylene blue dye (D) such as methylene blue (MB). In one embodiment, the pharmaceutical combination comprises dimethylbiguanide (R) and methylene blue (MB), wherein the dimethylbiguanide (R) can be administered at a concentration of 1% to 50%, and the MB can be administered at a concentration of 0.3% to 5%, preferably 0.5% to 5%; more preferably, the dimethylbiguanide (R) is administered at a concentration of 5% to 50%, and the MB is administered at a concentration of 0.5% to 5%. Preferably, dimethylbiguanide (R) is administered at a concentration of 5% to 50%, and methylene blue (MB) is administered at a concentration of 0.5% to 1%. Optionally, the pharmaceutical combination may also include one or more of the following at the indicated concentrations: 0.5% to 1% ifosfamide (IFO), 0.4% to 1% 5-fluorouracil (5-FU), and 0.3% osimertinib. Preferably, the pharmaceutical combination containing dimethylbiguanide (R) and methylene blue (MB) can be administered intratumorally and/or systemically. In some embodiments, the pharmaceutical combination includes dimethylbiguanide (R), MB, and any one of the following: IFO, 5-FU, and osimertinib, where dimethylbiguanide (R) is administered at a concentration of 5%, MB is administered at a concentration of 1%, IFO is administered at a concentration of 0.5%, 5-FU is administered at a concentration of 0.4%, and osimertinib is administered at a concentration of 0.3%. In some embodiments, in the above methods, the pharmaceutical combination includes dimethylbiguanide (R), SB, MB, and any one of the following: IFO, 5-FU, and osimertinib, where dimethylbiguanide (R) is administered at a concentration of 5%, SB is administered at a concentration of 5% to 10%, MB is administered at a concentration of 0.5%, IFO is administered at a concentration of 0.5%, 5-FU is administered at a concentration of 0.4%, and osimertinib is administered at a concentration of 0.3%.

In some embodiments, in the above methods, the pharmaceutical combination includes dimethylbiguanide (R) and an immunomodulator (E), where the immunomodulator includes or is selected from biological macromolecule immunomodulators (abbreviated as E1), immune cell immunomodulators (abbreviated as E2), and vaccine immunomodulators (abbreviated as E3). In some embodiments, the biological macromolecule immunomodulators can be, for example, immune checkpoint inhibitors, cytokines, and TLR agonists. In one embodiment, the immune checkpoint inhibitors can be Anti-PD-1 (CD279), the cytokines include interleukin-15, and the TLR agonists include CpG ODN 1826 (abbreviated as CpG), and the vaccine immunomodulators can be BCG.

In some embodiments, in the above methods, the pharmaceutical combination includes dimethylbiguanide (R) and CpG ODN 1826 (CpG), where the dimethylbiguanide (R) can be administered at a concentration of 1% to 50%, and the CpG can be administered at a concentration of 0.1%; preferably, the dimethylbiguanide (R) is administered at a concentration of 2.5% to 50%, and the CpG is administered at a concentration of 0.1%. Preferably, the pharmaceutical combination or composition further includes MB at a concentration of 1%, IFO at a concentration of 0.5%, and/or SB at a concentration of 10%. In some embodiments, the pharmaceutical combination or composition incudes dimethylbiguanide (R) and CpG and any one or two of the following: IFO, MB, and SB, where the dimethylbiguanide (R) is administered at a concentration of 2.5% to 50%, the IFO is administered at a concentration of 0.5%, the CpG is administered at a concentration of 0.1%, the MB is administered at a concentration of 1%, and the SB is administered at a concentration of 10%.

In some embodiments, in the above methods, the pharmaceutical combination or composition includes dimethylbiguanide (R) and the immune checkpoint inhibitor Anti-PD-1 (CD279), such as RMP1-14, where the dimethylbiguanide (R) can be administered at a concentration of 0.2% to 50%, and the RMP1-14 is administered at a concentration of 0.1%; preferably, the dimethylbiguanide (R) is administered at a concentration of 1% to 50%, and the RMP1-14 is administered at a concentration of 0.1%.

In some embodiments, in the above-mentioned methods, the pharmaceutical combination or composition comprises dimethylbiguanide (R) and BCG, wherein the dimethylbiguanide (R) can be administered at a concentration of 1% to 50%, preferably 2.5% to 50%, and more preferably 5% to 50%, and the BCG can be administered at a dose of 1×10⁶ CFU; preferably, the dimethylbiguanide (R) is administered at a concentration of 50%, and the BCG is administered at a dose of 1×10⁶ CFU. Optionally, the pharmaceutical combination or composition further includes any one of ifosfamide (IFO) at a concentration of 0.5% and SB at a concentration of 10%. In some embodiments, the pharmaceutical combination or composition comprises dimethylbiguanide (R) and BCG and any one of IFO and SB, wherein the dimethylbiguanide (R) is administered at a concentration of 5% to 50%, the BCG is administered at a dose of 1×10⁶ CFU, the IFO is administered at a concentration of 0.5%, and the SB is administered at a concentration of 10%.

In some embodiments, in the above-mentioned methods, the pharmaceutical combination or composition comprises dimethylbiguanide (R) and an immunomodulator (E), the immunomodulator includes or is selected from BCG, CpG ODN 1826 (CpG), and the immune checkpoint inhibitors is, such as anti-PD-1.

In some embodiments, in the above-mentioned methods, the pharmaceutical combination or composition comprises dimethylbiguanide (R) and BCG, wherein the dimethylbiguanide (R) can be administered at a concentration of 1% to 50%, preferably 2.5% to 50%, and more preferably 5% to 50%, and the BCG can be administered at a dose of 1×10⁶ CFU; preferably, the dimethylbiguanide (R) is administered at a concentration of 50%, and the BCG is administered at a dose of 1×10⁶ CFU. Optionally, the pharmaceutical combination or composition further includes any one of ifosfamide (IFO) at a concentration of 0.5%, MB at a concentration of 1%, or SB at a concentration of 10%. In some embodiments, the pharmaceutical combination or composition comprises dimethylbiguanide (R) and BCG and any one of IFO, MB, and SB, wherein the dimethylbiguanide (R) is administered at a concentration of 50%, the BCG is administered at a dose of 1×10⁶ CFU, the MB is administered at a concentration of 1%, the IFO is administered at a concentration of 0.5%, and the SB is administered at a concentration of 10%.

In some embodiments, in the above-mentioned methods, the pharmaceutical combination or composition comprises dimethylbiguanide (R) and CpG ODN 1826 (CpG), wherein the dimethylbiguanide (R) can be administered at a concentration of 1% to 50%, preferably 2.5% to 50%, and the CpG can be administered at a concentration of 0.1%; preferably, the dimethylbiguanide (R) is administered at a concentration of 2.5% to 50%, and the CpG is administered at a concentration of 0.1%. Preferably, the pharmaceutical combination or composition also includes either MB at a concentration of 1% or SB at a concentration of 10%. In some embodiments, the pharmaceutical combination or composition comprises dimethylbiguanide (R) and CpG and any one of MB and SB, wherein the dimethylbiguanide (R) is administered at a concentration of 50%, the CpG is administered at a concentration of 0.1%, the MB is administered at a concentration of 1%, and the SB is administered at a concentration of 10%.

Preferably, in the above-mentioned methods, the pharmaceutical combination or composition comprising dimethylbiguanide (R) and an immunomodulator such as BCG, anti-PD-1, or CpG ODN 1826 (CpG) is administered intralesionally or intratumorally, or intralesionally or intratumorally and systemically, such as by intraperitoneal injection or intravenous injection or intralesionally or intratumoral administration.

In some preferred embodiments, in the above-mentioned method, the contact or administration or intervention may include micro multi-point contact or administration or intervention, preferably micro multi-point injection, wherein the micro amount can be a medicament administration volume of ≤ 5% or 10% of the target volume, for example, including an amount of 5 µL - 1000 µL, such as 5 µL - 500 µL, 5 µL - 250 µL, 5 µL - 200 µL, 5 µL - 100 µL, 5 µL - 75 µL, 5 µL - 50 µL, 5 µL - 25 µL, 5 µL - 20 µL, 10 µL - 200 µL, 10 µL - 100 µL, 10 µL - 75 µL, 20 µL - 200 µL, 20 µL - 100 µL, 20 µL - 75 µL, 30 µL - 200 µL, 30 µL - 100 µL, 40 µL - 200 µL, 40 µL - 100 µL, 50 µL - 200 µL, 50 µL - 100 µL, or 50 µL - 75 µL, and the like. Preferably, micro multi-point contact or administration, more preferably, micro multi-point injection.

In some embodiments, the above-mentioned treatment, intervention or therapy can last for at least one course. The duration of one course can be approximately 7 to 40 days, for example, about 9 to about 37 days, such as 15, 20, 25, 27, 28, 29, 30, 31, 32, 33, 34, or 35 days.

In one embodiment, in one course, the contact or administration of the formulation of the present invention can be low-frequency, the low-frequency is, for example, administration about 1-6 times per course, preferably 1-5 times per course, 1-4 times per course, 1-3 times per course, or 1-2 times per course.

In some embodiments, in the above method, the dimethylbiguanide (R) and component (Z) in the pharmaceutical combination can be administered simultaneously, sequentially or intermittently.

In some embodiments, in the above method, the pharmaceutical combination, formulation or composition can be administered locally.

In some embodiments, in the above method, both the dimethylbiguanide (R) and component (Z) in the pharmaceutical combination are administered intralesionally or intratumorally (combination relationship I); the R and Z are administered intralesionally or intratumorally and systemically, respectively (combination relationship II); or the R and a portion of Z are administered intralesionally or intratumorally, and the other portion of Z is administered systemically (combination relationship III).

In some embodiments, in the above method, the tumor is a solid tumor suitable for intratumoral administration, preferably administered locally.

In some embodiments, in the above method, the tumor is selected from tumors where the tissue barrier function is not conducive to the cellular action of Z, preferably selected from refractory tumors of Z, including chemotherapy drug-resistant tumors, tumors with unfavorable microenvironments, tumors after discontinuation of antineoplastic agents, tumors without effective chemotherapeutic drugs, and tumors in patients with contraindication to or unsuitability for conventional chemotherapeutic drugs.

In some embodiments, in the above method, the tumor is selected from heterogeneous tumors, where the heterogeneity includes at least one of tissue heterogeneity, spatial heterogeneity, and safety heterogeneity. The heterogeneous tumor is a tumor in which different tumor bodies of the same type or different regions of the same tumor body are set as different target areas for differential treatment based on the heterogeneity, which is beneficial for treatment.

In some embodiments, in the above method, the contact or administration includes multi-point contact or administration, preferably, micro multi-point contact or administration, more preferably, micro multi-point injection.

In some embodiments, the micro includes micro volume.

In some embodiments, in the above method, the formulation or pharmaceutical combination is suitable for reduction (Y) of lesion volume (X) by micro-volume administration or intervention, a ratio of administration volume to target volume (V administration/ Vtarget) is approximately 0.02-0.056, 0.02-0.10 or 0.02-0.34.

In some embodiments, in the above method, when the lesion is ≧ about 3.5 cm³ or the maximum size is ≧ about 2 cm, the multi-point is ≧ 5 points, for example, 10, 15, 20, 25, 30, 35 or 50 points.

In some embodiments, in the above method, the contact or administration is low-frequency, preferably, the low-frequency is 1-6 times per course, more preferably, 1-5 times per course, 1-4 times per course, 1-3 times per course, or 1-2 times per course.

In some embodiments, in the above method, preferably, the maximum of administration concentration cR of the R is ≧ 5%, ≧ 10%, ≧ 20%, or ≦ 30%; the number of administration points is > 3, 10, 15, 20, 25, 30, 35 or 100 points.

In some embodiments, in the above method, the dimethylbiguanide R and the component Z can be provided by one or more formulations, and wherein:
the R and Z are administered intralesionally or intratumorally;
the R and Z are administered intralesionally or intratumorally and systemically, respectively; or
the R and a portion of Z are administered intralesionally or intratumorally, and the other portion of Z is administered systemically.

In some embodiments, in the above methods, the R and Z act synergistically, and wherein,
- the synergy is manifested by providing the following effects when the administration amount of dimethylbiguanide is at least halved (QR ≤ 1/2QR') and the administration amount of Z is at least reduced by 20%, compared to the conventional co-administered combination (R'/Z combination):
   1). Improved medication use performance, including increased adaptability to tumor heterogeneity, as evidenced by at least a 100% increase in at least one pharmaceutical adjustability index, where the pharmaceutical adjustability refers to an ability of the pharmaceutical to provide differentiated administration parameters to different target areas while maintaining efficacy; and/or
   2). Enhanced pharmaceutical efficacy, as evidenced by at least a 40% increase in at least one pharmaceutical efficacy index.

In some embodiments, in the above methods, the synergy further includes: at least a 10% increase in at least one pharmaceutical adjustability index and/or at least a 10% increase in at least one pharmaceutical efficacy index, compared to R and B.

In some embodiments, in the above uses and methods, N,N-dimethylbiguanide (R) is used to provide an active form distinct from a conventional metformin drug (whose active ingredient is a salt of N,N-dimethylbiguanide (R')), by the following limitations:
1). Not mixed with an acid that significantly converts R to R';
2). Local intralesional administration;
3). The administration amount for one course of treatment is: ${Q}_{R} = {\sum }_{i = 1}^{nR} {q}_{R \left(i\right)} ,$
   in the formula, Q_{R} is < 50% or 25% of the equivalent usage amount of the said R', n_{R} is the number of times that R is administered to the lesion or tumor in one course of treatment, and q_{R(i)} is the amount of R in the i-th (i = 1,..., nR) administration, and: ${q}_{R \left(i\right)} = {\sum }_{i i - 1}^{n R i} {c}_{R \left(ii\right)} \times {v}_{R \left(ii\right)} ,$
   in the formula, n_{Ri} represents the number of administration points, for example, the number of needle insertion points of R during the i-th administration, c_{R(ii)} and v_{R(ii)} represent the concentration and volume of R administered at the ii-th administration point, for example, the needle insertion point, respectively. The concentration threshold of c_{R(ii)} (W/V) is 0.1% or 0.2%, and the upper limit of the concentration threshold is 51%. The volume threshold of v_{R(ii)} is 1/800 or 1/300 of the target volume (abbreviated as v_{target(ii)}), and the upper limit of the volume threshold is 3 times of v_{target(ii)}.

Here, the active form of R' is presented as being fully acid-complexed in solution; while the active form of R is presented as being less complexed with acid in solution, mainly existing in a form with minimal or partial complexation. Thus, through the transient local action of this distinct active form, an activity (abbreviated as activity A) that is difficult for R' to provide is offered, including at least one of the following: effectively reducing the function of the biological barrier (abbreviated as activity A1), effectively damaging the lesional tissue (abbreviated as activity A2), and/or effectively releasing lesional immune substances and/or inflammatory signals within the lesional tissue (abbreviated as activity A3).

In some embodiments, in the above uses and methods, the R and the component (Z) are co-administered, where the administration concentration (w/w) of R is 0.1% - 51%, preferably 0.2% - 50%. The component (Z) includes at least one selected from the following group: cell-reactive antineoplastic agents (abbreviated as B), near-neutral or weakly basic sodium salts (abbreviated as C), methylene blue (abbreviated as D), and immunomodulators (abbreviated as E), where:
- the cell-reactive antineoplastic agents (B) provide the cellular reaction (abbreviated as activity B) through the following limitations, one key control step of which is crossing the aforementioned biological barrier:
   1). Intralesional administration and/or systemic administration;
   2). The administration amount for one course of treatment is: ${Q}_{B} = {\sum }_{j - l}^{nB} {q}_{B \left(j\right)} + {\sum }_{i ′ - l}^{nB ′} {c}_{B \left(j′\right)} \times {v}_{B \left(j′\right)}$ ,

In the formula, Q_{B} is ≦ the conventional administration amount of B in a sensitive tumor, n_{B} and q_{B(j)} are the number of systemic administrations and the amount of the j-th (j = 1,..., n_{B}) administration of B in one course of treatment, respectively, n_{B'}, c_{B(i')}, and v_{B(i')} are the number of tumor administrations, the concentration and the volume of the j'-th (j' = 1,..., nB') administration of B in one course of treatment, respectively.
- the C is used to provide the effect of improving a local efficacy-toxicity ratio of R (referred to as activity C) through the following limitation:
   1). Mixed administration with R;
   2). A ratio of the mixed amount of C to R (Q_{C}/Q_{R}) is 0.5 to 4.0;
- the D is used to provide the effect of enhancing the pharmaceutical efficacy of R through the following limitations:
   1). Intralesional administration, including mixed administration with R or separate administration;
   2). The administration amount in one course of treatment Q_{E} is ≦ the effective administration amount for transient local action; the C is used to provide the effect of improving a local efficacy-toxicity ratio of R (referred to as activity C) through the following limitation:
      1). Mixed administration with R;
      2). A ratio of the mixed amount of C to R (Q_{C}/Q_{R}) is 0.5 to 4.0;
- the D is used to provide the effect of enhancing the pharmaceutical efficacy of R through the following limitations:
   1). Intralesional administration, including mixed administration with R or separate administration;
   2). The administration amount in one course of treatment Q_{E} is ≦ the effective administration amount for transient local action,
- E is used to provide the effect of immune regulation benefiting from the effect of activity A1 and/or optimizing local and/or systemic immune responses (referred to as activity E) by utilizing the effect of activity A3 through the following limitations:
   1). Intralesional administration and/or systemic administration;
   2). The administration amount in one course of treatment is: ${Q}_{E} = {\sum }_{k = 1}^{n E} {q}_{B} \left(k\right) + {\sum }_{k ′ = 1}^{n E ′} cE \left(k′\right) \times vE \left(k′\right) ,$ where Q_{E} is ≦ the conventional administration amount of E in the anti-lesion application, n_{E} and q_{E(k)} are the number of systemic administrations and the amountof the k-th (k = 1,..., nE) administration of E in one course of treatment, respectively, n_{E'}, c_{E(k')}, and v_{E(k')} are the number of tumor administrations, the concentration and the volume of the k'-th (k' = 1,..., n_{K'}) administration of E in one course of treatment, respectively.

In some embodiments, in the above uses and methods, the R, the cell-reactive antineoplastic agent (referred to as B), and the immunomodulator (referred to as E) are co-administered, where:
- The cell-reactive antineoplastic agent (B) provides the cell reaction (referred to as activity B), one key control step of which is crossing the above-mentioned biological barrier, through the following limitations:
   1). Intralesional administration and/or systemic administration;
   2). The administration amount in one course of treatment is: ${Q}_{B} = {\sum }_{j - l}^{nB} {q}_{B \left(j\right)} + {\sum }_{i ′ - l}^{nB ′} {c}_{B \left(j′\right)} \times {v}_{B \left(j′\right)} ,$ where Q_{B} is ≦ the conventional administration amountof B in a sensitive tumor, n_{B} and q_{B(j)} are the number of systemic administrations and the amount of the j-th (j = 1,..., n_{B}) administration of B in one course of treatment, respectively, n_{B'}, c_{B(i')}, and v_{B(i')} are the number of tumor administrations, the concentration and the volume of the j'-th (j' = 1,..., n_{B'}) administration of B in one course of treatment, respectively;
- The immunomodulator (E) is used to provide the effect of immune regulation benefiting from the effect of activity A1 and/or optimizing local and/or systemic immune responses (referred to as activity E) by utilizing the effect of activity A3, through the following limitations:
   1). Intralesional administration and/or systemic administration;
   2). The administration amount in one course of treatment is: ${Q}_{E} = {\sum }_{k = 1}^{n E} {q}_{B \left(k\right)} + {\sum }_{k ′ = 1}^{n E ′} cE \left(k′\right) \times vE \left(k′\right) ,$ in the formula, Q_{E} is ≦ the conventional administration amount of E in the anti-intralesional application, n_{E} and q_{E(k)} are the number of systemic administrations of E in one course of treatment and the amount at the k-th (j = 1,..., nk) administration, respectively, while n_{E'}, c_{E(k')}, and v_{E(k')} are the number of tumor administrations of E in one course of treatment, the concentration and volume at the k'-th (k' = 1,..., nk')(k' = 1,..., nE') administration, respectively.

In some embodiments, in the above-mentioned method, the pharmaceutical adjustability indexes include at least one of the following intratumoral administration adjustable coefficients:
1). Concentration coefficient = a ratio of the maximum value to the minimum value of cR(ii), and the coefficient is > 50, 100 or 150, which enables the combination to provide highly variable administration concentrations of R to different tumors of the same type and/or different regions of the same tumor while maintaining its activity A stable;
2). Volume ratio coefficient = a ratio of the maximum value to the minimum value of the volume ratio of vR(ii) to Vtarget(ii), and the coefficient is > 5, 10 or 100, which enables the combination to provide highly variable administration volume ratios of R to different tumors of the same type and/or different regions of the same tumor while maintaining its activity A stable; and
3). The administration point density coefficient = a ratio of the maximum value to the minimum value of nRi within the vtarget (ii), and the coefficient is >5, 10 or 100, which enables the combination to provide highly variable needle point density of R to different tumors of the same type and/or different regions of the same tumor while maintaining its activity A stable.

In some embodiments, in the above method, the pharmaceutical efficacy indexes include at least one of the following:
1). Timeliness indicator, manifested as an onset time in mouse experiments (the day when the tumor proliferation rate is <42%), and the onset time is reduced by at least 25% compared to the R' scheme, R'/Z co-adminstration scheme, and single agent Z scheme, which makes the combination a fast-acting or further fast-acting combination, especially suitable for tumors that require rapid reduction in tumor size;
2). Effectiveness indicator, manifested as a tumor inhibition rate or objective response rate in the mouse experiments, and the tumor inhibition rate or objective response rate is increased by at least 25% compared to the R' scheme, R'/Z co-adminstration scheme, and single agent Z scheme, which makes the combination a highly effective or further highly effective combination, especially suitable for tumors that require maximum inhibition of tumor size;
3). Long-term efficacy indicator, manifested as the progression-free time in mouse experiments, and the progression-free time is prolonged by at least 25% compared to the R' scheme, R'/Z co-adminstration scheme, and single agent Z scheme, which makes the combination a long-acting or further long-acting combination, especially suitable for tumors that require maximum prolongation of progression-free time.

**According to the twelfth aspect of the present invention**, provided is a kit comprising the above-mentioned formulation, pharmaceutical combination or composition of the present invention, and instructions for carrying out the above-mentioned method of the present invention.

In some embodiments, the kit further comprises a micro-volume intervention device.

In some embodiments, in the above-mentioned kit, the micro-volume intervention device is suitable for micro-volume multi-point contact or administration, and preferably includes a micro-volume injection device.

**According to the thirteenth aspect of the present invention,** a method for preparing a formulation is provided, including: selecting N,N-dimethylbiguanide with a content of dimethylbiguanide salt <30%, ≤25%, preferably ≤10%, and more preferably ≤5% as the raw material; excluding and avoiding contact with acidic pH adjusting agents that can make the N,N-dimethylbiguanide meet the requirements of cell culture or the safety of conventional injection in the formula and during preparation; using a strong alkali-resistant preparation system; and dispersing the N,N-dimethylbiguanide in a solvent.

In some embodiments, the solvent is water.

In some embodiments, the N,N-dimethylbiguanide is dissolved in water.

**According to the fourteenth aspect of the present invention**, a use of N,N-dimethylbiguanide as a transient active ingredient in the preparation of a formulation, medicament, pharmaceutical combination or kit for treating tumors or nodules is provided, wherein the N,N-dimethylbiguanide is used to provide a transient effect on a lesion tissue and the cells contained therein.

In this application, the term "transient activity" or "transient effect" refers to an activity or effect provided by the active form of R within a transient period before it is converted to the active form R' in the body.

In one embodiment, N,N-dimethylbiguanide is an organic compound with the chemical formula C₄H₁₁N₅, which has the formula 1 as described in the first aspect above.

**According to the fifteenth aspect of the present invention**, a method for treating cells within a lesion in a subject is provided, including administering to the lesion the above-mentioned N,N-dimethylbiguanide or the formulation of the present invention, penetrating into the lesion, and contacting the cells within the lesion, wherein the N,N-dimethylbiguanide is used as a transient active ingredient to provide a transient effect on the lesion tissue and the cells contained therein, and the transient effect is, for example, a transient tissue penetration effect.

**According to the sixteenth aspect of the present invention**, a method for treating a lesion in a subject is provided, including: administering to the lesion the above-mentioned N,N-dimethylbiguanide or the formulation of the present invention, penetrating into the lesion, and contacting the components of the lesion, or administering to the lesion tissue the formulation of the present invention. Preferably, the administering or contacting can be systemic or local, such as by injection into the tumor or tumor microenvironment.

**According to the seventeenth aspect of the present invention**, a method for treating a lesion such as a tumor or nodule in a subject is provided, including introducing or administering to the subject the above-mentioned pharmaceutical combination, the composition or the formulation of the present invention.

In some embodiments, the interveningor administeringcan be systemic or local, preferably, the administeringor intervening may include intratumoral administration or intervention, preferably local intratumoral intervention or administration, preferably such as local injection administration or intervention into the tumor or tumor microenvironment.

In some embodiments, the injection administration may include infusion, liquid particle injection or implantation.

In some preferred embodiments, the above-mentioned formulation of the present invention is suitable for micro-volume administration or intervention administration, and a ratio of the administration volume to the target volume (V_{administration}/V_{target}) is approximately 0.02-0.05, 0.02-0.10 or 0.02-0.34.

In some embodiments, the above-mentioned contact or intervention or administration includes multi-point contact or intervention or administration, preferably, local multi-point contact or intervention.

The above-mentioned contact or administration may include multi-point contact or administration or intervention, where the multi-point refers to the number of micro-volume intervention sites where the efficacy of the total target area is observed to be significantly higher than that of conventional metformin drug. Specifically, the multi-point, for example, may be multiple sites at the target area, such as ≥5, ≥10, ≥20, ≥30, ≥50 or more sites, for example, at least 6, at least 7, at least 8, at least 9, at least 10, at least 11, at least 12, at least 13, at least 14, at least 15 or more sites, for example, 10 to 20, 10-30, 10-40, 10-50, 20-30, 20-40, 20-50, 25-50, 30-50 or more sites. Preferably, the contact points or administration sites or intervention points are uniformly distributed, especially to areas where conventional intervention cannot diffuse.

In some preferred embodiments, when the lesion is ≥ about 3.5 cm³ or the maximum size is ≥ about 2 cm, the multi-point is ≥ 5 sites, for example, 10, 15, 20, 25, 30, 35 or 50 sites.

In some preferred embodiments, the above-mentioned contact or administration or intervention may include micro-volume multi-point contact or administration or intervention, preferably, micro-volume multi-point injection, where the micro-volume is ≤ 5% or 10% of the pharmaceutical administration volume of the target volume, for example, including a quantity of 5 µL - 1000 µL, for example, 5 µL - 500 µL, 5 µL - 250 µL, 5 µL - 200 µL, 5 µL - 100 µL, 5 µL - 75 µL, 5 µL - 50 µL, 5 µL - 25 µL, 5 µL - 20 µL, 10 µL - 200 µL, 10 µL - 100 µL, 10 µL - 75 µL, 20 µL - 200 µL, 20 µL - 100 µL, 20 µL - 75 µL, 30 µL - 200 µL, 30 µL - 100 µL, 40 µL - 200 µL, 40 µL - 100 µL, 50 µL - 200 µL, 50 µL - 100 µL, or 50 µL - 75 µL, and the like. Preferably, micro-volume multi-point contact or administration, more preferably, micro-volume multi-point injection.

In some embodiments, the above-mentioned treatment can last for at least one course of treatment. The duration of one course of treatment can be about 7 to 40 days, for example, about 9 to about 37 days, for example, about 15, 20, 25, 27, 28, 29, 30, 31, 32, 33, 34, or 35 days.

In one embodiment, in one course of treatment, the contact or administration of the formulation of the present invention can be low-frequency, the low-frequency, for example, is administered about 1-6 times per course, preferably, 1-5 times per course, 1-4 times per course, 1-3 times per course, or 1-2 times per course.

**According to the eighteenth aspect of the present invention**, a kit is provided, comprising the formulation, pharmaceutical combination or composition as described above in the present invention, and instructions for carrying out the method of treatment or therapeutic method as described above in the present invention.

In one embodiment, the kit of the present invention further comprises a micro-volume intervention device, preferably, the device is suitable for micro-volume multi-point contact or administration, and preferably includes a micro-volume injection device such as a syringe.

In one embodiment, the volume intervention device may include, for example, a puncture needle, an injection needle, a catheter, or other devices with the same function.

In one embodiment, in the above uses, the above methods, and the above kits of the present invention, the quantity of one course of treatment (Q_{R}) of R in the formulation of the present invention is ≤ 20% or 50% of the quantity of one course of treatment (Q_{R'}) of the conventional metformin drug (R'), where R is a transient active ingredient beyond the expectation of "R and R' are pharmaceutically equivalent", characterized by a significant reduction in dosage but a significant increase in pharmaceutical effect, including at least one of the pharmaceutical adjustability indexes increased by at least 100% and at least one of the pharmaceutical efficacy indexes enhanced by at least 40%.

In this application, the term "transient activity" or "transient effect" refers to a local activity or local effect provided by the active form of R within a transient period before it is converted to the active form of R' in the body.

In one embodiment, in the above uses, the above methods, and the above kits of the present invention, the transient dosage (Q_{R} and Q_{Z}) of the combination of R and Z (abbreviated as R/Z) in the formulation of the present invention is significantly less than the dosage (Q_{R'} and Q_{Z'}) of the combination of conventional metformin drug (R') and Z (abbreviated as R'/Z): QR ≤ 25% or 50% Q_{R'}, and Q_{Z} ≤ Q_{Z'}, where R/Z is a synergistic combination beyond the expectation of "R/Z and R'/Z are pharmaceutically equivalent combinations", characterized by a significant reduction in dosage but a significant increase in pharmaceutical effect, including at least one of the pharmaceutical adjustability indexes increased by at least 100% and at least one of the pharmaceutical efficacy indexes enhanced by at least 25%, where the pharmaceutical adjustability refers to the performance of the medicament to provide differentiated dosing parameters to different target areas while still maintaining efficacy.

In one embodiment, in the above uses, the above methods, and the above kits of the present invention, at least one of the dosages (Q_{R} and Q_{Z}) of the combination of R and Z (abbreviated as R/Z) in the formulation of the present invention is lower than the dosages (Q_{R0} and Q_{Z0}) of R and Z as single agents: Q_{R} ≤ Q_{R0} and Q_{Z} ≤ 80% Q_{B0}, where R/Z is a synergistic combination beyond the expectation of the HSA method or Bliss method based on the effects of R and Z as single agents, characterized by a reduction in dosage but an increase in pharmaceutical effect, including at least one of the pharmaceutical adjustability indexes increased by at least 10% and at least one of the pharmaceutical efficacy indexes enhanced by at least 10%.

In one embodiment, in the above uses, the above methods, and the above kits of the present invention, the intervention or administration concentration (C, w/w) of N,N-dimethylbiguanide in the formulation of the present invention is 1.0% ≤ C ≤ 30%, for example, 1.0% ≤ C ≤ 20%, preferably 3.0% ≤ C ≤ 30%, preferably 3.0% ≤ C ≤ 20%, more preferably 5.0% ≤ C ≤ 30%, more preferably 5.0% ≤ C ≤ 20%, and particularly preferably 6.0% ≤ C ≤ 20%.

In one embodiment, in the above uses, methods, and kits of the present invention, the intervention or administration concentration (C, w/w) of N,N-dimethylbiguanide in the formulation of the present invention is 20% ≤ C ≤ 50%, preferably 25% ≤ C ≤ 50%, preferably 30% ≤ C ≤ 50%, more preferably 40% ≤ C ≤ 50%, and particularly preferably 45% ≤ C ≤ 50%.

In the present invention, its indication (Y, X) is suitable for application in treating (Y) lesion diseases or lesion symptoms (X), where the X is the volume of the lesion and the Y is effective or rapid reduction, and the lesion includes malignant and non-malignant lesions.

In the present invention, where X is the volume of the lesion and Y is effective or rapid reduction. In the present invention, the lesion is a lesion that is ineffective or has failed with conventional treatment, including at least one selected from the following group: a tumor that is ineffective or has failed with conventional treatment; a malignant tumor that is refractory to conventional chemotherapeutic drugs, a non-malignant nodule, preferably the lesion is a malignant tumor that is refractory to conventional chemotherapeutic drugs.

In the present invention, the refractory malignant tumor includes or is selected from at least one of the following groups: a tumor that is resistant to chemotherapeutic drugs, a tumor with an unfavorable microenvironment, a tumor after medication withdrawal, a tumor for which there is no effective chemotherapeutic drug, a tumor in a patient with contraindications to or unsuitability for conventional chemotherapeutic drugs.

The non-malignant nodule is a nodule including but not limit to at least one of the following non-malignant cells: connective tissue cells, secretory gland cells, and epithelial cells. In one embodiment, the nodule includes or is selected from, for example: organ nodules rich in organ cells (such as breast nodules, thyroid nodules, lung adenoid nodules), non-malignant tumors rich in connective tissue cells (such as lipomas, fibromas, etc.), connective tissue lesions rich in connective tissue cells (such as rheumatoid arthritis, systemic lupus erythematosus, polymyositis), and other nodules, preferably, breast hyperplasia nodules.

In the present invention, the lesion includes a tumor with non-resistant malignant tumor cells.

In the present invention, the malignant tumor cells are tumor cells that are not specific to the cytotoxicity of dimethylbiguanide.

In the present invention, the chemotherapy drug-resistant tumor includes a tumor that is resistant to conventional chemotherapeutic drugs, including but not limited to, for example, paclitaxel, 5-fluorouracil, cyclophosphamide, doxorubicin, and/or cisplatin, or a tumor that is resistant to targeted antineoplastic agents such as gefitinib, osimertinib, and/or trastuzumab, for example, including liver cancer tumors, breast cancer tumors, sarcoma tumors, pancreatic cancer, lung cancer, gastric cancer, and colon cancer tumors comprising an unfavorable microenvironment. In one embodiment, the refractory malignant tumor includes or is selected from liver cancer tumors, breast cancer tumors, sarcoma tumors, pancreatic cancer, lung cancer, gastric cancer, and colon cancer tumors that are resistant to chemotherapeutic drugs. Preferably, the chemotherapy drug-resistant tumor is 5-fluorouracil-resistant liver cancer.

In the above uses and methods of the present invention, the lesion includes any tumor at any stage due to any pathology (malignant and/or non-malignant), including, for example, the following groups classified by tumor cell type: epithelial cell tumors, sarcomas, lymphoid tumors, germ cell tumors, embryonic cell tumors; and tumors named according to the organ or tissue where the tumor cells are concentrated, including, for example, tumors named according to the following organs or tissues: skin, bone, muscle, breast, kidney, liver, lung, gallbladder, pancreas, brain, esophagus, bladder, large intestine, small intestine, spleen, stomach, prostate, testis, ovary, or uterus.

Specifically, the malignant tumors include, for example, breast cancer, pancreatic cancer, thyroid cancer, nasopharyngeal cancer, prostate cancer, liver cancer, lung cancer, intestinal cancer, oral cancer, esophageal cancer, gastric cancer, laryngeal cancer, testicular cancer, vaginal cancer, uterine cancer, ovarian cancer, sarcoma, and the like.

Furthermore, the aforementioned malignant tumors include refractory tumors, such as those that progress after chemotherapy. The tumors that progress after chemotherapy include those that progress after treatment with chemotherapeutic drugs such as paclitaxel, 5-fluorouracil, cyclophosphamide, doxorubicin, and cisplatin, or those that progress after treatment with targeted antineoplastic agents such as gefitinib, osimertinib, trastuzumab and derivatives thereof.

The non-malignant tumors include, for example, breast tumors, pancreatic tumors, thyroid tumors, prostate tumors, liver tumors, lung tumors, intestinal tumors, oral tumors, esophageal tumors, gastric tumors, nasopharyngeal tumors, laryngeal tumors, testicular tumors, vaginal tumors, uterine tumors, fallopian tube tumors, ovarian tumors, and the like.

In the above uses and methods of the present invention, according to one embodiment, the malignant cells include or are selected from conventional malignant cells that are ineffective with conventional dimethylbiguanide preparations alone, such as cancer cells and sarcoma cells other than liver cancer cells.

In the above uses and methods of the present invention, according to one embodiment, the non-malignant cells include or are selected from at least one of connective tissue cells and organ tissue cells.

In the above uses and methods of the present invention, according to one embodiment, the connective tissue cells include fibroblasts.

In the above uses and methods of the present invention, according to one embodiment, the organ tissue cells include or are selected from at least one of the following: liver cells, breast cells, thyroid cells, and skin cells.

In the above uses and methods of the present invention, the non-malignant lesions include or are selected from at least one of the following groups: benign tumors and nodules.

In the above uses and methods of the present invention, the non-malignant nodules include benign lesions other than benign tumors, including, for example, hyperplasia (such as hyperplasia of the breast, thyroid, parathyroid, prostate, and the like), cysts, abnormal venous masses (such as hemorrhoids, and the like), local inflammatory swelling, microbial infection swelling, and the like. The hemorrhoids include internal hemorrhoids, external hemorrhoids, and mixed hemorrhoids.

In one embodiment, the benign lesions include local inflammation, especially refractory inflammation. The refractory inflammation includes, but is not limited to, for example, alterative inflammation, exudative inflammation, and proliferative inflammation, which can be any suitable one known to those skilled in the art, such as including one or more of the following: arthritis, mastitis, pancreatitis, thyroiditis, prostatitis, hepatitis, pneumonia, enteritis, stomatitis, pharyngitis, periodontitis, esophagitis, gastritis, gastric ulcers, rhinitis, sinusitis, laryngitis, tracheitis, bronchitis, vaginitis, endometritis, salpingitis, oophoritis, and the like.

In one embodiment, the benign lesions include skin diseases, especially refractory skin diseases. The refractory skin diseases include, but are not limited to, for example, including one or more of the following: skin cancer, non-malignant skin tumors, viral skin diseases (such as herpes, warts, rubella, hand-foot-mouth disease), bacterial skin diseases (such as impetigo, furuncles, leprosy), fungal skin diseases (such as various tinea), sexually transmitted diseases (such as syphilis, gonorrhea, and condyloma acuminatum), allergic and autoimmune skin diseases (such as contact dermatitis, eczema, urticaria), physical skin diseases (such as solar dermatitis, chilblains, corns, chapped hands and feet, pressure sores), connective tissue diseases (such as lupus erythematosus), pigmentary skin disease (such as freckles, pigmented nevi, various spots), skin appendage diseases (such as acne, rosacea, seborrheic dermatitis, alopecia areata, alopecia, hyperhidrosis and bromhidrosis), and hyperkeratosis, such as spontaneous hyperkeratosis.

In the above uses and methods of the present invention, the subject includes mammals. Preferably, the subject includes humans. More preferably, the subject includes non-human mammals, such as non-human primates, domestic animals and pets, and the like. The domestic animals include, but are not limited to, for example, camels, pigs, cattle, sheep, horses, donkeys, goats, and sheep, etc; the pets include various mammalian pets such as dogs, cats, rabbits, monkeys, and chimpanzees, and the like.

In some embodiments, in the above formulations, pharmaceutical combinations, compositions, uses, methods, or kits, the intervention or administration concentration (C or C_{R}, w/w) of N,N-dimethylbiguanide is 1.0% ≤ C ≤ 20%, preferably 3.0% ≤ C ≤ 20%, more preferably 5.0% ≤ C ≤ 20%, and particularly preferably 6.0% ≤ C ≤ 20%.

In some embodiments, in the above-mentioned formulations, pharmaceutical combinations, compositions, uses, methods, or kits, the intervention or administration concentration (C or C_{R}, w/w) of N,N-dimethylbiguanide is 20% ≤ C ≤ 50%, preferably 30% ≤ C ≤ 50%, more preferably 40% ≤ C ≤ 50%, and particularly preferably 45% ≤ C ≤ 50%.

In some embodiments, in the above-mentioned formulations, pharmaceutical combinations, compositions, uses, methods, or kits, the tumor is a malignant tumor that is refractory to conventional chemotherapeutic drugs. Preferably, the refractory malignant tumor includes or is selected from at least one of the following groups: chemotherapy drug-resistant tumors, tumors with unfavorable microenvironments, tumors after drug withdrawal, tumors for which there is no effective chemotherapeutic drug, and tumors in patients with contraindication to or unsuitability for conventional chemotherapeutic drugs.

In some embodiments, in the above-mentioned formulations, pharmaceutical combinations, compositions, uses, methods, or kits, the nodules are nodules including at least one of the following non-malignant cells: non-malignant nodules of connective tissue cells, secretory gland cells, and epithelial cells.

In some embodiments, in the above-mentioned formulations, pharmaceutical combinations, compositions, uses, methods, or kits, the lesion includes a tumor containing non-drug-resistant malignant tumor cells.

### Brief descrption of the drawings

Figure 1A-C: HE staining pathological analysis (100×) of tumor samples from nude mice bearing human liver cancer 5-Fu-resistant cells (Bel-7409/5-Fu) after 1-hour administration of dimethylbiguanide hydrochloride, N,N-dimethylbiguanide, and 5-Fu, respectively, in Example 1.1 (X, Y, Z). X: administration of 1.3% dimethylbiguanide hydrochloride; Y: administration of 0.5% N,N-dimethylbiguanide; Z: administration of 0.25% 5-Fu (w/w).
Figure 2 A-C: Enlarged views of the HE staining images (400×) in Figure 1A-C.
Figure 3: Tumor samples from nude mice bearing human liver cancer 5-Fu-resistant cells (Bel-7409/5-Fu) after 1-hour administration of dimethylbiguanide hydrochloride, N,N-dimethylbiguanide, and 5-Fu, respectively, in Example 1.1 (A-G).
Figure 4: Therapeutic effect on beagle dogs with spontaneous cortical hyperplasia in Example 4.2 (A: lesion before treatment, B: lesion after treatment).
Figure 5: Therapeutic effect on rats with spontaneous fibroma in Example 4.2 (A: lesion before treatment, B: lesion after treatment).

### Detailed description

### Definitions

In this application, the term "activity" refers to the ability of a compound to exert a specific biological or pharmacological effect. The term "active form" refers to the specific form of a compound that provides a particular activity, such as the specific form that binds to a specific target and triggers a biological effect. The term "active ingredient" refers to the chemical entity in a medicament or formulation that directly provides a specific active form. The original state of a compound may not necessarily be active directly; for example, salicylic acid, which is produced by the hydrolysis of aspirin, is its active form, and sulfonamides need to be dissociated into the free form to exert their antibacterial effect.

In this application, the term "conventional dimethylbiguanide" (denoted as R' in this application) refers to metformin used in prior art. It is defined based on its main characteristics (can be administered systemically and can provide the desired pharmacological effect through systemic administration), and can only be a dimethylbiguanide salt. For instance, in the catalog of major suppliers like Sigma, the only compound under the metformin item is dimethylbiguanide hydrochloride. Any metformin sourced from Sigma, or any metformin that can be used in cell experiments, or can be administered systemically (including intraperitoneal administration), or has the same pharmaceutical mechanism as systemic administration for local administration, can only be a dimethylbiguanide salt. In this application, the most commonly used "dimethylbiguanide (metformin)", that is, dimethylbiguanide hydrochloride, is taken as the representative of conventional metformin drugs. In prior art, dimethylbiguanide salts and dimethylbiguanide (metformin) are often used interchangeably.

In this application, the term "the application N,N-dimethylbiguanide" (denoted as R in this application and can be used interchangeably with dimethylbiguanide compounds) is a distinct compound from conventional dimethylbiguanide (dimethylbiguanide salts). According to the International Union of Pure and Applied Chemistry (IUPAC), the dimethylbiguanide compound called N,N-dimethylbiguanide has the chemical formula C4H11N5, simplified structural formula as H3CNCNH3CNCH3, with a molecular weight of 129.2 (see formula 1 below). Dimethylbiguanide salts, on the other hand, are salts formed by N,N-dimethylbiguanide and different acids, with the chemical formula C4H11N5·acid, simplified structural formula as H3CNCNH3CNCH3·acid, and the specific IUPAC name depending on the acid contained. For example, the salt formed by N,N-dimethylbiguanide and hydrochloric acid has the chemical formula C4H11N5·HCl, simplified structural formula as H3CNCNH3CNCH3·HCl, and is named N,N-dimethylbiguanide hydrochloride by IUPAC, with a molecular weight of 165.6 (see formula 2 below).

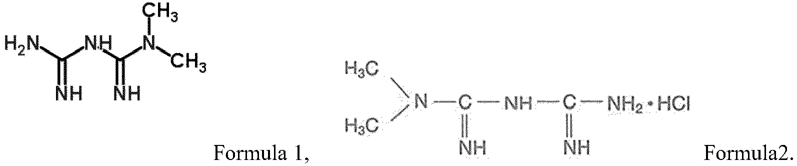

Different IUPAC standard nomenclatures correspond to distinct chemical structures. In N,N-dimethylbiguanide, "N,N-dimethyl" indicates that there are two methyl groups (CH₃-) attached to two nitrogen atoms respectively, and "biguanide" refers to a derivative of guanidine where the guanidine group is free. In contrast, in N,N-dimethylbiguanide hydrochloride, the guanidine group is bound to a chloride ion, forming an ion pair of a positively charged N,N-dimethylbiguanide ion and the corresponding chloride ion. The same applies to other N,N-dimethylbiguanide salts. This structural difference is also reflected in their distinct preparation methods. The preparation of N,N-dimethylbiguanide hydrochloride is relatively inexpensive, and N,N-dimethylbiguanide is usually prepared from N,N-dimethylbiguanide hydrochloride as the raw material.

In fact, when N,N-dimethylbiguanide enters body fluids such as blood, it can form dimethylbiguanide salts with the acid radicals therein. For instance, the guanidine group in N,N-dimethylbiguanide combines with a chloride ion (Cl⁻) to form an ion pair, resulting in N,N-dimethylbiguanide hydrochloride. In the expectation of the prior art, the above-mentioned structural differences in vitro do not produce significant differences in biological activity, and R and R' are regarded as pharmaceutical equivalents.

However, the results in the examples of this application unexpectedly show that this structural difference generates distinct biological activities under specific conditions, that is, distinct structure-activity relationships. Based on these results, it can be more comprehensively evaluated whether N,N-dimethylbiguanide and N,N-dimethylbiguanide salts are different active ingredients. If they are both administered systemically into the body and dispersed in the blood, have the same form, then exert the same effects, they are regarded as pharmaceutical equivalents and considered the same active ingredient. But if they are administered locally into the body and have different active forms in specific solutions (such as aqueous solutions), and the different forms can provide different core activities and exert different effects, then they are not pharmaceutical equivalents and need to be defined active ingredients, respectively, according to their different active forms.

In fact, under the conditions of this application, N,N-dimethylbiguanide (R) has a form of manifestation distinct from that of conventional metformin drugs (whose active ingredient is R'), where the active form of R' is shown as being a form completely complexed with acid in the solution (as demonstrated in Example 5 for systemic administration); conversely, the active form of R is shown as being less complexed with acid in the solution, mainly existing in a form of minimal or partial complexation (as demonstrated in Example 5 for local administration only, not for systemic administration), thereby providing an activity (activity A) that R' cannot provide, activity A includes transient local effects.

In the present application, the term "cell-reactive drug" (denoted as B in the present application) refers to a class of antineoplastic agents that act on tumor cells, mainly achieving antineoplastic effects by regulating the signaling pathways of growth, division or death of tumor cells and interfering with the key metabolic and biological processes of tumor cells. This class of drugs includes cytotoxic drugs and targeted antineoplastic agents. The term "cytotoxic drug" (denoted as B1 in this application) refers to a class of antineoplastic agents that kill tumor cells through non-specific mechanisms (directly damaging DNA, inhibiting RNA synthesis or interfering with protein polymerization). The term "targeted antineoplastic drug" (denoted as B2 in this application) refers to a class of medications that regulate cell signaling pathways by binding to specific targets on tumor cells (such as specific gene mutation products or abnormally expressed receptor proteins), thereby selectively inhibiting tumor cell growth or inducing their death. Cytotoxic drugs and targeted antineoplastic agents are generally regarded as quite different types of medicaments, but they share the following common features: they inhibit tumor progression by interfering with key signaling pathways in tumor cells (such as proliferation, metabolism, apoptosis or DNA repair), and thus face common problems.

In the present application, the term "near-neutral or weakly basic sodium salt" (abbreviated as C) refers to sodium salt compounds that can be used for injection and whose aqueous solutions exhibit near-neutral pH (5.0-8.5) within a certain concentration range. Such sodium salts include, but are not limited to, the following: sodium chloride, sodium bicarbonate, sodium dihydrogen phosphate, sodium lactate, sodium acetate, etc.

In this application, the term "immunomodulator" refers to substances that can optimize local and/or systemic immune responses by utilizing lesional immune substances and/or inflammatory signals released within the lesion, including, but are not limited to, the following three categories: biomacromolecular immunomodulators, immune cell immunomodulators, and vaccine immunomodulators. The term "biomacromolecular immunomodulators" (abbreviated as E1) refers to immunomodulators on proteins or nucleic acids, with representative medicaments for protein-based ones being immune checkpoint inhibitors and cytokines, and for nucleic acid-based ones being TLR agonists. The term "immune cell immunomodulators" (abbreviated as E2) refers to cell products used in treatments that directly or indirectly enhance immune responses through immune cell transplantation or injection, such as DC vaccines, NK cells, and CAR-T cells. The term "vaccine immunomodulators" (abbreviated as E3) refers to formulations that enhance, promote immune responses by inducing specific or non-specific immunity, such as vaccines based on lesional immune substance vaccines, oncolytic virus vaccines, and BCG vaccines.

In this application, the term "formulation" (Formulation) refers to pharmaceutical products that can be directly used after being prepared from pharmaceutical raw materials through specific processes. They can be, for example, tablets, capsules, injections, creams, suspensions, and the like. The term "optimized formulation" (Optimized formulation) refers to pharmaceutical formulations that have been selected through systematic research and optimized evaluation to use the most suitable pharmaceutical raw materials, excipients, and preparation processes, so that the product achieves the best state in terms of pharmaceutical use performance (preferably oral, followed by other systemic administration), efficacy, stability, and safety. After decades of selection, local administration formulations are far from being the preferred formulations for conventional metformin salts.

In this application, the term "scheme" refers to a specific pharmaceutical combination, pharmaceutical composition, formulation, method, kit, or use. The term "scheme of this application" refers to the pharmaceutical combination, pharmaceutical composition, formulation, method, kit, or use based on R of the present application, the term "control scheme" refers to the pharmaceutical combination, pharmaceutical composition, formulation, method, kit, or use used as a control for the scheme of the present application, and the term "conventional dimethylbiguanide scheme" refers to the scheme based on R' (commonly used as a control scheme). Indications are the core effects of a scheme and also its core value.

In this application, the term "indication" is distinct from "disease or disease symptoms". The latter refers to an abnormal life process, while the former refers to "a disease or condition suitable for a certain treatment measure" (from Cihai (1989 Edition)) or "the scope of disease symptoms for which a medicament is applicable" (from the National Committee for the Standardization of Scientific and Technical Terminology, Pharmacology Terminology (Second Edition)). In summary, the indication of a scheme refers to the scope of its applicability to specific diseases or disease symptoms, based on its effect characteristics (Y), to address the clinical needs of a specific disease (X) or its core pathological conditions, denoted as (X, Y). Those skilled in the art know that the effect characteristic (Y) is the core condition for defining the scope of applicable diseases or symptoms (X), and it must be specific, strict, clear and unambiguous. The authoritative institutions in various countries within this field have all made such regulations. For instance, the China Food and Pharmaceutical Administration (CFDA) stipulates in the "Detailed Rules for the Specifications of Instructions for Chemical Pharmaceuticals and Therapeutic Biological Products (2006 Edition)" that "[Indications] should be expressed accurately based on the use of the medicament, clearly stating its application in prevention, treatment, diagnosis, relief or auxiliary treatment of a certain disease (condition) or syndrome." Here, the effect characteristic (Y) of the medicament or method determines its use, for example, Y can be prevention, treatment, diagnosis, relief or auxiliary treatment. Within the field of "treatment", Y can be further subdivided according to more precise effect characteristic standards. For instance, it can be differentiated based on the satisfaction of specific effects as: effective treatment, rapid treatment, high-efficiency treatment, long-term treatment, and heterogeneous equivalence (scheme).

In this application, the term "structure-activity relationship" refers to the correlation between the indications (X, Y) and the set of pharmaceutical composition elements such as (α, β, γ). Indications are the most essential and valuable characteristics of the pharmaceutical "activity" set, and the necessary composition element set is obviously the characteristic pharmaceutical "structure" set. The pharmacological conditions that make the pharmaceutical "structure" display the pharmaceutical "activity" limit their relationship, that is, f: (α, β, γ) → (X, Y). Here, Y represents the activity characteristics, including interaction, dose-effectiveness, therapeutic effectiveness (timeliness, effectiveness, long-term efficacy), heterogeneous adaptability, etc.; X represents the scope of lesions suitable for the activity characteristics of Y; α represents the chemical characteristics or set of the medicament required to meet the indication X and the activity characteristics Y, such as the common chemical structure subset (A, B); β represents the conditions for providing activity in the pharmaceutical's active ingredients, such as the dosage of A and the functional combination or synergistic action conditions of (A, B), including, for example, the common concentration threshold of A and B and their synergistic concentration ratio range, etc.; γ represents the compatibility of the formulation (such as the exclusivity of A), ensuring the stability and efficacy of the medicament under specific conditions. The relationship f maps the "structure" set (α, β, γ) to the "activity" set (X, Y), f: (α, β, γ) → (X, Y), and f includes, for example, the administration form of the active form of the structure required to form the activity.

As mentioned in the "Background" above, the EC50 of the conventional dimethylbiguanide scheme in sensitive tumor cell experiments is at the mM level, which is over a hundred times higher than that of conventional antineoplastic agents (such as 5-FU). Apart from safety, it has no active advantages or activity characteristics (Y) that can be applied. For example, the indication of the conventional dimethylbiguanide scheme is limited to (X' = sensitive tumors, Y' = effective but not rapid, high, or long-term treatment), rather than the commonly expanded claim of (X = tumors, Y = treatment). If its indication (X', Y') is extended to (X, Y), it may lead to serious consequences in clinical practice due to the misuse of the scheme and the delay of treatment opportunities, and scientifically, it misleads technicians in this field.

The scientific combination of α, β, and γ (α, β, γ) is the key to achieve the expected pharmaceutical effect (activity), reflected in the precise matching of specific indications (X, Y). For example, the (α, β, γ) differences between R and R' include: different molecular structures (α) for providing active forms (N,N-dimethylbiguanide vs N,N-dimethylbiguanide hydrochloride), different concentration thresholds or dosages and solubility (β) (Q_{R}/Q_{R'} < 50% or more), and different stabilities of pharmaceutical formulations (γ) (with vs without mixing contraindications).

This application's scheme generates new therapeutic activity characteristics (Y) and diseases or conditions (X) suitable for treatment by providing new sets of pharmaceutical composition elements (α, β, γ) and new pharmacology (f), that is, f: (α, β, γ) → (X, Y).

The comparison system, evaluation methods, and standards for activity characteristics (Y), as well as related definitions, are as follows.

### 1. Comparison System

In this application, the term "conventional comparison" refers to the comparison between the research scheme and the existing or technologically predictable solutions (conventional schemes), assessing whether the research scheme exceeds the expection of the conventional scheme; the term "further comparison" refers to the in-depth evaluation of multiple research schemes on the basis of conventional comparison, further assessing whether a certain combination scheme exceeds the expected technical effect based on the component schemes through detailed and expanded comparisons.

The conventional comparison of single agents mainly involves the comparison between dimethylbiguanide medicament (R) and conventional metformin salt (R'). According to the teachings of the prior art, there are no structural differences between dimethylbiguanide medicaments, and R and R' are considered to be the same active components, expected to have the same efficacy. The actual efficacy of R in the research scheme is compared with the result of R', if the efficacy is the same, it is in line with expectations; if it is significantly stronger, it exceeds expectations and becomes an unconventional medicament that conflicts with the previous teachings.

The conventional comparison for the combination mainly is the comparison between the combination R/Z and the conventional combination R'/Z. As mentioned above, according to the teachings of the prior art, it is expected that R/Z and R'/Z have the same efficacy. The actual efficacy of R/Z in the research scheme is compared with the result of R'/Z, if the efficacy is the same, it is in line with expectations; if it is significantly stronger, it exceeds expectations and becomes an unconventional combination that conflicts with the previous teachings.

Under the condition that R becomes an unconventional metformin drug, a comparison between the combination R/Z and the single agents, especially R, is clearly beyond the expected range of conventional comparisons and is no longer applicable to conventional comparisons. It can only be further compared between different research schemes. The further comparison is based on the expected combination results calculated by the following HSA method or Bliss method according to the single agent results. The actual efficacy of R/Z in the research scheme is compared with the expected efficacy. If the efficacy is the same, it is in line with expectations; if it is stronger, it exceeds expectations and becomes a further unconventional combination.

### 2. Interaction

The expected interaction (antagonism, additivity, and synergy) between each active component (such as R and Z) in the co-administration scheme is highly uncertain, which is called Interaction. The probability of synergy is much lower than that of non-synergy. The core is the interaction beyond the expectations of the prior art scheme, which is usually an important purpose of the co-administration scheme research, a major technical difficulty and essential feature, and important evidence for unique innovation in the combination mechanism is also provided.

In this application, taking the mouse experiment in the Examples as an example, the evaluation of interation is as follows:
In this application, taking the mouse experiment in the examples as an example, the evaluation of quantity effectiveness is as follows.

### (1). Calculation of interaction index

The interaction index is a ratio of the actual co-administration effect (q₁) to an expected co-administration effect (q₂) calculated based on the prediction model, widely used in the industry: q = q1/q2.

### (2). General evaluation

When q > 1, it is synergy; otherwise, it is non-synergy (possibly independent action or antagonism).

### (3). Relative evaluation

### (A). Conventional comparison

In the conventional comparison, q₂ is the expected co-administration effect of R/Z (R/Z and R'/Z have the same effect) according to the prior art. The evaluation criterion is: when the actual effect of R/Z (q1) is greater than that of R'/Z, then q = q1/q2 > 1.0 is synergy; otherwise, it is non-synergy.

This synergy reflects the unexpected interaction produced by co-adminstration of R in place of R' (the metformin in the prior art) and Z, which is manifested as an unexpected enhancement effect. For example, the co-adminstration of the conventional metformin drug (R') and the cell-reactive drug (B) is expected to be the interaction of cellular reactive effect; while the combination of N,N-dimethylbiguanide (R) and B in this application provides a interaction between the weakened local biological barrier function of R and the cellular reactive antineoplastic effect of B, which exceeds the former expectation. This is the unconventional pharmacological mechanism of the above synergy.

### (B). Further Comparison

When the R/Z combination is synergistic in the conventional comparison, a further comparison is conducted. q₂ is the expected co-adminstration effect based on the efficacy of the single agents R and Z, where R is an active component that exceeds the expected efficacy of the existing dimethylbiguanide technical solution. The calculation of q₂ is used for the comparison between the combination scheme and the single agent scheme, and can be applied to both the conventional comparison and the further comparison. The two most commonly used methods in antineoplastic animal experiments are selected for this purpose. In this application, the actual single agent effects of R and Z are denoted as E_{R} and E_{Z}, respectively.

### (a). Highest Single Agent (HSA) Method

The HSA method is one of the most widely used methods in antineoplastic animal experiments. Its core idea is to compare the actual effect (q₁) of the combination with the predicted value (q₂) of the best single agent effect, where q₂ = max(E_{R}, E_{Z}). If the effect is the same, i.e., it is as expected; if the effect is stronger, it exceeds expectations. The evaluation criterion is: when the actual efficacy (q₁) of the combination is greater than max (E_{R}, E_{Z}), it is further synergistic; otherwise, it is not further synergistic.

### (B). Bliss Method

The Bliss independence model (Bliss, C. I. (1939). The toxicity of poisons applied jointly. Annals of Applied Biology, 26(3), 585-615.) is another widely used method in antineoplastic animal experiments. Its core assumption is that the effects of the two medicaments are independent and follow the principle of probability superposition: q2 = E_{R} + E_{Z} - E_{R} × E_{Z}. If the effect is the same, it is as expected; if the effect is stronger, it exceeds expectations. The evaluation criterion is: when the actual effect (q1) of the combination is greater than q2, it is further synergistic; otherwise, it is not further synergistic.

In this application, the terms "further synergistic" (scheme), "synergistic" (scheme), and "non-synergistic" (scheme) refer to the schemes that meet the above further synergistic, synergistic, and non-synergistic evaluation criteria, respectively.

In one embodiment, this application provides a pharmaceutical combination, pharmaceutical composition, formulation, method, kit, or use for treating solid tumors, wherein the combination of R and Z (R/Z combination) is a synergistic combination that exceeds the expected effect of a combination of the conventional metformin drug (R') and Z (R'/Z combination), characterized by a significant increase in pharmaceutical effect when the one-course dosage of R (Q_{R}) is ≤ 25% or 50% of the one-course dosage of R' (Q_{R***'***}) and the dosage of Z in the course does not increase, including at least a 100% increase in at least one pharmaceutical adjustability index and at least a 25% increase in at least one pharmaceutical efficacy index, or at least a 25% increase in the local efficacy-toxicity ratio.

In one embodiment, this application provides a combination, pharmaceutical composition, formulation, method, kit, or use for treating solid tumors, wherein the combination of R and Z (R/Z combination) is a further synergistic combination that exceeds the expected effect of the single agent R and single agent Z based on the HSA method or Bliss method, characterized by an increase in pharmaceutical effect when the one-course dosage of R (Q_{R}) does not increase and the one-course dosage of Z (Q_{B}) is reduced by at least 20%, including at least a 10% increase in at least one pharmaceutical adjustability index and at least a 10% increase in at least one pharmaceutical efficacy index, or at least a 10% increase in the local efficacy-toxicity ratio.

In one embodiment, the synergistic scheme or further synergistic scheme of the present application is preferably applicable to tumors that require the interaction between the intratumoral barrier function weakening effect of R and the intratumoral cellular reactivity antineoplastic effect of B to produce higher efficacy, including, for example, highly invasive tumors, advanced tumors, rapidly deteriorating tumors, and refractory tumors.

### 3. Dose-effectiveness

In the present application, the term "dose-effectiveness" refers to the intensity of the effect of a unit dose of a medicament. The expected dose-effectiveness of the scheme can range from highly effective to weakly effective to ineffective, with high uncertainty. The difference between normal cells and tumor cells is not significant, and the difference between the effective dose and the safe limit of antineoplastic agents is usually not large, and the expected intensity is usually not high, which is manifested as severe side effects when the patient's benefit is not significant. Improving dose-effectiveness is an important goal of the scheme research and a major technical difficulty and essential feature. If a new scheme exceeds the expected dose-effectiveness of the old scheme, it not only represents an unexpected increase in efficacy but also provides important evidence of unique innovation in the treatment mechanism or pharmaceutical design.

In the present application, taking the mouse experiments in the examples as an example, the dose-effectiveness evaluation is as follows.

### (1). Dose-effectiveness index

In the present application, the term "dose-effectiveness index" refers to the numerical representation of a specific pharmaceutical effect (such as therapeutic effect) produced by each unit of medicament (such as per mmol or per mg of medicament) in the body, used to measure the relationship between the minimum dose or its therapeutic effect (X) and the minimum dose or the minimum administration frequency related to it, usually for the quantity of one course of treatment.

### (2). Relative evaluation

### (A). Conventional comparison

Single agent comparison: the evaluation criterion is that when the dosage of R is halved (Q_{R} = 1/2Q_{R'}), at least one of its pharmaceutical effects increases by at least 30%; or at least one of the actual dose-effectiveness index of R as a single agent is at least 50% or 70% higher than that of R' as a single agent, then its dose-effectiveness exceeds the expected dose-effectiveness of the prior art (R and R' are regarded as pharmaceutically equivalent) and is considered potent.

Combination comparison: the evaluation criterion is that: when the dosage of at least one of R/Z is halved, at least one of the pharmaceutical effects of the combination increases by at least 30%; or the actual dose-effectiveness index of at least one of R/Z is at least 50% or 70% higher than that of the corresponding component of R'/B, then the dose-effectiveness of the combination exceeds the expected dose-effectiveness of the prior art (R and R' are regarded as pharmaceutically equivalent) and is considered potent.

### (B). Further comparison

In further comparison, the co-adminstration dose-effectiveness index of R/Z is the lowest of the dose-effectiveness index of R and Z as single agents based on the expected dose-effecitveness (HSA method). The evaluation criterion is that: when R/Z is highly effective, and when the dosage of at least one of R and Z is reduced by 10%, the actual dose-effectiveness index of the combination is at least 10% or 20% higher than the highest single agent, then its dose-effectiveness further exceeds the expectation and is considered further potent.

In this application, the terms "further potent" (scheme), "potent" (scheme), and "non-potent" (scheme) refer to the (schemes) that meet the above further potent, potent, and non-potent evaluation criteria respectively.

In one embodiment, the differential structure-activity relationship of the application's schemes can be simply expressed as f: (α, β, γ) → (X, Y), where the differential indication (X, Y) is the range (X) where the potent or further potent (Y) of R or R/Z is applicable, but the non-potent of R' or R'/Z is not applicable, to local lesion patients who need to benefit from low dosage or low frequency of high efficiency. The differential pharmacology (f) mainly includes the potent or further potent transient local effect of R distinct from R'; the differential component set (α, β, γ) is the potent or further potent component selection and its range limited by (X, Y) and f.

In one embodiment, the application provides a formulation, method, kit, or use for treating local lesions, wherein R is a potent active ingredient beyond the expected effect of conventional metformin drugs (R'), characterized by one course dosage (Q_{R}) of R being ≤ 25% or 50% of the one-course dosage (Q_{R'}) of R', but the pharmaceutical effect significantly increases, including at least one of the pharmaceutical adjustability indexes increasing by at least 100%, and at least one of the pharmaceutical efficacy indexes enhancing by at least 25%, or the local efficacy-toxicity ratio increasing by at least 25%.

In one embodiment, the application provides a combination, pharmaceutical composition, formulation, method, kit, or use for treating solid tumors, wherein the combination of R and Z (R/Z combination) is a potent combination beyond the expected effect of the combination of conventional metformin drugs (R') and Z (R'/Z combination), characterized by one course dosage (Q_{R}) of R being ≤ 25% or 50% of the one-course dosage (Q_{R'}) of R' and the one-course dosage of Z not increasing, but the pharmaceutical effect significantly increases, including at least one of the pharmaceutical adjustability indexes increasing by at least 100%, and at least one of the pharmaceutical efficacy indexes enhancing by at least 25%, or the local efficacy-toxicity ratio increasing by at least 25%.

In one embodiment, the present application provides a further dose-reduction and efficacy-enhancement combination, pharmaceutical composition, formulation, method, kit, or use for treating solid tumors, wherein the combination of R and Z (R/Z combination) is a further synergistic combination beyond the expected effect of R and Z as single agents based on the HSA method or Bliss method, characterized by the one-course dosage (Q_{R}) of R not increasing and the one-course dosage (Q_{Z}) of Z reducing by at least 20%, but the pharmaceutical effect increases, including at least one of the pharmaceutical adjustability indexes increasing by at least 10%, and at least one of the pharmaceutical efficacy indexes enhancing by at least 10%.

In one embodiment, the potent schemes or further potent schemes of the application are preferably applicable to tumors that require the strongest dose-effect relationship for treatment, including, for example, highly invasive tumors, advanced tumors, rapidly deteriorating tumors, and refractory tumors. If the present application's schemes are not selected but the above comparative schemes are chosen instead, there may be many risks in clinical practice, such as increased pharmaceutical dosage due to misusing the schemes (choosing non-potent when potent should be preferred), but the effect is not high, thus losing the treatment opportunity.

### 4. Therapeutic Efficacy

In this application, the term "therapeutic efficacy" refers to an ability to produce significant therapeutic effects on the target disease or symptom, including, for example, timeliness, effectiveness, and long-term efficacy, and the like. The level of therapeutic efficacy is influenced by multiple factors and is highly uncertain (high efficacy, low efficacy, no efficacy, etc.). High efficacy occurs with the lowest probability and is the main technical difficulty in pharmaceutical development, as well as an important basic indicator for scheme innovation. When the therapeutic efficacy expectation of the old scheme is not high (for example, non-quick-acting, non-effective, or non-persistent), if the new scheme exceeds this expectation to be quick-acting, effective, or persistent, this not only represents an unexpected increase in efficacy but also provides important evidence of unique innovation in the treatment mechanism or pharmaceutical design.

### 1). Timeliness

In this application, the term "timeliness" refers to the ability to show the effect (efficacy) of the target disease or symptom under the treatment scheme within a certain period of time, which is an important indicator for measuring the speed of therapeutic effect. The timeliness expectation of the scheme includes quick-acting, non-quick-acting, etc., and is highly uncertain. The condition of tumors usually progresses rapidly, and the timeliness expectation (for example, for antineoplastic agents) is usually not high. The probability of quick-acting is much lower than that of non-quick-acting, which is an important goal of the scheme research, as well as a major technical difficulty and essential feature. If the new scheme exceeds the non-quick-acting expectation of the old scheme to be quick-acting, this not only represents an unexpected increase in efficacy but also provides important evidence of unique innovation in the combination mechanism.

In this application, taking the mouse experiments in the examples as an example, the timeliness evaluation is as follows.

### (1). Timeliness index

In this application, the term "timeliness index" refers to the time to efficacy, where the efficacy refers to a relative tumor growth rate (T/C%) of ≤ 42% (according to NCI guidelines), and the time to efficacy is the date when efficacy is observed.

### (2). Relative Evaluation

### (A). Conventional Comparison

Single agent comparison: the evaluation criterion is that when the actual time to efficacy of single agent R is at least 25% or 40% shorter than that of single agent R', its timeliness exceeds the expectation of the prior art (R and R' are pharmaceutically equivalent) and is considered quick-acting.

Combination comparison: the evaluation criterion is that when the actual time to efficacy of R/Z is shorter than that of R'/Z, or at least 25% or 40% shorter, its timeliness exceeds the expectation of the prior art (R/Z and R'/Z are pharmaceutically equivalent) and is considered quick-acting.

### (B). Further Comparison

In further comparison, the co-adminstration timeliness of R/Z is the fastest of the timeliness expectations of single agents R and Z (HSA method). The evaluation criterion is that when the actual time to efficacy of R/Z is shorter than the fastest of single agents R and Z, or at least 10% or 20% shorter, its timeliness further exceeds the expectation and is considered further quick-acting.

In this application, the terms "further quick-acting" (scheme), "quick-acting" (scheme), and "non-quick-acting" (scheme) refer to the schemes that meet the above further quick-acting, quick-acting, and non-quick-acting evaluation criteria respectively. Medicaments with strong timeliness can better combine with other treatment methods and enhance the potential of combinations.

In one embodiment, the differential structure-activity relationship of the scheme in this application can be simply expressed as f: (α, β, γ) → (X, Y), where the differential indication (X, Y) is a range (X) where the quick-acting or further quick-acting (Y) of R or R/Z is applicable, but the non-quick-acting of R' or R'/Z is not applicable to patients with local lesions that need rapid effects during disease progression; the differential pharmacology (f) mainly includes the quick-acting or further quick-acting transient local effects of R distinct from R'; the differential component set (α, β, γ) is the selection and range of quick-acting or further quick-acting components limited by (X, Y) and f.

In one embodiment, the present application provides a formulation, method, kit, or use for treating local lesions, wherein the R is a dose reduction and efficacy enhancement active ingredient that exceeds the expected effect of the conventional metformin drug (R'), characterized by that when the one-course dosage of R (Q_{R}) is ≤ 25% or 50% of the one-course dosage of R' (Q_{R'}), the pharmaceutical effect is significantly increased, including a significant improvement in pharmaceutical timeliness indexes, such as a reduction in the effective time (the day when the tumor proliferation rate is < 42%) in mouse experiments by at least 25% compared to R'.

In one embodiment, the present application provides a combination, pharmaceutical composition, formulation, method, kit, or use for treating solid tumors, wherein the combination of R and Z (R/Z combination) is a dose reduction and efficacy enhancement combination that exceeds the expected effect of the conventional metformin drug (R') and Z (R'/Z combination), characterized by that when the one-course dosage of R (Q_{R}) is ≤ 25% or 50% of the one-course dosage of R' (Q_{R'}) and the one-course dosage of Z does not increase, the pharmaceutical effect is significantly increased, including a significant improvement in pharmaceutical timeliness indexes, such as a reduction in the effective time (the day when the tumor proliferation rate is < 42%) in mouse experiments by at least 25% compared to R'/Z.

In one embodiment, the present application provides a further dose reduction and efficacy enhancement combination, pharmaceutical composition, formulation, method, kit, or use for treating solid tumors, wherein the combination of R and Z (R/Z combination) is a further dose reduction and efficacy enhancement combination that exceeds the expected effect of R and Z single pharmphacetical based on the HSA method or Bliss method, and when the one-course dosage of R (Q_{R}) does not increase and the one-course dosage of Z (Q_{Z}) is reduced by at least 20%, the pharmaceutical effect is significantly increased, including a significant improvement in pharmaceutical timeliness indexes, such as a reduction in the effective time (the day when the tumor proliferation rate is < 42%) in mouse experiments by at least 25% compared to R'.

In one embodiment, the dose reduction and efficacy enhancement scheme of the present application is preferably applicable to tumors that require the scheme of strongest dose-effect relationship and the highest speed of effect for treatment, including, for example, highly invasive tumors, advanced tumors, rapidly deteriorating tumors, refractory tumors, and tumors with urgent symptoms that require rapid tumor burden reduction. If the conventional dimethylbiguanide scheme is chosen instead of the strong and fast-acting scheme of the present application, there may be many risks in clinical practice, such as increased pharmaceutical dosage without high efficacy and longer effective time, thus losing the treatment opportunity.

### 2). Efficacy

In the present application, the term "efficacy" refers to an ability to suppress the target disease or symptom. The expected efficacy of a scheme can be highly effective, low effective (effective but not highly effective), ineffective but useful (ineffective but with some therapeutic effect), and no expected efficacy (ineffective and with no reasonable expected therapeutic effect), with high uncertainty. The expected efficacy is usually not high. For example, in antineoplastic agents, the expected efficacy of first-line treatment medicaments is usually highly effective, while that of second-line treatment medicaments is mostly low effective. Medicaments that can still be used beyond first and second-line treatments are almost ineffective but useful (such as adjuvant therapy), and most medicaments are actually without expected efficacy. Therefore, the probability of highly effective is much lower than that of low effective, and the latter is much lower than that of ineffective but useful. Improving efficacy is an important goal of scheme research and a major technical difficulty and essential feature. If a new scheme exceeds the expected efficacy of an old scheme, this not only represents an unexpected increase in efficacy but also provides important evidence of unique innovation in treatment mechanisms or pharmaceutical design.

In the present application, taking the mouse experiments in the examples as an example, the efficacy evaluation is as follows.

### (1). Calculation of efficacy indexes

In the present application, the term "efficacy indexes" refers to a tumor inhibition rate or objective response rate. When a negative control group is present, the efficacy indexes are tumor inhibition rate and tumor weight; when no negative control group is present, the efficacy index is objective response rate (ORR). The latter classifies the therapeutic effect of the study group into the following four grades (RECIST guidelines) to observe: complete response (CR) when target lesions disappear; partial response (PR) when the volume of target lesions decreases by at least 30%; progressive disease (PD) when the volume of target lesions increases by at least 20%; stable disease (SD) when the change in the volume of target lesions is between PR and PD. ORR is the sum of CR and PR within a pre-determined shortest period of time (e.g., N days after medicament withdrawal).

### (2). General Evaluation

When a negative control group is present, the efficacy evaluation criteria for the study group are (CFDA's "Guidelines for Pharmacodynamics of Antineoplastic Medications"): effective if the tumor inhibition rate is ≥ 40% and the difference in tumor weight from the negative control group is statistically significant (P < 0.05); otherwise, ineffective. When no negative control group is present, the commonly used efficacy evaluation criteria are: effective if ORR is ≥ 60%; otherwise, ineffective.

For ineffective study groups, the evaluation criteria are: ineffective but useful if the tumor inhibition rate is > 25% or the objective response rate is > 40%; otherwise, no (efficacy) expectation.

### (3). Relative Evaluation

### (A). Conventional Comparison

Single-agent comparison: the evaluation criterion is: if the actual tumor inhibition rate or objective response rate of R single-agent is at least 25% or 50% higher than that of R' single-agent, then its efficacy exceeds expectations of the prior art (R and R' are pharmaceutically equivalent) and is considered highly effective.

Combination comparison: the evaluation criterion is: if the actual tumor inhibition rate or objective response rate of R/Z is at least 10%, 20%, or 40% higher than that of R'/Z, then its efficacy exceeds expectations of the prior art (R/Z and R'/Z are pharmaceutically equivalent) and is considered highly effective.

### (B). Further Comparison

In the further comparison, the co-adminstration efficacy of R/Z based on the expected efficacy of R and Z as monotherapies (HSA method) is the highest among them. The evaluation criteria are as follows: when R/Z is highly effective, and its actual tumor inhibition rate or objective response rate is at least 10% or 20% higher than the highest of R and Z as monotherapies, its efficacy is considered to exceed expectations and is further highly effective.

In this application, the terms "further highly effective" (scheme), "highly effective" (scheme), "ineffective" (scheme), "ineffective but useful" (scheme), and "no expectation" (scheme) refer to the schemes that meet the above further highly effective, highly effective, ineffective, ineffective but useful, and no expectation evaluation criteria, respectively.

In this application, the term "refractory tumor" refers to a tumor that is not suitable for effective treatment with a certain scheme such as a cell-reactive drug or conventional metformin drug, including tumors in patients with contraindication to or unsuitability for the scheme and tumors that are not within the effective treatment indications of the scheme.

In this application, the term "non-indication tumor" refers to a tumor that is not within the selected range of a certain scheme such as a cell-reactive drug or conventional metformin drug, for example, a tumor that is not included in the list of tumors for which it can be used as a first-line or second-line treatment medicament released by an authoritative institution, including ineffective but useful tumors and no expectation tumors. Ineffective but useful tumors include, for example, tumors with an unfavorable microenvironment, and no expectation tumors include, for example, drug-resistant tumors and tumors after pharmaceutical withdrawal.

In this application, the term "drug-resistant tumor" refers to a tumor containing drug-resistant cells or tissues (e.g., tumor tissue from patients with drug resistance to clinical antineoplastic agents or targeted medicaments), and drug-resistant tumors are usually insensitive to many other antineoplastic agents and thus ineffective, thereby becoming refractory tumors.

In this application, the term "unfavorable microenvironment tumor" refers to a tumor containing an unfavorable microenvironment for the action of antineoplastic agents, such as a tumor formed by a mixture of tumor cells and fibroblasts in animal experiments, or a tumor derived from clinical high stromal ratio tumor tissue (e.g., pancreatic cancer tissue). Such microenvironments are known to significantly reduce the sensitivity of tumor cells to conventional antineoplastic agents, thereby becoming refractory tumors.

In this application, the term "tumor after medicament withdrawal" refers to a tumor for which medicament administration has been stopped for any reason. There are many reasons for stopping tumor medication treatment, including the patient's physical condition no longer being suitable and medication treatment failure, and failure includes any reason for medication insensitivity. The mechanisms of this insensitivity are numerous, and existing research has not fully revealed them, involving both the cellular level (e.g., cellular medication resistance) and the tissue level (e.g., more difficult penetration within the tissue), thereby becoming refractory tumors.

In one embodiment, the differential structure-activity relationship of the present application can be simply expressed as f: (α, β, γ) → (X, Y), where the differential indication (X, Y) is the range (X) where the highly effective or further highly effective (Y) of R or R/Z is applicable, while the ineffective, ineffective but useful, or no expectation of R' or R'/Z is not applicable: patients with local lesions requiring highly effective or further highly effective treatment; the differential pharmacology (f) mainly includes the highly effective or further highly effective transient local effect of R compared to R'; the differential component set (α, β, γ) is the selection and range of highly effective or further highly effective component elements defined by (X, Y) and f, where X includes refractory tumors to cell-reactive drugs or conventional metformin drug.

In one embodiment, the present application provides a formulation, method, kit, or use for treating local lesions, wherein R is a dose reduction and efficacy enhancement active component that exceeds the expected effect of conventional metformin drug (R'), characterized by one course dosage (Q_{R}) of R being ≤ 25% - 50% of the one-course dosage (Q_{R'}) of R', while the pharmaceutical effect significantly increases, including a significant improvement in pharmaceutical efficacy indexes, as shown by a tumor inhibition rate or objective response rate in mouse experiments that is at least 25% - 50% higher than that of R'.

In one embodiment, the present application provides a combination, pharmaceutical composition, formulation, method, kit, or use for treating solid tumors, wherein the combination of R and Z (R/Z combination) is a dose reduction and efficacy enhancement combination that exceeds the expected effect of the combination of conventional metformin drugs (R') and Z (R'/Z combination), characterized by one course dosage (Q_{R}) of R being ≤ 25% - 50% of the one-course dosage (Q_{R'}) of R' and the dosage of Z not increasing, while the pharmaceutical effect significantly increases, including a significant improvement in pharmaceutical efficacy indexes, as shown by a tumor inhibition rate or objective response rate in mouse experiments that is at least 10%, 20%, 30%, or 40% higher than that of R'/Z.

In one embodiment, the present application provides a further dose reduction and efficacy enhancement combination, pharmaceutical composition, formulation, method, kit, or use for treating solid tumors, wherein the combination of R and Z (R/Z combination) is a further dose reduction and efficacy enhancement combination that exceeds the expected effect of R and Z as single agents based on the HSA method or Bliss method, characterized by one course dosage (Q_{R}) of R not increasing and one course dosage (Q_{Z}) of Z being reduced by at least 20%, while the pharmaceutical effect significantly increases, including an improvement in pharmaceutical efficacy index, as shown by a tumor inhibition rate or objective response rate in mouse experiments being at least 10% - 20% higher than the highest of the single agent.

In one embodiment, the dose reduction and efficacy enhancement scheme of the present application is preferably applicable to tumors that require the strongest dose-effect relationship and the highest efficacy for treatment, including, for example, highly aggressive tumors, advanced tumors, rapidly deteriorating tumors, drug-resistant tumors, tumors with an unfavorable microenvironment, and tumors that relapse after medicament withdrawal. If the conventional metformin scheme is chosen instead of the dose reduction and efficacy enhancement scheme of the present application, there may be many risks in clinical practice, such as increased pharmaceutical dosage due to incorrect scheme selection without a corresponding increase in efficacy, thereby losing the treatment opportunity.

### 3). Long lasting efficacy

In this application, the term "long lasting efficacy" refers to the ability to provide sustained inhibition of the target disease or symptom or sustained benefit to the patient. The expected long-lasting efficacy of the scheme can be long-acting, non-long-acting, etc., with high uncertainty. The growth of tumors is usually difficult to control, and the duration of pharmaceutical efficacy (e.g., antineoplastic agents) is mostly not long. The probability of long-acting is much lower than that of non-long-acting. This is an important purpose of the scheme research, as well as a major technical difficulty and essential feature. If a new scheme exceeds the non-long-acting expectation of the old scheme as long-acting, this not only represents an unexpected increase in efficacy but also provides important evidence of unique innovation in the treatment mechanism or pharmaceutical design.

In this application, taking the mouse experiments in the examples as an example, the long-lasting efficacy is evaluated as follows.

### (1). Calculation of long-lasting efficacy index

In this application, the term "long lasting efficacy index" refers to progression-free time or long-term survival rate (referred to as survival rate). Progression-free time is the number of days from the first administration to the day when progression is observed, where the progression is evaluated by relative tumor volume (RTV), and when RTV > 150% and continuously increases until the end, it is considered as progression. Survival rate is a ratio of the number of survivors observed in the study group at the time when most of the negative control group has died (e.g., on the 30th day) to the number of enrolled subjects. Conventionally, a tumor volume > 2000 mm³ is considered a death event.

### (2). Relative evaluation

Single agent comparison: in the prior art, R and R' are regarded as pharmaceutically equivalent, and are expected to have the same long-lasting efficacy. The evaluation criterion is: when the actual progression-free time or survival rate of R as a single agent is at least 20% - 40% higher than that of R' as a single agent, its long-lasting efficacy exceeds the expectation of the prior art and is considered long-acting.

Combination comparison: as mentioned above, R/Z is expected to have the same long-lasting efficacy as R'/Z according to the prior art. The evaluation criterion is: when the actual progression-free time or survival rate of R/Z is at least 20% - 40% higher than that of R'/Z, its long-lasting efficacy exceeds the expectation of the prior art and is considered long-acting.

### (B). Further comparison

In further comparison, the co-adminstration long-lasting efficacy of R/Z is the highest among the long-lasting efficacy expectations of single agents R and Z (HSA method). The evaluation criterion is: when the actual progression-free time or survival rate of R/Z is at least 10% - 20% higher than the highest of R and Z as single agents, its long-lasting efficacy further exceeds the expectation and is considered further long-acting.

In this application, the terms "further long-acting" (scheme), "long-acting" (scheme), and "non-long-acting" (scheme) refer to the schemes that meet the above further long-acting, long-acting, and non-long-acting evaluation criteria, respectively.

In one embodiment, the differential structure-activity relationship of the scheme in this application can be simply expressed as f: (α, β, γ) → (X, Y), where the differential indication (X, Y) is the range (X) where the long-acting or further long-acting (Y) of R or R/Z is applicable but the non-long-acting of R' or R'/Z is not applicable: patients with local lesions requiring prolonged therapeutic effect; the differential pharmacology (f) mainly includes the transient local effect of the long-acting or further long-acting of R compared to R'; the differential component set (α, β, γ) is the selection and range of long-acting or further long-acting components limited by (X, Y) and f.

In one embodiment, the application provides a formulation, method, kit, or use for treating local lesions, where R is a dose reduction and long acting active ingredient exceeding the expected effect of conventional metformin drug (R'), characterized by one course dosage (Q_{R}) of R being ≤ 25% - 50% of the one-course dosage (Q_{R'}) of R', while the pharmaceutical effect is significantly increased, including a significant improvement in the long lasting efficacy index, as shown by a progression-free time or survival rate in mouse experiments being at least 20% - 40% higher than that of R'.

In one embodiment, the present application provides a combination, a pharmaceutical composition, a formulation, a method, a kit, or use for treating solid tumors, wherein the combination of R and Z (R/Z combination) is a dose reduction and high efficacy combination that exceeds the expected effect of the conventional metformin drug (R') and Z combination (R'/Z combination), characterized by one course dosage of R (Q_{R}) being ≤ 25% - 50% of the one-course dosage of R' (Q_{R'}) and the one-course dosage of Z not increasing, while the pharmaceutical effect significantly increases, including a significant improvement in the long-term efficacy index, as shown by a progression-free time or survival rate in mouse experiments being at least 20% - 40% higher than that of the R'/Z combination.

In one embodiment, the application provides a further dose reduction and efficacy enhancement combination, a pharmaceutical composition, a formulation, a method, a kit, or use for treating solid tumors, wherein the combination of R and Z (R/Z combination) is a further dose reduction and quick acting combination that exceeds the expected effect of single agent R and single agent Z based on the HSA method or Bliss method, characterized by one-course dosage of R (Q_{R}) not increasing and one-course dosage of Z (Q_{Z}) being reduced by at least 20%, while the pharmaceutical effect significantly increases, including an improvement in the long-term efficacy index, as shown by a progression-free time or survival rate in mouse experiments being at least 10% - 20% higher than the highest of the single agents.

In one embodiment, the dose reduction and long-term efficacy scheme of the application is preferably applicable to tumors that require the strongest dose efficacy relationship and the longest sustained long-term efficacy for treatment, including, for example, highly aggressive tumors, advanced tumors, rapidly deteriorating tumors, drug-resistant tumors, tumors with unfavorable microenvironments, and tumors that relapse after medicament withdrawal. If the conventional metformin scheme is chosen instead of the strong and long-lasting scheme of the present application, there may be many risks in clinical practice, such as increased pharmaceutical dosage due to incorrect scheme selection without a corresponding increase in efficacy and short progression-free time, thereby losing the treatment opportunity.

### 4) Local Efficacy-toxicity Ratio

In the application, the term "local efficacy-toxicity ratio" (referred to as the "efficacy-toxicity ratio") refers to a balance between the therapeutic effect (local efficacy) and toxicity (local toxicity) of a medicament when administered locally. It is generally believed that classical chemical ablation agents (such as absolute ethanol) have a consistency between the required local efficacy and local toxicity. Improving the efficacy-toxicity ratio to break this consistency expectation is an important objective of the research and a significant technical difficulty and essential feature. If a new scheme exceeds the expected efficacy-toxicity ratio of the old scheme, it not only represents an unexpected increase in efficacy but also provides important evidence of unique innovation in the treatment mechanism or pharmaceutical design.

In this application, taking the mouse experiments in the examples as an example, the research on the efficacy-toxicity ratio is mainly conducted under the condition of Z=C, and the evaluation is as follows.

### (1) Efficacy-toxicity ratio index

In this application, the term "efficacy-toxicity ratio index" refers to the ratio (X/Y) of the required therapeutic effect (X, selected from therapeutic efficacy, such as >40% tumor inhibition rate) to the local tissue stimulation response (Y, local stimulation score) after the local administration of an equal amount of the medicament. When the local administration of the medication neither produces a significant therapeutic effect (such as a tumor inhibition rate <20%) nor causes significant local stimulation (such as a score <1.0), its efficacy-toxicity ratio index is considered to be close to zero in this application.

### (2) Relative evaluation

### (A) Conventional comparison

The evaluation criterion is: when the adminstaration amount of at least one component in R/C is reduced by 50% compared to the corresponding adminstaration amount in R'/C, and its efficacy-toxicity ratio index (such as X/Y) increases by at least 0.5 times or more, it is considered that the efficacy-toxicity ratio of R/C exceeds the expectation of the prior art (R/C and R'/C are pharmaceutically equivalent), and is a "high efficacy-toxicity ratio".

### (B) Further comparison

In further comparison, the expected value of the efficacy-toxicity ratio (q₂) is calculated based on the HSA method of the single agents R and C. The evaluation criterion is: when a ratio of actual efficacy-toxicity ratio index (q₁) of R/C to the expected index (q₂) (q = q₁/q₂) > 1, it is considered to be a further high efficacy-toxicity ratio beyond the expectation.

In one embodiment, the differential structure-activity relationship of the scheme in this application can be simply expressed as f: (α, β, γ) → (X, Y), where the differential indication (X, Y) is a range (X) where the high efficacy-toxicity ratio or further high efficacy-toxicity ratio (Y) of R or R/C is applicable, but R' or R'/C is not applicable due to ineffectiveness: for patients with local lesions intolerant to local irritation; the differential pharmacology (f) mainly includes a transient local effect of the high efficacy-toxicity ratio or further high efficacy-toxicity ratio of R compared to R'; the differential component set (α, β, γ) is the selection and range of the components of the high or further high efficacy-toxicity ratio limited by (X, Y) and f.

In one embodiment, this application provides a combination, pharmaceutical composition, formulation, method, kit, or use for treating local lesions, where the combination of R and C is a high efficacy-toxicity ratio combination that exceeds the expected effect of the combination of R' and C, characterized by a actual efficacy-toxicity ratio index (q₁) of R/C exceeding an expected index (q₂) based on the equivalence of R and R'.

In one embodiment, this application provides a further dose reduction and efficacy enhancement combination, pharmaceutical composition, formulation, method, kit, or use for treating solid tumors, where the combination of R and C is a further synergistic combination that exceeds the expectation based on the HSA method of the single agents R and C, characterized by an actual efficacy-toxicity ratio index (q1) of the combination exceeding an expected index (q₂) based on the single agents R and C.

In one embodiment, the high-efficiency-toxicity ratio scheme or further high-efficiency-toxicity ratio scheme of the present application is preferably applicable to patients or tumor bodies or tumor body parts that require a scheme with the highest possible efficiency-toxicity ratio for treatment, including, for example, highly invasive tumors, advanced tumors, rapidly deteriorating tumors, refractory tumors, or parts of the tumor that are prone to leakage of the pharmaceutical solution. If the scheme of the present application is not selected and the above-mentioned comparative scheme is chosen instead, there may be many risks of damage to normal tissues due to the misuse of the scheme (choosing the opposite when a high-efficiency-toxicity ratio should be preferred) in clinical practice.

### 4. Heterogeneous Adaptability

In the present application, the term "heterogeneous adaptability" refers to the ability of a scheme to exhibit convergent therapeutic effects (such as quick effect, long-lasting effect, high efficiency) in heterogeneous regions of the target disease or symptom (such as different tissues or lesions). The term "adjustability" refers to the ability of a scheme to adjust its therapeutic effect or administration parameters according to the dynamic changes of the disease state (including the heterogeneity) or external conditions. The heterogeneous adaptability or adjustability of a scheme is expected to be highly adaptable/highly adjustable, generally adaptable/adjustable, non-adaptable/non-adjustable, etc., with high uncertainty.

One of the basic characteristics of malignant tumors is high heterogeneity, and conventional antineoplastic schemes, including the conventional metformin scheme, usually have problems with heterogeneous adaptability. For intratumoral administration schemes (such as absolute ethanol schemes), there are also usually problems with heterogeneous adaptability, making it difficult to administer medicaments based on the nature of the tumor. Heterogeneity includes tissue heterogeneity, spatial heterogeneity, and safety heterogeneity. In summary, improving heterogeneous adaptability or adjustability is an important goal in the research of antineoplastic drug technical schemes, as well as a major technical difficulty and an essential feature. If a new scheme exceeds the adjustability expectation of an old scheme, this not only represents an unexpected efficacy enhancement but also enables adjustment and optimization under different conditions to meet the flexibility and diversity required for individualized clinical treatment, and provides important evidence of unique innovation in treatment mechanisms or pharmaceutical design.

In the present application, taking the mouse experiments in the examples as an example, the evaluation of heterogeneous adaptability/adjustability is as follows.

### 1). Calculation of heterogeneous adaptability/ adjustability index

In the present application, the term "heterogeneous adaptability index" or "adjustability index" refers to a difference in therapeutic effects of a scheme in different tissues or lesions (different tumors, different lesions, etc.), or an adjustability coefficient of local administration parameter for achieving convergent target therapeutic effects in the same tissue or lesion. These coefficients are the ratio of the highest value to the threshold in the adjustable range, including the local administration concentration adjustability coefficient (referred to as the concentration adjustability coefficient, for example, the highest value of cR/threshold), the local administration target volume adjustability coefficient (referred to as a target volume adjustability coefficient, the highest value of V_{target}/threshold), the local administration volume ratio adjustability coefficient (referred to as the volume ratio adjustability coefficient, for example, the highest value of VR/Vtarget/threshold), a local administration single dose adjustability coefficient (referred to as the single dose adjustability coefficient, for example, the highest value of c_{R}×v_{R}/threshold), a local administration point density adjustability coefficient (referred to as the administration point density adjustability coefficient, for example, the highest value of nᵢ/V_{target}/threshold), and the like.

### 2). Relative Evaluation

### (1). Conventional Comparison

Single-agent comparison: the evaluation criterion is that when R' as a single agent is effective on different tissues or lesions, or when the actual value of any of the above-mentioned adjustable coefficients of R as a single agent is at least 100% or 300% higher than that of R' as a single agent, then its heterogeneity adaptability or adjustability exceeds the expectations of the prior art (R and R' are pharmaceutically equivalent), it is highly adjustable. According to the teachings of the prior art, the therapeutic effects of R' as a single agent on different tissues or lesions (different tumors, different lesions, etc.) vary greatly (only limited to few tumors), and even for the few tumors that it is effective, the efficacy of R' is dose-dependent rather than dependent on local administration parameters, and its local adjustable coefficient is not high.

Combination comparison: the evaluation criterion is that when R/Z is effective on different tissues or lesions, or when the actual value of any of the above-mentioned adjustable coefficients of R/Z is at least 100% or 300% higher than that of R'/Z, then its heterogeneity adaptability or adjustability exceeds the expectations of the prior art (R/Z and R'/Z are pharmaceutically equivalent), it is highly adjustable; otherwise, it is not highly adjustable.

### (2). Further Comparison

In further comparison, the common adjustability of R/Z is the highest of the adjustability expectations of single agents R and Z (HSA method). The evaluation criterion is that when the actual value of any of the above-mentioned adjustable coefficients of R/Z is at least 20% higher than the highest of R and Z as single agents, then its adjustability further exceeds expectations, and is further highly adjustable.

In this application, the terms "further highly adjustable" (scheme), "highly adjustable" (scheme), and "not highly adjustable" (scheme) refer to the schemes that meet the above further highly adjustable, highly adjustable, and not highly adjustable evaluation criteria, respectively, which provide further highly heterogeneous equivalent, highly heterogeneous equivalent, and not highly heterogeneous equivalent pharmaceutical effects, respectively.

In one embodiment, the differential structure-activity relationship of the schemes in this application can be simply expressed as f: (α, β, γ) → (X, Y), where the differential indication (X, Y) is a range (X) where the highly adjustable or further highly adjustable (Y) of R or R/Z is applicable to, while the non-highly adjustable R' or R'/Z is not applicable to the patients with local lesions requiring optimizing the therapeutic schemes of pharmaceutical heterogenous adaptability and adjustability; the differential pharmacology (f) mainly includes the highly adjustable or further highly adjustable transient local effect of R that is different from R'; the differential component set (α, β, γ) is the selection and range of highly adjustable or further highly adjustable components limited by (X, Y) and f.

In one embodiment, this application provides a formulation, method, kit, or use for treating local lesions, where R is a dose reduction and efficacy enhancement active ingredient that exceeds the effect expectations of conventional metformin drugs (R'), characterized by one course dosage (Q_{R}) of R being ≤ 25% - 50% of the one-course dosage (Q_{R'}) of R', while the pharmaceutical effect is significantly increased, including at least one pharmaceuticaladjustability index being improved significantly, as as shown by an increase of at least one of the following adjustable coefficients by 100% or more in mouse experiments: concentration adjustable coefficient, target volume adjustable coefficient, volume ratio adjustable coefficient, single dose adjustable coefficient, administration point density adjustable coefficient .

In one embodiment, the present application provides a combination, a pharmaceutical composition, a formulation, a method, a kit, or use for treating solid tumors, wherein the combination of R and Z (R/Z combination) is a combination of dose reducction and efficacy enhancement beyond the expected effect of the combination of conventional metformin drugs (R') and Z (R'/Z combination), characterized by one course dosage of R (Q_{R}) being ≤ 25% - 50% of the one-course dosage of R' (Q_{R***'***}) and the one-course dosage of Z not increasing, while the pharmaceutical effect significantly increasing, including at least one pharmaceutical adjustability index significantly improving, as shown by an increase of at least one of the following adjustable coefficient by 100% or more in mouse experiments: concentration adjustable coefficient, target volume adjustable coefficient, volume ratio adjustable coefficient, single dose adjustable coefficient, and application point density adjustable coefficient.

In one embodiment, the present application provides a further dose reduction and efficacy enhancment combination, a pharmaceutical composition, a formulation, a method, a kit, or use for treating solid tumors, wherein the combination of R and Z (R/Z combination) is a further dose reduction and efficacy enhancement combination beyond the expected effect of single agent R and single agent Z based on the HSA method or Bliss method, characterized by one course dosage of R (Q_{R}) not increasing and one course dosage of Z (Q_{Z}) reducing by at least 20%, while the pharmaceutical effect significantly increaseing, including at least one pharmaceutical adjustability index significantly improving, as shown by an increase of at least one of the following adjustable coefficient by at least 10% - 20% in mouse experiments compared to the highest value of the monotherapies: concentration adjustable coefficient, target volume adjustable coefficient, volume ratio adjustable coefficient, single dose adjustable coefficient, and application point density adjustable coefficient .

In one embodiment, the local administration concentration adjustable coefficient of R (maximum value of c_{R} /threshold) is > 10, 50, 100, or 150, which enables R to provide highly variable concentrations in different regions while maintaining stable activity, including, for example, tailored medicament for heterogeneous tumors.

In one embodiment, the local administration target volume adjustable coefficient of R (maximum value of V_{target}/threshold) is > 2, 10, 50, or 100, which enables R to select different v_{target} for different treatment scenarios, including, for example, tailored medicament for heterogeneous tumors.

In one embodiment, the local administration volume ratio adjustable coefficient of R (maximum value of v_{R}/v_{target}/threshold) is > 5, 10, or 100, which enables R to provide highly variable volume ratios in different regions while maintaining stable activity, including, for example, tailored medicament for heterogeneous tumors.

In one embodiment, the local administration single dose adjustable coefficient of R (maximum value of c_{Ri} × v_{Ri}/threshold) is > 2, 6, or 10, which enables R to select different single doses for different treatment scenarios, including, for example, tailored medicament for heterogeneous tumors.

In one embodiment, the local administration point density adjustable coefficient (maximum value of nᵢᵢ/ v_{target}/threshold) of R is >3, 10, 50, or 100, which enables R to provide highly variable density to different regions while maintaining its activity stable, including for example tailored medicament for heterogeneous tumors.

In this application, the term "micro-volume" or "micro-dose" (scheme) refers to a scheme in the local administration volume highly adjustable (scheme) where v_{administration}/v_{target} is 0.02-0.056, or 0.02-0.10, otherwise it is a "normal volume" (scheme). The term "high-density" (scheme) refers to a scheme in the local administration point density highly adjustable (scheme) where nᵢᵢ/V_{target} is ≥1 or 2 points per cm³, otherwise it is a "normal density" (scheme).

In one embodiment, this application provides a formulation, method, kit, or use for treating solid tumors, which is a local administratin volume highly adjustable scheme, including micro-volume administration where v_{administration}/v_{target} is 0.02-0.056, or 0.02-0.10. It is suitable for intratumoral differential administration or administration based on heterogeneity, including based on shape (shape heterogeneity) administration, based on tissue (tissue heterogeneity) administration, or based on safety risk (safety risk heterogeneity) administration.

In one embodiment, this application provides a combination, pharmaceutical composition, formulation, method, kit, or use for treating solid tumors, which is a local administration point density highly adjustable scheme, including high-density administration where nᵢᵢ/V_{target} is ≥1 point per cm³. It is suitable for intratumoral differential administration or administration based on heterogeneity, including based on shape (shapeheterogeneity) administration, based on tissue (tissue heterogeneity) administration, or based on safety risk (safety risk heterogeneity) administration.

In one embodiment, for larger lesions (e.g., long diameter > 2 cm), especially large lesions (e.g., long diameter > 3 cm), the highly adjustable includes multi-point administration based on tissue heterogeneity, multi-point administration based on shape heterogeneity, and/or multi-point administration based on safety risk heterogeneity, where the multi-point refers to the number of administration points being >3, >5, or 10-200, and at least one point is a micro-volume administration.

In one embodiment, the highly adjustable includes low concentration (R administration concentration ≤ 3%) and/or micro-volume (R administration volume ≤ 250 µl) administration near the edge of the lesion.

In one embodiment, the highly adjustable includes high concentration (R administration concentration ≥ 10%) and/or large volume (R administration volume ≥ 1000 µL) administration near the center of the lesion.

In one embodiment, the dose reduction and efficacy enhancement scheme of this application is preferably suitable for heterogeneous tumors that require the strongest dose-efficacy and the highest adjustability for treatment, including for example highly aggressive tumors, advanced tumors, rapidly deteriorating tumors, drug-resistant tumors, tumors with unfavorable microenvironments, and tumors after medication withdrawal. If the conventional metformin scheme is chosen instead of the strong and long-lasting scheme of this application, there may be many risks in clinical practice, such as increased pharmaceutical dosage without high efficacy due to misused schemes, and low adjustability of administration based on heterogeneity, thus losing the treatment opportunity.

As used herein, other terms are intended to be defined by their well-known meanings in the field.

5) In this application, the term "lesion" refers to a local abnormality in the structure or function of the body, usually including lesional tissue, and the tissue contains cells. A lesion is a local manifestation of a disease.

In this application, the term "intervention" is distinguished from conventional injection and refers to the administration method of delivering therapeutic substances to the lesion site through specific administration devices (such as puncture needles, injection needles, catheters, or others) or other mediators (such as natural body orifices, minimally invasive incisions), including vascular intervention (such as vascular perfusion) and non-vascular intervention (such as percutaneous injection).

The term "conventional injection" refers to an administration method that does not exclude, or even mainly includes, or preferably includes absorption injection; the term "absorption injection" refers to an injection method that allows the medicament to be absorbed by the blood after entering the body, including, for example, intravenous injection, intramuscular injection, intraperitoneal injection, and the like.

The term " safety of conventional injection" refers to one of the quality standards of conventional injection, that is, no local side effects (such as local irritation, local tissue damage, and the like) occur during conventional injection, and therefore it must have, for example, isotonic (osmotic) property, isobaric (tonicity) property, and isoprotic (pH 4.5-9.0) property.

In this application, the term "dedicated" refers to being unable to be used, or even strictly prohibited from being used, outside the designated purpose. For example, "intervention dedicated" is limited to intervention and excludes use in absorption injection.

In this application, the term "pharmaceutical solution" refers to an aqueous solution of a medicament that can be injected into the human body and produce therapeutic effects, including the active ingredient and its aqueous solvent.

The term "composition" (or structure, abbreviated as "structure") refers to constituent elements that are necessary to provide a specific function ("effect") and cannot be selected based on common knowledge, including, for example, the active ingredient, its excipients, and the intervention concentration that must be limited.

The term "concentration" refers to the weight percentage concentration (W component/W solution, %) or molar concentration weight (W component/M component/1 liter) of the specified component in the solution.

The term "intervention concentration" refers to the concentration of the specified component in the pharmaceutical solution at the time of intervention (for example, at the injection needle hole or the outlet of the perfusion tube).

In this application, the term "prior art (of)" (or "conventional" (such as medicament, injection solution, injection agent, dosage form, method, use, or reaction) refers to a scope within the existing prediction (of) (such as medicament, injection solution, injection agent, dosage form, method, use, or reaction).

In this application, the term "transient active ingredient" is distinguished from "sustained active ingredient", the former and the latter refer to active ingredients that provide transient and sustained effects, respectively, the former, for example, chemical ablation agents, and the latter, for example, conventional antineoplastic agents (including antineoplastic agents); the term "transient effect" is distinguished from "sustained effect", the former and the latter refer to the effects provided by the active ingredient remaining at a specific concentration for a short time and for a relatively long time respectively, the required action times are relatively short and relatively long respectively, they are unrelated and closely related to pharmaceutical pharmacokinetics (such as half-life) respectively, they appear pharmaceutical effect relatively fast and relatively slow respectively, the former, for example, chemical ablation, and the latter, for example, conventional effects (including cytotoxic effects).

Within the scope of the present invention, the term "tumor" refers to a disease with a tumor as a lesion symptom, which can be any pathological (malignant and non-malignant) tumor at any stage, including, for example, the following groups classified by tumor cell type: epithelial cell tumors, sarcomas, lymphoid tumors, germ cell tumors, embryonic cell tumors; and it includes tumors named according to the organ or tissue where the tumor cells are concentrated, including for example tumors named according to the following organs or tissues: skin, bone, muscle, breast, kidney, liver, lung, gallbladder, pancreas, brain, esophagus, bladder, large intestine, small intestine, spleen, stomach, prostate, testis, ovary or uterus.

Specifically, the malignant tumors include for example breast cancer, pancreatic cancer, thyroid cancer, nasopharyngeal cancer, prostate cancer, liver cancer, lung cancer, intestinal cancer, oral cancer, esophageal cancer, gastric cancer, laryngeal cancer, testicular cancer, vaginal cancer, uterine cancer, ovarian cancer, sarcoma, and the like.

The non-malignant tumors include for example breast tumors, pancreatic tumors, thyroid tumors, prostate tumors, liver tumors, lung tumors, intestinal tumors, oral tumors, esophageal tumors, gastric tumors, nasopharyngeal tumors, laryngeal tumors, testicular tumors, vaginal tumors, uterine tumors, fallopian tube tumors, ovarian tumors, and the like.

The medicament of the present application is a therapeutic medicament. When it is used to treat solid tumor diseases, it is used as the main therapeutic medicament and can also be combined with other interventional therapies, systemic chemotherapy, immunotherapy, photodynamic therapy, sonodynamic therapy, surgical intervention or combinations of such therapies for administration to further improve the therapeutic effect.

According to one aspect of the present invention, the present invention provides a single agent dimethylbiguanide (R) product (single agent R scheme 1), specifically a formulation (referred to as "the single agent R formulation of the present application " in this application), the formulation contains N,N-dimethylbiguanide and optionally a solvent, the formulation does not contain an acidic pH-adjusting agent required to meet cell-culture requirements or conventional injection safety requirements, and the concentration of N,N-dimethylbiguanide contained in the formulation is ≥ its interventional or administration concentration, the interventional or administration concentration (w/w) is 0.5% ≤ C ≤ 51%, preferably 1% - 50%, more preferably 3% - 50%, 5% - 50%, or 5% - 30%.

The single agent R product (single agent R scheme 2) specifically for lesion intervention provided by the present invention includes dimethylbiguanide liquid (referred to as "the single agent R liquid of the present application" in this application), which can be an aqueous solution of N,N-dimethylbiguanide, wherein the aqueous solution does not contain an acidic pH-adjusting agent required to meet cell-culture requirements or conventional injection safety requirements, and the concentration of the N,N-dimethylbiguanide solution {[W dimethylbiguanide/(W dimethylbiguanide + W solvent)]%} is ≥ its intervention concentration, wherein the intervention concentration is 0.5% ≤ C ≤ the maximum solubility concentration (or saturation concentration) of N,N-dimethylbiguanide.

The present invention provides a single agent dimethylbiguanide specifically for lesion intervention (single agent R scheme 3) (referred to as "the single agent R of the present application" in this application), which is an aqueous solution of N,N-dimethylbiguanide, wherein the aqueous solution does not contain an acidic pH-adjusting agent required to meet cell-culture requirements or conventional injection safety requirements, and the concentration of the single agent N,N-dimethylbiguanide solution {[W dimethylbiguanide/(W dimethylbiguanide + W solvent)]%} is ≥ its intervention concentration, wherein the intervention concentration is 0.5% ≤ C ≤ the maximum solubility concentration of N,N-dimethylbiguanide. In fact, the single agent R of the present application is a medicament whose interventional form is the single agent R liquid of the present application.

The present invention provides a dimethylbiguanide single agent R pharmaceutical dosage form (single agent R scheme 4) (referred to as "single agent R dosage form of the present application " in this application), which is an aqueous solution of N,N-dimethylbiguanide, wherein the aqueous solution does not contain an acidic pH-adjusting agent required to meet cell-culture requirements or conventional injection safety requirements, and the concentration of the N,N-dimethylbiguanide Single agent R liquid {[W dimethylbiguanide/(W dimethylbiguanide + W solvent)]%} is ≥ its intervention concentration, wherein the intervention concentration is 0.5% ≤ C ≤ the maximum solubility concentration (saturation concentration) of N,N-dimethylbiguanide. In fact, single agent R dosage form of the present application is a dosage form for facilitating the intervention of the medicament R of the present application into a lesion in the form of liquid of the present application.

In the above-mentioned formulation, liquid, medicament, and/or pharmaceuticaldosage form, the intervention concentration (w/w) is 0.5% ≤ C ≤ 51%, preferably 1% - 50%, more preferably 3% - 50%, 3% - 40%, 5% - 50%, 5% - 35%, or 5% - 30%.

The above-mentioned formulation, liquid, medicament, and pharmaceutical dosage form is used for interventional treatment of lesions such as tumors or nodules in subjects.

In the above-mentioned formulation, the formulation contains a solvent, preferably the solvent is water, and more preferably water for injection.

In the above-mentioned formulation, liquid, medicament, and/or pharmaceutical dosage form, it does not include salts of N,N-dimethylbiguanide.

In the above-mentioned formulation, liquid, medicament, and/or pharmaceutical dosage form, the administration or intervention concentration (w/w) of dimethylbiguanide is ≤ 51%, ≤ 50%, ≤ 40%, ≤ 30%, ≤ 25%, ≤ 20%, ≤ 10%, ≤ 5%, or ≤ 2% such as 1%, 0.75%, or 0.5%.

In the above-mentioned formulation, liquid, medicament, and/or pharmaceutical formulation, the administration or intervention concentration (w/w) of dimethylbiguanide (R) is 0.2% - 51%, 0.5 - 50%, 1% - 40%, 5% - 50%, 5% - 40%, 5% - 30%, 5% - 25%, 5% - 20%, 1% - 30%, 1% - 25%, 1% - 20%, 0.5% - 10%, 1% - 5%, or ≤ 2% such as 1%, 0.75%, or 0.5%.

In the above-mentioned formulation, liquid, medicament, and/or pharmaceutical dosage form, the acidic pH adjusting agent includes or is selected from hydrochloric acid, sulfuric acid, acetic acid, citric acid, phosphoric acid, malic acid, tartaric acid, lactic acid, and carbonic acid.

In this application, the above-mentioned formulation, liquid, medicament, or the dosage form of the application is referred to as "single agent R of the present application" (single agent R scheme).

The single agent R of the present application is an interventional dedicated product and is not a general injection agent. It is even strictly prohibited for conventional injection (for example, it must be stated in the instructions for use).

According to one embodiment, the single agent R liquid in the single agent R of the present application is a liquid for providing a transient effect of dimethylbiguanide.

According to one embodiment, the single agent R dosage form of the present application is a low-frequency and rapid-acting interventional dedicated dosage form. The existing dosage form of dimethylbiguanide medicaments is normal frequency and normal acting dosage form mainly for frequent administration and normal acting treatment, and the existing anti-pathological effects of metformin, such as anti-cancer response, cannot provide low-frequency and rapid-acting effects, and can only provide high-frequency and normal-acting efficacy at most. In contrast, the low-frequency and rapid-acting interventional dedicated dosage form of the present application is strictly prohibited for highly frequent injection (for example, it must be stated in the instructions for use) and is not a normal frequency and normal acting dosage form for injection. The former must provide the low-frequency administration and rapid-acting property that the latter cannot anticipate. The differences between the two further expand on the basis of the above structure-effect characteristics: the latter must provide high frequency and normal acting efficacy including highly frequent administration and the beneficial and safe efficacy that appear after a long time, which must be implemented by a structural scheme that further ssatisfies the safety of highly frequent long-term conventional injection (for example, minimizing local irritation). The former must provide low-frequency and rapid effectiveness, which can only be achieved by a structural scheme that deviates more from the conventional injection safety requirements (1.5% ≤ CA < 10%).

According to one embodiment, the low-frequency and rapid-acting interventional dedicated dosage form of the present application is further applicable to patients who are not suitable for high-frequency intervention and normal acting treatment on the basis of the indications of the single agent R dosage form of the present application, such as patients with poor interventional compliance but in urgent need of therapeutic effects (unsuitable for no significant therapeutic effect after a long time, such as rapid disease progression or palliative therapy).

According to one embodiment, the single agent R dosage form of the present application is an interventional dedicated powder injection, which contains sterile powder and its solvent required for preparing the pharmaceutical solution of the present application, wherein the sterile powder contains N,N-dimethylbiguanide and is contained in a container filled with inert gas; the solvent includes pharmaceutically acceptable carriers such as solvents or solvents, and the solvent is a reagent for dissolving N,N-dimethylbiguanide, such as water for injection, and has a volume capable of dissolving N,N-dimethylbiguanide to the concentration of the single agent R liquid of the present application. The dimethylbiguanide powder injection of the present invention is strictly prohibited from being used as a conventional powder injection.

According to another aspect of the present invention, the present invention provides the use of N,N-dimethylbiguanide and its single agent liquid (single agent R scheme 2), spceifically:
The present invention provides the use of the pharmaceutical solution of the present application as a transient agent in the preparation of a medicament or dosage form of the present application (referred to as " use of single agent R liquid of the present application" in the present application), wherein the liquid is used to provide a transient effect of N,N-dimethylbiguanide on a lesion tissue and the cells contained therein.

The present invention provides a use of N,N-dimethylbiguanide as a transient active ingredient in the preparation of lesion interventional dedicated liquids, dedicated medicaments, or dedicated dosage forms (referred to as "use of dimethylbiguanide single agent of the present application" in the present application), wherein the N,N-dimethylbiguanide is used to provide a transient effect on the lesion tissue and the cells contained therein.

The present invention provides a method for treating cells within a lesion (referred to as "the single agent R cell treatment method of the present application"), including the following steps: introducing the N,N-dimethylbiguanide interventional dedicated liquid into a lesion or contacting a lesion with the N,N-dimethylbiguanide of the present invention, wherein the N,N-dimethylbiguanide is used as a transient active ingredient to provide a transient effect on a lesion tissue and the cells contained therein.

The present invention provides a method for treating a lesion (referred to as "the single agent R lesion treatment method of the present application"), including the following steps: introducing N,N-dimethylbiguanide interventional dedicated liquid into a lesion or contacting a lesion with the N,N-dimethylbiguanide of the present invention or administrating N,N-dimethylbiguanide to the lesion, wherein the N,N-dimethylbiguanide is used as a transient active ingredient to provide a transient effect on the lesion and the cells contained therein. In fact, the lesion treatment method of the present application includes the cell treatment method of the present application.

The present invention provides a method for treating a lesion disease (referred to as "the single agent R disease treatment method 1 of the present application"), including the following steps: introducing N,N-dimethylbiguanide interventional dedicated liquid into a lesion or contacting a lesion with the N,N-dimethylbiguanide of the present invention or administrating N,N-dimethylbiguanide to a lesion, wherein the N,N-dimethylbiguanide is used as a transient active ingredient to provide a transient effect on the lesion and the cells contained therein. In fact, the single agent R disease treatment method of the present application includes the single agent R lesion treatment method of the present application.

The present invention provides a method for treating a lesion such as a tumor or nodule in a subject (referred to as "the single agent R disease treatment method 2 of the present application"), including intervening or administrating the above-mentioned single agent R or formulation of the present application to the subject.

In some embodiments, the intervention or administration can be systemic or local. Preferably, the administration or intervention may include intratumoral administration or intervention, preferably local intratumoral intervention administration, preferably such as local injection administration or intervention into the tumor or tumor microenvironment.

In some embodiments, the injection administration may include infusion, liquid particle injection or implantation.

In some preferred embodiments, the above-mentioned formulation of the present invention is suitable for application in micro-volume administration or intervention to the micro-volume administration of reducing (Y) lesion (X), and a ratio of the administration volume to the target volume (V_{administration}/V_{target}) is approximately 0.02-0.056, 0.02-0.10 or 0.02-0.34.

In some embodiments, the above-mentioned contact or intervention or administration includes multi-point contact or intervention or administration, preferably local multi-point contact or intervention.

The above-mentioned contact or administration may include multi-(site) point contact or administration or intervention, where the multi-point refers to the number of micro-volume intervention points observed to have an effective total target area pharmaceutical efficacy evaluation being significantly higher than that of the conventional metformin drug. Specifically, the multi-point, for example, multiple sites at a target area, such as ≥5, ≥10, ≥20, ≥30, or ≥50 sites, for example, at least 6, at least 7, at least 8, at least 9, at least 10, at least 11, at least 12, at least 13, at least 14, at least 15 or more sites, for example, 10 to 20, 10-30, 10-40, 10-50, 20-30, 20-40, 20-50, 25-50, 30-50 or more sites. Preferably, the contact points or administration sites or intervention points are uniformly distributed, especially to areas where conventional intervention cannot diffuse.

In some preferred embodiments, when the lesion is ≥ about 3.5 cm³ or the maximum size is ≥ about 2 cm, the multi-(site) point is ≥ 5 sites, for example, 10, 15, 20, 25, 30, 35 or 50 sites.

In some preferred embodiments, the above-mentioned contact or administration or intervention may include micro-volume multi-(site) point contact or administration or intervention, preferably micro-volume multi-point injection. The micro-volume, for lesions larger than 3 cm, includes a volume of 5 µL - 1000 µL, for example, 5 µL - 500 µL, 5 µL - 250 µL, 5 µL - 200 µL, 5 µL - 100 µL, 5 µL - 75 µL, 5 µL - 50 µL, 5 µL - 25 µL, 5 µL - 20 µL, 10 µL - 200 µL, 10 µL - 100 µL, 10 µL - 75 µL, 20 µL - 200 µL, 20 µL - 100 µL, 20 µL - 75 µL, 30 µL - 200 µL, 30 µL - 100 µL, 40 µL - 200 µL, 40 µL - 100 µL, 50 µL - 200 µL, 50 µL - 100 µL, or 50 µL - 75 µL, and the like. Preferably, micro-volume multi-(site) point contact or administration, more preferably, micro-volume multi-(site) point injection.

In some embodiments, the above-mentioned treatment can last for at least one course. The duration of one course can be approximately 7 to 40 days, for example, about 9 to about 37 days, for instance, 15, 20, 25, 27, 28, 29, 30, 31, 32, 33, 34, or 35 days.

In one embodiment, in one course, the contact or administration of the formulation of the present invention can be at a low frequency. The low frequency, for example, about 1-6 times per course, preferably 1-5 times per course, 1-4 times per course, 1-3 times per course, or 1-2 times per course.

In the present application, the use of the single agent R liquid of the present application or the use of the single agent dimethylbiguanide of the present application is briefly referred to as the single agent R use of the present application; the single agent R cell treatment method, the single agent R lesion treatment method, or the single agent R disease treatment method of the present application is briefly referred to as the single agent R treatment method of the present application; the single agent R use or the single agent R treatment method of the present application is briefly referred to as the single agent R use (or method) of the present application; the transient effect provided by N,N-dimethylbiguanide on a lesion tissue and the cells contained therein is briefly referred to as the single agent R effect of the present application.

In the above single agent R use or the single agent R cell treatment method, the single agent R lesion treatment method, or the single agent R disease treatment method of the present application, the lesion includes malignant cells and/or non-malignant cells.

In the above uses and methods of the present invention, the lesion is a lesion that is ineffective or invalid to conventional treatment, including at least one of the following groups: a tumor that is ineffective or invalid to conventional treatment; a non-malignant nodule.

In the above uses and methods of the present invention, the tumor that is ineffective or invalid to conventional treatment includes at least one selected from the following groups: a tumor resistant to chemotherapeutic drugs, a tumor with a high tumor stromal ratio, a tumor that is ineffective or invalid to conventional chemotherapeutic drugs, a patient who is unfavorable for chemotherapy.

In the above uses and methods of the present invention, the non-malignant nodule is a nodule including at least one of the following non-malignant cells: connective tissue cells, secretory gland cells, epithelial cells.

In the above uses and methods of the present invention, the lesion contains a tumor of non-drug resistant malignant tumor cells.

In the above uses and methods of the present invention, the malignant tumor cells are tumor cells that are not specific to the cytotoxicity of dimethylbiguanide.

According to one embodiment, the liquid in the use (or method) of single agent R of the present application is the single agent R liquid of the present application.

According to one embodiment, the single agent R liquid of the present application is the single agent R liquid in the use (or method) of single agent R of the present application.

According to one embodiment, the transient effect in the use (or method) of single agent R of the present application includes transient tissue penetration effect and transient cell deformation effect.

According to one embodiment, the cell deformation includes effective cell deformation.

In this application, the term "cell deformation" refers to an observable change in the morphology (e.g., volume) of the cell, which includes reversible and irreversible deformation, where the irreversible deformation can develop into necrosis; the term "effective cell deformation" refers to cell deformation that can effectively cause the necrosis of the tissue composed of the cells.

According to one embodiment, in the above method of the present application, the dosage selection schemeof the single agent R liquid is: the volume of the liquid based on the lesion volume × the transient effective intervention concentration.

According to one embodiment, in the above method of the present application, the concentration selection scheme of the single agent R liquid is: ≥ the transient effective intervention concentration.

According to one embodiment, in the above method of the present application, the frequency selection scheme of the use of single agent R liquid is: the number of times required for transient treatment not reaching the lesion.

The single agent R liquid used in the method of the present application breaks through or even deviates from the existing methods of R'. The application methods of the former and latter pharmaceutical solution is closed (interventional) and open (any injection method that can form medicament-containing blood, preferably absorption injection) respectively, and the dosage selection schemes are (the volume of the pharmaceutical solution based on the lesion volume × transient effective intervention concentration) and (the patient's blood volume or body weight or surface area × continuous effective plasma drug concentration), respectively, and the concentration selection schemes of pharmaceutical solution are ≥ transient effective intervention concentration and (safety requirements) preferably lower concentration, respectively, and the use frequency selection schemes of pharmaceutical solution are (the number of times required for transient treatment not reaching the lesion) and (dozens to hundreds of times as required by the half-life of dimethylbiguanide and continuous effective plasma drug concentration) respectively.

The above technical solutions in the method of present application have produced technical effects exceeding the expectations of the existing methods, such as rapid effect, single agent effectiveness, single or low-frequency effectiveness, effectiveness for lesions that are ineffective or intolerant to existing metformin drug, and the like.

According to one embodiment, in the pharmaceutical solution of single agent R or formulation or the use (or method) of the present application, the concentration (C_{R}) of N,N-dimethylbiguanide is 1.0% ≤ CR ≤ the maximum solubility concentration (or saturation concentration) of N,N-dimethylbiguanide.

According to one embodiment, in the pharmaceutical solution of single agent R or single agent R formulation or the use (or method) of single agent R of the present application, the concentration (C_{R}) of N,N-dimethylbiguanide is 3.0% ≤ C_{R} ≤ the maximum solubility concentration (or saturation concentration) of N,N-dimethylbiguanide.

According to one embodiment, in the pharmaceutical solution of single agent R or the use (or method) of single agent R of the present application, the concentration (C_{R}) of N,N-dimethylbiguanide is 5.0% ≤ CR ≤ the maximum solubility concentration (or saturation concentration) of N,N-dimethylbiguanide.

According to one embodiment, the single agent R or the use (or method) of single agent R of the present application is the rapid-acting pharmaceutical solution, medication, dosage form, use, or method of N,N-dimethylbiguanide.

According to one embodiment, the single agent R or the use (or method) of single agent R of the present application is the low-frequency administration liquid, medicament, dosage form, use, or method of N,N-dimethylbiguanide. The medicament or use (or method) of the present application

According to one embodiment, the indication [(Y, X), hereinafter referred to as the indications of the present application] of the administration of the single agent R or the use (or method) of single agent R of the present application is suitable for treating (Y) lesion diseases or lesion symptoms (X), where the lesion contains malignant cells and/or non-malignant cells.

According to one embodiment, in the indications of the application, X is the volume of the lesion, and Y is effective or rapid reduction.

According to one embodiment, in the indications of the application, the lesion is a lesion that is effective or effective but not rapid or low-frequency effective and/or micro-volume interventional effective for the conventional action of dimethylbiguanide.

According to one embodiment, in the indications of the application, the lesion is a lesion that is ineffective when used alone but can be used as an adjuvant treatment for the conventional action of dimethylbiguanide.

According to one embodiment, in the indications of the application, the lesion is a lesion that is ineffective for the conventional action of dimethylbiguanide.

According to one embodiment, in the indications of the application, the lesion is a lesion that is ineffective or invalid for the conventional action.

According to one embodiment, in the indications of the application, the lesion that is ineffective or invalid for the conventional action includes or is selected from at least one of the following groups: tumors refractory to conventional chemotherapeutic drug, non-malignant nodules. According to one embodiment, in the indications of the application, the tumors refractory to the conventional chemotherapeutic drug includes or is selected from at least one from the following group: drug-resistant tumors, tumors with unfavorable microenvironments, tumors after medication withdrawal.

According to one embodiment, in the indications of the application, the drug-resistant tumor is a tumor containing drug-resistant malignant tumor cells.

According to one embodiment, in the indications of the application, the drug-resistant tumor is a tumor containing primary drug-resistant cells.

According to one embodiment, in the indications of the application, the drug-resistant tumor is a tumor containing a drug-resistant microenvironment.

According to one embodiment, in the indications of the application, the tumor containing a drug-resistant microenvironment is a tumor containing an unfavorable microenvironment.

According to one embodiment, in the indications of the application, the tumor containing an unfavorable microenvironment is a tumor in which a ratio of non-malignant cells to malignant cells is ≥ 43%.

According to one embodiment, in the indications of the application, the ineffective or failed tumor after chemotherapy is a tumor that is ineffective or failed after treatment with recognized first-line or second-line chemotherapeutic drugs.

According to one embodiment, in the indications of the application, the non-malignant nodules include or are selected from nodules of at least one of the following non-malignant cell groups: connective tissue cells, secretory gland cells, and epithelial cells.

According to one embodiment, in the indications of the application, the connective tissue cells include or are selected from at least one of the following: fibroblasts, fat cells, and macrophages.

According to one embodiment, in the indications of the application, the secretory gland cells include or are selected from at least one of the following: breast cells, thyroid cells, and prostate cells.

According to one embodiment, in the indications of the application, the epithelial cells include or are selected from keratinocytes.

According to one embodiment, in the indications of the application, the lesion includes or is selected from a tumor containing sensitive tumor cells.

According to one embodiment, in the indications of the application, the malignant tumor cells include or are selected from tumor cells that are not specific to the cytotoxicity of dimethylbiguanide.

According to one embodiment, in the indications of the application, the tumor containing sensitive tumor cells is a tumor that cannot be effectively treated by conventional metformin drug, especially a tumor that requires rapid-acting.

The medicament of the present application or the use (or method) of the present application can provide technical effects that cannot be achieved by conventional metformin drug or uses (or methods), including the type of action (transient action vs. sustained action), the speed of action (rapid vs. long-lasting), and the required administration method (low frequency vs. high frequency), to meet the treatment needs that cannot be met by its existing uses (such as high efficacy, even rapid effect).

According to one embodiment, in the indications of the application, the conventional tumor is a tumor that cannot or no longer can be effectively treated by conventional chemotherapeutic drugs.

According to one embodiment, in the indications of the application, the X is a disease or symptom of the lesion that is not suitable or no longer suitable to conventional treatment.

According to one embodiment, in the indications of the application, the X is a disease or symptom of the lesion that requires rapid treatment (unsuitable for no significant effect after a long time, such as rapid disease progression or palliative therapy).

According to one embodiment, in the indications of the application, the Y is a treatment produced by transient effect on a lesion tissue and the cells contained therein by the N,N-dimethylbiguanide liquid, especially rapid treatment.

Within the scope of the present invention, the term "tumor" refers to a disease with a tumor as a symptom, which can be any pathological (malignant and non-malignant) tumor at any stage, including, for example, the following groups classified by tumor cell type: epithelial cell tumors, sarcomas, lymphoid tumors, germ cell tumors, embryonic cell tumors; and including tumors named according to the organ or tissue where the tumor cells are concentrated, including, for example, tumors named according to the following organs or tissues: skin, bone, muscle, breast, kidney, liver, lung, gallbladder, pancreas, brain, esophagus, bladder, large intestine, small intestine, spleen, stomach, prostate, testis, ovary or uterus.

Specifically, the malignant tumors include, for example, breast cancer, pancreatic cancer, thyroid cancer, nasopharyngeal cancer, prostate cancer, liver cancer, lung cancer, intestinal cancer, oral cancer, esophageal cancer, gastric cancer, laryngeal cancer, testicular cancer, vaginal cancer, uterine cancer, ovarian cancer, sarcoma, and the like.

The non-malignant tumors include, for example, breast tumors, pancreatic tumors, thyroid tumors, prostate tumors, liver tumors, lung tumors, intestinal tumors, oral tumors, esophageal tumors, gastric tumors, nasopharyngeal tumors, laryngeal tumors, testicular tumors, vaginal tumors, uterine tumors, fallopian tube tumors, ovarian tumors, and the like.

According to one embodiment, the malignant cells include or are selected from conventional malignant cells that are ineffective with conventional metformin liquids alone, such as cancer cells other than liver cancer cells, and sarcoma cells.

According to one embodiment, the non-malignant cells include or are selected from at least one of connective tissue cells and organ tissue cells.

According to one embodiment, the connective tissue cells include fibroblasts.

According to one embodiment, the organ tissue cells include or are selected from at least one of the following: liver cells, breast cells, thyroid cells, skin cells.

According to one embodiment, the non-malignant lesions in the indications of the present application include or are selected from at least one of the following groups: benign tumors, nodules.

In the present application, the term "nodule" refers to benign lesions other than benign tumors, including, for example, hyperplasia (such as hyperplasia of the breast, thyroid, parathyroid, prostate, and the like), cysts, abnormal venous masses (such as hemorrhoids, among others.), local inflammatory swelling, microbial infection swelling, etc. The hemorrhoids include internal hemorrhoids, external hemorrhoids, and mixed hemorrhoids.

In one embodiment, the benign lesions include local inflammation, especially refractory inflammation. Within the scope of the present invention, the term "local inflammation" refers to non-tumorous inflammation in a local area, including, for example, alterative inflammation, exudative inflammation, and proliferative inflammation, which can be any suitable one known to those skilled in the art, such as including one or more of the following: arthritis, mastitis, pancreatitis, thyroiditis, prostatitis, hepatitis, pneumonia, enteritis, stomatitis, pharyngitis, periodontitis, esophagitis, gastritis, gastric ulcers, rhinitis, sinusitis, laryngitis, tracheitis, bronchitis, vaginitis, metritis, salpingitis, oophoritis, among others.

In one embodiment, the benign lesions include skin diseases, especially refractory skin diseases. Within the scope of the present invention, the term "skin disease" refers to lesions that are primary or secondary to the skin or skin appendages, which can be any suitable one known to those skilled in the art, such as including one or more of the following: skin cancer, non-malignant skin tumors, viral skin diseases (such as herpes, warts, rubella, hand-foot-mouth disease), bacterial skin diseases (such as impetigo, furuncles, leprosy), fungal skin diseases (such as various tinea), sexually transmitted diseases (such as syphilis, gonorrhea and condyloma acuminatum), allergic and autoimmune skin diseases (such as contact dermatitis, eczema, urticaria), physical skin diseases (such as solar skin diseases, chilblains, corns, hand-foot fissures, pressure sores), connective tissue diseases (such as lupus erythematosus), pigmentary disorders skin diseases (such as freckles, pigmented nevi, various macules), skin appendage diseases (such as acne, rosacea, seborrheic dermatitis, alopecia areata, alopecia, hyperhidrosis and bromhidrosis).

The present invention provides a pharmaceutical combination, the pharmaceutical combination comprising dimethylbiguanide (R) and component (Z), wherein the administration concentration (w/w) of the dimethylbiguanide is 0.2% - 51%, preferably 0.5% - 50%, and the component includes a cell-reactive antineoplastic agent (abbreviated as B).

In some embodiments, the pharmaceutical combination can be used to treat lesions, such as tumors or nodules in a subject.

In some embodiments, in the pharmaceutical combination, the tumor is a solid tumor suitable for tumor administration.

In some embodiments, in the above pharmaceutical combination, R provides specific activities that conventional metformin (with dimethylbiguanide salt as the active ingredient, abbreviated as R') is difficult to provide, including a specific effect of effectively reducing the function of biological barriers (abbreviated as activity A), through the following definitions:
1). tumor administration;
2).the administration amount is: ${Q}_{R} = {\sum }_{i = 1}^{nR} {q}_{R \left(i\right)}$,
   wherein n_{R} is the number of times of tumor administraton in one course of treatment, qR(i) is the amount of R in the i-th (i = 1,..., n_{R}) administration, and: ${q}_{R \left(i\right)} = {\sum }_{i i - 1}^{n R i} {c}_{R \left(ii\right)} \times {v}_{R \left(ii\right)} ,$
   wherein n_{Ri} is the number of needle insertion points during the i-th administration of R, c_{R(ii)} and v_{R(ii)} are the concentration and volume of R administered at the ii-th needle insertion point, respectively, wherein the concentration threshold of c_{R(ii)} (W/W) is 0.1% or 0.2%, with an upper limit of concentration of 51%, and the volume threshold of v_{R(ii)} is 1/800 or 1/300 of a target volume (denoted as V_{target(ii))}, with an upper limit of volume of 3 times of V_{target(ii)};

- one of key control steps provided by Z through the following definitions is the specific antineoplastic effect (denoted as activity B) of crossing the tumor tissue barrier:
   1). intratumoral administration and/or systemic administration;
   2). the administration amount is: ${Q}_{Z} = {\sum }_{j - l}^{nB} {q}_{Z \left(j\right)} + {\sum }_{i ′ - l}^{nB ′} {c}_{Z \left(j′\right)} \times {v}_{Z \left(j′\right)} ,$,
   wherein n_{Z} and q_{B(j)} are the number of systemic administrations of Z in one course of treatment and the amount in the j-th (j = 1,..., n_{B}) administration, respectively, and n_{Z'}, c_{Z(j')}, and v_{Z(j')} are the number of tumor administrations of Z in one course of treatment, the concentration and volume in the j'-th (j' = 1,..., n_{Z'}) administration, respectively;
- the type of the combination is a synergistic combination where the pharmaceutical effects of activity A of R and activity B of B enhance each other.

In some embodiments, in the above pharmaceutical combination, the N,N-dimethylbiguanide (R) comprises < 30%, preferably ≤ 5% of a salt of dimethylbiguanide (R'), or does not comprise a salt of dimethylbiguanide (R'), and preferably, N,N-dimethylbiguanide (R) is in a form of a powder injection.

In some embodiments, in the above pharmaceutical combination, the tumor is selected from a tumor where the tissue barrier function is not conducive to the inhibitory effect of Z on tumor cells, preferably from refractory tumors of Z, including chemotherapy drug-resistant tumors, tumors with unfavorable microenvironments, tumors after withdrawl of antineoplastic agents, tumors without effective chemotherapeutic drugs, and tumors in patients with contraindication to or unsuitability for conventional chemotherapeutic drugs.

In some embodiments, in the above pharmaceutical combination, the tumor is selected from heterogeneous tumors, where the heterogeneity includes at least one of tissue heterogeneity, spatial heterogeneity, and safety heterogeneity, and the heterogeneous tumor is a tumor where different tumor bodies of the same type or different regions of the same tumor body are set as different target areas for differential administration based on the heterogeneity, which is beneficial for treatment.

In some embodiments, in the above pharmaceutical combination, the component (Z) includes antineoplastic agents, which are selected from cell-reactive drugs one of whose key control steps is crossing the tumor tissue barrier.

In some embodiments, in the above pharmaceutical combination, the cell-reactive drugs include cytotoxic drugs and targeted antineoplastic agents.

In some embodiments, in the above pharmaceutical combination, the cytotoxic drugs include DNA damaging agents, antimetabolites, microtubule inhibitors, and topoisomerase inhibitors.

In some embodiments, in the above pharmaceutical combination, representative compounds of the DNA damaging agents include cisplatin, carboplatin, oxaliplatin, ifosfamide, and doxorubicin.

In some embodiments, in the above pharmaceutical combination, representative compounds of the antimetabolites include fluorouracil, gemcitabine, and methotrexate.

In some embodiments, in the above pharmaceutical combination, representative compounds of the microtubule inhibitors include paclitaxel and vincristine.

In some embodiments, in the above-mentioned pharmaceutical combination, the agent includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin, mitoxantrone, gefitinib, osimertinib, larotrectinib, pazopanib, trastuzumab, and derivatives thereof.

In some embodiments, in the pharmaceutical combination, the administration concentration (w/w) of dimethylbiguanide (R) can be 0.2%-51%, 0.2%-50%, 0.5%-50%, 0.5%-45%, 1%-45%, 5%-45%, 5%-40%, 5%-30%, 5%-25%, 5%-20%, 1%-30%, 1%-25%, 1%-20%, 0.5%-10%, 1%-5%, or ≤2% such as 1%, 0.75% or 0.5%.

In some embodiments, in the above-mentioned pharmaceutical combination, the dimethylbiguanide (R) and the component (Z) can be administered simultaneously or sequentially, preferably administered at certain time intervals.

In some embodiments, in the above-mentioned pharmaceutical combination, the dimethylbiguanide (R) and the component (Z) can be administered intratumorally, or the dimethylbiguanide (R) is administered intratumorally and a portion of the component (Z) is administered intratumorally, while the other portion of the component (Z) is administered systemically, for example, intraperitoneally, or the dimethylbiguanide (R) is administered intratumorally and the component (Z) is administered systemically, for example, intraperitoneally.

In some embodiments, in the above-mentioned pharmaceutical combination, the dimethylbiguanide (R) and the component (Z) can be administered intratumorally simultaneously or sequentially, or the dimethylbiguanide (R) and a portion of the component (Z) can be administered intratumorally simultaneously or sequentially, while the other portion of the component (Z) is administered systemically, for example, intraperitoneally simultaneously or sequentially, or the dimethylbiguanide (R) is administered intratumorally simultaneously or sequentially and the component (Z) is administered systemically, for example, intraperitoneally or via the gastrointestinal tract.

In some embodiments, in the above-mentioned pharmaceutical combination, the dimethylbiguanide (R) and the component (Z) can be administered intratumorally sequentially, or the dimethylbiguanide (R) and a portion of the component (Z) can be administered intratumorally sequentially, while the other portion of the component (Z) is administered systemically sequentially, for example, intraperitoneally, or the component (Z) is administered systemically, for example, intraperitoneally, before or after the dimethylbiguanide (R) is administered intratumorally.

In some embodiments, in the above-mentioned pharmaceutical combination, the dimethylbiguanide (R) and the component (Z) are both administered intratumorally (combination relationship I); the R and Z are administered intratumorally and systemically, respectively (combination relationship II); or the R and a portion of Z are administered intratumorally, and the other portion of Z is administered systemically (combination relationship III).

The present invention also provides a pharmaceutical combination, which comprises formulation I and formulation II, wherein the formulation I comprises N,N-dimethylbiguanide (R) or N,N-dimethylbiguanide (R) and component (Z), and the formulation II contains Z but not R, wherein:
1) in the formulation I, the N,N-dimethylbiguanide (R) comprises ≤5% or does not include a salt of N,N-dimethylbiguanide;
2) the coadministration methods of the formulation I and the formulation II are:
   Formulation I is only used for intratumoral administration and cannot be used for systemic administration,
   Formulation II can be used for intratumoral administration and/or systemic administration;
3) the coadministration amount of the formulation I and the formulation II is limited by the coadministration amountof Z and R:
   (1). the administration amount of R is: ${Q}_{R} = {\sum }_{i = 1}^{nR} {q}_{R \left(i\right)}$,
      wherein n_{R} is the number of intratumoral administrations of R in one course of treatment, q_{R(i)} is the amount of R in the i-th (i = 1,..., n_{R}) administration, and: ${q}_{R \left(i\right)} = {\sum }_{i i - 1}^{n R i} {c}_{R \left(ii\right)} \times {v}_{R \left(ii\right)} ,$
      wherein n_{Ri} is the number of administration points, for example, needle insertion points, of R in the i-th administration, c_{R(ii)} and v_{R(ii)} are the concentration and volume of R administered at the ii-th administration point, for example, needle insertion point, respectively, and v_{R(ii)} ≤ 3 times of the target volume (denoted as v_{target(ii)}) of the ii-th administration point;
   (2). the administration amount of Z is: ${Q}_{Z} = {\sum }_{k = 1}^{n Z} {q}_{Z \left(k′\right)} + {\sum }_{k ′ = 1}^{n Z ′} {c}_{Z \left(k′\right)} \times {v}_{Z \left(k′\right)}$
      wherein n_{Z} and q_{Z(j)} are the number of systemic administrations of Z in one course of treatment and the administration amount of Z in the j-th (j = 1,..., nB) administration, respectively, and n_{Z'}, c_{Z(j')}, and v_{Z(j')} are the number of intratumoral administrations of Z in one course of treatment, the concentration and volume of Z administered at the j'-th (j' = 1,..., n_{Z'}) administration, respectively.

In some embodiments, in the above-mentioned pharmaceutical combination, the component Z includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin, mitoxantrone, gefitinib, osimertinib, larotrectinib, pazopanib, trastuzumab, and derivatives thereof.

In some embodiments, in the above-mentioned pharmaceutical combination, the medicament C includes or is selected from at least one of the following: sodium chloride, sodium bicarbonate, sodium dihydrogen phosphate, sodium lactate, sodium acetate.

In some embodiments, in the above-mentioned pharmaceutical combination, the medicament E includes or is selected from at least one of the following: biological macromolecule immunomodulators such as immune checkpoint inhibitors, cytokines, and TLR agonists; immune cell immunomodulators; and vaccine immunomodulators such as BCG.

In some embodiments, in the above-mentioned pharmaceutical combination, the mixing contraindications of R are any one of the following groups:
1) a salt of N,N-dimethylbiguanide (R');
2) acidifying agents that significantly convert R to R', where the acidifying agents include acidic pH adjusting agents and other acidic substances;
3) conventional dimethylbiguanide pharmaceutical excipients with a dosage ratio (Q excipient/QR) of >3.0 or >4.0, including their conventional sustained-release dosage form carriers, such as gel carriers and nano-carriers;
4) excipients unstable in strong alkaline aqueous solutions, including, for example, reducing sugars, cellulose, liposomes, carbomers, and polyethylene glycol-modified nano-carriers,
where the mixing contraindications refer to the need to avoid mixing with them throughout the entire process of formulation, preparation, product, and use.

In some embodiments, in the above-mentioned pharmaceutical combination, the coadministration methods include:
1) R and Z are mixed for intratumoral administration,
2) R and Z are administered intratumorally separately,
3) R and Z are administered intratumorally and systemically, respectively,
4) R and a portion of Z are mixed or administered intratumorally separately, while the other portion of Z is administered systemically.

In some embodiments, in the above-mentioned pharmaceutical combination, the coadministration threshold is 0.5%, 1%, 3.3%, 5%, or 10%.

According to the present invention, a pharmaceutical composition (the composition of the present invention) comprising any of the above-mentioned pharmaceutical combinations of the present invention and a formulation (referred to as the R combination formulations of the present invention) comprising any of the above-mentioned pharmaceutical combination or composition of the present invention are provided.

According to the present invention, a use of N,N-dimethylbiguanide (R) and component (Z) for medicaments, pharmaceutical combinations, or kits for treating tumors or nodules (referred to as the R combination therapeutic use 1 of the present invention) is provided.

According to the present invention, a use of the above-mentioned pharmaceutical combination or composition of the present invention in the preparation of medicaments, agent combinations, or kits for treating lesions such as tumors or nodules in a subject (referred to as the R combination therapeutic use 2 of the present invention) is provided.

In some embodiments, in the above-mentioned R combination therapeutic uses of the present invention, the administration concentration of dimethylbiguanide (w/w) is ≤51%, ≤50%, ≤40%, ≤30%, ≤25%, ≤20%, preferably ≤10%, preferably ≤5%, more preferably ≤2% such as 1%, 0.75%, or 0.5%.

In some embodiments, the administration concentration (w/w) of dimethylbiguanide (R) in the above-mentioned combination therapeutic uses of the present invention is 0.2% - 51%, 0.5% - 50%, 1% - 40%, 5% - 50%, 5% - 40%, 5% - 30%, 5% - 25%, 5% - 20%, 1% - 30%, 1% - 25%, 1% - 20%, preferably 0.5% - 10%, preferably 1% - 5%, more preferably ≤ 2%, such as 1%, 0.75%, or 0.5%.

In some embodiments, the concentration (w/w) of N,N-dimethylbiguanide salt in the above-mentioned combination therapeutic uses of the present invention is ≤ 30%, preferably ≤ 20%, preferably ≤ 10%, more preferably ≤ 5%, ≤ 4%, or ≤ 3%, more preferably ≤ 2%, particularly preferably ≤ 1%, such as 1% or 0.5%, most preferably 0%, that is, the R of the pharmaceutical combination does not contain a salt of N,N-dimethylbiguanide.

In some embodiments, the R composition of the present invention comprises N,N-dimethylbiguanide and optionally a solvent, and the composition does not comprise additional agent(s) required for cell culture or the safety of conventional injection. The concentration of N,N-dimethylbiguanide contained in the composition is ≥ its intervention concentration, where the administration or intervention concentration (w/w) is 0.5% ≤ CA ≤ 51%, preferably 1% - 51%, 2% - 50%, 3% - 50%, 4% - 50%, 5% - 50%, 6% - 50%, 7% - 50%, 8% - 50%, 9% - 50%, 10% - 50%, 15% - 50%, 20% - 50%, 25% - 50%, 30% - 50%, 35% - 50%, 40% - 50%, 1% - 40%, 5% - 50%, 10% - 40%, 15% - 40%, 20% - 40%, 25% - 40%, 30% - 40%, 35% - 40%, or 1% - 35%, 5% - 30%, etc., or any value and range in between, such as 1.5%, 2%, 3%, 3.5%, 4%, 4.5%, 5%, 5.5%, 6%, 6.5%, 7.7.5%, 8.8.5%, 9.9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, 21%, 21.5%, 22%, 22.5%, 23%, 23.5%, 24%, 24.5%, 25%, 26%, 27%, 28%, 29%, 30%, 31%, 32%, 33%, 34%, 35%, 36%, 37%, 38%, 39%, 40%, 45%, 50%.

In some embodiments, the composition comprises a solvent, such as a pharmaceutically acceptable carrier, preferably water.

In some embodiments, the composition does not include a salt of N,N-dimethylbiguanide.

In some embodiments, the concentration (w/w) of the salt of N,N-dimethylbiguanide contained in the composition is ≤ 25%, preferably ≤ 10%, preferably ≤ 5%, preferably ≤ 4%, more preferably ≤ 3%, more preferably ≤ 2%, particularly preferably ≤ 1%, and most preferably 0%, that is, the composition does not comprise a salt of N,N-dimethylbiguanide.

In one embodiment, the additional agent includes an acidic pH adjusting agent.

In one embodiment, the acidic pH adjusting agent comprises or is selected from hydrochloric acid, sulfuric acid, acetic acid, citric acid, phosphoric acid, malic acid, tartaric acid, lactic acid, carbonic acid, etc.

In some embodiments, the pharmaceutical combination or composition for the above uses includes component Z, which includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin, mitoxantrone, gefitinib, osimertinib, larotrectinib, pazopanib, trastuzumab, and derivatives thereof.

In some embodiments, the pharmaceutical combination or composition for the above uses includes medicament C, which includes or is selected from at least one of the following: sodium chloride, sodium bicarbonate, sodium dihydrogen phosphate, sodium lactate, sodium acetate.

In some embodiments, the pharmaceutical combination or composition for the above uses includes medicament E, which includes or is selected from at least one of the following: biological macromolecule immune modulators such as immune checkpoint inhibitors, cytokines, and TLR agonists; immune cell immune modulators; and vaccine immune modulators such as BCG.

According to the present invention, a method for treating cells of a lesion such as a tumor or nodule (referred to as the R combination treatment method of the present invention) is provided, including administering or introducing the above pharmaceutical combination or composition of the present invention to the lesion, penetrating into the lesion, and contacting with the cells within the tissue.

According to the present invention, a method for treating a tumor or nodule (referred to as the R combination treatment method 1 of the present invention) is also provided, including: administering the above pharmaceutical combination and composition of the present invention to the tumor or nodule, preferably, the administration includes administering the formulation of the present invention to the tumor or nodule, and/or administering dimethylbiguanide to the tumor or nodule and administering the medicament intratumorally or systemically.

According to the present invention, a method for treating a tumor in a subject (referred to as the R combination treatment method 2 of the present invention) is provided, including administering the above pharmaceutical combination or composition of the present invention to the subject.

In the above R combination treatment methods, the pharmaceutical combination or composition includes dimethylbiguanide and optionally a solvent, wherein the formulation does not contain an acidic pH adjusting agent required to meet cell-culture requirements or conventional injection safety requirements, and the concentration of N,N-dimethylbiguanide is greater than or equal to its intervention concentration, and the intervention concentration (w/w) is 0.5% ≤ C ≤ 51%, preferably 1% - 50%, more preferably 3% - 40% or 5% - 35%.

In some embodiments, the pharmaceutical combination or composition may comprise a solvent, preferably the solvent is water, and more preferably water for injection.

In some embodiments, the pharmaceutical combination or composition may not include a dimethylbiguanide salt.

In some embodiments, the concentration (w/w) of dimethylbiguanide (R) in the pharmaceutical combination or composition can be ≤ 51%, ≤ 50%, ≤ 40%, ≤ 30%, ≤ 25%, ≤ 20%, preferably ≤ 10%, preferably ≤ 5%, more preferably ≤ 2% such as 1%, 0.75% or 0.5%.

In some embodiments, the concentration (w/w) of dimethylbiguanide (R) in the pharmaceutical combination or composition can be 0.2% - 51%, 0.2% - 50%, 0.5% - 50%, 1% - 40%, 5% - 50%, 5% - 40%, 5% - 30%, 5% - 25%, 5% - 20%, 1% - 30%, 1% - 25%, 1% - 20%, preferably 0.5% - 10%, preferably 1% - 5%, or preferably ≤ 2% such as 1%, 0.75% or 0.5%.

In some embodiments, the acidic pH adjusting agent can include or be selected from hydrochloric acid, sulfuric acid, acetic acid, citric acid, phosphoric acid, malic acid, tartaric acid, lactic acid and carbonic acid.

In some embodiments, in the above methods, the R is contained in a powder.

In some embodiments, in the above methods, the powder consists of a dry powder containing R and a solvent.

In some embodiments, in the above methods, the dry powder is R dry powder and the solvent is water for injection.

In some embodiments, the pharmaceutical combination or composition does not contain an acid that can significantly convert N,N-dimethylbiguanide (R) in the aqueous solution into dimethylbiguanide salt.

In the above R combination treatment method or R combination processing method, the administered pharmaceutical combination or composition contains dimethylbiguanide (R) and component (Z), where the administration concentration of dimethylbiguanide is 0.2% - 51%, preferably 0.5% - 50% by weight/weight (w/w), and the component includes a cell-reactive antineoplastic agent (abbreviated as B).

In some embodiments, in the above R combination treatment method or R combination processing method, in the pharmaceutical combination:
- the R provides an activity (abbreviated as activity A) that conventional dimethylbiguanide (active ingredient of which is dimethylbiguanide salt, abbreviated as R') cannot provide, including effectively reducing the function of biological barriers (abbreviated as activity A1), through the following limitation:
   1). tumor administration;
   2). the administration amount for one course of treatment is: ${Q}_{R} = {\sum }_{i = 1}^{nR} {q}_{R \left(i\right)}$,
      where n_{R} represents the number of times of R administered to a tumor in one course of treatment, and q_{R(i)} represents the amount of R in the i-th (i = 1,..., n_{R}) administration, and: $qR \left(i\right) = {\sum }_{i i - 1}^{n R i} cR \left(ii\right) \times vR \left(ii\right) ,$
      where n_{Ri} represents the number of administration points, for example, the number of needle insertion points in the i-th administration of R, and c_{R(ii)} and v_{R(ii)} represent the concentration and volume of R administered at the ii-th administration point such as the needle insertion point, respectively. The concentration threshold of c_{R(ii)} (w/w) is 0.1% or 0.2%, and the upper limit of the concentration threshold is 51%. The volume threshold of v_{R(ii)} is 1/800 or 1/300 of the target volume (abbreviated as V_{target(ii)}), and the upper limit of the volume threshold is 3 times of V_{target(ii)};
- the cell-reactive antineoplastic agent (B) provides the cell reaction (abbreviated as activity B) through the following limitations, and one of the key control steps of this reaction is to cross the above-mentioned biological barrier:
   1). intratumoral administration and/or systemic administration;
   2). the administration amount for one course of treatment is: ${Q}_{B} = {\sum }_{j - l}^{nB} {q}_{B \left(j\right)} + {\sum }_{i ′ - l}^{nB ′} {c}_{B \left(j′\right)} \times {v}_{B \left(j′\right)} ,$ where Q_{B} is≦the conventional amount in a sensitive tumor of B, n_{B} and q_{B(j)} represent the number of systemic administrations and the amount in the j-th (j = 1,..., n_{B}) administration of B in one course of treatment, respectively, and n_{B'}, c_{B(j')}, and v_{B(j')} represent the number of tumor administrations, the concentration and volume in the j'-th administration (j' = 1,..., n_{B***'***}) of B in one course of treatment, respectively;
- the type of the combination is a synergistic combination where the pharmaceutical effects of the activity A of R and the activity B of B enhance each other.

In some embodiments, the N,N-dimethylbiguanide (R) comprises <30% (w/w), preferably ≦5% of a salt of N,N-dimethylbiguanide (R'), or does not comprise a salt of N,N-dimethylbiguanide (R'), and preferably, the N,N-dimethylbiguanide (R) is in a form of a powder for injection.

In some embodiments, the component (Z) in the pharmaceutical combination includes an antineoplastic drug, which is selected from a cell-reactive drug (B) that one of whose key control steps is to cross the tumor tissue barrier.

In some embodiments, the cell-reactive drug (B) includes cytotoxic drugs and targeted antineoplastic agents.

In some embodiments, the cytotoxic drugs include DNA-damaging agents, antimetabolites, microtubule inhibitors, and topoisomerase inhibitors.

In some embodiments, representative compounds of the DNA-damaging agents include cisplatin, carboplatin, oxaliplatin, ifosfamide, and doxorubicin.

In some embodiments, representative compounds of the antimetabolites include fluorouracil, gemcitabine, and methotrexate.

In some embodiments, representative compounds of the microtubule inhibitors include paclitaxel and vincristine.

In some embodiments, the targeted antineoplastic agents include small molecule targeted-antineoplastic agents, preferably kinase inhibitors.

In some embodiments, the kinase inhibitors include gefitinib, osimertinib, larotrectinib, pazopanib, trastuzumab, and derivatives thereof.

In some embodiments, in the above-mentioned R combination treatment method, the component Z includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin, mitoxantrone, gefitinib, osimertinib, larotrectinib, pazopanib, trastuzumab, and derivatives thereof.

In some embodiments, in the above-mentioned R combination treatment method, the medicament C includes or is selected from at least one of the following: sodium chloride, sodium bicarbonate, sodium dihydrogen phosphate, sodium lactate, and sodium acetate.

In some embodiments, in the above-mentioned R combination treatment method, the medicament E includes or is selected from at least one of the following: biological macromolecule immune modulators such as immune checkpoint inhibitors, cytokines, and TLR agonists; immune cell immune modulators; and vaccine immune modulators such as BCG.

In some embodiments, in the above-mentioned R combination treatment method, the administration concentration of dimethylbiguanide (R) (w/w) can be 0.2%-51%, 0.2%-50%, 0.5-51%, 0.5-50%, 0.5-45%, 1%-45%, 5%-45%, 5%-40%, 5%-30%, 5%-25%, 5%-20%, 1%-30%, 1%-25%, 1%-20%, 0.5%-10%, 1%-5%, or ≦2% such as 1%, 0.75%, or 0.5%.

In some embodiments, in the above-mentioned R combination treatment method, the pharmaceutical combination includes dimethylbiguanide (R) and doxorubicin (DOX), where the dimethylbiguanide (R) can be administered at a concentration(w/w) of 0.2%-50%, and the doxorubicin (DOX) can be administered at a concentration(w/w) of 0.025%-1%, preferably, the dimethylbiguanide (R) is administered at a concentration(w/w) of 5%-50%, and the doxorubicin (DOX) is administered at a concentration of 0.1%-1%, optionally, both dimethylbiguanide (R) and doxorubicin (DOX) are administered intratumorally.

In one embodiment, dimethylbiguanide (R) is administered intratumorally at a concentration (w/w) of 0.2% to 50%, the doxorubicin (DOX) is administered intratumorally at a concentration (w/w) of 0.025% to 1%, and the doxorubicin (DOX) is administered systemically at a concentration of 0.05% to 0.06% (w/w), such as intraperitoneally.

In some embodiments, in the above-mentioned R combination treatment method, the pharmaceutical combination includes dimethylbiguanide (R) and ifosfamide (IFO), where the dimethylbiguanide (R) can be administered at a concentration (w/w) of 0.2% to 50%, and the ifosfamide (IFO) can be administered at a concentration (w/w) of 0.1% to 10%. Preferably, the dimethylbiguanide (R) is administered at a concentration (w/w) of 0.2% to 45%, and the ifosfamide (IFO) is administered at a concentration (w/w) of 0.1% to 10%. More preferably, the dimethylbiguanide (R) is administered at a concentration (w/w) of 5% to 45%, and the ifosfamide (IFO) is administered at a concentration (w/w) of 0.5% to 10%, optionally, both dimethylbiguanide (R) and ifosfamide (IFO) are administered intratumorally.

In one embodiment, the dimethylbiguanide (R) is administered intratumorally at a concentration (w/w) of 5% to 50%, the ifosfamide (IFO) is administered intratumorally at a concentration (w/w) of 0.5% to 10%, and the ifosfamide (IFO) is administered systemically such as intraperitoneally or intragastrically at a concentration (w/w) of 0.5%.

In some embodiments, in the above-mentioned R combination treatment method, the pharmaceutical combination includes dimethylbiguanide (R) and cisplatin (DDP), where the dimethylbiguanide (R) can be administered at a concentration (w/w) of 0.2% to 50%, and the cisplatin (DDP) can be administered at a concentration (w/w) of 0.01% to 0.4%. Preferably, the dimethylbiguanide (R) is administered at a concentration (w/w) of 0.5% to 50%, and the cisplatin (DDP) is administered at a concentration (w/w) of 0.01% to 0.04%, optionally, both dimethylbiguanide (R) and cisplatin (DDP) are administered intratumorally.

In one embodiment, the dimethylbiguanide (R) is administered intratumorally at a concentration (w/w) of 0.5% to 50%, the cisplatin (DDP) is administered intratumorally at a concentration (w/w) of 0.013% to 0.4%, and the cisplatin (DDP) is administered systemically, such as intraperitoneally at a concentration (w/w) of 0.015%.

In some embodiments, in the above-mentioned R combination treatment methods, the pharmaceutical combination includes dimethylbiguanide (R) and 5-fluorouracil (5-FU), wherein the dimethylbiguanide (R) can be administered at a concentration (w/w) of 0.2% to 50%, and the 5-fluorouracil (5-FU) can be administered at a concentration (w/w) of 0.05% to 9%. Preferably, the dimethylbiguanide (R) is administered at a concentration (w/w) of 0.2% to 45%, and the 5-fluorouracil (5-FU) is administered at a concentration (w/w) of 0.5% to 9%. More preferably, the dimethylbiguanide (R) is administered at a concentration (w/w) of 5% to 45%, and the 5-fluorouracil (5-FU) is administered at a concentration (w/w) of 0.9% to 9%. Optionally, both dimethylbiguanide (R) and 5-fluorouracil (5-FU) are administered intratumorally.

In one embodiment, the dimethylbiguanide (R) is administered intratumorally at a concentration (w/w) of 5% to 45%, the 5-fluorouracil (5-FU) is administered intratumorally at a concentration (w/w) of 0.9% to 9%, and the 5-fluorouracil (5-FU) is administered systemically at a concentration (w/w) of 0.5%, such as intraperitoneally.

In some embodiments, in the above-mentioned R combination treatment methods, the pharmaceutical combination includes dimethylbiguanide (R), cisplatin (DDP), and gemcitabine (GEM), wherein the dimethylbiguanide (R) can be administered at a concentration (w/w) of 0.2% to 50%, the cisplatin (DDP) can be administered at a concentration (w/w) of 0.05% to 1%, and the gemcitabine (GEM) can be administered at a concentration (w/w) of 0.5% to 2%, preferably at a concentration (w/w) of 1%. Optionally, or, dimethylbiguanide (R) and cisplatin (DDP) are administered intratumorally and gemcitabine (GEM) is administered systemically, such as intraperitoneally.

In some embodiments, in the above-mentioned R combination treatment methods, the pharmaceutical combination includes dimethylbiguanide (R), paclitaxel (PTX), and 5-fluorouracil (5-FU), wherein the dimethylbiguanide (R) can be administered at a concentration (w/w) of 0.2%0 to 50%, the 5-fluorouracil (5-FU) can be administered at a concentration (w/w) of 0.1% to 10%, and the paclitaxel can be administered at a concentration (w/w) of 0.02% to 1%, preferably at a concentration (w/w) of 0.03%. Optionally, the dimethylbiguanide (R) and 5-fluorouracil (5-FU) are administered intratumorally and paclitaxel (PTX) is administered systemically, such as intraperitoneally.

In some embodiments, in the above-mentioned R combination treatment methods, the pharmaceutical combination includes dimethylbiguanide (R) and osimertinib, wherein the dimethylbiguanide (R) can be administered at a concentration (w/w) of 0.2%0 to 50%, and the osimertinib can be administered at a concentration (w/w) of 0.05% to 2%. Preferably, the dimethylbiguanide (R) is administered at a concentration (w/w) of 5%, and the osimertinib is administered at a concentration (w/w) of 0.15%. Optionally, the dimethylbiguanide (R) is administered intratumorally, and the osimertinib is administered systemically, such as intraperitoneally.

In some embodiments, in the above-mentioned R combination treatment methods, the pharmaceutical combination includes dimethylbiguanide (R) and gefitinib (GR), wherein the dimethylbiguanide (R) can be administered at a concentration (w/w) of 0.2%0 to 50%, and the gefitinib (GR) can be administered at a concentration (w/w) of 0.1% to 2%. Preferably, the dimethylbiguanide (R) is administered at a concentration (w/w) of 1% to 50%, and the gefitinib (GR) is administered at a concentration (w/w) of 0.5%. Optionally, the dimethylbiguanide (R) is administered intratumorally, and the gefitinib (GR) is administered systemically, for example, intraperitoneally.

In some embodiments, in the above-mentioned R combination treatment methods, the pharmaceutical combination includes dimethylbiguanide (R), cisplatin (DDP), and gefitinib (GR), wherein the dimethylbiguanide (R) is administered at a concentration(w/w) of 5% (for example, intratumorally), the gefitinib (GR) is administered at a concentration (w/w) of 0.5% (systemically, for example, intraperitoneally), and the cisplatin (DDP) is administered at a concentration (w/w) of 0.15 (for example, intratumorally).

In some embodiments, in the above-mentioned R combination treatment methods, the pharmaceutical combination includes dimethylbiguanide (R), ifosfamide (IFO), and gefitinib (GR), wherein the dimethylbiguanide (R) is administered at a concentration (w/w) of 5% (for example, intratumorally), the ifosfamide (IFO) is administered at a concentration (w/w) of 1% (for example, intratumorally), and the gefitinib (GR) is administered at a concentration (w/w) of 0.5% (systemically, for example, intraperitoneally).

In some embodiments, in the above-mentioned R combination treatment methods, the pharmaceutical combination includes dimethylbiguanide (R), doxorubicin (DOX), and gefitinib (GR), wherein the dimethylbiguanide (R) is administered at a concentration (w/w) of 5% (for example, intratumorally), the doxorubicin (DOX) is administered at a concentration (w/w) of 0.05% (for example, intratumorally), and the gefitinib (GR) is administered at a concentration (w/w) of 0.5% (systemically, for example, intraperitoneally).

In some preferred embodiments, in the above-mentioned R combination treatment methods, the contact or administration or intervention may include micro multi-point contact or administration or intervention, preferably, micro multi-point injection. The micro amount for lesions larger than 3 cm may include the amount of 5 µL to 1000 µL, for example, 5 µL to 500 µL, 5 µL to 250 µL, 5 µL to 200 µL, 5 µL to 100 µL, 5 µL to 75 µL, 5 µL to 50 µL, 5 µL to 25 µL, 5 µL to 20 µL, 10 µL to 200 µL, 10 µL to 100 µL, 10 µL to 75 µL, 20 µL to 200 µL, 20 µL to 100 µL, 20 µL to 75 µL, 30 µL to 200 µL, 30 µL to 100 µL, 40 µL to 200 µL, 40 µL to 100 µL, 50 µL to 200 µL, 50 µL to 100 µL, or 50 µL to 75 µL, and the like. Preferably, micro multi-point contact or administration, more preferably, micro multi-point injection.

In some embodiments, the above-mentioned treatment can last for at least one course of treatment. The duration of one course of treatment can be about 7 to 40 days, for example, about 9 to about 37 days, for example, about 15, 20, 25, 27, 28, 29, 30, 31, 32, 33, 34, or 35 days.

In one embodiment, during one course of treatment, the contact or administration of the formulation of the present invention can be at a low frequency. The low frequency is for example, administered about 1-6 times per course of treatment, preferably 1-5 times per course of treatment, 1-4 times per course of treatment, 1-3 times per course of treatment, or 1-2 times per course of treatment.

In some embodiments, in the above-mentioned R combination treatment method, the dimethylbiguanide (R) and component (Z) in the pharmaceutical combination can be administered simultaneously, sequentially, or at intervals.

In some embodiments, in the above-mentioned R combination treatment method, both the dimethylbiguanide (R) and component (Z) in the pharmaceutical combination are administered intratumorally (combination relationship I); R and Z are administered intratumorally and systemically, respectively (combination relationship II); or the R and a portion of Z are administered intratumorally and the other portion of Z is administered systemically (combination relationship III).

In some embodiments, in the above-mentioned method, the tumor is a solid tumor suitable for intratumor administration.

In some embodiments, in the above-mentioned R combination treatment method, the tumor is selected from a tumor where the tissue barrier function is not conducive to the inhibitory effect of Z on tumor cells, preferably selected from refractory tumors of Z, including non-indicated tumors, drug-resistant tumors, tumors with unfavorable microenvironments, and tumors after medication withdrawal.

In some embodiments, in the above-mentioned R combination treatment method, the tumor is selected from heterogeneous tumors, where the heterogeneity includes at least one of tissue heterogeneity, spatial heterogeneity, and safety heterogeneity. The heterogeneous tumor is a tumor in which different tumor masses of the same type or different regions of the same tumor mass are set as different target areas for differentiated administration to facilitate treatment based on the heterogeneity.

In some embodiments, in the above-mentioned R combination treatment method, the contact or adminstration includes multi-point contact or adminstration, preferably, micro multi-point contact or administration, and more preferably, micro multi-point injection.

In some embodiments, in the above-mentioned R combination treatment method, the formulation or pharmaceutical combination is suitable for micro-volume administration or interventional reduction (Y) of lesion volume (X), with a ratio of administration volume to target volume (V_{administration}/V_{target}) being approximately 0.02-0.056, 0.02-0.10, or 0.02-0.34.

In some embodiments, in the above-mentioned R combination treatment method, when the lesion is ≧ about 3.5 cm³ or the maximum size is ≧ about 2 cm, the multi-point is ≧ 5 points, for example, 10, 15, 20, 25, 30, 35, or 50 points.

In some embodiments, in the above-mentioned R combination treatment method, the contact or administration is low-frequency, preferably, the low frequency is 1-6 times per course, more preferably, 1-5 times per course, 1-4 times per course, 1-3 times per course, or 1-2 times per course.

In some embodiments, in the above-mentioned R combination treatment method, preferably, the maximum of administration concentration cR of R is ≧ 5%, ≧ 10%, ≧ 20%, or ≦ 30%; the number of administration points is > 3, 10, 15, 20, 25, 30, 35, or 100 points.

In some embodiments, in the above-mentioned R combination treatment method, the dimethylbiguanide R and component Z can be provided by one or more formulations, and wherein:
R and Z are administered intratumorally;
R and Z are administered intratumorally and systemically, respectively; or
R and a portion of Z are administered intratumorally, and the other portion of Z is administered systemically.

In some embodiments, in the above-mentioned R/Z combination treatment method, the combination of R and Z (R/Z combination) is a synergistic combination that exceeds the expected effect of the conventional combination of metformin drug (R') and Z (R'/Z combination), manifested as when the one-course dosage of R (Q_{R}) is ≦ 25% or 50% of the one-course dosage of R' (Q_{R***'***}) and the dosage of Z per course does not increase, the pharmaceutical effect significantly increases, including at least one of the pharmaceutical adjustability indexes increasing by at least 100% and at least one of the pharmaceutical efficacy indexes enhancing by at least 25%, where the pharmaceutical adjustability refers to the performance of the medication in providing differentiated administration parameters to different target areas while still maintaining efficacy.

In some embodiments, in the above-mentioned R/Z combination treatment method, the R/Z combination is also a synergistic combination that exceeds the expected effect based on the HSA method or Bliss method of single agent R and single agent Z, manifested as when the one-course dosage of R (Q_{R}) does not increase and the one-course dosage of Z (Q_{B}) is reduced by at least 20%, the pharmaceutical effect increases, including at least one of the pharmaceutical adjustability indexes increasing by at least 10% and at least one of the pharmaceutical efficacy indexes enhancing by at least 10%.

In some embodiments, in the above-mentioned R or R/Z combination treatment method, the pharmaceutical adjustability indexes include at least one of the following local administration parameter adjustability coefficients (a ratio of the maximum value in the adjustability range to the threshold thereof):
1). the concentration adjustable coefficient of R, which is a ratio of the maximum value of c_{R} to the threshold (maximum value of c_{R}/threshold), and the increase in the adjustability index is an increase in the coefficient, preferably the coefficient is increased to > 10, 50, 100 or 150, which enables R to provide highly variable concentrations to different regions while maintaining its activity stable, including for example, drug aministration based on the nature of the heterogeneous tumor;
2). the target volume adjustable coefficient of R, which is a ratio of the maximum value of the target volume (v_{target}) to the threshold (maximum value of v_{target} /threshold), and the increase in the adjustability index is an increase in the coefficient, preferably the coefficient is increased to > 2, 10, 50, or 100, which enables R to select different v_{target} for drug aministration based on different treatment situations, including for example, heterogeneous tumor;
3). the administration volume ratio adjustable coefficient of R, which is a ratio of the maximum value of a ratio of the administration volume (v_{R}) to the target volume (v_{target}) to the threshold (maximum value of v_{R} / v_{target} / threshold), and the increase in the adjustability index is an increase in the coefficient, preferably the coefficient is increased to > 5, 10, or 100, which enables R to provide a highly variable volume ratio to different regions while maintaining its activity stable, including for example, drug aministration based on the nature of the heterogeneous tumor;
4). the single dosage adjustable coefficient of R, which is a ratio of the maximum value of the single dosage (c_{Ri} × v_{Ri}) to the threshold (maximum value of c_{Ri} × v_{Ri} / threshold), and the increase in the adjustability index is an increase in the coefficient, preferably the coefficient is increased to > 2, 6, or 10, which enables R to select different single dosages for drug aministration based on different treatment situations, including for example, heterogeneous tumor; and
5). the adjustable coefficient of the administration point density of R, which is a ratio of the maximum value of the administration point density (nᵢᵢ/v_{target}) to the threshold (maximum value of nᵢᵢ/v_{target}/threshold), and the increase in the adjustability index is an increase in the coefficient. Preferably, the coefficient is increased to >3, 10, 50, or 100, which enables R to provide highly variable densities to different regions while maintaining its activity stable, including, for example, drug aministration based on the nature of the heterogeneous tumor.

In some embodiments, in the above-mentioned R or R/Z combination treatment methods, the pharmaceutical efficacy indexes include at least one of the following:
1). Timeliness indexes, manifested as the onset time in mouse experiments (the day when the tumor proliferation rate is <42%), and the enhancement of the efficacy indexes is the shortening of the onset time, making the scheme a rapid-acting scheme, especially suitable for tumors that require rapid reduction in tumor size;
2). Effectiveness indexes, manifested as the tumor inhibition rate or objective response rate in mouse experiments, and the enhancement of the efficacy indexes is an increase in the tumor inhibition rate or objective response rate, making the scheme a highly effective scheme, especially suitable for tumors that require maximum inhibition of tumor size;
3). Long-term efficacy indexes, manifested as the progression-free time in mouse experiments, and the enhancement of the efficacy indexes is an extension of the progression-free time, making the scheme a long-term scheme, especially suitable for tumors that require maximum extension of the progression-free time.

According to the present invention, a kit comprising the formulation of the present invention as described above and instructions for implementing the above-mentioned methods of the present invention is provided.

According to the present invention, a kit is provided, comprising the pharmaceutical combination or composition of the present invention as described above, and instructions for implementing the above-mentioned treatment or therapy method of the present invention.

In one embodiment, the above-mentioned kit further includes a micro-volume interventional device, preferably, the device is suitable for micro-volume multi-point contact or administration, and preferably includes a micro-injection device such as a syringe.

In one embodiment, the volume interventional device may include, for example, a puncture needle, an injection needle, a catheter, or other devices with the same function.

According to the present invention, the medicament of this application is a therapeutic drug. When used for treating lesional diseases, it serves as a main therapeutic drug and can be combined with other interventional therapies, systemic chemotherapy, immunotherapy, photodynamic therapy, sonodynamic therapy, surgical intervention, or combinations of such therapies to further enhance efficacy.

### Beneficial effects of the present invention

1. It should be noted that since the R can only be locally administered and not systemically administered like R', the scheme of the present application is only applicable to patients who can be administered intratumorally for tumor treatment. The treatment problems of such patients still need to be addressed, although intratumoral administration currently accounts for less than 1% in clinical practice. The beneficial effects provided by this scheme for such patients are as follows:
   1. Compared with the prior art of dimethylbiguanide: the active ingredient in the formulation of the present invention is N,N-dimethylbiguanide (R), while the active ingredient in the prior art is a salt of N,N-dimethylbiguanide (R'). Within the transient time period of R being converted to R' after local administration, it has an active form that is different from the latter, exhibiting a dosage reduction and efficacy enhancement exceeding the expectations of the prior art (R and R' are pharmaceutically equivalent). When the dosage of R in one course of treatment (Q_{R}) is less than 25% to 50% of the dosage of R' (Q_{R***'***}), the pharmaceutical effect is significantly increased, including at least one of the pharmaceutical adjustability indexes increasing by at least 100% and at least one of the pharmaceutical efficacy indexes enhancing by at least 40%. The efficacy indexes include the onset time, tumor inhibition rate or objective response rate, and progression-free time. The improved efficacy enables R to solve the problems that R' cannot solve: fast-acting, high efficacy, and/or long-lasting efficacy. The adjustability indexes include: the maximum value of c_{R}/threshold, the maximum value of v_{target}/threshold, the maximum value of v_{R}/v_{target}/threshold, the maximum value of c_{Ri}×v_{Ri} /threshold, and the maximum value of nᵢᵢ/v_{target}/threshold. The improved adjustability enables R to solve the problems that R' cannot solve: providing highly variable administration parameters to different tumor regions while maintaining stable efficacy, including, for example, drug aministration based on the nature of the heterogeneous tumors.
   2. Comparasion of the present invetnion with the prior art of transient local action medicaments (such as anhydrous ethanol): 1). the solubility concentration (e.g., 51%) of R in the present invention is dozens of times higher than the threshold concentration (e.g., 0.2% or 0.5%), solving the long-standing problem of low adjustability of such medicaments. 2). the transient local action of R in the present invention has better complementarity with the local action of other medicaments (such as methylene blue) in terms of mechanism, providing better synergistic effects. 3). the efficacy-toxicity ratio of R of the present invention is higher, and the efficacy-toxicity ratio of the combination of R and C (such as SB) further significantly increases, providing a more preferred technical solution for patients sensitive to local stimulation.
   3. Comparasion of the present invention with the prior art ofco-administration of dimethylbiguanide (R') and B (R'/B) : the transient activity of R in the combination of R and B in the present scheme can provide a function of reducing the biological barrier function that R' cannot provide, even in tumor tissues that cannot be completely cause necrosis, which is beneficial for Z to break through this key control step, and, in turn, beneficial for the development of the pharmaceutical effect of R in this region. The combination of R and B thus provides a synergistic effect that enhances each other beyond the expectations of the prior art (R/B and R'/B are pharmaceutically equivalent), manifested as the beneficial effects of R described in the above 1 (the dosage reduction and efficacy enhancement) being exerted, and at least one of the efficacy indexes such as tumor inhibition rate and progression-free time being further improved due to co-administration with B compared with R. Even compared with single agent R and cell-reactive antineoplastic agents (B), the combination in the present invention further enhances the efficacy than the strongest efficacy in the single agent in at least B-resistant tumors, manifested as at least one of the adjustability indexes increasing by at least 10% and/or at least one of the efficacy indexes enhancing by at least 10%.
   4. Comparasion of the present invention with the prior art of co-administration of dimethylbiguanide (R') and E (R'/E): the above transient activity of R in the combination of R and E in this scheme can provide the effect of effectively releasing lesional immune substances and inflammatory signals that R alone cannot provide, even in tumor tissues where it cannot completely cause necrosis. Coupled with the aforementioned effect of effectively reducing the function of biological barriers, it is conducive to the infiltration of immune cells. These effects provide an important basis and synergistic mechanism for the action of E. The combination of R and the immunomodulator (E) provides a synergistic effect that enhances each other and exceeds the expectation of the prior art (R/E and R'/E are pharmaceutically equivalent), as manifested by the beneficial effects of R/E (such as long-term survival rate) being increased by more than 50% compared to R'/E. Even when compared with single agent R and single agent E, the combination has a further enhanced effect than the strongest effect of the single agents, as manifested by an increase in long-term survival rate of at least 25%.

Based on the study described in greater detail below, although the specific mechanism still needs further research, R in the medicament of this application shows a transient effect, especially a transient penetration effect on lesion structures. This effect enables the pharmaceutical solution to reach the cells within the tissue and cause effective morphological changes, ultimately leading to the effective destruction or volume reduction of the lesion tissue, as well as direct damage to tumor cells.

Other purposes, advantages, and novel features of the present invention will become apparent to those skilled in the art upon examination of the following examples, which are not intended to be limiting. Additionally, each of the various embodiments and aspects of the invention as mentioned above and as claimed in the claims section will find experimental support in the following examples.

### Examples

The following specific examples are provided to further illustrate the present invention, but are not intended to limit the invention. In the following examples, all experimental animals are handled in accordance with relevant regulations and industry self-discipline.

Unless otherwise specified, the cells, materials, reagents, etc. used in the following specific examples can all be obtained from commercial sources.

In the following examples, unless otherwise specified, all cells are purchased from West China Hospital of Sichuan University and its wholly-owned company (Sichuan Kangcheng Biotechnology Co., Ltd.), among which the drug-resistant cells have a drug resistance index of 4 or above; the tumor tissue of the patient-derived xenograft models (referred to as PDX in this application) and their drug resistance verification are provided by West China Hospital of Sichuan University and related experiments are conducted in collaboration with the West China hospital, Sichuan University.

In the following examples, the research substance of the tissue barrier function reducing agent is N,N-dimethylbiguanide (abbreviated as R, CAS 657-24-9), and its comparison substance is dimethylbiguanide hydrochloride (abbreviated as R', CAS 1115-70-4). The raw materials of both are purchased from Shanghai Macklin Biochemical Technology Co., Ltd. According to the supplier, the dry powder of N,N-dimethylbiguanide is prepared by removing hydrochloric acid from dimethylbiguanide hydrochloride, with a purity quality standard of ≥95% (the quality inspection result is 95.02%). It is detected as a sterile powder. The highest concentration that does not precipitate after dissolving under rapid stirring and leaving at room temperature (25°C) for 2 hours is 51%, so in this application, 51% (w/w) represents the maximum solubility of N,N-dimethylbiguanide in water.

In the following examples, the cell-reactive drugs (abbreviated as B) are selected from a sufficient number of representative medicaments to fully demonstrate that the following results can be reasonably extended to this class of medicaments: the tumor tissue barrier function is reduced by the same compound (R), which is conducive to the pharmaceutical effect of any representative medicament on the antineoplastic response at the tumor cell level. The representative medicaments of B are selected from cytotoxic drugs (B1) and targeted-antineoplastic agents (B2), and their raw materials are purchased from Shanghai Macklin Biochemical Technology Co., Ltd. and Shanghai Weihuan Biotechnology Co., Ltd.

In the following examples, the representative medicaments of cytotoxic drugs are selected from DNA damaging agents, antimetabolites, and microtubule inhibitors. Among them, the representative compounds of DNA damaging agents include: cisplatin (abbreviated as DDP, CAS 15663-27-1), carboplatin (abbreviated as CBP, CAS 41575-94-4), ifosfamide (abbreviated as IFO, CAS 3778-73-2), doxorubicin (abbreviated as DOX, CAS 23214-92-8); the representative compounds of antimetabolites include: fluorouracil (abbreviated as 5-FU, CAS 51-21-8), gemcitabine (abbreviated as GEM, CAS 95058-81-4), methotrexate (abbreviated as MTX, CAS 59-05-2); the representative compounds of microtubule inhibitors include: paclitaxel (abbreviated as PTX, CAS 33069-62-4), vincristine (abbreviated as VCR, CAS 57-22-7).

In the following examples, the representative drugs of targeted antineoplastic agents are selected from kinase inhibitors, etc. Among them, representative compounds of kinase inhibitors include: Gefitinib (GR, 184475-35-2), Osimertinib (AZD9291, CAS 1421373-65-0), Larotrectinib (CAS 223403-58-4), Pazopanib (CAS 444731-52-6), and Trastuzumab (HER, CAS 180288-69-1).

In the following examples, the immunomodulator (E) is selected from one or more of the following: biologic macromolecule immunomodulators (E1), immune cell immunomodulators (E2), and vaccine immunomodulators (E3). E1 is selected from immune checkpoint inhibitors including Anti-mouse PD-1 (CD279)-InViVo (Selleck, abbreviated as RMP1-14), cytokines including interleukin-15 (abbreviated as IL-15, CAS 150801-17-9), and TLR agonists including CpG ODN1826 (abbreviated as CpG). Vaccine based immunomodulators include Bacillus Calmette-Guérin (abbreviated as BCG, Chengdu Institute of Biological Products Co., Ltd).

In the following examples, methylene blue (CAS 615-731-6) is abbreviated as MB.

In the following examples, unless otherwise specified, the experimental animals are purchased from Chengdu Dasuo Biotechnology Co., Ltd., and the animal experiments are also conducted in the company's animal laboratory. Balb/c nude mice, Balb/c mice, and C57BL/6 mice are 5-6 weeks old healthy animals with normal weight (18-22g), normal diet (non-fasting), and no diseases (including no metabolic system diseases or diabetes). The subcutaneous tumor formation method is carried out in accordance with the guidelines issued by the pharmaceutical regulatory authorities (such as the CFDA's "Guidelines for the Pharmacodynamics of Antineoplastic Pharmaceuticals"), and unless otherwise specified, the subcutaneous tumor model is established in the right axilla of the animals. Unless otherwise specified, when the lesion grows to the required volume (e.g., 100-300mm³), the animals are randomly divided into several groups using PEMS 3.2 software, with 6 animals in each group. During intratumoral injection, the pharmaceutical solution is evenly distributed within the tumor as much as possible. The items observed, measured, and analyzed in the experiments includes general condition, body weight, food intake, lesion volume, tumor weight, etc. Unless otherwise specified, no abnormalities are found in general condition, body weight, and food intake. The subcutaneous nodular animal experiments are conducted according to the above-mentioned subcutaneous tumor animal experiment methods.

In the following examples, the experimental results (such as tumor weight) are expressed as mean ± standard error (x ± SEM), and the differences between the means of two experimental animal groups are compared using the statistical software The SPSSAU project (2023) (SPSSAU (version 23.0) [Online Application Software]. Retrieved from https://www.Spssau.Com), with a significance level of 0.05.

The formula for calculating tumor volume is as follows:
Tumor volume (V) = 1/2 × a × b², where a represents the length of the tumor and b represents the width of the tumor.

The formula for calculating relative tumor volume (RTV) is as follows:
RTV = Vₜ / V₀, where Vₜ and V₀ are the tumor volumes on the study day (t) and the random grouping day, respectively.

The formula for calculating relative tumor proliferation rate (T/C%) is as follows:
T/C% = [(Tₜ - T₀) / (Cₜ - C₀)] × 100, where Tₜ, T₀ and Cₜ, C₀ are the tumor volumes of the study group and the negative control group on the study day (t) and the random grouping day, respectively.

The formula for calculating tumor inhibition rate is as follows:
Tumor inhibition rate Y (%) = (CW - TW) / CW × 100%, where TW is the average tumor weight of the study group; and CW is the average tumor weight of the negative control group.

In the following examples, the evaluation criteria for various pharmaceutical effects are as described above.

### Example 1. Study of R: Using Drug-Resistant Tumors as Lesion Models

In this example, the conventional metformin salt- refractory lesion model (abbreviated as the refractory lesion in this application) was a tumor resistant to conventional antineoplastic chemotherapeutic drugs (such as cytotoxic drugs and targeted antineoplastic agents) (abbreviated as drug-resistant tumor in this application), including drug-resistant liver cancer tumors, drug-resistant lung cancer tumors, drug-resistant gastric cancer tumors, and drug-resistant breast cancer tumors. The conventional antineoplastic chemotherapeutic drugs included traditional chemotherapeutic drugs (such as 5-fluorouracil, doxorubicin, cisplatin, paclitaxel, and the like) and the targeted antineoplastic agents (such as gefitinib, osimertinib, and the like).

### Example 1.1 Individual Case Study of Drug-Resistant Tumors

In one experiment, the drug-resistant tumor was a drug-resistant liver cancer tumor containing drug-resistant cells (human liver cancer drug-resistant cells Bel-7409/5-Fu). The experimental animals were nude mice, and the tumor formation method in the animals was the conventional subcutaneous transplantation tumor method. Briefly, the cell transplantation amount was 10⁶ cells per mouse, and the transplantation method was cell suspension injection, with the tumor formation site being subcutaneous in the animals. The animals with successful modeling were enrolled (average tumor volume was 178 mm³) and randomly divided into Group 1 (42 mice) and Group 2 (18 mice). Group 1 was further randomly divided into 7 subgroups (6 mice per subgroup), labeled as Subgroups A, B, C, D, E, F, and G. On the 11th day after the gouping of Group 1, the volume of Group 2 was measured (average tumor volume was 828 mm³), and then they were randomly divided into 3 subgroups (6 mice per subgroup), labeled as Subgroups X, Y, and Z. Drug administration began on the day of grouping, which was considered as the first day of treatment, and so on. The pharmaceutical solutions given to the animals in each group were all aqueous solutions prepared by conventional methods.

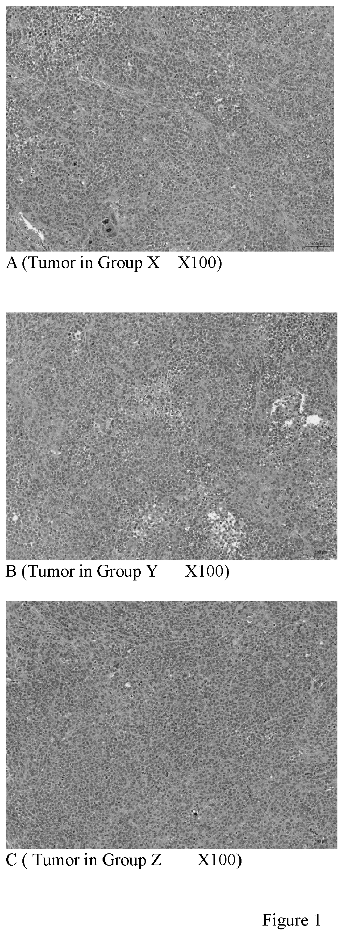

Treatment of Subgroups X-Z: Drug was administered once, all by intratumoral injection. The injected pharmaceutical solution was shown in the following table, with an injection volume of 500 µL per mouse. One hour after injection (t1), the animals in each group were euthanized and dissected to remove the tumors. Macroscopic observations were made and samples were taken for pathological analysis. The pathological analysis method was the conventional HE staining method. Briefly, the tumors were fixed, and then dehydrated, trimmed, embedded, sectioned, stained, and sealed according to the standard operating procedures (SOP) for pathological examination. Finally, microscopic examination was performed, and the tumor tissues and cells were observed under a light microscope. The results were shown in Figure 1 and Table 1.

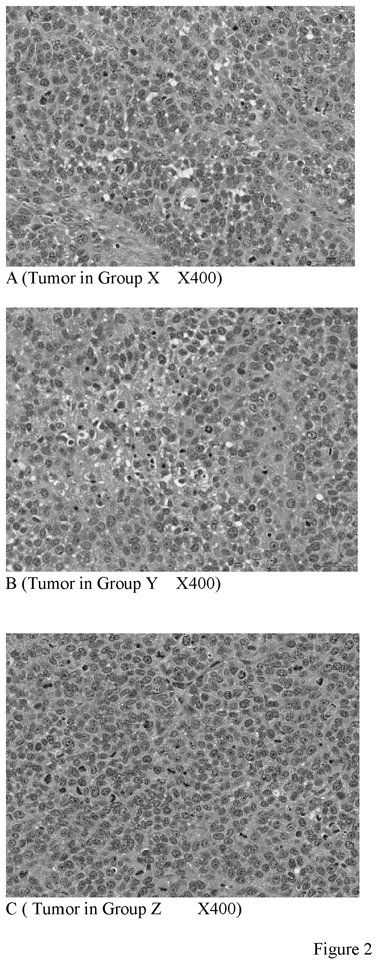

Treatment of Subgroups A-G: drug was administered once a day for 3 consecutive days. The maximum doseS per administration of 5-Fu, dimethylbiguanide hydrochloride, and N,N-dimethylbiguanide were 0.4, 4.2, and 3.2 (mg per administration per mouse), respectively. The daily injection volume for each mouse in Subgroups G and C was 350 µL of 0.25% and 0.5% N,N-dimethylbiguanide by intratumoral injection (approximately 2 times the tumor volume), and 230 µL and 140 µL of 1.3% dimethylbiguanide hydrochloride by intraperitoneal injection, respectively. The other subgroups were all given the pharmaceutical solutions shown in the following table by intratumoral injection, with the administration volume = dose / pharmaceutical concentration. On the 15th day (t2), the animals were euthanized, and the tumors were dissected (Figure 2) to measure the tumor weight. The tumor inhibition rate was calculated using 5-Fu as the negative control, and the results were shown in Table 1.

**Table 1**

| Groups | Intratumoral administered drugs | t1 pharmaco logical effects | t2 pharmacological effect | |
|---|---|---|---|---|
| | | | Tumor weight (g, mean ±SEM) | Tumor inhibition rate (%) |
| Z | 0.20% 5-Fu | - | | |
| X | 1.3% R ' | + | | |
| Y | 0.5% R | ++ | | |
| A | 0.20% 5-Fu | | 0.75±0.11 | - |
| B | 1.3% R' | | 0.68±0.09 | 9.3 |
| C | 0.5% R | | 0.40±0.06 | 46.7 |
| D | 1%R | | 0.22±0.09 | 70.7 |
| E | 21.3% R | | 0.15±0.02 | 80.0 |
| F | 32% R | | 0.17±0.04 | 77.3 |
| G | 0.25% R | | 0.55±0.07 | 26.7 |

| | | | | |
|---|---|---|---|---|
| *: Results of pathological analysis, which were recorded as normal (-), slight (+), mild (++), moderate (+++), and severe (++++) using the conventional four-level grading method for lesions. | | | | |

As shown in the above table, after administration for 1 hour, comparing R (group Y) with R'(group X) at 2 times molar dose, with 5-Fu (group Z) as a negative control, the results of four level grading method for lesion were mild cell atrophy (++) and slight cell atrophy (+), respectively. The pathological analysis of the tumor samples showed that there were differences in the morphology of cells in the tumor (FIG. 1). The groups Y, X and Z were significant abnormal, insignificant abnormal, and normal, respectively. Specifically, group Y showed significant abnormal cell volume reduction, loose cell arrangement, and immune cell infiltration within the tumor. These results indicated that, compared with R'(group X), R (group Y) showed a significant dose reduction and efficacy enhancement, manifested as R dosage reduced by 50% and the effect (X, for example, the result of the four level grading method for lesion) increased by at least 50%.

As shown in the above table, on day 25 after administration, compared with R' (group B), R (group D, E, F) showed a significant dose reduction and efficacy enhancement, manifested as the dose-effect index (inhibition rate /mmol) increased by at least 100%, the adjustability index increased, such as the adjustable coefficient of local administration concentration increased to ≧30, and the adjustable coefficient of local administration volume increased to ≧30. These results showed that R was an active ingredient of dose reduction and efficacy enhancement beyond the expectation of the prior art (R and R' are pharmaceutical equivalents).

In addition, groups C and G were mixed administration of R' and R, and the total administration dosage was the same as that of group D, but the tumor inhibition rate was lower.

### Example 1.2 Extension to drug-resistant tumors

In one experiment, the drug-resistant tumor was a drug-resistant lung cancer tumor containing drug-resistant human lung cancer cells A549/ADM, and the experimental animal was nude mice, and the tumor was transplanted tumors of the cells. The method for establishing the experimental animal model was described in Example 1.1 and would not be described here any longer. The animals with successful modeling (conventional chemotherapeutic drug refractory tumor, average volume of 183mm³) were enrolled and randomly divided into 4 groups, which were recorded as group A, B, C, and D. Drug administration was started on the day of grouping, which was recorded as the first day of treatment, and so on. The pharmaceutical solution administered to the animals in each group was aqueous and did not contain additional agents (such as pH adjusting agent) required for cell culture or the safety of conventional injection, and it was prepared according to the conventional method.

The drug was administered once a day for 3 consecutive days. The administration dosage of ADM, dimethylbiguanide hydrochloride, and N,N-dimethylbiguanide were 0.05, 4.6, and 3.5 mg per dose per animal, respectively. Administration volume = dose/drug concentration. All groups were intratumoral injected with the pharmaceutical solution as described in the table below. On day 15, animals were euthanized and dissected to determine tumor weight, and the tumor inhibition rate was calculated using R' as a negative control; the results were shown in the table below.

**Table 2**

| Groups | Intratumoral administration of drugs | Tumor weight (g, mean ±SEM) | Tumor inhibition rate (%) |
|---|---|---|---|
| A | 1.3%R' | 0.81 ± 0.20 | 0 |
| B | 0.8% R | 0.25 ± 0.11 | 69.1% |
| C | 1%R | 0.23 ± 0.09 | 71.6% |
| D | 0.05% ADM | 0.76 ± 0.18 | 6.2% |

Comparing R (groups B and C) with R' (group A), the results showed in the above table were consistent with Example 1.1, further showing that R was an active ingredient of dose reduction and efficacy enhanment beyond the expectations of the prior art (R and R' were pharmaceutical equivalents), which was applicable to drug-resistant tumors that can be intratumorally administered and not limited to the tumor cells contained therein and the specific types of drugs to which they were resistant. Wherein efficacy enhancement comprises improving efficacy (high efficiency) and adjustability (highly adjustable effective adminstration concentration, volume, etc.) as described in Example 1.1.

In one study, the drug-resistant tumor was a drug-resistant gastric cancer tumor containing drug-resistant gastric cancer cells MKN45/DDP, and the tumor was a transplanted tumor of the cells. The method for establishing the experimental animal model was described in Example 1.1 and would not be described here any longer. The animals with successful modeling (average volume of 197 mm³) were enrolled and randomly divided into 4 groups, which were denoted as groups A, B, C, and D. Drug administration started on the day of grouping, which was recorded as the first day of treatment, and so on. The pharmaceutical solution administered to the animals in each group was aqueous solution and did not contain additional agents (such as pH adjusting agent) required for cell culture or the safety of conventional injection, and it was prepared according to the conventional method.

The drug was administered once a day for 3 consecutive days. The administration dosage of DDP, dimethylbiguanide hydrochloride, and N,N-dimethylbiguanide were 0.04, 4.6, and 3.5 mg per dose per animal, respectively. Administration volume = dose/pharmaceutical concentration. On day 15, animals were euthanized and dissected to determine tumor weight, and the tumor inhibition rate was calculated using R' as a negative control; the results were shown in the table below.

**Table 3**

| Groups | Intratumoral administration of drugs | Tumor weight (g, mean ±SEM) | Tumor inhibition rate (%) |
|---|---|---|---|
| A | 1.3%R ' | 0.89±0.22 | 0 |
| B | 0.8% R | 0.24±0.10 | 73.0 |
| C | 1%R | 0.21±0.09 | 76.4 |
| D | 0.04% DDP | 0.86±0.18 | 3.4 |

Comparing R (groups B and C) with R' (group A), the results showed in the above table were consistent with Example 1.1, further showing that R was an active ingredient of dose reduction and efficacy enhancement beyond the expectations of the prior art (R and R' are pharmaceutical equivalents), which was applicable to drug-resistant tumors that can be intratumorally adminstered and not limited to the tumor cells contained therein and the specific types of drugs to which they were resistant. Wherein, the efficacy enhancement comprises improving efficacy (high efficiency) and adjustability (highly adjustable effective pharmaceutical concentration, volume, etc.) as described in Example 1.1.

### Example 2. Study of R: Extension of lesion model to tumors containing an unfavorable microenvironment

In this example, a conventional dimethylbiguanide-refractory lesion model was a tumor containing an unfavorable microenvironment, including liver cancer, breast cancer, sarcoma, pancreatic cancer, etc. which was transplanted from a 7:3 mixture of the corresponding malignant tumor cells and connective tissue cells (fibroblasts), respectively. It was significantly different from conventional tumors generated from 100% tumor cells and was therefore sometimes referred to as high tumor-stromal ratio tumors. It is well known that connective tissue cells (e.g., fibroblasts) and their products (e.g., fibers) participated in the formation of microenvironments that were not conducive to the action of chemotherapeutic drugs on tumor cells therein, sometimes referred to as drug-resistant microenvironments. For example, the extracellular matrix (ECM) containing the fiber network was considered to be a key barrier for the transport of chemotherapeutic drugs to tumor cells (Chen Jie et al., Role of Extracellular Matrix in tumor development and treatment, Chemistry of Life, 2022, 42(3): 385-393): Firstly, the rich and dense fiber network blocked the drug around the tumor blood vessels, which significantly reduced the pharmaceutical diffusion efficiency. In addition, the abnormally increased ECM further compressed blood vessels and lymphatic vessels, resulting in increased interstitial fluid pressure in the center of the tumor and hindering the delivery of drugs in interstitial transport. Finally, the dense ECM and TGF-p could activate the transformation of fibroblasts into CAFs. CAFs could aggravate matrix sclerosis on the one hand, and the cytokines secreted by CAFs could mediate the expression of drug resistance genes on the other hand. In summary, tumors containing unfavorable microenvironment desensitized conventional effects, such lesions were refractory to conventional antitumor chemotherapeutic drugs, especially conventional metformin drug (chemotherapy sensitivity was lower than commonly used chemotherapeutic drugs), and were not suitable for effective, low frequency, fast acting, and/or microvolume intervention reduction (Y). This type of lesions (X) could even be used as a negative control in this example.

### Example 2.1 Individual case study of tumors with unfavorable microenvironment

In one study, the refractory tumor model was a hepatocellular carcinoma containing an unfavorable microenvironment, which was generated by a mixture of 70% mouse hepatocellular carcinoma cells H22 and 30% mouse fibroblast cells (3T3). The experimental animal was mice, and the tumor was formed by the conventional subcutaneous tumor transplantation method. In brief, the number of transplanted tumor cells was 10⁶ cells per mouse, the method of transplantation was injection of cell suspension, and the modeling site was subcutaneous. On the 5th day after inoculation, the tumor formation rate was 100%, and 6 animals were randomly selected as the test group, and 5-Fu was administered daily consecutively. After 10 days, when the 3-day tumor volume growth rate of each group reached more than 125%, it was considered that the animal model (refractory tumor) was established successfully. Then the other animals were enrolled and randomly divided into 14 groups, named A, B, C, D, E, F, G, H, I, J, K, L, M, N. The average tumor volume was 141mm³. The administration was started on the day of grouping, which was recorded as the first day of treatment, and so on. The pharmaceutical solution administered to the animals in each group was aqueous solution and did not contain additional agents (such as pH adjusting agent) required for cell culture or the safety of conventional injection, and it was prepared according to the conventional method.

Administration began on day 1, and the number of adminstration was as shown in the table below (1 or 6 doses). The 6 doses were given once a day for 6 consecutive days. The administration doses of L-lysine (Lys), sodium hydroxide, 5-Fu, dimethylbiguanide hydrochloride, N,N-dimethylbiguanide, and glycine were 23.3, 2.0, 0.4, 5.3, 4.1, and 4.1 mg per dose per animal, respectively. Administration volume = dose/pharmaceutical concentration. Except for group M, which was injected intraperitoneally, the other groups were injected intratumorally. On day 15, the animals were euthanized and dissected to determine the tumor weight, and the tumor inhibition rate was calculated using 5-Fu as a negative control; the results were shown in the table below.

**Table 4**

| Groups | pharmaceutical solution | Number of doses | Tumor weight (g, mean ±SEM) | Tumor inhibition rate (%) |
|---|---|---|---|---|
| A | 7.3% Lys (500mM) | 1 | 1.97±0.37 | 8.8 |
| B | 1.3% R '(78mM) | 1 | 1.74±0.55 | 19.4 |
| C | 1.3%R ' | 6 | 1.58±0.42 | 26.9 |
| D | 0.62% NaOH (156mM) | 1 | 1.31±0.53 | 39.4 |
| E | 0.62% NaOH | 6 | 0.34±0.27 | 84.3 |
| F | 1% R(78mM) | 1 | 0.61 ±0.32 | 51.8 |
| G | 1% R | 6 | 0.16±0.16 | 92.6 |
| H | 5% R | 6 | 0.05±0.03 | 97.7 |
| I | 20% R | 6 | 0.01±0.01 | 99.5 |
| J | 40%R | 6 | 0.02±0.02 | 99.1 |
| K | 51%R | 6 | 0.04±0.04 | 98.1 |
| L | 1R-HAc (pH11.5) | 6 | 0.48±0.33 | 77.8 |
| M | 0.2% 5-Fu | 1 | 2.16±0.31 | 0 |
| N | 1%R/1% glycine | 6 | 0.81±0.26 | 72.5 |

Compared with R' (groups B, C), the results of R (groups F to K) showed in the above table were consistent with Example 1, further showing that R was an active ingredient of dose reduction and efficacy enhancement beyond the expectations of the prior art (R and R' are pharmaceutical equivalents), which was applicable to tumors that could be intratumoral administered and not limited to previous drug-resistant tumors. Wherein, the efficacy enhancement included improving efficacy (high efficiency) and adjustability (highly adjustable effective administered concentration, volume, etc.) as described in Example 1.1.

Compared with lysine and sodium hydroxide (groups A and D), the inhibition rate of R (group F) was also significantly improved, and the adjustability (administration concentration adjustable coefficient > 50, administration volume adjustable coefficient > 50) was at least 100% higher than that of lysine, which was suitable for administration of drugs to heterogeneous tumors based on their nature. The tumor inhibition rate of R (group G) was 15% (HAc) to more than 200% (HCl) higher than that of R/acidifier mixture groups (group C, L, N), indicating that acidifier was the mixed contraindication of R.

### Example 2.2. Extension to the tumors containing unfavorable microenvironment

In one experiment, breast cancer tumors containing an unfavorable microenvironment were generated from a mixture of 70% mouse breast cancer cells (4T1) and 30% mouse fibroblast cells (3T3), the modeling method for experimental animals was as described in Example 2.1 and would not be described here any more. The animals with successful modeling (refractory tumors, average volume of 282mm³) were enrolled and randomly divided into three groups, denoted as group A, B, and C. Drug administration was started on the day of grouping, which was recorded as the first day of treatment, and so on. The pharmaceutical solution administered to the animals in each group was aqueous solution and did not contain additional agents (such as pH adjusting agent) required for cell culture or the safety of conventional injection, and it was prepared according to the conventional method.

Dimethylbiguanide hydrochloride and N,N-dimethylbiguanide were administered once at doses of 5.9 and 4.5 mg per animal on day 1, 2, and 3, respectively. Administration volume = dose/drug concentration. Group A and group C received intratumoral injection of the drugs as shown in the table below. Group B was injected 550µL of 0.5%N,N-dimethylbiguanide intratumorly (about twice the tumor volume) and 170µL of 1.3% dimethylbiguanide hydrochloride intraperitoneally. On day 9, the animals were euthanized and the tumor weights were measured to calculate the tumor inhibition rate. The results were shown in Table 5 below.

**Table 5**

| Groups | Intratumoral administration of drugs | Tumor weight (g, mean ±SEM) | Tumor inhibition rate (%) |
|---|---|---|---|
| A | 1.3%R' | 0.29±0.05 | 0 |
| B | 0.5% R | 0.17±0.06 | 51.4% |
| C | 1%R | 0.14±0.03 | 86.2% |

Comparing R (groups B and C) with R' (group A), the results shown in the above table were consistent with Examples 1 and 3.1, further showing that R was an active ingredient of dose reduction and efficacy enhancement beyond the expectations of the prior art (R and R' are pharmaceutical equivalents), which was applicable to tumors that could be intratumorally administered and not limited to previously drug-resistant tumors. Therein, the efficacy enhancement included improving efficacy (high efficiency) and adjustability (highly adjustable effective administered concentration, volume, etc.) as described in Example 1.1.

In the following examples 6-9, there were further comparative studies of R versus R'.

### Example 3. Preliminary study of R coadminstered with a cell-reactive antineoplastic agent (B)

In this example, R and R' were respectively studied in comparison with chemotherapeutic drugs combinations, and also in comparison with the tumor after discontinuation of the chemotherapeutic drug as a lesion model.

### Example 3.1 5-Fu used as the representative of B

In one study, 5-Fu was used as the study drug B, and the tumor was hepatoma containing mouse hepatoma cells (H22). The experimental animals were mice, and the tumor formation method was conventional subcutaneous tumor transplantation method. In brief, the number of transplanted tumor cells was 10⁶ cells per mouse, the method of transplantation was injection of cell suspension, and the tumor formation site was subcutaneous. On the 4th day after inoculation, the tumor formation rate was 100%, and 0.2% 5-Fu (150µL/mouse) was administered intraperitoneally every day for 4 consecutive days. On the 7th day after injection, the animals whose tumor volume increased by more than 125% for 3 days (the average tumor volume was 298mm³) were enrolled in the study. The animals were randomly divided into 7 groups, named as the group shown in the table below. The ratio of the highest value to the lowest value of the target volume (V target) distribution in each group was about 200%.

The drug was administered on the day of grouping, which was recorded as the first day of treatment, and so on. The pharmaceutical solution for each group was aqueous solution, which was prepared according to the conventional method. The drug was administered once a day for 4 consecutive days. The maximum doses of 5-Fu, dimethylbiguanide hydrochloride and N,N-dimethylbiguanide were 0.4mg, 5.3 mg and 5.1 mg/time per animal, respectively. Maximum volume administered = maximum dose/drug concentration. Group T received intraperitoneal injection of 200µL/animal each time. Group C received intratumoral injection of 600µL of 0.35% N,N-dimethylbiguanide (about 2 times the tumor volume) and intraperitoneal injection of 200µL of 1.3% dimethylbiguanide hydrochloride. Other groups received intratumoral injection of the pharmaceutical solution as shown in the table below. The daily injection volume of each animal in group A, B, D, E, F and G was 410µL, 410µL, 410µL, 410µL, 25µL and 10µL, respectively. On the 15th day, the animals were euthanized and the tumor weight was measured. The tumor inhibition rate was calculated with 5-Fu as a negative control, and the results were shown in Table 6 below.

**Table 6**

| Groups | Intratumoral administered drugs | Tumor weight (g, mean ±SEM) | Tumor inhibition rate (%) |
|---|---|---|---|
| T | 0.2% 5-Fu | 1.84±0.29 | - |
| A | 1.3%R' | 1.43±0.63 | 22.3 |
| B | 1%R-HCl (pH7.4) | 1.37±0.48 | 25.5 |
| C | 0.35% | 1.17±0.44 | 36.4 |
| D | 0.5% R | 0.78±0.36 | 57.6 |
| E | 1%R | 0.53±0.35 | 71.2 |
| F | 20%R | 0.26±0.25 | 85.9 |
| G | 51%R | 0.21±0.16 | 88.6 |

In the above table, compared with R'/B (group A), R/B (groups D to G) were significant dose reduction and efficacy enhancement, as indicated by a reduction in R dosage of at least 50% compared with R' but an increase in the inhibition ratio (X) of at least 50% (e.g. Group D), or an increase in the dose-effect index of at least one active ingredient (inhibition ratio/mmol) of at least 100% compared with R'/B (e.g. Group E to G). The dose reduction and efficacy enhancement also included the improvement of adjustability indexes, such as the adjustable coefficient of local administration concentration increased to ≧ 10 or 100, the adjustable coefficient of local administration volume increased to ≧10 or 200. These results showed that R/B was a synergistic combination beyond the expectation of the prior art (R/B and R'/B were pharmaceutical equivalent combination), and even a synergistic combination of dose reduction and efficacy enhancement.

In addition, group C was a mixture of R' and R, and the total dosage of drugs in group C was higher than that in group D, but the tumor inhibition rate was lower. The mixed molar ratio of R' to R (R'/R %) in intratumorally administered drug of group A, B and E was 100%, 30.6% and 0, respectively, and their results were ineffective, ineffective and effective, respectively, which showed that R' was the contraindication of R mixture.

### Example 3.2 Extension study of B

In one experiment, IFO was used as study B, the tumor was breast cancer, containing mouse breast cancer cells (4T1), and the experimental animal was mice, and the method of tumor formation in the experimental animal was as described in Example 3.1. On the 4th day after inoculation, the tumor formation rate was 100%, and 1% IFO (150µL/mouse) was administered intraperitoneally daily for 3 consecutive days. On the 8th day after injection, the animals with tumor growth rate of more than 125% for 3 days (average tumor volume was 328mm³) were enrolled in the study and randomly divided into 3 groups, which were recorded as group A, B and C, respectively. Treatment was started on the day of grouping, which was recorded as the first day of treatment, and so on. The pharmaceutical solution given to the animals in each group was aqueous solution and did not contain additional agents (such as pH adjusting agent) required for cell culture or the safety of conventional injection, and it was prepared according to the conventional method.

Dimethylbiguanide hydrochloride and N,N-dimethylbiguanide were administered intratumorally once a day for 4 consecutive days at doses of 6.6 and 5.1 mg per animal, respectively. Administration volume = dose/drug concentration. On day 15, animals were euthanized and dissected and tumor weights were measured to calculate the tumor inhibition rate, and the results were shown in Table 7 below.

**Table 7**

| Groups | Intratumoral administration of drugs | Tumor weight (g, mean ±SEM) | Tumor inhibition rate (%) |
|---|---|---|---|
| A | 1.3%R' | 0.45±0.02 | 0 |
| B | 1%R | 0.14±0.04 | 68.9% |
| C | 51%R | 0.17±0.05 | 62.2% |

The results showed in the above table were consistent with Example 3.1, further showing that the R/B combination was a synergistic combination beyond the expectation of the prior art of dimethylbiguanide (R/B and R'/B were pharmaceutical equivalent combinations), and that B was selected from any DNA damaging agent with a cellular response as the primary antineoplastic response and was not limited to an antimetabolite as described in Example 3.1. Wherein the synergistic effect or synergy included increased efficacy as described in Example 3.1 (high efficiency) and enhanced adjustability (highly adjustable effective dosing concentration, volume, etc.).

In the following example 6-9, there were further studies of R and R' separately in combination with chemotherapeutic agents.

### Example 4. Study of R: Extension of the lesion model to non-malignant lesions

In this example, the conventional metformin -refractory lesion model was a nonmalignant lesion, which was a lesion that does not contain malignant tumor cells (let alone dimethylbiguanide-sensitive tumor cells), but instead contained connective tissue cells (fibroblasts, muscle cells, etc.) and/or organ cells (skin keratinocytes, mammary cells, thyroid cells), including, for example: organ nodules rich in organ cells (breast nodules, thyroid nodules, lung gland nodules), non-malignant tumors rich in connective tissue cells (lipomas, fibromas, etc.), connective tissue lesions rich in connective tissue cells (rheumatoid arthritis, systemic lupus erythematosus, polymyositis) and other nodules, etc. Conventional chemotherapeutic drugs, as well as conventional metformin drug, were not suitable for effective, low frequency, fast acting, and/or micro-volume interventional treatment (Y) of such lesions (X), and the latter may also be used as a negative control in this example.

### Example 4.1 Studies using nodules containing fibroblasts and their related products as a lesion model

In one experiment, the nonmalignant lesions were nodules containing fibroblasts and their related products, modeled by mouse fibroblast 3T3 cells. The experimental animals were mice, and the nodules were generated by the same method as conventional subcutaneous tumor transplantation. In brief, the volume of transplanted cells was 10⁶ cells per mouse, the method of transplantation was injection of cell suspension, and the modeling site was subcutaneous. The animals with successful modeling (average volume of 209mm³) were enrolled and randomly divided into 6 groups, which were recorded as group A, B, C, D, E, and F. Drug administration was started on the day of grouping, which was recorded as the first day of treatment, and so on. The pharmaceutical solution administered to the animals in each group was aqueous solution and did not contain additional agents (such as pH adjusting agent) required for cell culture or the safety of conventional injection, and it was prepared according to the conventional method.

The drug was administered once a day for 4 consecutive days. Dimethylbiguanide hydrochloride and N,N-dimethylbiguanide were administered at maximum doses of 6.6 and 5.1mg/time per animal, respectively. In group B, each animal received intranodal injection of 425µL of 0.25% N,N-dimethylbiguanide (about twice the tumor volume) and intraperitoneal injection of 400µL of 1.3% dimethylbiguanide hydrochloride, respectively. The other groups were intratumoral injected with the pharmaceutical solution as shown in the table below. Maximum volume administered = maximum dose/drug concentration. The daily injection volume of each animal in group A, C, D, E, and F was 510µL, 510µL, 510µL, 15µL, and 10µL, respectively. On day 15, the animals were euthanized and dissected to determine the nodule weight, and the inhibition rate of nodules was calculated using dimethylbiguanide hydrochloride as a negative control; the results were shown in Table 8 below.

**Table 8**

| Group | Intranodal administered Drug | Nodule weight (g, mean ±SEM) | Inhibition rate (%) |
|---|---|---|---|
| A | 1.3%R' | 2.01±0.40 | - |
| B | 0.25% R | 1.47±0.41 | 26.9 |
| C | 0.75% R | 0.53±0.26 | 73.6 |
| D | 1%R | 0.40±0.25 | 80.1 |
| E | 30%R | 0.33±0.25 | 83.6 |
| F | 51%R | 0.10±0.10 | 95.0 |

Unexpectedly, as shown in the above table, compared with R' (group A), the anti-nodule results of R (groups C to F) were consistent with the antineoplastic results in Examples 1 and 2, further showing that R was an active ingredient of dose reduction and efficacy enhancement beyond the expectation of the prior art (R and R' are pharmaceutical equivalents), wherein the efficacy enhancement included increased efficacy (high efficiency) and enhanced adjustability (highly adjustable effective dosing concentration, volume, etc.) as described in Examples 1, 2.

### Example 4.2 Extension to other non-cancerous lesions

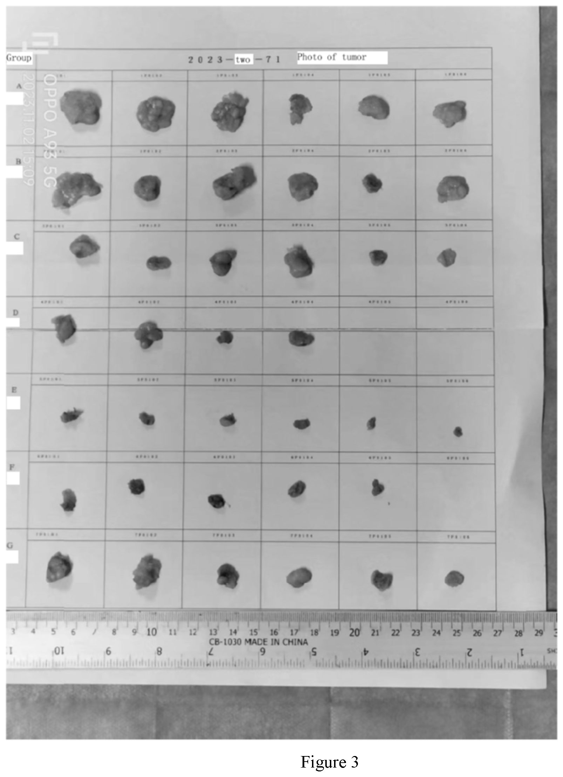

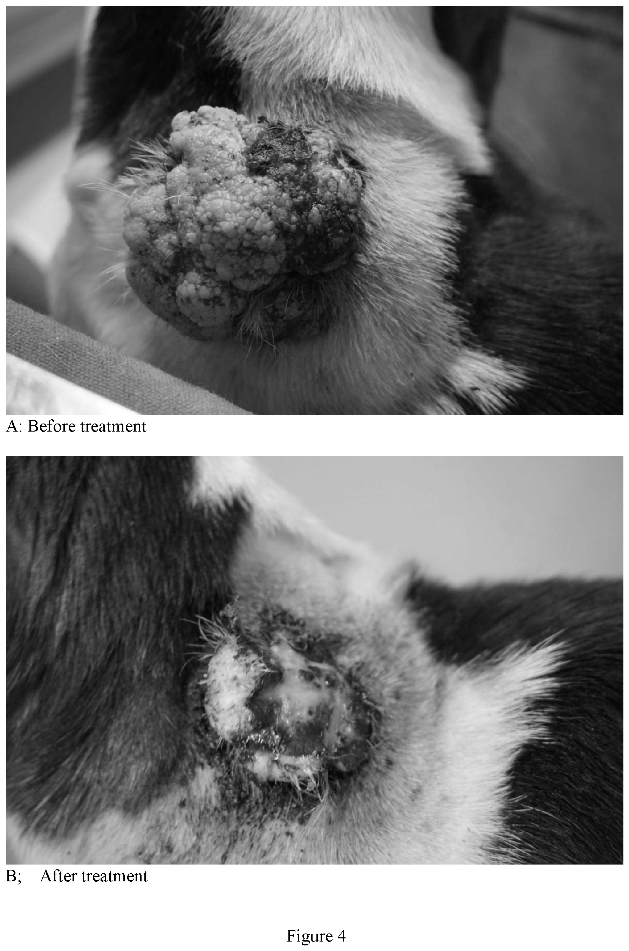

In one experiment, the nonmalignant lesion was a papillary hyperplasia (containing skin keratinocytes) determined by pathological analysis from an inoperable beagle dog (9.5 kg of body weight). The size of the hyperplasia was 54×52×26mm (Figure 4A), and the volume was approximately 73,000 mm³. The nodular hyperplasia was firm, and multiple interventions with different concentrations of pharmaceutical solution was considered. The interventional solution was aqueous solution and did not contain any additional agents (e.g., pH adjusting agents) required for cell culture or the safety of conventional injections and was prepared in a conventional method. One dose was administered on day 1, 9, and 17. The first administration was 1.5 ml of 51% of N,N - dimethylbiguanide aqueous solution, injected at 30 point (distributed as evenly as possible in the nodules, with V_{target} of about 2400 mm³per point), with an average of 50 µL per point (Vᵢₙₜₑᵣᵥₑₙₜᵢₒₙ/V_{target} was about 0.02). The second administration was 2.5mL of 30% N,N-dimethylbiguanide aqueous solution, injected at 15 points (distributed as evenly as possible in the nodules, with V_{target} of about 1800 mm³per point), with an average of 167 µL per point (Vᵢₙₜₑᵣᵥₑₙₜᵢₒₙ /V_{target} was about 0.09)_{.} The third administration was 25 mL of 1% N,N-dimethylbiguanide aqueous solution, divided into 4 points (as evenly distributed as possible in the nodules). Thirty-seven days after the first administration, the nodule volume had decreased to 23,600 mm³, a reduction of more than 68% (Figure 3B).

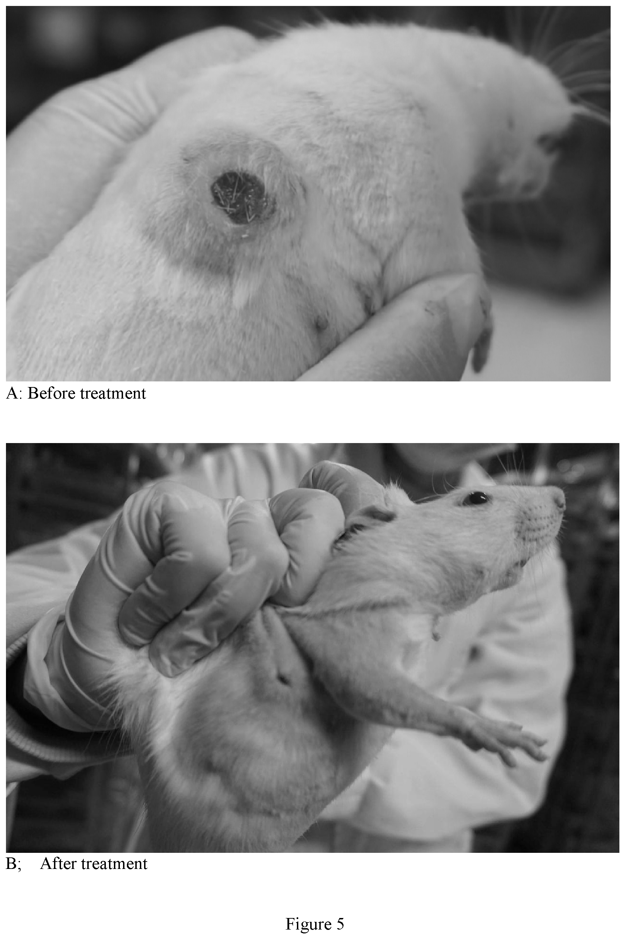

In one experiment, the nonmalignant lesion was a fibroma from a rat, with a size of 19mm long by 19mm wide (Figure 5A), and a V of approximately 3400 mm³. The interventional solution was aqueous solution and did not contain any additional agents (e.g., pH adjusting agents) required for cell culture or the safety of conventional injections and was formulated according to conventional method. One dose was administered on day 1 and day 15, respectively. The first administration was 120µL of 40% N,N-dimethylbiguanide aqueous solution, which was injected at 4 points (as evenly distributed as possible in the fibroma, and the V_{target} of each point was about 850 mm³), with an average of 30µL per point (Vᵢₙₜₑᵣᵥₑₙₜᵢₒₙ /V_{target} was about 0.04). The second administration was 120µL of 40% of N,N - dimethylbiguanide aqueous solution, which was injected at 4 points ( as evenly distributed as possible in the fibroma, and the V_{target} of each point was about 500 mm³), with an average of 30µL per point (Vᵢₙₜₑᵣᵥₑₙₜᵢₒₙ /V_{target} was about 0.06). Thirty-two days after the first administration, the fibroma had disappeared (Figure 5B).

### Example 5 Comparative study of systemically administered drugs

In one experiment, 36 mice were randomly divided into 6 groups (6 mice in each group), including 2 groups of tail vein injection (group A1 and B1), 2 groups of intraperitoneal injection (group A2 and B2), and 2 groups of right lower limb intramuscular injection (group A3 and B3). The two injections were 0.65% dimethylbiguanide hydrochloride aqueous solution (injection A) and 0.5% N,N-dimethylbiguanide aqueous solution (injection B), which were prepared according to the conventional method. Injection A (400µL/animal) and B (400µL/animal) were via tail vein injection, intraperitoneal injection, and intramuscular injection into the right lower limb of the mice in the corresponding groups A and B, respectively.

One hour after injection, no obvious vascular injury was observed in group A1 (0/6) but significant vascular injury was observed in group B1 (6/6), and no muscle injury was observed in group A3 (0/6) but some muscle injury was observed in group B3 (6/6). On the next day, death was not observed in group A2 (0/6) but was observed in group B2 (3/6). These results indicated that dimethylbiguanide hydrochloride aqueous solution could be used for systemic administration, but dimethylbiguanide hydrochloride aqueous solution with a concentration of ≧0.5% could not be used for systemic administration.

Illustration of the results of Examples 1-5:
1. R could only be used for local and not systemic administration, which greatly limits its application (for lesions that were administered locally). However:
2. Compared with R', the local administration of R was a dose reduction and enhancement of the antineoplastic effect, which showed that the dosage of R was reduced by at least 50% compared with R' but at least one effect (X) was increased by more than 50% (group B), or at least one dose-effect index (X/mmol) of R was increased by at least 100% compared with R' (groups B and C). The effect (X) included an efficacy index and an adjustability index. The improvement of the efficacy index such as the inhibition rate increased by more than 50%, the improvement of the adjustability index such as the local drug concentration adjustable coefficient increased to ≧10 or 50 or 100, the local pharmaceutical target volume adjustable coefficient increased to ≧100, the local pharmaceutical volume adjustable coefficient increased to ≧10 or 200. These results showed that R was an active ingredient of dose reduction and efficacy enhancement that exceeded the expectation of the prior art (R and R' were pharmaceutical equivalents); and
3. Compared with R', the dose reduction and efficacy enhancement of R was shown in multiple refractory disease models of the latter, which not only in the degree of efficacy but also in the nature of efficacy exceeded the expectation of the prior art (the pharmacological equivalents R and R' had the same antineoplastic specificity), and showed completely different pharmacology (the transient local action of R vs the cytotoxic antineoplastic effect of R'); and
4. Comparison of local administration of R and R', pathological analysis also showed that R produced expanded tumor cell intercellular space and more infiltration of immune cells (such as T cells, macrophages, etc.). The intercellular space expansion led to decrease of the osmotic resistance, and the more infiltration of immune cells showed increase of the permeability of tumor microenvironment, and immune cells could secrete a variety of cytokines and enzymes, further damaging the close connection between cells and extracellular matrix. These pharmaceutical effects obviously weaken the barrier function of tumor tissue, providing a new sharing mode for the drug constrained by this function. The coadministration (R/B) of locally administered R and cell-reactive antineoplastic agents B showed a coadministration of dose reduction and efficacy enhancement that exceeded the expectations of the prior art (pharmaceutical equivalent R'/B and its antineoplastic specificity). Its higher inhibitory rate also reflected the direct damage of tissues and cells, and thus pathological related antigen, the danger signal molecules (DAMPs), and inflammatory signals were induced to release, which provided favorable conditions for its coadministration with immune regulator (E). These are further confirmed by the following coadministration examples.

### Example 6 Study of R and B pharmaceutical combination: B model drug being DNA damaging agent

In this example, the representative medication of cell reactivie antineoplastic agents (B) was selected from DNA inhabitors approved by National Medical Products Administration, which was the DNA damaging agents, its representative drug was one or more of the following: cisplatin (a commonly used drug for metal complex), ifosfamide (a commonly used drug for alkylating agent), and doxorubicin (a commonly used drug for antineoplastic antibiotics).

In this example, solid tumors were selected from tumors that could be administrated intratumorally, including tumors that were sensitive to DNA damaging agents and refractory tumors. DNA damaging agent refractory tumors were tumors that were not used for treatment options, especially the tumors that were not treated in the first and second line scheme, including invalid tumor and ineffective tumors of B. Nonrespond tumors including, for example, tumors that were resistant to DNA-damaging agents; Invalid tumors including, such as DNA damaging agents resistant tumors; ineffective tumors including such as conventional glioma, liver cancer, pancreatic cancer, stomach cancer, melanoma, colorectal cancer, they contained tumor cells that were not sensitive to DNA damaging agents, and these cells had efficient DNA repair mechanisms, active antioxidant enzyme system, or strong efflux ability of drug and the like which were resistance to DNA damaging mechanism.

### Example 6.1 Study of R and representative drug IFO of B pharmaceutical combination

In one trial (a horizontal collaboration project with West China Hospital, Sichuan University), IFO was used as B, and the solid tumors were osimertinib-resistant tumors, which were proved to be sensitive to IFO. Experimental animals were nude mice, tumor formation method was conventional subcutaneous transplantation of patient derived xenograft (PDX). Briefly, the model tissue was derived from tissue of tyrosine kinase inhibitors (TKIs) resistant lung cancer, the transplantation volume was 1 particle (diameter was about 1 mm) per animal, and the transplantation of the tissue particle was injected with an implantation needle, and the modelling site was subcutaneous of animals. The animals with successful modeling (an average tumor volume of 323 mm³) were enrolled, and were randomly divided into 4 groups (with 5 animals in each group) as shown in the following table, the ratio of highest value to the lowest value of target volume (V targe) distribution is > 400%. Drug administration was started on the day of grouping, which was recorded as the first day of treatment, and so on. The administered pharmaceutical solution shown in the table below was prepared according to the routine method. The N,N-dimethylbiguanide drug solution does not contain any additives, including pH regulator which could convert it to dimethylbiguanide salt, as it could no longer be used for routine administration.

The administration method was intratumor injection, and the drugs and concentrations were shown in the table below, and the administration was once a day with 3 times consecutively, and the volume (V _{dose}) was 100 µL/time/only_{.}

The tumor volume was measured daily, and the day when the relative tumor proliferation rate (T/C %) < 42% observed was the date of effective time, and the day when relative tumor volume (RTV) > 150% from day 1 to the end of the observation was the date of progression-free time. The animals were enthanized at day 9, tumors were taken and tumor weight was measured. The inhibitory rate was calculated, and the result was shown in table 9.

**Table 9**

| Gro ups | Pharmaceutical composition | Effective time (day) | Progression-free time (day) | Tumor weight (g, average + / - SEM) | Tumor inhibition rate (%) |
|---|---|---|---|---|---|
| A | Normal saline | - | - | 2.04±0.80 | - |
| B | 0.5% IFO | 6 | 7 | 0.83±0.46 | 59.3 |
| C | 5%R | 4 | > 9 | 0.34±0.15 | 83.3 |
| D | 5% R0.5% IFO | 4 | > 9 | 0.00 | 100 |

In the above table, compared with the single agent control group (group B and group C), the actual tumor inhibition rate k₁ (100%) of group D was higher than the expected tumor inhibition rate k₂ of HSA method and Bliss method, which were max (group B, group D) =83.3% and ≦E_{B}+Ec-E_{B}×E_{C}=93.2%, respectively. Its actual progression-free time k₁ (> 9) was greater than the expected k₂ of HSA method and Bliss method, respectively, which was (Max (R', B) = 9 and 0, shown as further high efficiency and further prolonged efficiency.

In the following trials and experiments of following examples, unless otherwise stated, the number of administrations of said R was reduced to 2 or 1, to better study its combination with Z.

In one trial, IFO was used as B, and the representative of refractory tumor of B was an IFO-insensitive tumor (pancreatic cancer). The experimental animals were nude mice, and the tumor formation method was the conventional subcutaneous tumor transplantation method. In brief, IFO insensitive cells (human pancreatic cancer cell line PANC-1) were transplanted at a volume of 2×10⁶ cells per mouse by injection of cell suspension. The successful models were enrolled (the average tumor volume was 201mm³) and randomly divided into 17 groups (6 mice in each group), which were recorded as the groups showed in the table below. The ratio of the highest to the lowest value of the target volume (Vtarget) distribution in each group was about 200%. Drug administration was started on the day of grouping, which was recorded as the first day of treatment, and so on. Each group was administrated with the pharmaceutical solution showed in the table below, which was the intratumoral pharmaceutical solution I and the intraperitoneal pharmaceutical solution II showed in the table below, respectively. The pharmaceutical solution was prepared according to routine method. The solution containing N, N-dimethylbiguanide did not contain any additive, that is, did not contain a pH regulator that would convert it to dimethylbiguanide salts, since it is no longer available for routine administration.

Group A, E and Q received intratumoral administration of 200µL each time on days 1, 3, 5 and 7. Group B and F received intratumoral administration of 400µL each time on days 1, 3, and 5. Group C and H received intratumoral administration twice, on the 1st and 3rd day, 10µL each time. Group D received intratumoral administration and intraperitoneal administration, with intratumoral administration twice on the 1st and 3rd day, 10µL each time; and intraperitoneal administration (200µL/time) four times on the 5th, 7th, 9th and 11th day. Group G and P received intratumoral administration twice, 100µL each time, on the 1st and 3rd day. Group I received intratumoral administration and intraperitoneal administration. The intratumoral administration was the same as group A, intraperitoneal administration was given 5 times at day 1, 3, 5, 7, 9, with 200µL each time. Group J and group K received intratumoral administration and intraperitoneal administration. The intratumoral administration was the same as group B and group C, respectively, and intraperitoneal administration was given 4 times at day 1, 3, 5, 7, with 200µL each time. Group L received intratumoral administration and intraperitoneal administration. The intratumoral administration was the same as group E; and intraperitoneal administration was given twice on the 4th and 6th day, with 200µL each time. Group M received intratumoral administration and intraperitoneal administration. The intratumoral administration was the same as group F; and intraperitoneal administration was given 3 times on day 7, 9 and 11. Group N received intratumoral administration and intraperitoneal administration. The intratumoral administration was the same as group G; and intraperitoneal administration was given 4 times at day 5, 7, 9, 11, with 200µL each time. Group O received intratumoral administration and intraperitoneal administration, and intratumoral administration was given twice on the 1st and 3rd day, with 10µL each time. Intraperitoneal administration was given twice, with 200µL each time on the 5th and 7th day.

The tumor volume was measured at two-day intervals, the day when the relative tumor proliferation rate (T/C %) < 42% observed was the date of effective time. The day when the relative tumor volume (RTV) > 150% from day 1 to the end of the observation was the date of progress free time. The animals were euthanized on day 15. Tumors were taken and tumor weight was measured. Inhibitory rate was calculated and the result was shown in table 10.

**Table 10**

| Gro ups | Drug, administration route | | Dosage (Q) | | Efficacy (X) | | |
|---|---|---|---|---|---|---|---|
| | Pharmac eutical solution I for | Pharmace utical solution II for | Q_{R} or Q_{R'} | QB | Effecti ve time (days) | Progressi on-free time (days) | Tumor inhibition rate |
| | intratum oral administr ation | intraperito neal administra tion | | | | | (%) |
| A | 2.5% R' | - | 0.12 | | > 15 | 3 | 9.4 |
| B | 0.2%R | - | 0.02 | | 5 | 5 | 37.1 |
| C | 50% R | - | 0.08 | | 5 | 13 | 62.7 |
| D | 5.0% IFO | 0.5% IFO | | 5.00 | > 15 | 3 | 21.4 |
| E | 2.5% R ' 0.5% IFO | - | 0.12 | 4.00 | > 15 | 3 | 25.1 |
| F | 0.2%R 0.1% IFO | - | 0.02 | 1.20 | 5 | 9 | 54.7 |
| G | 5% R 0.5% IFO | - | 0.08 | 1.00 | 3 | > 15 | 85.4 |
| H | 45% R 10%IFO | - | 0.07 | 2.00 | 3 | > 15 | 81.3 |
| I | 2.5% R' | 0.5% IFO | 0.12 | 5.00 | > 15 | 3 | 29.1 |
| J | 0.2% R | 0.5% IFO | 0.02 | 4.00 | 5 | 9 | 61.3 |
| K | 50%R | 0.5% IFO | 0.08 | 4.00 | 5 | > 15 | 87.5 |
| L | 2.5% R' 0.375% IFO | 0.5% IFO | 0.12 | 5.00 | > 15 | 5 | 31.8 |
| M | 0.2% R 0.05% IFO | 0.5% IFO | 0.02 | 3.60 | 5 | 13 | 79.2 |
| N | 5% R 0.5% IFO | 0.5% IFO | 0.08 | 5.00 | 5 | > 15 | 95.7 |
| ○ | 45% R 10%IFO | 0.5% IFO | 0.07 | 4.00 | 3 | > 15 | 91.6 |
| P | 3.35% R2.15% R' 0.5% IFO | - | 0.08 | 1.00 | 7 | 9 | 61.7 |
| Q | normal saline | - | 0 | 0 | - | - | - |

| | | | | | | | |
|---|---|---|---|---|---|---|---|
| *: Unit of Q_{R} or Q_{R} was mmol, and the unit of Q_{B} was mg | | | | | | | |

### 1. Single agent research:

The single-agent R results as shown in the above table were consistent with Examples 1, 2, and 4. For example, compared with R' (group A), R (group B, C) was an obvious dose reduction and efficiency enhancement, manifested as the dose being reduced at least 75%, but at least one effect (X) increased by more than 50% (group B). Wherein effect (X) including efficacy coefficient and the adjustability coefficient, with efficacy improving, such as efficiency time shortened by more than 50%, inhibition rate increased more than 50%, progression-free time prolonged more than 50%, adjustability coefficient improving, such as the adjustability concentration coefficient increased to ≧ 100 or 200, adjustable dosing volume coefficient increased to ≧ 100 or 200. These results showed that R again was the active ingredient of dose reduction and efficiency enhancement beyond the expectation of prior art (R and R' were pharmaceutical equivalents).

The single-agent representative group (Group D) of the antineoplastic drug B (Group B) aligned with the expectation in B-resistant tumors, demonstrating limited efficacy as indicated by a tumor inhibition rate of 21.4%, or Q_{B} < Q_{B0} (minimum effective dose). According to well known knowledge of the art, the efficacy of drug B was dose-dependent. Therefore, it was reasonable to infer that single agent groups receiving doses lower than Q_{B} would not exhibit higher tumor inhibition rates.

### 2. Regular comparison of combinations:

In the above table, the results of R and B combination were consistent with Example 3. For example, compared R/B (Group F to H, J to K, M to O) with R'/B (Group E, I, L), the dose of R/B was obvious reduced, and efficacy was enhanced, manifested as, such as the dosage of R reducing to at least 75% of R' and dosage of B reducing by 20%, but at least one effect (X) increased by more than 50% (Group F, J, M). The effect (X) including efficacy coefficient and the adjustability coefficient, with efficacy improving such as efficacy time shortened by more than 50%, inhibition rate increased more than 50%, progression-free time prolonged more than 50%, adjustability coefficient improving, such as R adjustability concentration coefficient increased to ≧ 100 or 200, R adjustability concentration volume ratio coefficient increased to ≧ 100 or 200. These results again showed that R/B was synergistic combination, even for combination of dose reduction and efficiency enhancement that beyond the expectation of prior art (R/B and R'/B were pharmaceutical equivalents) .

### 3. Further comparison of combination:

The results as shown in the table were consistent with example 3. For example, in comparision R/B (Group F to H, J to K, M to O) with Single agent R group (group B) and single agent B group (group E), R/B was dose reduction and efficacy enhancement, manifested as, such as compared with single agent B, dosage of B reduced at least 10% but at least one effect (X) increased by more than 10%. Wherein the effect (X) including efficacy coefficient and the adjustability coefficient, with efficacy improving such as efficacy time shortened by more than 10%, inhibition rate increased more than 10%, progression-free time prolonged more than 10%, adjustability coefficient improving, such as B adjustability concentration coefficient increased at least 10% or 20%, B administration volume ratio increased at least 10% or 20%.

These results exceeded the expectations (k2) of the HSA or Bliss method of combination versus component comparison, respectively, even though the R was already the active ingredient exceeded the expectation of prior art of dimethylbiguanide (R and R' were pharmaceutical equivalents). Taking group F as an example, the actual tumor inhibition rate k₁ (54.7%) was higher than the expected value of HSA method k2 = max (group B, group D) = 37.1%, and the expected value of Bliss method k₂ < E_{B}+E_{D}-E_{B}×E_{D} = 50.6%. The actual progression-free time k₁ (9 days) was longer than the expected value k₂ by HSA method and Bliss method, max (group B, group D) = 5 days and E_{B}+E_{D}-E_{B}×E_{D} < 9 days, respectively. These results again suggested that R/B may be a further synergistic combination beyond the expectation (HSA or Bliss method), or even a further synergistic combination for dose reduction and efficacy enhancement.

In addition, the mixed Molar ratio of R and R' (R'/R%) of intratumoral drug in Group E, P, G were 100%, 30.6%, 0, respectively, the results of combination with B were 25.1%, 61.7%, and 85.4% respectively, which showed R' was a contraindication for mixture with R.

### Example 6.1 Study of R and its pharmaceutical combination with B: Extension of B to DNA damaging agents

In one study, B was DOX, and refractory tumor to B was DOX-resistant breast cancer. The experimental animals were nude mice. The tumor formation method was the conventional subcutaneous tumor transplantation method. In brief, drug-resistant cells (human breast cancer cell line MCF-7/DOX) were transplanted at a dose of 2×10⁶ cells per mouse by injection of cell suspension, and the modeling site was subcutaneous. The animals with successful tumor modeling were enrolled (the average tumor volume was 181mm³) and randomly divided into 18 groups (6 animals in each group), which were recorded as the groups as shown in the table below. The ratio of the highest value to the lowest value in the distribution of target volume (v target) in each group was about 200%. Administration was started on the day of grouping, which was recorded as day 1 of treatment, and so on. Each group was administered with the pharmaceutical aqueous solution as shown in the table below, which was the intratumoral pharmaceutical solution I and the intraperitoneal pharmaceutical solution II as shown in the table below, respectively, which were prepared according to the conventional method. The liquid containing N,N-dimethylbiguanide did not contain any additives, including a pH regulator that converted it to dimethylbiguanide salts, since it was no longer available for routine administration.

Group A, F and R received intratumoral administration of 200 µL each time on day 1, 3, 5 and 7. Group B, G and H received intratumoral administration of 400µL each time on day 1, 3 and 7. Group C and I received intratumoral administration twice on the 1st and 3rd day, 100 µL each time. Group D and J received intratumoral administration twice, on the 1st and 3rd day, 10µL each time. Group E received intratumoral administration and intraperitoneal administration, intratumoral administration was performed twice on the 1st and 3rd day with 10µL each time; intraperitoneal administration was performed four times (200µL /time) on day 5, 7, 9 and 11. Group K received intratumoral administration and intraperitoneal administration, the intratumoral administration was the same as group A, intraperitoneal administration was perfomed 6 times at day 1, 3, 5, 7, 9, 11 with 200 µL each time. Grous L, M and N received intratumoral or intraperitoneal administration. Intratumoral administration was the same as group B, C and D, respectively, and intraperitoneal administration was performed 4 times at day 1, 3, 5 and 7, with 200 µL each time. Group O received intratumoral administration and intraperitoneal administration. The intratumoral administration was the same as group F, and intraperitoneal administration was performed twice on the 9th and 11th day, with 200 µL each time. Group P and Q received intratumoral administration and intraperitoneal administration, the intratumoral administration was the same as group H and J, respectively. The intraperitoneal administration was performed twice on day 7 and 9, with 200 µL each time.

Tumor volume was measured every 2 days. The day when the relative tumor proliferation rate (T/C %) was less than 42% was recorded as the effective time, and the day when the relative tumor volume (RTV) was observed to increase to more than 150% from day 1 until the end point was the progression-free time. The animals were euthnized at day 15. The tumor was dissected, and the tumor weight was determined. The tumor inhibition rate was calculated. Results were shown in Table 11.

**Table 11**

| Gro up | Phamaceuticals, method of administration | | dosage | | Efficacy (X) | | |
|---|---|---|---|---|---|---|---|
| | Pharmaceutical solution I for intratumoral administration | Pharmaceutical solution II for intraperitoneal administration | Q_{R} or Q_{R'} | Q_{B} | Effectiv e time (days) | Progr ession -free time (days) | Tum or inhi bitio n rate ( %) |
| A | 2.5%R' | - | 0.12 | | > 15 | 3 | 8.3 |
| B | 0.2% R | - | 0.02 | | 5 | 5 | 33.1 |
| C | 5%R | - | 0.08 | | 5 | 13 | 62.3 |
| D | 50%R | - | 0.08 | | 5 | 13 | 61.9 |
| E | 0.5% DOX | 0.025% DOX | - | 0.30 | > 15 | 3 | 16.1 |
| F | 2.5%R ' 0.03% DOX | - | 0.12 | 0.24 | > 15 | 3 | 17.5 |
| G | 0.1% R 0.0115% DOX | - | 0.01 | 0.14 | 5 | 7 | 35.9 |
| H | 0.2% R 0.0115% DOX | - | 0.02 | 0.14 | 5 | 9 | 45.3 |
| I | 5%R 0.05% DOX | - | 0.08 | 0.10 | 3 | > 15 | 83.1 |
| J | 50%R 0.5% DOX | - | 0.08 | 0.10 | 3 | > 15 | 81.7 |
| K | 2.5%R ' | 0.025% DOX | 0.12 | 0.30 | > 15 | 3 | 29.3 |
| L | 0.2% R | 0.025% DOX | 0.02 | 0.20 | 5 | 9 | 55.8 |
| M | 5%R | 0.025% DOX | 0.08 | 0.20 | 5 | > 15 | 87.2 |
| N | 50%R | 0.025% DOX | 0.08 | 0.20 | 5 | > 15 | 88.3 |
| O | 2.5%R ' 0.025% DOX | 0.025% DOX | 0.12 | 0.30 | > 15 | 5 | 31.4 |
| P | 0.2% R 0.0115% DOX | 0.025% DOX | 0.02 | 0.24 | 5 | > 15 | 86.3 |
| Q | 50%R 0.5% DOX | 0.025% DOX | 0.08 | 0.20 | 5 | > 15 | 96.4 |
| R | Normal saline | - | 0 | 0 | - | - | - |

| | | | | | | | |
|---|---|---|---|---|---|---|---|
| * : The unit of Q_{R} or Q_{R'}was mmol and the unit of Q_{B} was mg | | | | | | | |

Except for group B, various concentrations of Single agent R groups were effective, indicating that the effective drug concentration threshold of Single agent R was > 0.2%. Except for goup G, various concentration of R in R/B combination groups were effective, indicating the effective share concentration threshold of R was > 0.1% and ≦ 0.2%.

In comparison R (Group B, C, D) with R' (Group A), as well as R/B combinations (Group G to J, L to N, P to Q) with R'/B combinations (Group F, K, O), and in comparison with single agents (Group B, C, D, E), the results presented in the aforementioned table were consistent with those in Examples 1, 2, 3, 4, and 6.1, further demonstrating that R was an active ingredient of dose reduction and efficacy enhancement that exceeded the expectations of prior art (R and R' were pharmaceutical equivalents), that R/B was a synergistic combination exceeded the expectations of prior art of dimethylbiguanide (R/B and R'/B were pharmaceutical equivalent combinations), and further synergistic combinations exceeded the expectations of the single agents of R and B (as determined by the HSA or Bliss methods). Moreover, B was selected from any drug whose primary antineoplastic response was cellular responses, without limitation to specific DNA-damaging agents. The dose-reducing and efficacy-enhancing or synergistic effects included dose reduction and efficacy enhancement (rapid, high, and/or long-lasting efficacy) and increased adjustability (highly adjustable effective administration concentrations, volumes, etc.), as described in Example 6.1.

Taking Group H as an example, its actual tumor inhibition rate k₁ (45.3%) exceeded the expected value k₂ = max (Group B, Group E) = 33.1% HSA method, and the expected value k₂ < E_{B} + E_{E} - E_{B} × E_{E} = 44.8% of Bliss method; its actual progression-free time k₁ (9 days) exceeded the expected values k2 of both the HSA and Bliss methods, which were max (Group B, Group E) = 5 days and E_{B} + E_{E} - E_{B} × E_{E} < 8 days, respectively.

In one experiment, B was DDP, the representative tumor of refractory tumor to B was liver cancer containing tumor cells that were not sensitive to DDP and the micro environment was further desensitized. The experimental animals were nude mice, and the tumor formation method was conventional subcutaneous transplantation tumor. In short, the 1:1 mixture of mouse liver cancer cells (mouse liver tumor H22 cell lines) and mouse fibroblast was transplanted with 2 x 10⁶ cells/mice. The transplantation method was cell suspension injection, and the modeling site was subcutaneous tissue of animals. Animals with successful modeling (average tumor volume of 193 mm³) were enrolled and randomly divided into 17 groups (6 in each group) as recorded in groups of the following table. The ratio of the highest and the lowest value of the target volume (Vtarget) distribution was about 200%. The administration began on the day of grouping, and recorded as the first day of treatment, and so on. Each group was administrated with the pharmaceutical solution as shown in the table below, which was the intratumoral pharmaceutical solution I and the intraperitoneal pharmaceutical solution II as shown in the table below, respectively. The pharmaceutical solution was prepared by conventional method. The pharmaceutical solution containing N, N-dimethylbiguanide did not contain any additives, including a pH regulator which converted it to dimethylbiguanide, as it was no longer used for conventional administration.

Group A, F, R received intratumoral administration four times on day 1, 3, 5, 7, with 200 µl/time. Group B and G received intratumoral administration 3 times on day 1, 3, 5, with 400 µL/time. Group C, H received intratumoral administration 2 times on day 1, 3, with 300 µL/time. Group D, I received intratumoral administration 2 times on day 1, 3, with 10 µL/time. Group E received intratumoral administration and intraperitoneal administration, the intratumoral administration was twice on the 1st and 3rd day, 200 µL each time; and the intraperitoneal administration was four times on day 5, 7, 9, 11, 200 µL /time. Group J received intratumoral administration and intraperitoneal administration, the intratumoral administration was the same as group A, and the intraperitoneal administration was 6 times on day 1, 3, 5, 7, 9, 11, 200 µL /time. Group K, L, M received intratumoral administration and intraperitoneal administration, the intratumoral administration was the same as group B, C, D respectively, and intraperitoneal administration was four times on day 1, 3, 5, 7, 200 µL/time. Group N received intratumoral administration and intraperitoneal administration, the intratumoral administration was the same as group F, and intraperitoneal administration was twice on day 9, 11, 200 µL /time. Group O received intratumoral administration and intraperitoneal administration, the intratumoral administration was the same as group G, and intraperitoneal administration was once on day 9, 200 µL /time. Group P and Q received intratumoral administration and intraperitoneal administration, the intratumoral administration was the same as group H, I respectively, and intraperitoneal administration was twice on day 5, 7, 200 µL /time.

Tumor volume was measured every 2 days. The day when the relative tumor proliferation rate (T/C %) was less than 42% was recorded as the effective time, and the day when the relative tumor volume (RTV) was observed to increase more than 150% from day 1 to the end point was progression-free time. The tumor was dissected and the tumor weight was determined and the tumor inhibition rate was calculated. The results were shown in Table 12.

**Table 12**

| Gro up | pharmacutical, method of administration | | dosage | | Efficacy (X) | | |
|---|---|---|---|---|---|---|---|
| | Pharmace utical solution I for intratumor al administra tion | Pharmaceutical solution II for intraperitoneal administration | Q_{R} or Q_{R'} | Q_{B} | effecti ve time (days) | Progr ession -free time (days) | Tumor inhibiti on rate (%) |
| A | 2.5% R' | - | 0.12 | | > 15 | 3 | 15.4 |
| B | 0.2%R | - | 0.02 | | 5 | 5 | 36.2 |
| C | 0.5% R | | 0.02 | | 5 | 5 | 40.5 |
| D | 50%R | - | 0.08 | | 5 | 15 | 59.3 |
| E | 0.015% DDP | 0.015% DDP | | 0.18 | > 15 | 3 | 25.7 |
| F | 2.5% R' 0.015% DDP | - | 0.12 | 0.12 | > 15 | 3 | 29.3 |
| G | 0.2% R 0.0075% DDP | - | 0.02 | 0.09 | 5 | 9 | 53.9 |
| H | 0.5% R 0.013% DDP | - | 0.02 | 0.08 | 5 | 13 | 63.7 |
| I | 50%R 0.4% DDP | - | 0.08 | 0.08 | 5 | > 15 | 85.7 |
| J | 2.5% R' | 0.015% DDP | 0.12 | 0.18 | > 15 | 5 | 29.5 |
| K | 0.2% R | 0.015% DDP | 0.02 | 0.12 | 5 | 11 | 57.4 |
| L | 0.5% R | 0.015% DDP | 0.02 | 0.12 | 5 | 13 | 66.3 |
| M | 50%R | 0.015% DDP | 0.08 | 0.12 | 5 | > 15 | 87.4 |
| N | 2.5% R '0.015% DDP | 0.015% DDP | 0.12 | 0.18 | > 15 | 3 | 33.7 |
| O | 0.2%R0.0 075% DDP | 0.015% DDP | 0.02 | 0.12 | 5 | 13 | 70.6 |
| P | 0.5% R 0.013% DDP | 0.015% DDP | 0.02 | 0.14 | 5 | > 15 | 86 .3 |
| Q | 50%R 0.4% DDP | 0.015% DDP | 0.08 | 0.14 | 5 | > 15 | 93 .7 |
| R | Normal saline | - | 0 | 0 | - | - | - |

| | | | | | | | |
|---|---|---|---|---|---|---|---|
| * : the unit of Q_{R} or Q_{R'}was mmol, the unit of Q_{B} was mg | | | | | | | |

Comparison of R (group B, C, D) with R' (group A), comparison of R/B combination (group G to I, K to M, O to Q) with R'/B combination (group F, J, N), and comparison with single agents (group B, C, D, E), the results shown in the above table were consistent with the corresponding results in Examples 1, 2, 3, 4, and 6.1. This further indicated that R was an active ingredient of dosage reduction and efficacy enhancement beyond the expectations of the prior art (R and R' were pharmaceutically equivalent), R/B was a synergistic combination beyond the expectations of the prior art of dimethylbiguanide (R/B and R'/B are pharmaceutically equivalent combinations), and further a synergistic combination beyond the expectations based on the single agents of R and B (by HSA method or Bliss method). Moreover, B was selected from any drug with a cellular response as the main antineoplastic response, not limited to specific DNA damaging agent types. The dosage reduction and efficacy improvement or synergism included the reduction and efficacy improvement (rapid, efficient, and/or long-lasting) and adjustability ability improvement (highly adjustable effective administration concentration, volume, etc.) as described in Example 6.1.

### Example 7. Study of pharmaceutical combination of R and B: model B drugs were extended to antimetabolites

In the following experiments, the representative drugs of the antimetabolite drug (B) were selected from the antimetabolite drugs approved by the pharmaceutical regulatory authorities, namely fluorouracil and gemcitabine. The B-refractory tumors were selected from the refractory tumors of antimetabolite drug (B), that is, the tumors that were usually not used as treatment plans, especially the first-line and second-line plans, including the ineffective and failed tumors of B. The ineffective tumors of antimetabolite drugs include, for example, conventional non-small cell lung cancer, pancreatic cancer, melanoma, and the like, which contained tumor cells that were insensitive to antimetabolite drugs, and these cells had strong drug metabolism ability, active DNA repair mechanism, etc. The failed tumors of antimetabolite drugs included, for example, its resistant tumors. Many cell experiment studies had shown that increasing the concentration of B in direct contact with these cells, or damaging their cell membranes, was conducive to improving the efficacy of the drug.

### Example 7.1 Study of pharmaceutical combination of R and the representative drug 5-Fu of the antimetabolite drugs

In one experiment, the study used 5-Fu as B, and the representative tumor for 5-Fu-resistant tumors was the 5-Fu-resistant tumor. The experimental animals were nude mice, and the tumor formation method was the conventional subcutaneous transplantation method. In brief, the transplantation volume of IFO-insensitive cells (human colorectal cancer cell Fu-resistant strain sw620/Fu) was 1.5×10⁶ cells per mouse, and the transplantation method was cell suspension injection, with the modeling site being the subcutaneous of the animals. The animals with successful modeling were included (the average tumor volume was 227 mm³) and randomly divided into 17 groups (6 mice per group), as shown in the following table. The ratio of the highest value to the lowest value in the distribution of target volume (Vtarget) in each group was approximately 200%. On the day of grouping, the drug was administered, which was recorded as the first day of treatment, and so on. Each group was administered the pharmaceutical solution as shown in the following table, which was either the intratumoral pharmaceutical solution I or the intraperitoneal pharmaceutical solution II, all prepared according to conventional methods. The pharmaceutical solution containing N, N-dimethylbiguanide did not contain any additives, including a pH regulator that could convert it to a dimethylbiguanide salt, and thus could no longer be used for conventional administration.

Group A, F, and Q were intratumoral administration 4 times, on day 1, 3, 5, and 7, 200 µLper time. Group B, G received intratumoral administration 3 times, on day 1, 3, and 5, 400 µLper time. Group C, H received intratumoral administration 2 times, on day 1 and 3, 100 µL per time. Group D, I received intratumoral administration 2 times, on day 1 and 3, 10 µLper time. Group E was intratumoral administration 2 times and intraperitoneal administration 4 times, the intratumoral administration on day 1 and 3, 200 µLper time; the intraperitoneal administration on day 5, 7, 9, and 11, 200 µLper time. Group J was intratumoral administration and intraperitoneal administration, the intratumoral administration was the same as Group A, the intraperitoneal administration was 6 times on day 1, 3, 5, 7, 9, and 11, 200 µLper time. Groups K and L received intratumoral administration and intraperitoneal administration, the intratumoral administration was the same as Group B and D, the intraperitoneal administration was 4 times on day 1, 3, 5, and 7, 200 µLper time. Group M was intratumoral administration and intraperitoneal administration, the intratumoral administration was the same as Group F, the intraperitoneal administration was 2 times on day 9 and 11, 200 µLper time. Group N was intratumoral administration and intraperitoneal administration, the intratumoral administration was the same as Group G, the intraperitoneal administration was 2 times on day 7 and 9, 200 µLper time. Group O and P received intratumoral administration and intraperitoneal administration, the intratumoral administration was the same as Group H and I, the intraperitoneal administration was 3 times on day 5, 7, and 9, 200 µLper time.

The tumor volume was measured every two days. The day when the relative tumor proliferation rate (T/C%) was less than 42% was recorded as the effective time. The observation day when the relative tumor volume (RTV) continued to increase from the 1st day until it exceeded 150% was recorded as the progression free time. On the 15th day, the animals were euthanized, the tumor was dissected, the tumor weight was measured, and the tumor inhibition rate was calculated. The results were shown in Table 13.

**Table 13**

| Gro up | Drug, administration route | | Dosage | | Efficacy (X) | | |
|---|---|---|---|---|---|---|---|
| | Pharmaceutical solution I for intratumoral administration | Pharmaceuti cal solution II for intraperiton eal administrati on | Q_{R} or Q_{R'} | Q_{B} | Effectiv e time (days) | Progre ssionfree time (day) | Tumor inhibiti on rate (%) |
| A | 2.5% R' | - | 0.12 | | > 15 | 3 | 11.3 |
| B | 0.2%R | - | 0.02 | | 5 | 5 | 36.8 |
| C | 5% R | - | 0.08 | | 5 | 11 | 61.7 |
| D | 50%R | - | 0.08 | | 5 | 13 | 60.3 |
| E | 0.35% 5-Fu | 0.35% 5-Fu | - | 4.20 | > 15 | 3 | 21.4 |
| F | 2.5% R' 0.35% 5-Fu | - | 0.12 | 2.80 | > 15 | 3 | 17.5 |
| G | 0.2% R 0.05% 5-Fu | - | 0.02 | 0.60 | 5 | 9 | 53.6 |
| H | 5% R 0.9% 5-Fu | - | 0.08 | 1.80 | 5 | > 15 | 76.8 |
| I | 45% R 9.0% 5-Fu | - | 0.07 | 1.80 | 5 | > 15 | 75.2 |
| J | 2.5% R' | 0.35% 5 - Fu | 0.12 | 4.20 | > 15 | 3 | 26.4 |
| K | 0.2% R | 0.35% 5 - Fu | 0.02 | 2.80 | 5 | 9 | 52.6 |
| L | 50%R | 0.35% 5-Fu | 0.08 | 2.80 | 5 | 15 | 73.4 |
| M | 2.5% R' 0.35% 5-Fu | 0.35% 5-Fu | 0.12 | 4.20 | > 15 | 3 | 32.7 |
| N | 0.2% R | 0.35% 5 - | 0.02 | 2.70 | 5 | > 15 | 83.1 |
| | 0.05% 5 - Fu | Fu | | | | | |
| ○ | 5% R0.9%5 - Fu | 0.35% 5 - Fu | 0.08 | 3.90 | 5 | > 15 | 91.7 |
| P | 45% R 9% 5 - Fu | 0.35% 5 - Fu | 0.07 | 3.90 | 5 | > 15 | 93.5 |
| Q | Saline solution | - | 0 | 0 | - | - | - |

| | | | | | | | |
|---|---|---|---|---|---|---|---|
| * : the unit of Q_{R} or Q_{R'} was mmol, and the unit of Q_{B} was mg | | | | | | | |

Comparison of R (group B, C, D) with R' (group A), comparison of R/B combination (group G to I, K to L, N to P) with R'/B combination (group F, J, M), and comparison with single agents (group B, C, D, E), the results shown in the above table were consistent with the corresponding results in Examples 1, 2, 3, 4, and 6. This further indicated that R was an active ingredient of dose reduction and efficacy enhancement beyond the expectations of the prior art (R and R' were pharmaceutically equivalent), R/B was a synergistic combination beyond the expectations of the prior art of dimethylbiguanide (R/B and R'/B were pharmaceutically equivalent combinations), and further a synergistic combination beyond the expectations based on the single agents of R and B (by HSA method or Bliss method). Moreover, B was selected from any drug with a cellular response as the main antineoplastic response, not limited to specific types (further extension from DNA damaging agents to antimetabolic drugs). The dose reduction and efficacy enhancement or synergism included the reduction and efficacy improvement (rapid, efficient, and/or long-lasting) and adjustability improvement (highly adjustable effective administration concentration, volume, etc.) as described in Example 6.1.

### Example 7.2 Study of pharmaceutical combination of R and B: extension of B to antimetabolite drugs

In one experiment, the study used GEM as B, and the refractory tumors of B were GEM-resistant tumors. The experimental animals were nude mice, and the tumor formation method was the conventional subcutaneous transplantation method. In brief, the transplantation volume of GEM-insensitive cells (human pancreatic cancer cell line ASPC-1/GEM with gemcitabine resistance) was 1.5×10⁶ cells per mouse. The transplantation method was cell suspension injection, and the modeling site was under the animal's skin. The successfully modeled animals were enrolled (with an average tumor volume of 191 mm³) and randomly divided into 16 groups (6 mice per group), as shown in the following table. The ratio of the highest value to the lowest value in the target volume (Vtarget) distribution of each group was approximately 200%. On the day of grouping, the drug was administered, which was recorded as the first day of treatment, and so on. Each group was administered the pharmaceutical solution as shown in the following table, which was either the intratumoral pharmaceutical solution I or the intraperitoneal pharmaceutical solution II, prepared according to conventional methods. The pharmaceutical solution containing N, N-dimethylbiguanide did not contain any additives, including a pH regulator that could convert it to dimethylbiguanide salt, and thus could no longer be used for conventional administration.

Group A, P received intratumoral administration 4 times, on days 1, 3, 5, and 7, 200 µL per time. Group B received intratumoral administration 3 times, on day 1, 3, and 5, 400 µL each time. Group C, D received intratumoral administration 2 times, on day 1 and 3, 200 µLand 10 µLrespectively. Group E, F, G received intratumoral administration 2 times, on day 1 and 3, 200µL, 100µL, and 10 µLrespectively. Group H received intraperitoneal administration 6 times, on day 1, 3, 5, 7, 9, and 11, 300 µLper time. Group I received intratumoral administration and intraperitoneal administration, the intratumoral administration was the same as in Group A, and the intraperitoneal administration was the same as Group H. Groups J, K, L received intratumoral administration and intraperitoneal administration, the intratumoral administration was the same as Groups B, C, and D respectively, and the intraperitoneal administration was 4 times on day 1, 3, 5, and 7, 300 µLper time. Groups M, N, O received intratumoral administration and intraperitoneal administration, the intratumoral administration was the same as in Groups E, F, and G respectively, and the intraperitoneal administration was 3 times on day 5, 7, and 9, 300 µL per time.

The tumor volume was measured twice every two days. The day when the relative tumor proliferation rate (T/C%) was < 42% was recorded as the effective time. The day when the relative tumor volume (RTV) increased continuously from the 1st day until > 150% to the end point was recorded as the progression-free time. On the 15th day, the animals were euthanized, the tumors were dissected, the tumor weight was determined, and the tumor inhibition rate was calculated. The results were shown in Table 14.

**Table 14**

| Group | Drug, administration route | | Dosage | | Efficacy (X) | | |
|---|---|---|---|---|---|---|---|
| | Pharmace utical solution I for intratumor al administra tion | Pharmaceuti cal solution II for intraperiton eal administrati on | Q_{R} or Q_{R'} | Q_{GEM} | Effectiv e time (days) | Progress ion-free time (days) | Tumo r inhibi tion rate (%) |
| A | 2.5% R' | - | 0.12 | | > 15 | 3 | 10.2 |
| B | 0.2%R | - | 0.02 | | 5 | 5 | 32.7 |
| C | 1% R | - | 0.03 | | 5 | 9 | 42.4 |
| D | 50%R | - | 0.08 | | 5 | 11 | 63.8 |
| E | 1% R 0.05% DDP | - | 0.03 | | 5 | > 15 | 71.4 |
| F | 5% R0.03% DDP | - | 0.08 | | 5 | > 15 | 81.6 |
| G | 50% R0.3% DDP | - | 0.08 | | 5 | > 1 | 79.2 |
| H | - | 1% of the GEM | | 18.00 | > 15 | 3 | 23.7 |
| I | 2.5% R' | 1% of the GEM | 0.12 | 18.00 | > 15 | 3 | 21.5 |
| J | 0.2% R | 1% GEM | 0.02 | 12.00 | 5 | 9 | 50.3 |
| K | 1% R | 1% GEM | 0.03 | 12.00 | 5 | 13 | 68.1 |
| L | 50%R | 1% GEM | 0.08 | 12.00 | 5 | 13 | 73.9 |
| M | 1% R 0.05% DDP | 1% GEM | 0.03 | 9.00 | 5 | > 15 | 85.2 |
| N | 5% R0.03% DDP | 1% of the GEM | 0.08 | 9.00 | 5 | > 15 | 93.7 |
| ○ | 50% R0.3% DDP | 1% GEM | 0.08 | 9.00 | 5 | > 15 | 95.1 |
| P | Normal saline | - | 0 | 0 | - | - | - |

| | | | | | | | |
|---|---|---|---|---|---|---|---|
| * : the unit of Q_{R} or Q_{R'}was mmol and the unit of Q_{GEM} was mg | | | | | | | |

Comparison of R (group B, C, D) with R' (group A), comparison of R/B combination (J, K, L) and R'/B combination (group I) with single agent (group B, C, D, H), the results showed in the table, respectively were consistent with the corresponding results of example 1, 2, 3, 4, 6, and 7.1, further indicating that R was the active ingredients of dose reduction and efficacy enhancement beyond the expectations of the prior art (R and R' were pharmaceutical equivalents), and R/B was synergistic combination beyond the expectation of the prior art of dimethylbiguanide (R/B and R'/B were pharmaceutical combination equivalents), and further synergistic combination beyond the expectation of the prior art of the single-agents R and B (HSA or Bliss method). Moreover, B was selected from any drug with a cellular response as the primary antineoplastic response and was not limited to specific types (further expanded from DNA damaging agent, antimetabolism pharmaceutical to antimicrotubules pharmaceutical). Wherein said dose reduction and efficacy enhancement or synergism included dose reduction and efficacy enhancement (rapid, highly effective, and/or long-acting) and adjustability enhancement (highly adjustable effective administration concentration, volume, etc.) as described in Example 6.1.

The drugs of R intratumoral administration and a portion of B intraperitoneal administration, and a portion of groups (group M, N, O) of intragastric administration (group E, F, G) were dose reduction and efficacy enhancement combination shown in the previous examples. These groups further improved efficacy, especially increased tumor inhibitory rate compared to these dose reduction and efficacy enhancement combination.

### Example 8: Study of the Pharmaceutical Combinations of R and B: Extension of B Model Drugs to Anti-Microtubule Drugs

In the following experiment, the drug (B) was selected from paclitaxel, which was the most widely used and representative drug. The B refractory tumors were selected from tumors resistant to anti-microtubulin drugs, namely tumors that were not usually used in treatment plans, especially first-line and second-line treatments, including invalid and ineffective tumors of B. The invalid tumors of anti-microtubulin drug included, for example, conventional liver cancer, colorectal cancer, melanoma, pancreatic cancer, and the like, which contained tumor cells that were not sensitive to anti-microtubulin drugs, and these cells had drug resistance mechanisms such as changes in microtubule dynamic homeostasis and overexpression of pharmaceutical efflux proteins. The ineffective tumors of anti-microtubulin pharmaceuticals included, for example, its resistant tumors.

In one experiment, the drug B (B) was TAX, and the representative tumors of B refactory tumors were resistant to TAX. The experimental animals were nude mice, and the tumor formation method was the conventional subcutaneous transplantation method. In brief, the transplantation volume of GEM insensitive cells (paclitaxel-resistant human ovarian cancer cell strain SKOV3/TAX) was 1×10⁶ cells per mouse, and the transplantation method was cell suspension injection, with the modeling site being the subcutaneous of the animal. Animals with successful modeling were enrolled (average tumor volume of 183 mm³) and randomly divided into 16 groups (6 mice per group), as shown in the following table, and the ratio of the highest value to the lowest value in the target volume (Vtarget) distribution of each group was approximately 200%. On the day of grouping, the pharmaceutical was administered, and it was recorded as the first day of treatment, and so on. Each group was administered the pharmaceutical solution as shown in the following table, which was either the intratumoral administration solution I or the intraperitoneal administration solution II, all prepared according to conventional methods.

Group A, P received intratumoral administration 4 times, on day 1, 3, 5, and 7, 200 µL per time. Group B received intratumoral administration 3 times, on day 1, 3, and 5, 400 µL each time. Group C, D received intratumoral administration 2 times, on day 1 and 3, 200 µL and 10 µL each time. Group E, F, G received intratumoral administration 2 times, on day 1 and 3, 200µL, 100µL, and 10 µL each time. Group H received intraperitoneal administration 6 times, on day 1, 3, 5, 7, 9, and 11, 200 µL per time. Group I received intratumoral administration and intraperitoneal administration, the intratumoral administration was the same as Group A and the intraperitoneal administration was the same as Group H. Group J, K, L received intratumoral administration and intraperitoneal administration, the intratumoral administration was the same as group B, C, and D, and the intraperitoneal administration was 4 times on day 1, 3, 5, and 7, 200 µL per time. Group M, N, O received intratumoral administration and intraperitoneal administration, the intratumoral administration was the same as group E, F, and G, and the intraperitoneal administration was 3 times on day 7, 9, and 11, 200 µL per time.

The tumor volume was measured twice every two days. The day when the relative tumor proliferation rate (T/C%) was < 42% was the effective time, and the day when the relative tumor volume (RTV) continued to increase from the 1st day until > 150% and then reached the end point was the progression-free time. On the 15th day, the animals were euthanized, the tumor was dissected, the tumor weight was determined, and the tumor inhibition rate was calculated. The results were shown in Table 15.

**Table 15**

| group | Drug, administration route | | Dosage | | Efficacy (X) | | |
|---|---|---|---|---|---|---|---|
| | Pharmaceutic al solution I for intratumoral administratio n | Pharmaceutical solution II for intraperitoneal administration | QR or Q R' | Q T A X | Effectiv e time (days) | Progres sion-free time (days) | Tum or inhi bitio n rate (%) |
| A | 2.5% R' | - | 0.12 | | > 15 | 3 | 11.4 |
| B | 0.2%R | - | 0.02 | | 5 | 5 | 30.1 |
| C | 1% R | - | 0.03 | | 5 | 7 | 43.7 |
| D | 50%R | - | 0.08 | | 5 | 11 | 64.2 |
| E | 1% R 0.1% 5 - Fu | - | 0.03 | | 5 | 13 | 68.6 |
| F | 5% R0.5%5-Fu | - | 0.08 | | 5 | 15 | 76.4 |
| G | 45% R10%5-Fu | - | 0.07 | | 5 | > 15 | 75.1 |
| H | - | 0.03% TAX | | 0.3 6 | > 15 | 3 | 26.1 |
| I | 2.5% R' | 0.03% TAX | 0.12 | 0.3 6 | > 15 | 3 | 23.9 |
| J | 0.2%R | 0.03% TAX | 0.02 | 0.2 4 | 5 | 9 | 52.7 |
| K | 1% R | 0.03% TAX | 0.03 | 0.2 4 | 5 | 13 | 64.3 |
| L | 50%R | 0.03% TAX | 0.08 | 0.2 4 | 5 | 15 | 78.1 |
| M | 1% R 0.1% 5 - Fu | 0.03% TAX | 0.03 | 0.1 8 | 5 | > 15 | 81.2 |
| N | 5% R0.5%5 - Fu | 0.03% TAX | 0.08 | 0.1 8 | 5 | > 15 | 92.1 |
| O | 45% R 10%5-Fu | 0.03% TAX | 0.07 | 0.1 8 | 5 | > 15 | 97.3 |
| P | Normal saline | - | 0 | 0 | - | - | - |

| | | | | | | | |
|---|---|---|---|---|---|---|---|
| * : the unit of Q_{R} or Q_{R'}was mmol and the unit of Qₜₐₓ was mg | | | | | | | |

Comparison of R (group B, C, D) with R'(group A), R/B combination (group J, K, L) with R'/B combination (group I), and single agents (group B, C, D, H), the results shown in the above table respectively were consistant with the corresponding results of Example 1, 2, 3, 4, 6, 7, and 8.1. This further indicated that R was an active ingredient of dose reduction and efficacy enhancement beyond the expectations of the prior art (R and R' were pharmaceutically equivalents); R/B was a synergistic combination beyond the expectations of the prior art of dimethylbiguanide (R/B and R'/B were pharmaceutically equivalent combinations); and was a further synergistic combination beyond the expectations of the prior art of single agent (HSA method or Bliss method). Moreover, B was selected from any drug with a cellular response as the main antineoplastic response, without being limited to its specific types (further expanding from DNA damaging agents, antimetabolite drugs to anti-microtubulin drugs). The dose reduction and efficacy enhancement or the synergistic effect included the dose reduction and efficacy enhancement (rapid, efficient, and/or long-lasting) and the improvement of adjustability (highly adjustable effective administration concentration, volume, etc.) as described in Example 6.1.

The drugs of R intratumoral administration and part of B intraperitoneal administration, and part of groups (group M, N, O) of intragastric administration (group E, F, G) were dose reduction and efficacy enhancement combination shown in the previous examples. These research groups further enhanced efficacy, especially increased tumor inhibitory rate compared to these dose reduction and efficacy enhancement compositions.

### Example 9 Study of pharmaceutical combination of R and B: extension of B model drug to targeted antineoplastic agents

The following experiment, drug (B) was the targeted antineoplastic agents, the refractory tumor was selected from the refractory tumors resistant to the targeted antineoplastic agents, namely tumors that were not usually used in treatment plans, especially first-line and second-line treatments, including invalid and ineffective tumors of B. Targeted invalid tumor included such as conventional pancreatic cancer, liver cancer, colorectal cancer, non-small cell lung cancer (wild type EGFR and ALK), three negative breast cancer, melanoma, and so on, their cells lacked effective targets and exhibited high tumor heterogeneity, etc. Tumor targeted ineffective tumors included, for example, its drug-resistant tumors.

### Example 9.1 combination of R and osimertinib (AZD9291), the representative targeted-antineoplastic agents

In an experiment (in collaboration with the West China Hospital of Sichuan University), the study used osimertinib as B, and B refractory tumors were tyrosine kinase inhibitors (TKIs)-resistant tumors. The experimental animals were nude mice, and the tumor formation method was the conventional human tumor tissue subcutaneous transplantation method (PDX). In brief, the modeling tissue was derived from human lung cancer resistant to tyrosine kinase inhibitors (TKIs), and the transplantation volume was 1 particle (diameter approximately 1mm) per mouse. The transplantation method was to implant the tissue particles through a transplantation needle, and the modeling site was subcutaneous of the animal. Animals with successful modeling were enrolled (average tumor volume of 323mm³), and were randomly divided into 4 groups (5 mice per group), as shown in the table below. The ratio of the highest value to the lowest value in the target volume (Vtarget) distribution of each group was approximately 300%. On the day of grouping, the drug was administered, which was recorded as the first day of treatment, and so on. The drug administration for each group was as shown in the table below, namely, intratumoral administration of the pharmaceutical solution I and intragastric administration of the pharmaceutical solution II. Osimertinib was a suspension, and the other drugs were aqueous solution, all prepared according to conventional methods. The pharmaceutical solution containing N,N-dimethylbiguanide did not contain any additives, including pH regulators that could convert it to dimethylbiguanide salt, and thus could no longer be used for conventional administration.

Group A, C, and D received intratumoral administration 3 times, on day 1, 2, and 3, with 100µL each time; Group B received intragastric administration once a day until the end, approximately 200µL each time; Group E and F received intratumoral administration 3 times and intragastric administration once a day, the intratumoral administration was once a day on day 1, 2, and 3, 100µL each time, and intragastric administration was approximately 200µL each time, until the end.

The tumor volume was measured once a day, and the day when the relative tumor proliferation rate (T/C%) was <42% was recorded as the effective time, and the day when the relative tumor volume (RTV) continued to increase from the 1st day until >150% to the end point was recorded as the progression-free time. On the 7th day, the animals were euthanized, the tumors were dissected, the tumor weight was determined, and the tumor inhibition rate was calculated. The results were shown in Table 16.

**Table 16**

| Group | Pharmaceutica | Pharmaceutical | Effective | Progression- | Tumo |
|---|---|---|---|---|---|
| | l solution I for intratumoral administration | solution II for intragastric administration | time (days) | free time (days) | r inhibit ion rate (%) |
| A | Saline solution | | - | - | - |
| B | - | 0.15% AZD9291 | > 7 | 0 | 4.2 |
| C | 5% R | - | 4 | > 7 | 87.4 |
| D | 6.4% R' | - | > 7 | 0 | 10.6 |
| E | 5% R | 0.15% AZD9291 | 4 | > 7 | 95.8 |
| F | 6.4% R' | 0.15% AZD9291 | > 7 | 0 | 15.3 |

When comparing R (group C) with R'(group D), R/B combination (group E) with R'/B combination (group F), and with single agents (groupB and C), as shown in the results in the above table respectively, they were consistent with the corresponding results in Example 1, 2, 3, 4, 6, 7, and 8. This further indicated that R was an active ingredient of dose reduction and efficacy enhancement beyond the expectations of the prior art (R and R' were pharmaceutically equivalents), R/B was a synergistic combination beyond the expectations of the prior art of dimethylbiguanide (R/B and R'/B were pharmaceutically equivalent combinations), and further synergistic combination beyond the expectations of single agent expectations (HSA method or Bliss method). Moreover, B was selected from any drug with a cellular response as the main antineoplastic response, without being limited to specific types (further expanding to targeted antineoplastic agents from DNA damaging agents, antimetabolite drugs, and anti-microtubulin drugs). The dosage reduction and efficacy enhancement or synergism included the reduction and efficacy improvement (rapid, efficient, and/or long-lasting) or the improvement of adjustability (highly adjustable effective administration concentration, volume, etc.) as described in Example 6.1.

### Example 9.2 Extension of B in combinations with targeted antineoplastic agents

In one experiment, the study used GR as B, and the representative tumor of B refractory tumors was GR-resistant tumors. The experimental animals were C57BL/6 mice, and the tumor formation method was the conventional subcutaneous transplantation method. In brief, the transplantation volume of GR-insensitive cells (the gefitinib resistant human lung adenocarcinoma cell line PC-9/GR) was 1×10⁶ cells per mouse. The transplantation method was cell suspension injection, and the modeling site was subcutaneous of the animal. The animals with successful modeling were enrolled (the average tumor volume was 173 mm³) and randomly divided into 16 groups (6 mice per group), as shown in the following table. On the day of grouping, the treatment began, which was recorded as the first day of treatment, and so on. Each group received the following drugs as shown in the table: pharmaceutical solution I for intratumoral administration, pharmaceutical solution II for intraperitoneal administration, and pharmaceutical solution III for intragastric administration. GR was in suspension, and the other drugs were aqueous solutions. All were prepared according to conventional methods.

Group A, P received intratumoral administrations 4 times on day 1, 3, 5, and 7, 200 µLper time. Group B received intratumoral administrations 3 times once daily on day 1, 3, and 5, 400 µL each time. Group C, D received intratumoral administrations 2 times once daily on day 1 and 3, 200 µLand 10 µLrespectively. Group E, F, G received intratumoral administrations (5% R) 2 times and intraperitoneal administrations (0.5% IFO, 0.05% DDP, 0.05% DOX) 4 times, the intratumoral administration received once daily on day 1 and 3 (100 µl each time) and the intraperitoneal administration was once daily on day 1, 3, 5, and 7 days (200µL, 200µL, 150 µL each time respectively). Group H received intragastric administration once daily until day 14, 250 µL per time. Group I received intratumoral administration and intragastric administration, the intratumoral administration was the same as Group A, and intragastric administration was the same as Group H. Group J, K, L received intratumoral administration and intragastric administration, the intratumoral administration was the same as group B, C, and D, and the intragastric administration was once every other day (250 µL each time) for a total of 7 times. Group M, N, O received intratumoral administrations 2 times, intraperitoneal administrations 4 times, and intragastric administrations 7 times, the intratumoral administration and intraperitoneal administration were the same as group E, F, and G, and the intragastric administration was once every other day (250 µL each time) for a total of 7 times.

The tumor volume was measured every 2 days. The day when the relative tumor proliferation rate (T/C%) <42% was recorded as the effective time. The day when the relative tumor volume (RTV) increased continuously from the 1st day until >150% to the end point was recorded as the progression-free time. On the 15th day, the animals were euthanized, the tumors were dissected, the tumor weight was determined, and the tumor inhibition rate was calculated. The results were shown in Table 17.

**Table 17**

| Gr ou p | Drugs and administration route | | | Dosage | | Efficacy (X) | | |
|---|---|---|---|---|---|---|---|---|
| | Pharmaceu tical solution I for intratumor al administra tion | Pharma ceutical solutio n II for intraper itoneal adminis tration | Pharmaceuti cal solution for III for intragastric administrati on | QR or QR' | QG R | Effect ive time (days) | Pro gres sion - free time (day s) | Tumor inhibiti on rate (%) |
| A | 2.5% R' | - | - | 0.12 | | > 15 | 3 | 9.6 |
| B | 0.2%R | - | - | 0.02 | | 5 | 5 | 32.7 |
| C | 1% R | - | - | 0.03 | | 5 | 9 | 41.3 |
| D | 50%R | - | - | 0.08 | | 5 | 11 | 60.8 |
| E | 5% R | 0.5% IFO | - | 0.08 | | 5 | > 15 | 82.3 |
| F | 5% R | 0.05% DDP | - | 0.08 | | 5 | > 15 | 79.1 |
| G | 5% R | 0.05% DOX | - | 0.08 | | 5 | 15 | 71.9 |
| H | - | - | 0.5% GR | | 17.5 | > 15 | 3 | 17.3 |
| I | 2.5% R' | - | 0.5% GR | 0.12 | 17.5 | > 15 | 3 | 16.9 |
| J | 0.2% R | - | 0.5% GR | 0.02 | 8.75 | 5 | 7 | 45.6 |
| K | 1% R | - | 0.5% GR | 0.03 | 8.75 | 5 | 11 | 61.5 |
| L | 50%R | - | 0.5% GR | 0.08 | 8.75 | 5 | > 15 | 72.6 |
| M | 5% R | 0.5% IFO | 0.5% GR | 0.08 | 8.75 | 3 | > 15 | 91.2 |
| N | 5% R | 0.05% DDP | 0.5% GR | 0.08 | 8.75 | 3 | > 15 | 93.1 |
| ○ | 5% R | 0.05% DOX | 0.5% GR | 0.08 | 8.75 | 3 | > 15 | 94.6 |
| P | Normal saline | - | - | 0 | 0 | - | - | - |

| | | | | | | | | |
|---|---|---|---|---|---|---|---|---|
| * : the unit of Q_{R} or Q_{R'}was mmol and the unit of Q_{B} was mg | | | | | | | | |

Comparison of R (group B, C, D) with R'(group A), comparison of R/B (group J, K, L) with R'/B groups (group I), and with single agents (group B, C, D, H), the results as shown in the above table were consistent with the corresponding results in Example 1, 2, 3, 4, 6, 7, 8 and 9.1. This further demonstrated that R was an active ingredient of dose reduction and efficacy enhancement beyond the expectations of the prior art (R and R' were pharmaceutically equivalents), R/B was a synergistic combination beyond the expectations of the prior art of dimethylbiguanide (R/B and R'/B were pharmaceutically equivalent combinations), and a further synergistic combination beyond the expectations of the single agents of R and B (HSA method or Bliss method).

The drugs of intratumoral administration of R and the intratumoral administration (group E, F, G) in study groups (group M, N, O) of partial intraperitoneal administration and partial intragastric administration of B had been shown in the previous examples as dose reduction and efficacy enhancement combinations. These study groups further enhanced efficacy compared to these dose reduction and efficacy enhancement combinations, especially in terms of tumor inhibition rate improvement.

In one experiment, the study used trastuzumab (TZB) as B, the experimental animals were nude mice, and the tumor formation method was the conventional subcutaneous transplantation tumor method. In brief, the transplantation amount of drug-resistant human breast cancer cells (BT-474/TR) was 1×10⁶ cells per mouse, the transplantation method was cell suspension injection, and the modeling site was subcutaneous of the animal. The animals with successful modeling were enrolled (average tumor volume of 196 mm³⁾ and randomly divided into 10 groups (6 mice per group), as shown in the table below, and the ratio of the highest value to the lowest value in the target volume (Vtarget) distribution of each group was approximately 200%. On the day of grouping, the administration began, which was recorded as the first day of treatment, and so on. Each group was administered the pharmaceutical solution as shown in the table below, all prepared according to conventional methods.

Group A to E was administered pharmaceutical solution I, administered intratumorally once on the 1st and 2nd days, with each administration volume of 200µL, 200µL, 200µL, 100µL, 10 µL per mouse. Group F was administered pharmaceutical solution II, administered by tail vein injection once on the 1st and 7th days, 100 µLper mouse each time. Group G to J was both administratered pharmaceutical solution I and solution II, the pharmaceutical solution I was administered the same as group B to E, and the pharmaceutical solution II was administered the same as group H.

The tumor volume was measured once every 2 days, and the day when the relative tumor proliferation rate (T/C%) was < 42% was recorded as the effective time, and the day when the relative tumor volume (RTV) increased continuously from the 1st day until > 150% and then increased until the end point was recorded as the progression-free time. On the 15th day, the animals were euthanized, the tumor was dissected, the tumor weight was determined, and the tumor inhibition rate was calculated. The results were shown in Table 18.

**Table 18**

| Groups | Composition of drugs | | Pharmaceutical effects | | |
|---|---|---|---|---|---|
| | pharmaceutical solution I | pharmaceutical solution II | Effective time (days) | Progress ion-free time (days) | Tum or inhib ition rate (% ) |
| A | Normal Saline | - | - | - | - |
| B | 6.5% R' | - | > 15 | 3 | 11.5 |
| C | 0.2%R | - | 5 | 5 | 32.1 |
| D | 5% R | - | 5 | 11 | 61.3 |
| E | 50%R | - | 5 | 11 | 63.7 |
| F | - | 0.2% TZB | > 15 | 3 | 13.6 |
| G | 6.5% R' | 0.2% TZB | 5 | 1 | 10.9 |
| H | 0.2%R | 0.2% TZB | 5 | > 15 | 43.1 |
| I | 5% R | 0.2% TZB | 5 | > 15 | 75.4 |
| J | 50%R | 0.2% TZB | 5 | > 15 | 78.5 |

Comparison of R (group C, D, E) with R' (group B), comparison of R/B combination (group H, I, J) with R'/B combination (group G), as well as single agents (group C, D, E, H), the results showed in the above table were consistent with the corresponding results in Example 1, 2, 3, 4, 6, 7, 8 and 9.1, further indicating that R was an active ingredient of dose reduction and efficacy enhancement (R and R' were pharmaceutically equivalents) beyond the expectations of the prior art, R/B was a synergistic combination beyond the expectation of the prior art of dimethylbiguanide (R/B and R'/B were pharmaceutically equivalent combinations), and further a synergistic combination beyond the expectation of the single agents of R and B (HSA method or Bliss method). Moreover, B was selected from any drug with cellular response as a main antineoplastic reaction, not limited to its specific types (further expanding from DNA damaging agents, antimetabolite drugs, and anti-microtubulin drugs to targeted antineoplastic agents). The efficacy enhancement or synergy included the efficacy improvement (rapid, efficient, and/or long-lasting) and the adjustablility improvement (highly adjustable effective administration concentration, volume, etc.) as described in Example 6.1.

In Example 6-9, the dosage of R (Q_{R}) (concentration of 0.2-50%) was consistent with that in Example 1-9, and the dosage of B (Q_{B}) was ≤ the conventional dosage for treating its sensitive tumors.

In these examples, N,N-dimethylbiguanide in free-base form (R) universally showed the aforementioned transient local effects on various diseases (tumors, nodules) of different animals (mouse, rat, dog), making it possible to meaningfully reduce the biological barrier function within the lesion, providing a basis for the synergistic effect with any drug that reduced effect for the barrier function, especially cell-reactive drugs.

Representative drugs of cell-reactive drugs (B) started from IFO and expanded to DNA damaging agents, then to antimetabolite drugs, then to anti-tubulin drugs, and then to targeted antineoplastic agents, including: cisplatin, doxorubicin, ifosfamide, fluorouracil, gemcitabine, paclitaxel, gefitinib, osimertinib, and trastuzumab. They have significant structural differences, including small molecules and large molecules, compounds and antibodies, etc., representing the structural diversity of cell-reactive drugs. Their antineoplastic mechanisms vary greatly, including DNA damaging agents, antimetabolite drugs, anti-microtubulin agents, kinase inhibitors, etc., representing the mechanism diversity of cell-reactive drugs. They shared one common point, that is, the biological barrier within the lesion was a key control factor for pharmaceutical efficacy.

In these examples, the technical results of the R/B combination of R with the above representative drugs of structural-mechanism diversity went beyond the expectations of the prior art of dimethylbiguanide (R/B and R'/B were pharmaceutically equivalent combinations, R'/B combination was a combination of cell-reactive drugs) and went beyond the expectations of the individual drug of R and B (HSA method or Bliss method). The wide consistency of the synergistic effect produced by the combination of R and B indicated that the pharmacological mechanism of the combination of R and B had commonality: their respective pharmaceutical effects could enhance each other. In fact, according to common knowledge of pharmacology, the common control pathway of cell-reactive drugs was the biological barrier within the lesion, and drugs that reduced the biological barrier function had an enhancing effect on the active form of any cell-reactive drug passing through this common control pathway, which made the latter more likely to enter the tumor tissue and contact target cells to improve efficacy, which was conducive to the development of the former pharmaceutical effect such as tissue destruction.

Therefore, B in the combination of this application could be reasonably inferred from the combination effects of the representative drugs as applicable to cell-reactive drugs, at least including DNA damaging agents, antimetabolite drugs, anti-microtubulin drugs, and targeted antineoplastic agents. More specifically:
1). Based on the consistency of the combined effects of the aforementioned representative drugs of this class, even the consistency with the representative drugs of targeted antineoplastic agents of other classes, it could be reasonably inferred that the cytotoxic drugs in this application combination were applicable to the category of cytotoxic drugs, at least including DNA damaging agents, antimetabolites, and anti-microtubulin drugs, among which:
   The DNA damaging agents in this application combination could be reasonably inferred from the combined effects of the class of aforementioned representative drugs to be applicable to DNA damaging agents. The antimetabolites in this application combination could be reasonably inferred from the aforementioned representative drugs to be applicable to the class of antimetabolites. The anti-microtubulin drugs in this application combination could be reasonably inferred from the aforementioned representative drugs to be applicable to the class of anti-tubulin drugs.
2). Based on the consistency of the combined effects of the aforementioned representative drugs of this class, even the consistency with representative drugs of cytotoxic drugs of other classes, the targeted antineoplastic agents in this application combination could be reasonably inferred from the combined effects of the aforementioned representative drugs to be applicable to the class of targeted antineoplastic agents, at least including tyrosine kinase inhibitors.

### Example 10. Study of pharmaceutical combination containing R and near neutral or weak alkaline sodium salt (C)

### Example 10.1 Study of combination containing C

In one experiment, the experimental animals were BALB/c mice. The local stimulation model was the leg muscle tissue, and the anti-disease model was the breast cancer tumor. The tumor formation method was the conventional subcutaneous tumor transplantation method. The transplantation amount of 4T1 breast cancer cells was 1×10⁶ cells per mouse. The transplantation method was cell suspension injection, and the modeling site was subcutaneous of the animal. Normal mice and those successfully modeled with tumors (with an average tumor volume of 161 mm³) were grouped as Series I and Series II respectively, and each was randomly divided into 15 groups (from A to P, with 6 mice in each group), and were labeled as the groups as shown in the following table respectively. The same groups in different series meant the administration of the same drug to different models. On the day of grouping, administration began, and it was recorded as the first day of treatment, and so on. Each group was administered the pharmaceutical solution as shown in the following table, all prepared according to the conventional method.

On the first day, all mice in Series I received local injection in the right hind leg muscle once, with an injection volume of 100 µg per mouse; all mice in Series II received intratumoral injection, with an injection volume of 200 µg per mouse. the local stimulation at the injection site of series I was evaluated according to the conventional local stimulation scoring method (0-4 points) 24 hours after the injection, and the mean score (M) was calculated. The animals of series II were euthanized on the 7th day, tumors were dissected. Tumor weight was measured, and the tumor inhibition rate (X) was calculated. The results of M, X, and X/M were shown in Table 19.

**Table 19**

| Group | Composition of pharmaceutical solution | | Tissue destruction score (M) | Tumor inhibition rate (X) | Effect - toxicity ratio (X/M) |
|---|---|---|---|---|---|
| | R or R ' | C or other | | | |
| A | - | Normal saline | 0 | - | - |
| B | - | Anhydrous ethanol | 3.6 points | 51.3% | 14.3% /point |
| C | | 2.4% NaCl | 0.3 points | 7.9% | Approximately 0 |
| D | - | 3.0% SA | 0.5 points | 13.1% | Approximately 0 |
| E | - | 2.4% MSP | 0.7 points | 10.9% | Approximately 0 |
| F | - | 2.4% SB | 0.6 points | 13.7% | Approximately 0 |
| G | 2.6% R' | - | 0.5 points | 11.7% | Approximately 0 |
| H | 1% R | - | 2.4 points | 42.1% | 17.5% /point |
| I | 2.6% R' | 2.4%SB | 0.9 points | 17.8% | Approximately 0 |
| J | 1% R | 2.4% NaCl | 2.4 points | 53.7% | 22.4% /point |
| K | 1% R | 1.7% SA | 2.3points | 51.8% | 22.5 %/point |
| L | 1% R | 3.0% of the SA | 2.4 points | 58.2% | 24.2% /point |
| M | 1% R | 1.2% MSP | 1.9 points | 44.3% | 23.3% /point |
| N | 1% R | 0.5% SB | 2.0 points | 45.3% | 22.6% /point |
| ○ | 1% R | 1% SB | 1.5 points | 41.3% | 27.5% /point |
| P | 1% R | 4% SB | 1.5 points | 53.7% | 35.8% /point |

In the above table, normal saline and anhydrous ethanol were negative and positive control respectively.

### 1. Single agent research:

As shown in the table above, the efficacy of the near-neutral sodium salt single was very weak (the tumor inhibition rate was all < 20%), and it did not show any therapeutic significance. The ratio of its effectiveness to toxicity was thus meaningless, and the efficacy-to-toxicity index of these drugs was therefore approximately 0. R' was actually also a near-neutral salt, and its efficacy-toxicity index was also approximately 0. The single agent of R showed effectiveness (the tumor inhibition rate > 40%), and compared with the classic anhydrous ethanol, its efficacy-toxicity index increased by more than 50%, demonstrating relatively higher local action specificity, which exceeded the non-specific expectations of local action. These results once again indicated that R was an active ingredient with reduced dosage and enhanced efficacy (efficacy-toxicity ratio) that exceeded the expectations of the prior art (R and R' were pharmaceutical equivalents).

### 2. Regular comparison of combinations:

In the above table, when the amount of R and C (Q_{C}/Q_{R}) was 0.5 to 4.0 (group J and P), C exhibited significant toxicity reduction toward R without markedly compromising efficacy, or showed no increase in toxicity but provided certain efficacy enhancement. This was manifested as an improved efficacy-to-toxicity ratio or enhanced local-action specificity depending on the combination. However, in R'/C (group I), C demonstrated neither toxicity reduction nor efficacy enhancement toward R', indicating that the efficacy-to-toxicity ratio did not improve as a result of the combination. These results further illustrated that the R/C combination was a synergistic combination that exceeded the expectations of the prior art (R/C and R'/C were pharmaceutically equivalent combinations) or even an efficacy-to-toxicity ratio synergistic combination.

### 3. Further comparison of combination:

In the above table, when the ratio of R to C (Q_{C}/Q_{R}) was from 0.5 to 4.0 (Group J to P), group R/C (Group J to P) was a synergistic combination exceeding the expected efficacy-to-toxicity ratio (k₂) of the HSA method compared to the single agent R group (Group H) and the single agent B group (Group C to F). Taking Group O as an example, its actual efficacy-to-toxicity ratio k₁ (27.5%/point) was greater than the expected value k2= max (group H, F) = 18.2%/point of the HSA method. These results once again demonstrated that the R/C was a further synergistic combination beyond expectation (HSA method) and even a synergistic combination of efficacy-toxicity ratio.

In the above examples, N,N-dimethylbiguanide in free-base form (R) universally showed the aforementioned transient local effects for different diseases (tumors, nodules) in different animals (mice, rats, dogs), including tissue destruction effects. In this example, the near-neutral or weakly alkaline sodium salts (C) used included: sodium chloride, sodium bicarbonate (abbreviated as SB), monosodium phosphate (abbreviated as MSP), sodium lactate (abbreviated as Nal), sodium acetate (abbreviated as SA), and they could cover the main categories of near-neutral or weakly alkaline sodium salts due to their chemical structure differences. They shared one common point, that is, they could provide a weak transient local effect, such as hypertonic effect.

In this example, the technical results of the R/C combination of R and the above representative drugs of diverse structures were all beyond the expectations of the prior art of dimethylbiguanide (R/C and R'/C were pharmaceutically equivalent combinations), as well as beyond the expectations of single agents of R and C (HSA method), especially the expected efficacy-toxicity ratio. The wide consistency of the synergistic effect produced by the combination of R and C indicated that the pharmacological mechanism of the combination of R and C had commonality: their respective pharmaceutical effects could enhance each other. In fact, according to common knowledge of pharmacology, the hypertonic effect could provide a hypertonic environment, which may enhance the efficacy of drugs in the tumor, especially transient local effect drugs. This further explained the essential difference between R and R'. Therefore, the C in the combination of this application could be reasonably inferred from the combination effects of the representative drugs to be applicable to the injectable near-neutral sodium salts.

Even more unexpectedly, SB significantly increased the efficacy-toxicity ratio of R, which exceeded the expected hypertonic effect of near-neutral sodium salts (C) in the prior art (for example, Group O and Group K had the same efficacy-toxicity ratio). In summary, the combination containing R and C of this application provided a more preferred technical solution for patients who were sensitive to local stimulation.

### Example 10.2 Study extension to combination of R, B, C

In one experiment, pancreatic cancer was selected as the representative tumor for refractory tumors. The experimental animals were nude mice. The tumor formation method was the conventional subcutaneous transplantation method. In brief, the transplantation volume of the 5-Fu insensitive cells (human pancreatic cancer cell line PANC-1) was 1×10⁶ cells per mouse. The transplantation method was cell suspension injection, and the modeling site was subcutaneous of the animal. Normal mice and those with successfully modeling tumors (with an average tumor volume of 159 mm³) were respectively grouped as Series I and Series II, and each was randomly divided into 5 groups (group A, E, I, M, Q) and 20 groups (group A to T), with 6 mice in each group. The groups were shown in the following table. The same groups in different series meant the administration of the same drug to different models. On the day of grouping, the drug was administered, and it was recorded as the first day of treatment, and so on. Each group was administered the pharmaceutical solution as shown in the table below, all prepared according to the conventional method.

All mice in Series I were injected with pharmaceutical solution I via intramuscular injection into the right hind leg with a single injection at a dosage of 100 µl per animal per time. All mice in Series II were injected with pharmaceutical solution I intratumorally and pharmaceutical solution II systematically, the intratumoral injection volume was 200 µLper mouse per time, and the systemic administration was intraperitoneal administration (IFO and 5-Fu) and gavage (osimertinib). Intraperitoneal administration was performed on day 1, 3, 5, and 7 for a total of 4 times, 200 µL per mouse per time. Gavage was administered once a day for a total of 12 times, 200 µL per mouse per time. In Series I, local irritation at the injection site was assessed at 24 hours according to the conventional local irritation scoring method (0-4 points), and the mean score (M) was calculated. In Series II, the animals were euthanized on the 15th day. The tumors were dissected, and the tumor weight was measured. The tumor inhibition rate (X) was calculated. Results of M, X, and X/M were shown in Table 20.

**Table 20**

| Group | Drug | | Tissue destruction score (M) | tumor inhibition rate (X) | Effect - toxicity ratio (X/M) |
|---|---|---|---|---|---|
| | pharmaceutical solution I | pharmaceutical solution II | | | |
| A | (normal saline) | - | 0 points | - | - |
| B | - | 0.5% IFO | - | 16.4% | - |
| C | - | 0.4% 5 - Fu | - | 11.3% | - |
| D | - | 0.3% AZD9291 | - | 7.9% | - |
| E | 3% R | - | 2.6 points | 60.7% | 23.3%/point |
| F | 3% R | 0.5% IFO | - | 83.6% | - |
| G | 3% R | 0.4% 5-Fu | - | 79.3% | - |
| H | 3% R | 0.3% AZD9291 | - | 71.8% | - |
| I | 3% R/1.5%SB | - | 2.1 points | 61.4% | 29.2%/point |
| J | 3% R/1.5%SB | 0.5% IFO | - | 81.9% | - |
| K | 3% R/1.5%SB | 0.4% 5-Fu | - | 78.2% | - |
| L | 3% R / 1.5% SB | 0.3% AZD9291 | - | 72.6% | - |
| M | 3% R/3%SB | - | 1.6 points | 58.3% | 36.4%/point |
| N | 3% R/3%SB | 0.5% IFO | - | 82.6% | |
| O | 3% R / 3% SB | 0.4% 5-Fu | - | 76.3% | |
| P | 3% R/3% SB | 0.3% AZD9291 | - | 70.8% | |
| Q | 3% R / 9% SB | - | 1.5 points | 60.8% | 40.5% /point |
| R | 3% R/9%SB | 0.5% IFO | - | 84.5% | - |
| S | 3% R / 9% SB | 0.4% 5 - Fu | - | 77.9% | - |
| T | 3% R/9%SB | 0.3% AZD9291 | - | 72.4% | - |

As shown in the table above, the results of the R/C and B combination groups (group J to L, N to P, R to T) were consistent with those of the R and B combination groups (group F to H), and the latter were shown to be a synergistic combination exceeding the expectations of the prior art of dimethylbiguanide (R/B and R'/B were pharmaceutically equivalent) in Example 6-9. Furthermore, the results of the R/C and B combination groups (group J to L, N to P, R to T) also exceeded the expectations of the HSA method or Bliss method based on the effect of the components (k₂). Taking the group N as an example, its actual tumor inhibition rate k₁ (82.6%) was greater than the expected value k₂ =max(group B, group M) = 58.3% of the HSA method and the expected value k₂ =E_{B} + E_{M} - E_{B} × E_{M} = 65.1% of the Bliss method. These results once again showed that R/C was consistent with R, and its combination with B was a further synergistic combination beyond the expectation (HSA method or Bliss method).

In this example, the dosage of R (QR) was consistent with that in Examples 1-9, the dosage of C (QC) was ≤ its conventional dosage, and the ratio of R to C (QC/QR) was 0.5 to 4.0. The R/C and R showed a consistent transient local effect, which also made it possible to meaningfully reduce the biological barrier function within the lesion. In this example, the representative drugs of the cell-reactive drug (B) were IFO, 5-Fu, and osimertinib, which could represent the chemical structural diversity of cellular reactive pharmaceticals, antineoplastic mechanism diversity (DNA damaging agents, antimetabolites, targeted antineoplastic agents), and consistency of key control steps of the antineoplastic response (biological barrier within the lesion), covering the entire class B drugs. The combination of R/C and these B representative drugs showed consistent combined effects with the combination of R and B in Examples 3, 6, 7, 8, and 9, all went beyond the expectation of synergistic combination of the prior art of dimethylbiguanide, and beyond the further synergistic combination based on the single agent expectations (HSA method or Bliss method).

Consistent with Example 9, the combination of R/C and very different structure-mechanism of B drugs showed consistent synergistic effects, which also indicated that the pharmacological mechanism of the combination of R/C and any cell-reactive drug B had commonality: their respective pharmaceutical effects could enhance each other. In fact, according to common knowledge of pharmacology, the common control pathway of cell-reactive drugs was the biological barrier within the lesion, and drugs that reduced the biological barrier function had an enhancing effect on the active form of any cell-reactive drug passing through this common control pathway, which made the latter more likely to enter the tumor tissue and contacted target cells to improve the efficacy, which was conducive to the development of the former pharmaceutical effect such as tissue destruction.

Therefore, B of the combination containing R and C and B in this application could be reasonably inferred to be applicable to the cell-reactive drugs from the above representative pharmaceutical combination effects, at least including the previously mentioned DNA damaging agents, antimetabolites, anti-microtubulin drugs, and targeted-antineoplastic agents.

### Example 11: Research on the Pharmaceutical Combination of R and Methylene Blue (D)

### Example 11.1: Research on the Combination Containing D

In one experiment, the experimental animals were BALB/c mice. The tumor formation method was the conventional subcutaneous transplantation method. The transplantation amount of 4T1 breast cancer cells was 1×10⁶ cells per mouse. The transplantation method was cell suspension injection, and the modeling site was subcutenous of the animals. Normal mice and those that successfully developed tumors (with an average tumor volume of 184 mm³) were enrolled, and they were randomly divided into 14 groups (6 mice per group), as shown in the table below. The ratio of the highest value to the lowest value in the distribution of target volume (Vtarget) was approximately 200%. On the day of grouping, the pharmaceutical solution was administered, which was recorded as the first day of treatment, and so on. Each group was administered the pharmaceutical solution as shown in the table below, all prepared according to conventional methods. Arg was arginine, and MB was methylene blue.

Groups A to H and group N all received intratumoral administered pharmaceutical solution I on the 1st and 3rd day, twice each, with the volume of each administration per mouse as follows: group B 150 µL, group C and F 75 µL and 10 µL respectively, group H and N 100 µL, and the rest of the groups 200 µL. Group I to M were all intratumoral administered twice, on the 1st and 3rd day, with pharmaceutical solution II was administered first, and then pharmaceutical solution I after 20 minutes. Pharmaceutical solution II was administered to each mouse as follows: except for group J which was 150 µL, the others were 75 µL each. Pharmaceutical solution I was administered the same as group D to G.

The tumor volume was measured twice every 2 days. The day when the relative tumor volume (RTV) increased continuously from the 1st day until it exceeded 150% to the end point was counted as the progression free time. On the 15th day, the animals were euthanized, the tumors were dissected, the tumor weight was determined, and the tumor inhibition rate was calculated. The results were shown in Table 21.

**Table 21**

| Group | Composition of drugs | | Progression-free time (days) | Tumor inhibition rate (X) |
|---|---|---|---|---|
| | Pharmaceutical solution I | pharmaceutical solution II | | |
| A | normal saline | - | - | - |
| B | 0.5% MB | - | 9 | 39.1% |
| C | 1% MB | - | 9 | 47.3% |
| D | 3% R' | - | 3 | 7.4% |
| E | 1% R | - | 9 | 41.7% |
| F | 50% R | - | 9 | 59.3% |
| G | 2.5% R / 10% SB | - | 9 | 56.7 % |
| H | 10% Arg | - | 9 | 31.3 % |
| I | 3% R' | 1% MB | 9 | 42.1% |
| J | 1% R | 0.5% MB | > 15 | 79.3% |
| K | 25% R | 1% MB | > 15 | 81.6% |
| L | 50%R | 1% MB | > 15 | 87.9% |
| M | 10% Arg | 1% MB | 9 | 45.8% |
| N | 2.5% R/10%SB/0.5% MB | - | 9 | 79.3% |

### 1. Conventional comparison of combinations:

In the above table, compared with R'/D (Group I), R/D and R/C/D showed a significant dose reduction and efficacy enhancement (from group J to O), manifested as, for example, the dosage of R being reduced by at least 50% compared to R' while at least one effect (tumor suppression rate) increased by more than 50%. These results indicated that both R/D and R/C/D were synergistic combinations beyond the expectations of prior art (R/D and R'/D. R/C/D and R'/C/D were pharmaceutically equivalent combinations), and even synergistic combinations of dose reduction and efficacy enhancement.

### 2. Further comparison of combinations:

In the above table, compared with the component groups (groupB, C, E to H), the effects of the combination groups (group J to L, group N) exceeded the expectations of the effects of the components (k2) based on the HSA method or Bliss method. Even though R was already beyond the expectations of prior art of dimethylbiguanide (R and R' were pharmaceutically equivalents). Taking the group N as an example, its actual tumor inhibition rate k1 (85.3%) was greater than the expected value k₂ ≦max (group B, group H) = 63.7% of the HSA method and the expected value k₂<E_{B} + E_{H} - E_{B} × EH = 77.9% of the Bliss method. These results once again showed that R/B was a further synergistic combination exceeded the expectation (based on the HSA method or Bliss method), and even a further synergistic combination of dose reduction and efficacy enhancement. Additionally, when comparing R with Agr (which also contains guanidine), the former (group J) was the described synergistic combination, while the latter (group M) was not a synergistic combination.

### Example 11.2 Extension to study of combination containing B

In one experiment, pancreatic cancer was selected as the representative tumor for refactory tumors. The experimental animals were nude mice, and the tumor formation method was the conventional subcutaneous transplantation method. In brief, the transplantation volume of the human pancreatic cancer cell line PANC-1 was 1×10⁶ cells per mouse, and the transplantation method was cell suspension injection, with the modeling site being the subcutaneous of the animal. Animals with successful tumor modeling (average tumor volume of 183 mm³) were enrolled and randomly divided into 15 groups (6 mice per group), as shown in the following table. On the day of grouping, the treatment began, which was recorded as the 1st day of treatment, and so on. Each group was given the pharmaceutical solution as shown in the table below, all prepared according to conventional methods.

Group A to C received pharmaceutical solution I intratumorally once, on the 1st day, with a volume of 100 µL per mouse. Group D received intratumoral administration once on the 1st day, with pharmaceutical solution II given first to each mouse at 75µL, followed by pharmaceutical solution I at 100 µL after 20 minutes. Group E to F received intratumoral administration once on the 1st day, with pharmaceutical solution I was administered to each mouse at 10µL, and pharmaceutical solution II was administered on the opposite side of the tumor to each mouse at 75 µL and 10 µL respectively. Group G to H received intraperitoneal administration on day 1, 3, 5, and 7 for a total of 4 times, with 200 µG per time per mouse. Group I received pharmaceutical solution III by gavage 10 times, once a day, 200 µG per mouse. Group J to L received pharmaceutical solution I and pharmaceutical solution III, with pharmaceutical solution I was administered as in Group B, and pharmaceutical solution III was administered as in Group G, H, and I. Group M to O received pharmaceutical solution I, pharmaceutical solution II, and pharmaceutical solution III, with pharmaceutical solution I, pharmaceutical solution II was administered as in Group D, and pharmaceutical solution III was administered as in Group G, H, and I. Group P received pharmaceutical solution I and pharmaceutical solution III, with pharmaceutical solution I was administered as in Group C, and pharmaceutical solution III was administered as in Group G. Group Q to R received pharmaceutical solution I, pharmaceutical solution II, and pharmaceutical solution III, and pharmaceutical solution I was administratered as in Group E and F, and pharmaceutical solution III was administered as in Group G and H. Group S to T received pharmaceutical solution I and pharmaceutical solution II once on the 1st day, with pharmaceutical solution I administered to each mouse at 10 µL, and pharmaceutical solution II was administered on the opposite side of the tumor to each mouse at 10 µL and 75 µL respectively. On the 18th day, the animals were euthanized, the tumors were dissected, the tumor weight was measured, and the tumor inhibition rate was calculated. The results were shown in Table 22.

**Table 22**

| group | Drugs | | | Tumor Inhibition rate(X) |
|---|---|---|---|---|
| | Pharmaceutical solution I | Pharmaceutical solution II | Pharmaceutical solution III | |
| A | Normal saline | - | - | - |
| B | 5% R | - | - | 41.3% |
| C | 5%R/10%SB/0.5%MB | - | - | 64.9% |
| D | 5% R | 1% MB | - | 68.4% |
| | | | | |
| E | 50% R | 1% of MB | - | 71.8% |
| F | 50% R | 5% of MB | - | 73.9% |
| G | - | - | 0.5% IFO | 13.6% |
| H | - | - | 0.4% 5-Fu | 10.9% |
| I | - | - | 0.3% Osimertinib | 7.1% |
| J | 5% R | - | 0.5% IFO | 71.3% |
| K | 5% R | - | 0.4% 5-Fu | 73.6% |
| L | 5% R | - | 0.3% Osimertinib | 65.8 percent |
| M | 5% R | 1% MB | 0.5% IFO | 91.3% |
| N | 5% R | 1% MB | 0.4% 5 - Fu | 90.9% |
| ○ | 5% R | 1% MB | 0.3% Osimertinib | 87.6% |
| P | 5% R / 10% SB / 0.5% MB | - | 0.5% IFO | 87.4% |
| Q | 50% R | 1% MB | 0.5% IFO | 95.1% |
| R | 50% R | 5% of MB | 0.4% 5-Fu | 93.8% |
| S | 50% R | 5% IFO / 5% | - | 98.1% |
| | | MB | | |
| T | 50% R | 5% 5-Fu / 0.8% MB | - | 93.2% |

As shown in the table above, the combination results of the combination groups containing R, B, and D (group M to O) were consistent with those of the combination groups containing R and B (group J to L), and the latter were shown to be a synergistic combination exceeding the expectations of the prior art for dimethylbiguanide (R/B and R'/B were pharmaceutically equivalents) in Example 6-9. Furthermore, the results of the combination groups containing R, B, and D (group M to T) also exceeded the expectations of effects of component groups (group B to I) (k2) based on the HSA method or Bliss method. Taking the group R as an example, its actual tumor inhibtion rate k₁ (93.8%) was greater than the expected value k₂ = max(group F, group H) = 73.9% of the HSA method and the expected value k₂ = E_{F} + E_{H} - E_{F} × E_{H}=76.7% of the Bliss method. These results once again showed that R/C was consistent with R, and its combination with B was a further synergistic combination exceeding the expectations of the HSA method or Bliss method.

In the above examples, the dosage of R (QR) (concentration 1-50%) was consistent with that in Example 1-9, and the dosage of methylene blue (QD) (concentration 0.5-5%) was the effective dosage for transient local effect. As shown in Example 1-9, N,N-dimethylbiguanide in free-base form (R) universally showed the aforementioned transient local effect for different animals (mice, rats, dogs) and different lesions (tumors, nodules), including tissue destruction effects. It was shown that MB could provide transient local effects. The technical result of the combination of R and D with a very different structure exceeded the expectations of the prior art of dimethylbiguanide (R/D and R'/D were pharmaceutically equivalent combinations) and also exceeded the expectations of the single agent (HSA method or Bliss method). This indicated that the pharmaceutical effects of R and D could mutually enhance each other. This further indicated the essential difference between R and R'.

In this example, both R/D or R/C/D and R exhibited consistent transient local effects, making it possible to meaningfully reduce the biological barrier function within the lesion. In this example, the representative drugs of cell-reactive drugs (B) were IFO, 5-Fu, and osimertinib, which could represent the chemical structure diversity, antineoplastic mechanism diversity (DNA damaging agents, antimetabolites, targeted antineoplastic agents), and consistency of key control steps of antineoplastic response (biological barrier within the lesion) of the entire class of B drugs. Combinatin of R/D or R/C/D and these representative drugs of B showed consistent combined effects with the combinations of R and B in Example 3, 6, 7, 8, and 9, all were synergistic combinations exceeding the expectation of the prior art of dimethylbiguanide and further synergistic combinations of the single agent (HSA method or Bliss method).

Consistent with R or R/C in Example 6-10, the combination of R/D or R/C/D and B with very different structures and mechanisms produced consistent synergistic effects, which also indicated that they shared common pharmacological mechanisms with any cell-reactive drug B: their respective pharmaceutical effects could mutually enhance each other. Therefore, B of the combination containg R and D and B in this application could be reasonably inferred from the effects of the above representative pharmaceutical combination to be applicable to cell-reactive drugs, at least including the previously mentioned DNA damaging agents, antimetabolites, anti-tubulin drugs, and targeted antineoplastic agents.

### Example 12 Study of pharmaceutical combination containing R and immunomodulator (E): E is a biomacromolecule immunomodulator

In this example, E was used for biological macromolecules immune modulators (E1), such as immune checkpoint inhibitors, cytokines, TLR agonist.

### Example 12.1 Study of combination containg immune checkpoint inhibitor

In one experiment, B1 was used as an immune checkpoint inhibitor (RMP1-14), and C57BL/6 mice were the experimental animals. The tumor formation method was the conventional subcutaneous tumor transplantation method. In summary, the transplantation volume of mouse colon cancer MC38 cells was 1 × transplantation⁵ cells per mouse, the transplantation method was cell suspension injection, and the transplantation site was subcutaneous of the animal. The animals with successful modeling were enrolled (average tumor volume was 104 mm³) and randomly divided into 10 groups (8 mice per group), as shown in the table below. The ratio of the highest value to the lowest value in the target volume (Vtarget) distribution of each group was approximately 200%. On the day of grouping, the drug was administered, which was recorded as the first day of treatment, and so on. Each group was administered the pharmaceutical solution as shown in the table below, all prepared according to the conventional method.

Group A to E all received solution I, with intratumoral administration once on day 1 and 2, with each administration volume being 200 µL except for group E, which was 10 µL. Group F received solution II, with tail vein injection once on day 1 and 7, with 40 µL per mouse each time. Groups G to J received solution I and solution II, with the administration of solution I as in group B to E, and solution II as in group H. On the 30th day, the survival rate was calculated according to the observation results (a tumor volume > 2000 mm³ was considered a death event), and the results were shown in Table 23.

**Table 23**

| Group | Composition of drugs | | Survival rate (%) |
|---|---|---|---|
| | pharmaceutical solution I | pharmaceutical solution II | |
| A | Normal saline | - | 0 |
| B | 6.5% R' | - | 0 |
| C | 0.2%R | - | 25.0 |
| D | 1% R | - | 37.5 |
| E | 50%R | - | 50.0 |
| F | - | 0.1% RMP1-14 | 0 |
| G | 6.5% R' | 0.1% RMP1-14 | 0 |
| H | 0.2%R | 0.1% RMP1-14 | 50.0 |
| I | 1% R | 0.1% RMP1-14 | 62.5 |
| J | 50%R | 0.1% RMP1-14 | 75.0 |

### 1. Single agent research:

As shown in the table, single agent R and E' (group B, F) failed to provide significant effect (survival rate of 0), the tumor inhibition effect of Single agent R (group C to E) was consistent with those in the previous examples, and survival rate was increased by 50% or more than that of R'. These results again showed that R was the active ingredient of dose reduction and efficacy enhancement (survival rate) beyond the expectation of the prior art (R and R' were pharmaceutical equivalents).

### 2. Regular comparison of combinations:

In the above table, compared with R/E (group G), the combination containing R and E (group H to J) was an obvious dose reduction and efficacy enhancement, characterized by that such as the dosage of R was reduced at least 50% than that of R' but at least one effect (survival rate) was increased by more than 50%. These results showed that the combination containing R and E was a synergistic combination beyond the expectation of the prior art (R/E and R'/ E were pharmaceutical equivalent combination), even for synergistic combination of dose reduction and efficacy enhancement.

### 3. Further comparison fo combination:

In the above table, compared with the component (group C to F), the effect of combination containing R and E (group H to J) went beyond the expectation of components based on HSA method or Bliss method (k₂), even if the R was already the active ingredient exceeding the expectation of the prior art (R and R' were pharmaceutical equivalents). Taking group I as an example, the actual survival rate k₁ (62.5%) was higher than expected value k₂ ≦ Max (group D, group F) = 37.5% of HSA method, and expected value k₂ = E_{D} + E_{F} - E_{D} * E_{F} = 37.5% of Bliss method. These results again suggested that R/B was a further synergistic combination beyond the expectation (HSA method or Bliss method), or even a further synergistic combination of dose reduction and efficacy enhancement.

### Example 12.2 Extension to biological macromolecular immunomodulators

In one experiment, the immunomodulator E was extended to Toll-like receptor (TLR) agonists. The well-known representative drug was CpGODN1826 (abbreviated as CpG), and the experimental animals were C57BL/6 mice. The tumor formation method was the conventional subcutaneous tumor transplantation method. In brief, the transplantation volume of B16-F10 melanoma cells in mice was 1×10⁵ cells per mouse, the transplantation method was intratumoral injection of cell suspension, and the modeling site was subcutaneous of the animal. The mice with successful tumor formation were enrolled in the study (average tumor volume was 109 mm³) and randomly divided into 12 groups (8 mice per group), as shown in the table below. The ratio of the highest to the lowest value in the target volume (V_{target}) distribution of each group was approximately 200%. The pharmaceutical solution was administered on the day of grouping, which was recorded as the first day of treatment, and so on. Each group was administered the pharmaceutical solution as shown in the table below, all prepared according to conventional methods.

Groups A to E all received pharmaceutical solution I intratumorally once on day 1 and 2, with each injection volume being 200 µL for all groups except E, which was 10 µL per mouse. Group F received pharmaceutical solution I and pharmaceutical solution II. The pharmaceutical solution I was intratumoral administered on day 1 and 2 (10 µL per mouse each time), and pharmaceutical solution II was intraperitoneal administered once on day 1, 4, 7, and 10 (200 µL per mouse each time). Group G received pharmaceutical solution III intratumorally once on day 3 and 5 (100 µL per mouse). Group H to K received pharmaceutical solution I and pharmaceutical solution III, the pharmaceutical solution I was administered the same as groups B to E, and pharmaceutical solution III was administered the same as group G. Group L received pharmaceutical solution I, pharmaceutical solution II and pharmaceutical solution III, the pharmaceutical solution I and pharmaceutical solution II was administered the same as group F, and pharmaceutical solution III was administered the same as group G. On the 30th day, survival rates were calculated based on the observation results (a tumor volume > 2000 mm³ was considered a death event). The results were shown in Table 24.

**Table 24**

| Group | Composition of drugs | | | Survival rate (%) |
|---|---|---|---|---|
| | pharmaceutical solution I | pharmaceutical solution II | pharmaceutical solution III | |
| A | Normal saline | - | - | 0 |
| B | 3% R' | - | - | 0 |
| C | 1% R | - | - | 37.5 |
| D | 2.5% R/10%SB | - | - | 50.0 |
| E | 50% R | - | - | 50.0 |
| F | 50% R | 0.5% IFO | - | 50.0 |
| G | - | - | 0.1% CpG | **0** |
| H | 3% R' | - | 0.1% CpG | **0** |
| I | 1% R | - | 0.1% CpG | 50.0 |
| J | 2.5% R / 10% SB | - | 0.1% CpG | 62.5 |
| K | 50% R | - | 0.1% CpG | 62.5 |
| L | 50% R | 0.5% IFO | 0.1% CpG | 75.0 |

### 1. Single-agent studies:

As shown in the table above, neither R nor E alone (group B and G) provided a significant pharmaceutical effect (survival rate was 0), while R alone (group C to E) was consistent with the tumor inhibition effect described in previous examples, with a survival rate increasing more than 50% than that of R'. These results again showed that R was an active ingredient of dose reduction and efficacy enhancement (survival rate) beyond the expectation of the prior art (R and R' were pharmaceutical equivalents).

### 2. Regular comparison of combinations:

In the table above, combinations containing R and E (group I to L) were significant dose reduction and efficacy enhancement compared with R'/E (group H), manifested as, for example, at least a 50% reduction in R dosage compared with R' but a 50% or greater improvement in at least one effect (survival). These results showed that the combination containing R and E was a synergistic combination beyond the expectation of the prior art (R/E and R'/E were pharmaceutical equivalent combinations), and even a synergism combination of dose-reduction and efficacy enhancement.

### 3. Further comparison of combinations:

In the above table, compared with the component (group C to F), the effect of the combination containing R and E (group I to L) exceeded the expectation of the effect of components of HSA or Bliss method (k₂), even if the R was already the active ingredient exceeding the expectation of the prior art of dimethylbiguanide (R and R' were pharmaceutical equivalents). Taking group J as an example, its actual survival k ₁ (62.5%) was greater than the expected value k₂≦ Max group (group D, G) = 50.0% of HSA method, and expected value k₂ = E_{D} + E_{G} - E_{D} * E_{G} = 50% of Bliss method. The results showed that the combination containing R and E again was further synergistic combinations exceeding the expectation (HSA or Bliss method).

In one experiment, a cytokine (IL-15) was used as an immune regulator, with the combination of R also produced similar results, namely the similar efficacy enhanced synergistic results (including tumor inhibition effect and survival rate, and the results were not shown).

In this example, the dosage (Q_{R}) of R was the same as in Example 1-9, and the dosage (Q_{E1}) of biomacromolecule immunomodulator (E1) was ≦ its usual dosage. Q_{E1} used by E as a single agent did not show significant efficacy. The transient local effect shown by R was consistent with that in Example 1-9, as follows: 1). As mentioned earlier, the biological barrier function within the lesion was reduced significantly, which was beneficial to the immune cells; 2). The high tumor inhibition rate also reflected the direct killing of tumor tissues and tumor cells, thereby inducing the release of pathological immune substances (such as tumor-associated antigens (TAA) and damage associated molecular patterns (DAMPs) released from necrotic tissues and cell damage) and inflammatory signals. These pharmacologic effects (lowering the biologic barrier function within the lesion and releasing phathological immune substances and inflammatory signals) provided a synergistic basis for coadministration with any biomacromolecular immunomodulator that could enhance or benefit from them.

In the above example, the representative drugs of biological macromolecules immune modulators extended from immune checkpoint inhibitors to cytokines and TLR agonists. It is well known that the structures of these representative drugs were proteins (monoclonal antibodies, natural proteins or their modified forms) and oligodeoxynucleotides (ODNs), which could represent the structural diversity of biomacromolecular immunomodulators. Their main mechanisms were reversal of immunosuppression, activation of effector immune cells, and enhancement of antigen presentation, respectively, which could represent the mechanism diversity of biomacromolecular immunomodulators. In the combination of the application, they had in common that the pathological immune substances and inflammatory signals released within the lesion could be used to optimize local and systemic immune responses.

In the above experiments, the coadministration of R and E1 produced a synergistic combination effect exceeded the expectation of the prior art of dimethylbiguanide (R/E1 and R'/E1were pharmaceutical equivalent combination), and a further synergistic combination effect exceeded the expectation of the single agent (HSA or Bliss method).

Therefore, the E in combination of the application could be reasonably inferred from the combination effect of the above representative drug to be applicable to immune regulator, at least including biological macromolecules immune modulators, further at least including immune checkpoint inhibitors, cytokines, and TLR agonists.

### Example 13 Study on pharmaceutical combinations containing R and immunomodulators (E): Extension of E to vaccines (E3)

In one experiment, the immunomodulators were expanded to vaccine based immunomodulatos (E3), with the well-known representative drug being Bacille Calmette-Guérin (BCG). The experimental animals were BALB/c mice, and the tumor formation method was the conventional subcutaneous tumor transplantation method. In brief, the transplantation volume of 4T1 breast cancer cells in mice was 1×10⁵ cells per mouse, the transplantation method was cell suspension injection, and the modeling site was subcutaneous of the animals. The successfully modeled animals were enrolled (with an average tumor volume of 124 mm³) and randomly divided into 10 groups (8 mice per group), as shown in the following table. The pharmaceutical solution was administered on the day of grouping, denoted as treatment day 1, and so on. Each group received the pharmaceutical solution as shown in the table below, all prepared according to conventional methods.

Group A to E all received pharmaceutical solution I, with intratumoral administration once on day 1 and 2, with each administration volume being 200 µL for all except group D, which was 10 µL. Group F received pharmaceutical solution I and solution II, with intratumoral administration of pharmaceutical solution I once each on day 1 and 2 (100 µL per mouse each time), and intraperitoneal administration of pharmaceutical solution II once each on day 1, 4, 7, and 10 (200 µL per mouse each time). Group G received pharmaceutical solution III, with tail vein injection once each on day 2 and 9, with each administration volume being 1×10⁶ CFU per mouse. Group H to K received pharmaceutical solution I and pharmaceutical solution III, with administration of pharmaceutical solution I as in groups B to E, and administration of pharmaceutical solution III as in group G. Group L received pharmaceutical solution I, solution II, and solution III, with administration of pharmaceutical solution I and solution II as in group F, and administration of pharmaceutical solution III as in group G. On the 30th day, based on the observation results (a tumor volume > 2000 mm³ was considered a death event), the survival rate was calculated, and the results were shown in Table 25.

**Table 25**

| Group | Composition of drugs | | | Survival rate (%) |
|---|---|---|---|---|
| | pharmaceutical solution I | pharmaceutical solution II | pharmaceutical solution III | |
| A | Normal saline | - | - | 0 |
| B | 3% R' | - | - | 0 |
| C | 1% R | - | - | 25.0 |
| D | 2.5% R / 10% SB | - | - | 37.5 |
| E | 50% R | - | - | 37.5 |
| F | 5% R | 0.5% IFO | - | 50.0 |
| G | - | - | 1×10⁶ CFUBCG | 0 |
| H | 3% R' | - | 1×10⁶ CFUBCG | 0 |
| I | 1% R | - | 1×10⁶ CFUBCG | 50.0 |
| J | 2.5% R / 10% SB | - | 1×10⁶ CFUBCG | 62.5 |
| K | 50% R | - | 1×10⁶ CFUBCG | 62.5 |
| L | 5% R | 0.5% IFO | 1 x 10 ⁶ CFUBCG | 75.0 |

### 1. Single-pharmaceutical studies:

As shown in the table, single agent R' and BCG (group B, G) failed to provide significant effect (survival rate of 0), the tumor inhibition rate of Single agent R (group C and E) was consistent with the previous examples, and survival rate was increased by 50% or more than R'. These results again showed that R was the active ingredient of dose reduction and efficacy enhancement (survival) beyond the expectation of the prior art (R and R' were pharmaceutical equivalents).

### 2. Regular comparison of combinations:

In the table above, compared with R'/E (group H), combinations containing R and E (groups I to K) were significant dose reduction and efficacy enhancement, as indicated for example by a reduction in R dosage of at least 50% over R' but a greater than 50% increase in at least one effect (survival rate). These results showed that the combination containing R and E was a synergistic combination beyond the expectation of the prior art (R/E and R '/E were pharmaceutical equivalent combinations), and even a synergism combination of dose-reduction and efficacy enhancement.

### 3. Further comparison of combinations:

In the above table, compared with the component (group C to G), the effect of the combination containing R and E (group I to L) exceed the expectation of effect of components based on HSA or Bliss method (k₂), even if the R was the active ingredient already exceeded the expectation of the prior art of dimethylbiguanide (R and R' were pharmaceutical equivalents). Taking group L as an example, the actual survival rate k₁ (75.0%) was higher than the expected value k₂ ≦ max (F group, G group) = 50.0% of the HAS method, and the expected value k₂ = E_{F}+E-E×E_{GFG} = 50.0% of the Bliss method. The results showed again that the combination containing R and E was a further synergistic combination beyond the expectation (HSA or Bliss method).

In addition to those described herein, according to the foregoing description, a variety of modifications of the present invention will be apparent to those skilled in the art. Such modifications are also intended to fall within the scope of the attached claims. All references cited in this application (including all patents, patent applications, journal articles, books, and any other publications) are incorporated herein by reference in their entirety.

## Claims

1. A formulation comprising N,N-dimethylbiguanide (abbreviated as R) and optionally a solvent, wherein the formulation does not comprise an acidic pH adjusting agent required to meet cell-culture or conventional injection safety, wherein a concentration of the N,N-dimethylbiguanide is greater than or equal to its administration concentration, wherein the administration concentration (w/w) is 0.5% ≦ CR ≦ 51%, preferably, 1% to 50%, more preferably 3% to 50% or 5% to 30%.

2. A formulation specifically for lesion intervention, comprising N,N-dimethylbiguanide (abbreviated as R) and optionally a solvent, wherein the formulation does not comprise an acidic pH adjusting agent required to meet cell-culture or conventional injection safety, wherein a concentration of the N,N-dimethylbiguanide is greater than or equal to its intervention concentration, wherein the intervention concentration (w/w) is 0.5% ≤ C ≤ 51%, preferably 1% - 50%, more preferably 3% - 40% or 5% - 35%, and is preferably used for interventional treatment of a lesion, such as a tumor or a nodule, in a subject.

3. The formulation according to any one of claims 1-2, wherein the formulation comprises a solvent, preferably the solvent is water, more preferably water for injection.

4. The formulation according to any one of claims 1 to 3, wherein the formulation does not comprise a salt of N,N-dimethylbiguanide and an acid that causes R significantly convert to R'.

5. The formulation according to any one of claims 1 to 3, wherein the concentration (w/w) of the N,N-dimethylbiguanide in the formulation is ≤ 51%, ≤ 50%, ≤ 40%, ≤ 30%, ≤ 25%, ≤ 20%, preferably ≤ 10%, and more preferably ≤ 5%.

6. The formulation according to any one of claims 1 to 3, wherein the concentration (w/w) of the N,N-dimethylbiguanide (R) in the formulation is 0.5-50%, 1%-40%, 5%-50%, 5%-40%, 15%-50%, 25%-50%, 35%-50%.

7. The formulation according to any one of claims 1 to 7, wherein the acidic pH adjusting agent or acid comprises or is selected from hydrochloric acid, sulfuric acid, acetic acid, citric acid, phosphoric acid, malic acid, tartaric acid, lactic acid and carbonic acid.

8. The formulation according to any one of claims 1 to 7, wherein the formulation consists of N,N-dimethylbiguanide (R) and water.

9. The formulation according to claims 1-8, wherein the formulation is used to provide an active form of R distinct from the conventional metformin formulation (whose active ingredient is R'), wherein an active form of R' is exhibited as a form that is completely complexted with an acid in solution; while the active form of R is exhibited as a form that is less complexed with a acid in solution, mainly existing in a form of minimal or partial complexation.

10. The formulation according to any one of claims 1 to 9 for use as a pharmaceutical component of a pharmaceutical combination, wherein the pharmaceutical combination further comprises a component (Z), and wherein the Z is selected from at least one of the following groups: cell-reactive antineoplastic agents (abbreviated as B), near-neutral or weakly basic sodium salts (abbreviated as C), methylene blue dyes (abbreviated as D), and immunomodulators (abbreviated as E).

11. A pharmaceutical combination for treating a local lesion, comprising N,N-dimethylbiguanide (abbreviated as R) and a component (Z), wherein an administration concentration (w/w) of the N,N-dimethylbiguanide is 0.1% - 51%, preferably 0.2% - 50%, and the component (Z) comprises at least one selected from the following group: cell-reactive antineoplastic agents (abbreviated as B), near-neutral or weakly basic sodium salts (abbreviated as C), methylene blue dyes (abbreviated as D), and immunomodulators (abbreviated as E).

12. The pharmaceutical combination according to claim 11, wherein the N, N-dimethylbiguanide is used to provide an active form distinct from a conventional metformin drug (whose active ingredient is a salt of N,N-dimethylbiguanide (R')), through the following limitations:
1). is not mixed with an acid that causes significant conversion of R to R';
2). local intralesional administration;
3). an administration amount for one course of treatment is: ${Q}_{R} = {\sum }_{i = 1}^{nR} {q}_{R \left(i\right)}$ ,
wherein Q_{R} < 50% or 25% of the equivalent dosage of R', n_{R} is the number of intratumoral administrations of R in one course of treatment, q_{R(i)} is the amount of R in the i-th (i = 1,..., n_{R}) administration, and: ${q}_{R \left(i\right)} = {\sum }_{i i - 1}^{n R i} {c}_{R \left(ii\right)} \times {v}_{R \left(ii\right)} ,$
wherein n_{Ri} is the number of administration points in the i-th administration, such as the number of injection points, c_{R(ii)} and v_{R(ii)} are the concentration and volume administered at the ii-th administration points, such as the injection points, respectively, wherein a concentration threshold of the c_{R(ii)} (W/W) is 0.1% or 0.2%, the upper limit of the concentration threshold is 51%, a volume threshold of the V_{R(ii)} is 1/800 or 1/300 of a target volume (denoted as v_{Target(ii)}), and the upper limit of the volume threshold is 3 times of v_{Target(ii)}.
wherein an active form of the R' is presented as a form that is fully complexed with an acid in a solution; while an active form of the R is presented as a form that is less complexed with an acid in a solution, mainly existing in a form of minimal or partial acid complexation, thereby, through a transient local action of this distinct active form, R provides an activity (referred to as activity A) that is difficult for R' to provide, comprising at least one of the following: an action that effectively reduces the function of biological barriers (abbreviated as activity A1), an action that effectively damages pathological tissue (abbreviated as activity A2), and/or an action that effectively releases lesional immune substances and/or inflammatory signals within the lesion (abbreviated as activity A3).

13. The pharmaceutical combination according to claim 11 or 12, for use ina treatment of a solid tumor, comprises R and B, wherein
- the activity A of the R at least comprises effectively reducing the function of the biological barrier (activity A1);
- the cell-reactive antineoplastic agent (B) provides the cellular activity (abbreviated as activity B) through the following definitions: one key control step of which is crossing the above-mentioned biological barrier:
1). intratumoral administration and/or systemic administration;
2). an administration amount for one course of treatment is: $QB = {\sum }_{j = 1}^{nB} {q}_{B \left(j\right)} + {\sum }_{j ′ = 1}^{nB ′} {c}_{B \left(j′\right)} \times {V}_{B \left(j′\right)}$ ,
wherein Q_{B} is ≦ a conventional dosage of B for a sensitive tumor, n_{B}, q_{B} (j) are the number of systemic administrations of B in one course of treatment and an amount at the j-th (j = 1,..., nB) administration, respectively,n_{B'},cB_{(j')} and v_{B(j')} are the number of intratumoral administrations of B in one course of treatment, a concentration and a volume at the j' th (= 1,..., n_{B'}) administration, respectively.

14. The pharmaceutical combination according to any one of claims 11-13, comprising R, C, and/or D, wherein:
- the activity A of R at least comprises an action effectively damaging a lesional tissue (activity A2);
- C is used to provide an effect of improving a local efficacy-toxicity ratio of the R (abbreviated as activity C) through the following definitions:
1). co-administration with R;
2). a mixture ratio of C to R (Q_{C}/Q_{R}) is 0.5 to 4.0;
- the D provides the pharmacological effect of enhancing the efficacy of R through the following definitions:
1). intralesional administration, comprising administration mixed with R or administration separately;
2). the dosage Q_{E} for one course of treatment is ≤ the effective dosage for a transient local action.

15. The pharmaceutical combination according to any one of claims 11-14, comprising R and E, wherein:
- the activity A of R at least includes an action of effectively reducing the function of the biological barrier (activity A1), and/or an action of effectively releasing lesional immune substances and/or inflammatory signals in the lesional tissue (activity A3);
- the E is used to provide an immunomodulatory effect benefiting from the effect of activity A1, and/or to optimize a local and/or systemic immune response by utilizing the effect of activity A3 (abbreviated as activity E), through the following definitions:
1). intralesional administration and/or systemic administration;
2). an administration amount for one course of treatment is: ${Q}_{E} = {\sum }_{k = 1}^{n E} {q}_{B \left(k\right)} + {\sum }_{k ′ = 1}^{n E ′} {c}_{E \left(k′\right)} \times {v}_{E \left(k′\right)} ,$ wherein Q_{E} is ≦ the conventional dosage of E in anti-lesion application, n_{E}, q_{E(k)} are the number of systemic administrations of E in one course of treatment and the amount at the k-th (k = 1,..., n_{K}) administration, respectively, n_{E'}, c_{E(k')}, and v_{E(k')} are the number of intratumoral administrations of E in one course of treatment, and a concentration and a volume at the k' th (k' = 1,..., n_{k'}) administration, respectively.

16. The formulation according to any one of claims 1 -10 or the pharmaceutical combination according to any one of claims 11-15, wherein the N,N-dimethylbiguanide (R) comprises < 30% (w/w), preferably ≦ 5% (w/w) of a salt of N,N-dimethylbiguanide (R'), or more preferably does not comprise a salt of N,N-dimethylbiguanide (R'), optionally, the N,N-dimethylbiguanide (R) is in the form of a powder for injection.

17. The pharmaceutical combination according to any one of claims 11-16, wherein the cell-reactive antineoplastic agent includes or is selected from at least one of the following: cisplatin, cyclophosphamide, carboplatin, doxorubicin, ifosfamide, mechlorethamine, fluorouracil, capecitabine, gemcitabine, methotrexate, pemetrexed, fludarabine salt, paclitaxel, docetaxel, vincristine, vinblastine, vindesine, etoposide, irinotecan, topotecan, daunorubicin, mitoxantrone, gefitinib, osimertinib, larotrectinib, pazopanib, trastuzumab, and derivatives thereof.

18. A composition comprising the formulation according to any one of claims 1 - 10 or the pharmaceutical combination according to any one of claims 11-17.

19. A formulation comprising the pharmaceutical combination according to any one of claims 11-17 or the composition according to claim 18.

20. Use of N,N-dimethylbiguanide (abbreviated as R) as a transient active ingredient in the manufacture of a formulation, a medicament, a pharmaceutical combination or a kit for the treatment of a tumor or a nodule.

21. Use of the formulation according to any one of claims 1 110, the pharmaceutical combination according to any one of claims 11-17, the composition according to claim 18, or the formulation according to claim 19 in the manufacture of a medicament, a pharmaceutical combination or a kit for treating a lesion such as a tumor or nodule in a subject.

22. The use according to claim 20 or 21, wherein the N,N-dimethylbiguanide is used to provide a morphological manifestation distinct from a conventional metformin drug (whose active form is R'), wherein an active form of R' is in a form that completely complexed with an acid in solution; while an active form of R is in a form that is less complexed with an acid in solution, mainly existing in the form of minimal or partial acid complexation, thereby through a transient local action of this distinct active form, providing an activity (abbreviated as activity A) that is difficult to be provided by R', including an activity of effectively reducing the function of the biological barrier (abbreviated as activity A1), an activity of effectively damaging the pathological tissue (abbreviated as activity A2), and/or an activity of effectively releasing the lesional immune substances and/or inflammatory signals within the lesion tissue (abbreviated as activity A3).

23. A method for treating cells within a lesion, such as tumor or nodule cells, comprising administering or intervening in the N,N-dimethylbiguanide (R) in the formulation according to any one of claims 1-10, the pharmaceutical combination according to any one of claims 11-17, the composition according to claim 18, or the formulation according to claim 19 into the lesion, allowing it to permeate the lesion tissue and to contact and kill cells within the tissue.

24. A method for treating a lesion, comprising: administering or intervening in the N,N-dimethylbiguanide (R) in the formulation according to any one of claims 1-10, the pharmaceutical combination according to any one of claims 11-17, the composition according to claim 18, or the formulation according to claim 19 to the lesion, allowing it to permeate the lesion tissue and to contact and kill cells within the tissue.

25. A method for treating a tumor in a subject, comprising:
intervening in or administering the formulation according to any one of claims 1-10 to the tumor in the subject, or
intervening in or administering the N,N-dimethylbiguanide (R) in the formulation according to any one of claims 1 to 10, the pharmaceutical combination according to any one of claims 11 to 17, the composition according to claim 18, or the formulation according to claim 19 to the tumor in the subject, and systemically administering one or more of B, D, and E to the subject and/or intervening in or administering them to the tumor.

26. The method according to any one of claims 23-25, wherein the intervention or administration comprises multi-point intervention or administration, preferably, comprises micro multi-point intervention or administration, and more preferably, includes micro multi-point injection.

27. The method according to any one of claims 23-26, wherein the formulation is suitable for micro-volume administration or intervention to reduce lesion volume, and a ratio of the administration volume to the target volume (V_{administration}/V_{target}) is about 0.02 - 0.056, 0.02 - 0.10 or 0.02 - 0.34.

28. The method according to any one of claims 23-27, when when the lesion is ≧ about 3.5 cm³ or the maximum dimension is ≧ about 2 cm, the multiple points are ≧ 5 points, for example, 10, 15, 20, 25, 30, 35, 50, or 100 points.

29. The method according to any one of claims 20-28, wherein the intervention or administration is low-frequency, preferably, the low-frequency is 1-6 times per course of treatment, more preferably, it is 1-5 times per course of treatment, 1-4 times per course of treatment, 1-3 times per course of treatment, or 1-2 times per course of treatment.

30. The method according to any one of claims 23 to 29, wherein R and B can be provided by one or more formulations, and wherein:
the R and B are administered intralesionally or intratumorally;
the R and B areadministered intralesionally or intratumorally and systemically, respectively; or
the R and a portion of B are administered intralesionally or intratumorally, and the other portion of B is administered systemically.

31. The method according to any one of claims 23-29, wherein the R and C may be provided by one or more formulations, and both the R and C are administered intralesionally or intratumorally.

32. The method according to any one of claims 23-29, wherein the R and D may be provided by one or more formulations, and both the R and D are administered intralesionally or intratumorally

33. The method according to any one of claims 23-29, wherein the R and E can be provided by one or more formulations, and wherein:
the R and E are both administered intralesionally or intratumorally;
the R and E are administered intralesionally or intratumorally and systemically, respectively; or
the R and a portion of E are administered intralesionally or intratumorally, and the other portion of E is administered systemically.

34. The formulation according to any one of claims 1 to 10, the pharmaceutical combination according to any one of claims 11 to 17, the composition according to claim 18, the formulation according to claim 19, or the use according to any one of claims 20 to 22 or the method according to any one of claims 23 to 33, wherein R is not mixed with a sufficient amount of R' or an acidifying agent capable of converting R to a sufficient amount of R', where the sufficient amount refers to an amount such that an amount ratio of R' to R (R'/R) is ≥ 1/3.

35. The use according to any one of claims 20 to 22 or the method according to any one of claims 23 to 34, wherein the N,N-dimethylbiguanide (R) is used to provide an active form distinct from the conventional metformin drug (whose active ingredient is R'), through the following definitions:
1). not mixed with an acid that significantly converts R to R';
2). local intralesional administration;
3). an administration amount for one course of treatment is: ${Q}_{R} = {\sum }_{i = 1}^{nR} {q}_{R \left(i\right)} ,$
wherein Q_{R} < 50% or 25% of the equivalent dosage of R', n_{R} is the number of times of R being administered intralesionally or intratumorally in one course of treatment, q_{R(i)} is an amount of R in the i-th (i = 1,..., nR) administration, and: ${q}_{R \left(i\right)} = {\sum }_{ii - l}^{nRi} {C}_{R \left(ii\right)} \times {v}_{R \left(ii\right)} ,$
wherein n_{Ri} is the number of administration points in the i-th administration of R, such as the number of needle insertion points, C_{R(ii)} and V_{R(ii)} are the concentration and volume of R administered at the ii-th administration point, respectively, such as the ii-th needle insertion point, where a concentration threshold of C_{R(ii)} (W/V) is 0.1% or 0.2%, and the upper limit of the concentration threshold is 51%; a volume threshold of V_{R(ii)} is 1/800 or 1/300 of the target volume (abbreviated as v_{Target(ii)}), and the upper limit of the volume threshold is 3 times of V_{Target(ii)}.
wherein an active form of the R' is presented as a form being completely complexed with an acid in solution; while an active form of the R is presented as a form being less complexed with an acid in solution, mainly existing in a form with minimal or partial acid complexation, thereby providing an activity (abbreviated as activity A) that is difficult for R' to provide through a transient local effect of this distinct active form, including at least one of the following: an action of effectively reducing the function of the biological barrier (abbreviated as activity A1), an action of effectively damaging the lesional tissue (abbreviated as activity A2), and/or an action of effectively releasing lesional immune substances and/or inflammatory signals within the lesional tissue (abbreviated as activity A3).

36. The use or method according to claim 35, wherein the R and the drug (Z) are co-administered, wherein an administration concentration (w/w) of the R is 0.1% - 51%, preferably 0.2% - 50%, and the drug (Z) includes at least one selected from the following group: cell-reactive antineoplastic agents (abbreviated as B), near-neutral or weakly basic sodium salts (abbreviated as C), methylene blue (abbreviated as D), and immunomodulators (abbreviated as E), wherein:
- the cell-reactive antineoplastic agent (B) provides the cellular reaction (abbreviated as activity B) through the following limitations, one key control step of which is crossing the above-mentioned biological barrier:
1). intralesional administration and/or systemic administration;
2). an administration amount for one course of treatment is: ${Q}_{B} = {\sum }_{j - l}^{nB} {q}_{B \left(j\right)} + 5 {\sum }_{i ′ - l}^{nB ′} {C}_{B \left(j′\right)} \times {V}_{B \left(j′\right)}$ ,
wherein Q_{B} is ≦ the conventional dosage of B for a sensitive tumor, n_{B}, q_{B(j)} are the number of systemic administrations and the amount of B in the j th (j = 1,... nB) administration, respectively, in one course of treatment, n_{B'}, c_{B(j')}, and v_{B(j')} are the number of tumoral administrations, the concentration and the volume of B in the j' th (j' = 1,..., nB') administration respectively in one course of treatment;
- the C provides an action of improving a local efficacy-toxicity ratio of R (abbreviated as activity C) through the following limitations:
1). co-administration with R;
2). a ratio of the mixed amount of C to R (Q_{C}/Q_{R}) is 0.5 to 4.0;
- the D provides the pharmacological effect of enhancing the pharmaceutical effect of R through the following limitations:
1). intralesional administration, including administration mixted with R or administration separately;
2). an administration amount for one course of treatment Q_{E} is ≦ the effective dosage for transient local action;
- the E provides the immunomodulatory effect benefiting from the effect of activity A1 and/or the effect of optimizing local and/or systemic immune response by utilizing the effect of activity A3 (abbreviated as activity E) through the following limitations:
1). intralesional administration and/or systemic administration;
2). an administration amount for one course of treatment is: ${Q}_{E} = {\sum }_{k = 1}^{n E} q B \left(k\right) + {\sum }_{k ′ = 1}^{n E ′} cE \left(k′\right) \times vE \left(k′\right) ,$ wherein Q_{E} is ≦ the conventional dosage in the anti-lesion application of E, n_{E}, q_{E(k)} are the number of systemic administrations and the amount of the k th (k = 1,..., nₖ) administration of E respectively in one course of treatment, n_{E'}, c_{E(k')}, and v_{E(k')} are the number of intratumoral administrations, the concentration and the volume of the k' th (k' = 1,..., n_{k'}) administration of E respectively in one course of treatment.

37. The use or method according to claim 35 or 36, wherein the R and the cell-reactive antineoplastic agent (abbreviated as B) and the immunomodulator (abbreviated as E) are coadminstered, wherein:
- the cell-reactive antineoplastic agent (B) provides the cellular reaction (abbreviated as activity B) through the following limitations, one of the key control steps of which is to cross the above-mentioned biological barrier:
1). intralesional administration and/or systemic administration;
2). an administration amount for one course of treatment is: ${Q}_{B} = {\sum }_{j - l}^{nB} {q}_{B \left(j\right)} + {\sum }_{i ′ - l}^{nB ′} {C}_{B \left(j′\right)} \times {V}_{B \left(j′\right)} ,$ wherein Q_{B} is ≦ the conventional dosage of B for a sensitive tumor, n_{B}, q_{B(j)} are the number of systemic administrations and the amount of B in the j th (j = 1,..., n_{B}) administration respectively in one course of treatment, n_{B'}, c_{B(j')}, and v_{B(j')} are the number of intratumoral administrations, the concentration and the volume of the j' th (j' = 1,..., n_{B'}) administration of B respectively in one course of treatment,
- the immunomodulator (E) is used to provide the immunomodulatory effect benefiting from the effect of activity A1 and/or to optimize the local and/or systemic immune response by utilizing the activity A3 (abbreviated as activity E) through the following limitations:
1). intralesional administration and/or systemic administration;
2). an administration amount for one course of treatment is: ${Q}_{E} = {\sum }_{k = 1}^{n E} qB \left(k\right) + {\sum }_{k ′ = 1}^{n E ′} cE \left(k′\right) \times {v}_{E \left(k′\right)} ,$ wherein Q_{E} is ≦ the conventional dosage in the anti-lesion application of E, n_{E}, q_{E(k)} are the number of systemic administrations and the amount of the k th (k = 1,..., nₖ) administration of E respectively in one course of treatment, n_{E'}, c_{E(k')}, and v_{E(k')} are the number of tumoral administrations, the concentration and the volume of the k' th (k' = 1,..., n_{k'}) administration of E respectively in one course of treatment.

38. The formulation according to any one of claims 1 to 10, the pharmaceutical combination according to any one of claims 11 to 17, the composition according to claim 18, the formulation according to claim 19, the use according to any one of claims 20 to 22, or the method according to any one of claims 23 to 37, wherein R is contained in a powder.

39. The composition according to claim 10, the pharmaceutical combination according to any one of claims 11 to 17, the composition according to claim 18, the formulation according to claim 19, the use according to any one of claims 20 to 22, or the method according to any one of claims 23 to 37, wherein the cell-reactive antineoplastic agent (B) comprises cytotoxic drugs and targeted antineoplastic agents.

40. The pharmaceutical combination, composition, formulation, use or method according to claim 39, wherein the cytotoxic drug comprises DNA damaging agents, antimetabolites or antitubulin agents, wherein the DNA damaging agent comprises cisplatin, carboplatin, oxaliplatin, ifosfamide, doxorubicin; and the antimetabolites comprise fluorouracil, gemcitabine, methotrexate; the antitubulin agents include paclitaxel, and vincristine.

41. The pharmaceutical combination, composition, formulation, use or method according to claim 39, wherein the targeted antineoplastic drug includes a kinase inhibitor.

42. The pharmaceutical combination, composition, formulation, use or method according to claim 41, wherein the kinase inhibitor includes gefitinib, osimertinib, larotrectinib, pazopanib, trastuzumab, and derivatives thereof.

43. The composition according to claim 10, the pharmaceutical combination according to any one of claims 11 to 17, the composition according to claim 18, the formulation according to claim 19, the use according to any one of claims 20 to 22, or the method according to any one of claims 23 to 37, wherein the near-neutral or weakly basic sodium salt (C) includes: sodium chloride, sodium bicarbonate (abbreviated as SB), monosodium phosphate (MSP), sodium lactate (abbreviated as Nal), and sodium acetate (abbreviated as SA).

44. The formulation according to claim 10, the pharmaceutical combination according to any one of claims 11 to 17, the composition according to claim 18, the formulation according to claim 19, the use according to any one of claims 20 to 22, or the method according to any one of claims 23 to 37, wherein the near-neutral or weakly basic sodium salt (C) is sodium bicarbonate (SB).

45. The formulation according to claim 10, the pharmaceutical combination according to any one of claims 11 to 17, the composition according to claim 18, the formulation according to claim 19, the use according to any one of claims 20 to 22, or the method according to any one of claims 23 to 37, wherein the methylene blue dye (D) includes methylene blue, patent blue, isosulfan blue and new methylene blue.

46. The formulation according to claim 10, the pharmaceutical combination according to any one of claims 11 to 17, the composition according to claim 18, the formulation according to claim 19, the use according to any one of claims 20 to 22, or the method according to any one of claims 23 to 37, wherein the methylene blue dye (D) is methylene blue.

47. The composition according to claim 10, the pharmaceutical combination according to any one of claims 11 to 17, the composition according to claim 18, the formulation according to claim 19, the use according to any one of claims 20 to 22, or the method according to any one of claims 23 to 37, wherein the immunomodulator (E) includes biomacromolecule immunomodulators (abbreviated as E1), immune cell immunomodulators (abbreviated as E2), and vaccine immunomodulators (abbreviated as E3).

48. A kit comprising the formulation according to any one of claims 1 to 10 or the pharmaceutical combination according to any one of claims 11 to 20, and instructions for carrying out the method according to any one of claims 23 to 37.

49. The kit according to claim 48, further comprising a micro-volume intervention device.

50. The kit according to claim 49, wherein the micro-volume intervention device is suitable for micro-volume multi-point contact or administration, preferably including a micro-injection device, wherein the micro-volume is ≦ 5% or 10% of the drug administration volume of the target volume.

51. The formulation according to any one of claims 2 to 10, the pharmaceutical combination according to any one of claims 11 to 17, the composition according to claim 18, the formulation according to claim 19, the use according to any one of claims 20 to 22, the method according to any one of claims 23 to 37, or the kit according to any one of claims 48 to 50, wherein the local lesion or tumor is a malignant tumor that is refractory to conventional chemotherapeutic drugs, preferably, the refractory malignant tumor comprises or is selected from at least one of the following groups: chemotherapeutic drug-resistant tumors, tumors with an unfavorable microenvironment, tumors after discontinuation of antineoplastic agents, tumors for which there are no effective chemotherapeutic drugs, and tumors in patients with contraindication to or who are not suitable for conventional chemotherapeutic drugs.

52. The formulation according to any one of claims 2 to 10, the pharmaceutical combination according to any one of claims 11 to 17, the composition according to claim 18, the formulation according to claim 19 or the use according to any one of claims 20 to 22, and the method according to any one of claims 23 to 37, wherein the nodules are at least one of the nodules comprising the following non-malignant cells: non-malignant nodules of connective tissue cells, secretory gland cells, and epithelial cells.

53. The formulation according to any one of claims 2 to 10, the pharmaceutical combination according to any one of claims 11 to 17, the composition according to claim 18, the formulation according to claim 19, or the use according to any one of claims 20 to 22, or the method according to any one of claims 23 to 37, wherein the lesion comprises a solid tumor.
